# EUROPEAN PATENT SPECIFICATION

(11) **EP 4 321 504 B1**
(45) Date of publication and mention of the grant of the patent: **18.02.2026**
(21) Application number: 22784092.3
(22) Date of filing: 07.04.2022
(51) Int. Cl.: C07C 219/06, C07C 219/16, C07C 229/26, C07C 319/20, C07C 271/16, C07C 271/18, C07C 271/20, C07C 271/22, C07D 207/452, C07D 233/60, C07D 487/04, C07D 495/04, A61K 9/127, A61P 35/00, C08G 65/332, C08G 65/333, C08G 65/337

(54) **PEGYLATED LIPID AND LIPOSOME MODIFIED THEREBY, AND PHARMACEUTICAL COMPOSITION COMPRISING LIPOSOME AND PREPARATION AND USE THEREOF**
PEGYLIERTES LIPID UND DAMIT MODIFIZIERTES LIPOSOM SOWIE PHARMAZEUTISCHE ZUSAMMENSETZUNG MIT LIPOSOM SOWIE HERSTELLUNG UND VERWENDUNG DAVON
LIPIDE PEGYLÉ ET LIPOSOME AINSI MODIFIÉ, COMPOSITION PHARMACEUTIQUE COMPRENANT UN LIPOSOME, PRÉPARATION ET UTILISATION ASSOCIÉES

(30) Priority: 08.04.2021 CN 202110379685; 09.07.2021 CN 202110780189; 23.07.2021 CN 202110839008
(43) Date of publication of application: 14.02.2024
(73) Proprietor: Xiamen Sinopeg Biotech Co., Ltd., Xiamen, Fujian 361100 (CN)
(72) Inventor: WENG, Wengui, Xiamen, Fujian 361100 (CN); LIU, Chao, Xiamen, Fujian 361100 (CN); WANG, Ailan, Xiamen, Fujian 361100 (CN); LIN, Minggui, Xiamen, Fujian 361100 (CN); LIU, Qiaoyan, Xiamen, Fujian 361100 (CN)
(74) Representative: Algemeen Octrooi- en Merkenbureau B.V.
(86) International application number: PCT/CN2022/085528
(87) International publication number: WO 2022/214026

(56) References cited:
- WO-A1-2020/072605
- CN-A- 102 006 890
- CN-A- 102 884 041
- CN-A- 103 096 875
- CN-A- 103 881 084
- CN-A- 103 974 719
- CN-A- 104 159 615
- CN-A- 106 795 096
- CN-A- 108 368 028
- CN-A- 110 167 922

## Description

### TECHNICAL FIELD

The present invention relates to the field of drug delivery, specifically to a PEGylated lipid as pharmaceutical carrier, in particular to a PEGylated lipid with a nitrogen-atom branching center and preparation method thereof, cationic liposomes containing the PEGylated lipid, liposome pharmaceutical compositions containing the PEGylated lipid, formulations of the compositions and application thereof.

### BACKGROUND OF THE INVENTION

Liposome is a microvesicle formed by encapsulating drugs in the lipoid bilayer. Liposomal nanoparticles contain liposomes and drugs, especially nucleic acid drugs, and their structures are similar to biofilms, which makes liposomal nanoparticle a biocompatible and non-toxic nanomaterial. They can encapsulate water-soluble and lipo-soluble drugs, with advantages such as reduced drug dose, sustained release, targeted drug release, and protection of encapsulated nucleic acids from degradation and clearance in serum. In addition, nanoliposome is also an excellent antigen carrier, not only encapsulating a series of antigens with different physicochemical properties and immune adjuvants, protecting protein polypeptide antigens from degradation, but also promoting the phagocytosis and presentation of antigen-presenting cells to antigen and enhancing the specific immune response of the body as well. Therefore, liposomal nanoparticles are widely used in the field of drug delivery.

Cationic lipids bearing positive charges on their surface form cationic liposome-nucleic acid drug complexes by electrostatic interaction with the negatively charged nucleic acids, while the whole cationic liposome-nucleic acid drug complexes bearing positive charges on their surface are prone to generate non-specific adsorption with serum proteins in plasma to form large-size aggregates, and the large-size aggregates are easily cleared by the reticuloendothelial system (RES) to cause short blood circulation time, poor stability and low transportation efficiency. For these reasons, the surfaces of cationic liposomes need to be modified to prepare long circulating cationic liposomes. Currently, the commonly used long circulating cationic liposomes are modified by PEGylated lipids, such as PEG-DMG. PEG interacts with the water molecules in solvent via hydrogen bonding to form a hydrated layer on the surface of the modified cationic liposome, and the hydrated layers conceal the positive charges on the surfaces of cationic liposomes to inhibit the protein adsorption and reduce the recognition by the phagocytic system. Therefore, PEGylated lipids are important for preparing long circulating cationic liposomes and further improving the nucleic acid transport efficiency.

Although PEGylated lipids, such as PEG-DMG, and PEGylated lipids of formula (II) of CN108368028A, have made great progress in drug delivery, there is still a need for novel PEGylated lipids that are suitable for regular therapeutic uses in this field.

### SUMMARY OF THE INVENTION

The present invention provides novel PEGylated lipids and preparation methods thereof, cationic liposomes containing the PEGylated lipids, pharmaceutical compositions containing the cationic liposome and formulations thereof, especially the nucleic acid pharmaceutical compositions containing the cationic liposome and formulations thereof. The formulations of cationic liposome-nucleic acid pharmaceutical compositions can deliver nucleic acid drugs into cells, and improve the transportation efficiency of nucleic acid drugs, thereby improving the treatment effect of nucleic acid drugs.

The above-mentioned aim can be achieved as follows.

**In** one embodiment according to the claimed invention, provided herein is a PEGylated lipid:
A PEGylated lipid having the structure represented by the general formula (2): 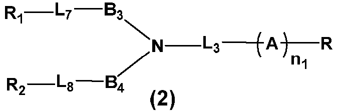
or a pharmaceutically acceptable salt, tautomer, or stereoisomer thereof,
wherein, one of L₇ and L₈ is -OC(=O)O-, -C(=O)-, -O-, -O(CR_{c}R_{c})ₛO-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, or -NR_{c}C(=O)S, and the other is a linking bond, -OC(=O), -C(=O)O-, -OC(=O)O-, -C(=O)-, -O-, -O(CR_{c}R_{c})ₛO-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, or -NR_{c}C(=O)S-, wherein, R_{c} is independently, at each occurrence, a hydrogen atom or an alkyl group, s is 2, 3, or 4.
L₃ is a linking bond, -L₄-, -Z-L₄-, -L₄-Z-, -Z-L₄-Z-, -L₄-Z-L₅-, -Z-L₄-Z-L₅-, -L₄-Z-L₅-Z-, -Z-L₄-Z-L₅-Z-, or -L₄-Z-L₄-Z-L₅-Z-; said L₄ and L₅ are carbon-chain linking groups, which are each independently represented by -(CRₐR_{b})ₜ-(CRₐR_{b})ₒ-(CRₐR_{b})ₚ-, wherein, t, p, and q are each independently an integer from 0 to 12, and t, o, and p are not simultaneously 0; Rₐ and R_{b} are, at each occurrence, each independently a hydrogen atom or a C₁₋₁₂ alkyl group; said Z is, at each occurrence, independently selected from the group consisting of -C(=O)-, -OC(=O)-, -C(=O)O-, -OC(=O)O-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NR_{c}C(=O)S-, and 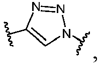 wherein, R_{c} is, at each occurrence, independently a hydrogen atom or a C₁₋₁₂ alkyl group;
B₃ and B₄ are each independently a linking bond or a C₁₋₃₀ alkylene group;
R₁ and R₂ are each independently a C₁₋₃₀ aliphatic hydrocarbon group;
R contains any atom or group selected from the group consisting of a hydrogen atom, an alkyl group, an alkoxy group, an alcoholic hydroxyl group, a protected alcoholic hydroxyl group, a thiol group, a protected thiol group, a carboxyl group, a protected carboxyl group, an amino group, a protected amino group, an aldehyde group, a protected aldehyde group, an ester group, a carbonate group, a urethane group, a succinimidyl group, a maleimide group, a protected maleimide group, a dimethylamino group, an alkenyl group, an alkenoate group, an azido group, an alkynyl group, a folate group, a rhodamine group, a biotinyl group, a monosaccharide group, and a polysaccharide group;
A is -(CRₐR_{b})ₛO- or -O(CRₐR_{b})ₛ-, wherein s is 2, 3, or 4;
n₁ is an integer approximately from 20 to 250;
said alkyl group, alkylene group, alkoxy group, and aliphatic hydrocarbon group are each independently substituted or unsubstituted.

Four preparation methods of the aforementioned PEGylated lipids are disclosed herein, specifically as follows:
Method-1: The foregoing PEGylated lipids can be prepared by the following steps:
   Step 1: Activating the carboxyl terminus of the acid A-1 or A-1' containing an unprotected carboxyl group with carboxyl activating agents to obtain the ester A-2 or A-2' containing an activated carboxyl terminus, wherein B₃' and B₄' are each independently a linking bond or an alkylene group having one less methylene group than B₃ and B₄; R₁' and R₂' are each independently R₁, R₂ or an aliphatic hydrocarbon group having one less methylene group than R₁ and R₂; R₇ is a carboxyl-activating group; and, when either B₃' or L₇ is not a linking bond, R₁' is R₁; when both B₃' and L₇ are linking bonds; R₁' is an aliphatic hydrocarbon group having one less methylene group than R₁; when either B₄' or L₈ is not a linking bond, R₂' is R₂; when both B₄' and L₈ are linking bonds, R₂' is an aliphatic hydrocarbon group having one less methylene group than R₂;
   Step 2: Carrying out the condensation reaction between the ester A-2 or A-2' containing an activated carboxyl terminus and the primary amine derivative A-3 or A-3' containing a nitrogen-source terminal group to obtain the amide intermediate A-4 or A-4';
   Step 3: Using reducing agents to reduce the amide intermediate A-4 or A-4' to the secondary amine intermediate A-5 or A-5';
   Step 4: Carrying out the coupling reaction between the secondary amine intermediate and the dual end-functionalized PEG derivative which is, the biLPEG molecule A-6, to obtain the PEGylated lipid derivative A-7 or A-7'; wherein, the biLPEG can be monodisperse or polydisperse; wherein, the two terminal functional groups of biLPEG can be the same or different; wherein, the R' end of biLPEG contains the reactive group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation; wherein, the F₁ contains active functional groups, which can react with the secondary amino group of the secondary amine intermediate A-5 or A-5' to obtain the nitrogen-atom branching center and the divalent linking group L₃;
   when R' is R, the structure of A-7 or A-7' is represented by the general formula (2);
   when R' is not R, A-8 or A-8' represented by the general formula (2) is obtained via terminal micro-modification of A-7 or A-7'; said terminal micro-modification is selected from chemical reactions including deprotection, salt complexation and decomplexation, ionization, protonation, deprotonation, and leaving group transformation.
   Step 1: 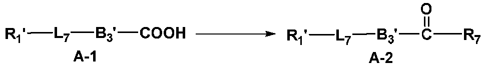 or 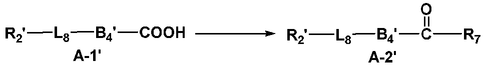
   Step 2: 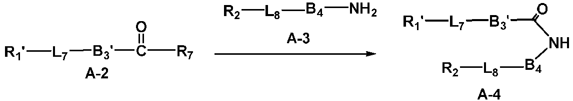 or 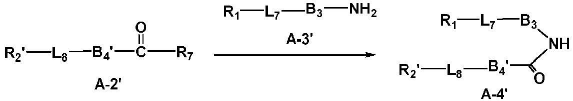
   Step 3: 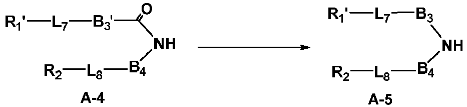 or or 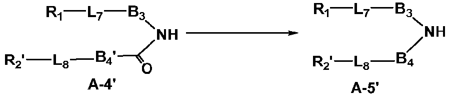
   Step 4: 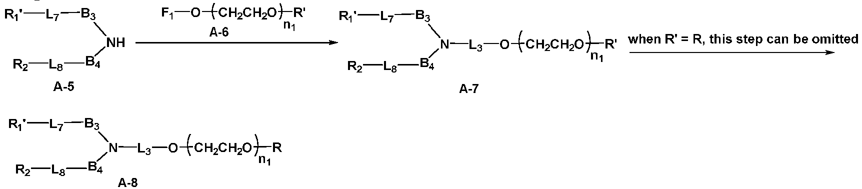 or 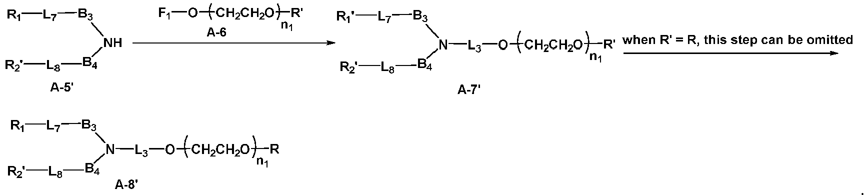
Method-2: The foregoing PEGylated lipids can be prepared by the following steps:
   Step 1: Carrying out the coupling reaction between the bifunctional PEG derivative biLPEG molecule B-1 and the primary amine derivative B-2 or B-2' containing a nitrogen-source terminal group to obtain the PEGylated secondary amine derivative B-3 or B-3'; wherein, biLPEG can be monodisperse or polydisperse, the two terminal functional groups of biLPEG can be the same or different, and the R' end of biLPEG contains the reactive group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation; wherein, the F₁ contains active functional groups, which can react with the amino group of the primary amine B-2 or B-2' to obtain the secondary amine derivative B-3 or B-3' containing the divalent linking group L₃;
   Step 2: Carrying out the alkylation reaction between the secondary amine derivative B-3 or B-3' and the compound B-4 or B-4' with the reactive group F_{N} to obtain the PEGylated lipid derivative B-5 or B-5'; said F_{N} is a reactive group which can react with amino group or secondary amino group, preferably -OMs, -OTs, -CHO, -F, -Cl, or -Br;
   when R' is R, the structure of B-5 or B-5' is represented by the general formula (2);
   when R' is not R, B-6 or B-6' represented by the general formula (2) is obtained via terminal micro-modification of B-5 or B-5'; said terminal micro-modification is selected from chemical reactions including deprotection, salt complexation and decomplexation, ionization, protonation, deprotonation, and leaving group transformation.
   Step 1: 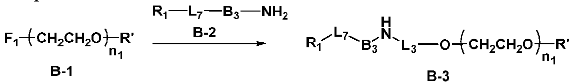 or 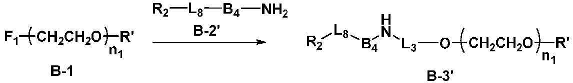
   Step 2: 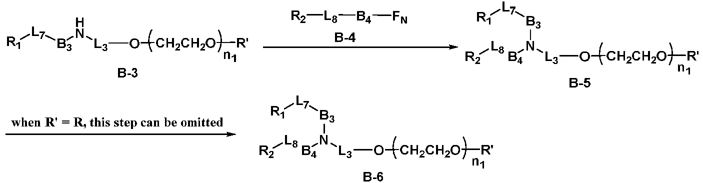 or 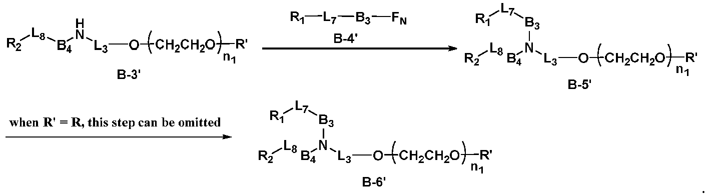
Method-3: The foregoing PEGylated lipids can be prepared by the following steps:
   Step 1: Carrying out the reaction between the small molecule C-1 and the small molecule C-2 to obtain the small molecule intermediate C-3 which contains the divalent linking group L₇, the reactive group F_{N} at one end, and an aliphatic hydrocarbon group R₁ at the other end; wherein, F₃ and F₄ are each independently a reactive group, which can form the divalent linking group L₇ via reaction; wherein, C-2 contains a pair of heterofunctional groups F₃ and F_{N}, and said F_{N} is the reactive group which can react with amino group or secondary amino group, preferably -OMs, -OTs, -CHO, -F, -Cl, or -Br;
   Step 2: Carrying out the alkylation reaction between two molecules of the small molecule intermediate C-3 and the PEGylated primary amine derivative C-4 containing a nitrogen-source terminal group to obtain the PEGylated lipid C-5, wherein the R' end contains the reactive group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation;
   when R' is R, the structure of C-5 is represented by the general formula (2);
   when R' is not R, C-6 represented by the general formula (2) is obtained via terminal micro-modification of C-5; said terminal micro-modification is selected from chemical reactions including deprotection, salt complexation and decomplexation, ionization, protonation, deprotonation and leaving group transformation; wherein, R₁ is the same as R₂, B₃ is the same as B₄, and L₇ is the same as L₈.
   Step 1: 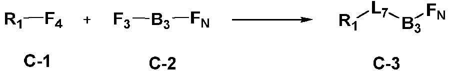
   Step 2: 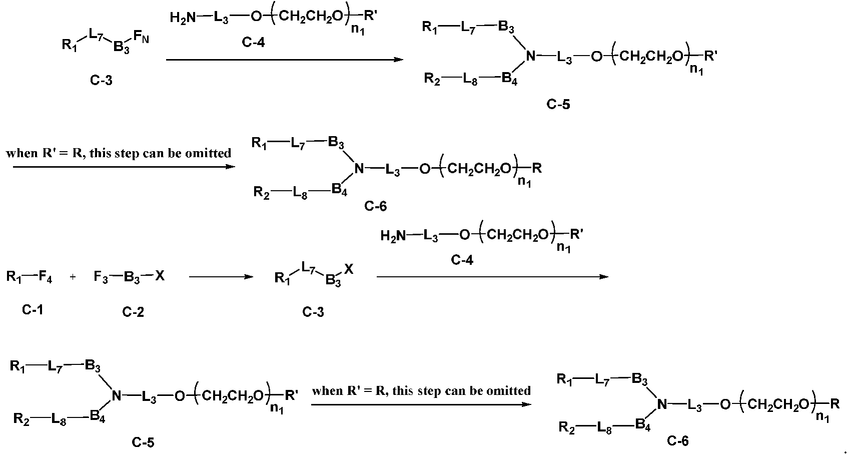
Method-4: The foregoing PEGylated lipids can be prepared by the following steps:
   Step 1: Carrying out the reaction between the small molecule D-1 and the small molecule D-2 to obtain the small molecule intermediate D-3 which contains the divalent linking group L₇, a hydroxyl group at one end, and an aliphatic hydrocarbon group R₁ at the other end; wherein, F₃ and F₄ are each independently a reactive group, which can form the divalent linking group L₇ via reaction; wherein, D-2 contains a pair of heterofunctional groups F₃ and a hydroxyl group;
   Step 2: Carrying out the reaction in which the hydroxyl group of the small molecule intermediate D-3 is oxidized to an aldehyde group to obtain the small molecule intermediate D-4 containing an aldehyde group, wherein B₃' is an alkylene group having one less methylene group than B₃;
   Step 3: Carrying out the addition reaction between two molecules of the small molecule intermediate D-4 containing an aldehyde group and the PEGylated primary amine derivative D-5 containing a nitrogen-source terminal group to obtain the PEGylated lipid D-6, wherein, the R' end contains the reactive group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation;
   when R' is R, the structure of D-6 is represented by the general formula (2);
   when R' is not R, D-7 represented by the general formula (2) is obtained via terminal micro-modification of D-6; said terminal micro-modification is selected from chemical reactions including deprotection, salt complexation and decomplexation, ionization, protonation, deprotonation and leaving group transformation; wherein, R₁ is the same as R₂, B₃ is the same as B₄, and L₇ is the same as L₈.
   Step 1: 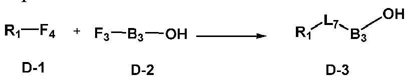
   Step 2: 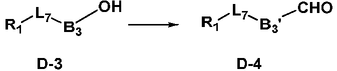
   Step 3:

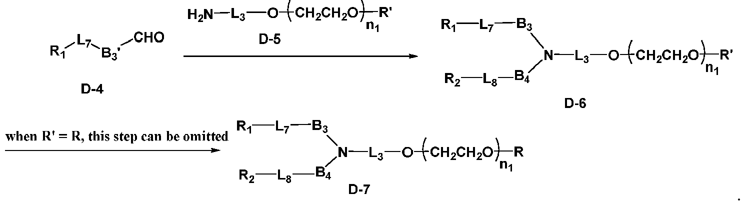

An embodiment as disclosed herein provides a cationic liposome:
A cationic liposome, containing the PEGylated lipid represented by the formula (2).

An embodiment as disclosed herein provides a cationic liposome pharmaceutical composition:
A liposome pharmaceutical composition, containing cationic liposomes and drugs, wherein said cationic liposomes contain the PEGylated lipid represented by the formula (2).

An embodiment as disclosed herein provides a formulation of liposome pharmaceutical composition:
A formulation of liposome pharmaceutical composition, containing the foregoing liposome pharmaceutical composition and pharmaceutically acceptable diluents or excipients.

### Compared with the prior art, the present invention brings the following beneficial effects:

The nitrogen atom as the branching center of the novel PEGylated lipid in the present invention is easily protonated under physiological pH conditions to produce partial positive charges, and thus the lipid is capable to bind negatively charged nucleic acids, thereby improving the loading efficiency of nucleic acid drugs.

The surfaces of the cationic liposomes can be modified by the novel PEGylated lipids of the present invention to obtain PEGylated cationic liposomes. The presence of the long-chain PEG overcomes the shortcoming of conventional cationic liposomes which are cleared by the phagocytes because of the serum proteins in plasma, improves the stability of cationic liposomes in serum, prolongs the circulation time in vivo, and further improves the transportation efficiency and treatment effect of drugs.

In the present invention, formulations of cationic liposome pharmaceutical compositions containing PEGylated lipids have stronger gene complexation ability and higher biocompatibility, and contribute to improving the therapeutic effect of drugs.

In the present invention, the terminals of the polyethylene glycol chains of the PEGylated lipids can also contain fluorescent groups or targeting groups, which furtherly improve the targeted therapeutic or diagnostic effect of the modified cationic liposome pharmaceutical compositions.

In the present invention, the polyethylene glycol chains of the PEGylated lipids can be degradable. In certain therapeutic areas, such as tumor therapy, the stable PEG coated on the surface of the liposome can keep the liposome components from leaving the endosome so that it prevents the delivery of the liposome contents into the cytoplasm, while the degradable PEG can be degraded to leave the liposome surface in the unique acidic environment of endosomal vacuoles or tumor tissues so that the therapeutic agent loaded in liposomes can be delivered to the target site efficiently.

### DETAILED DESCRIPTION OF THE INVENTION

### Description of terms

In the present invention, unless otherwise defined, all terms are defined as follows.

In the present invention, when a structure has isomers, it may refer to any form of the isomers unless otherwise specified. For example, when *cis-* and *trans-* isomers are present, it can refer to either a cis-structure or a trans-structure; when E and Z isomers are present, it can refer to either an (E)-structure or a (Z)-structure; and, when the structure has optical activity, it can be either a laevoisomer or a dextroisomer.

In the present invention, the definition of the numerical interval includes both the numerical interval indicated by a dash (e.g., 0-12) and the numerical interval indicated by a wavy line (e.g., 0~12). In the present invention, an integer interval denoted as an interval can represent the group consisting of all integers within the range of the interval unless otherwise specified, and said range includes two endpoints as well. For example, the integer interval 0-12 represents the group consisting of 0, 1, 2, 3, 4, 5, 6, 7, 8, 9, 10, 11 and 12. The numerical interval in the present invention includes but is not limited to the numerical intervals represented by integers, non-integers, percentages and fractions, and all of the foregoing numerical intervals include two endpoints unless otherwise specified.

In the present invention, a numerical value described with "about" or "approximately" generally indicates a numerical range of ±10% which, in some cases, can be amplified to ±15%, not exceeding ±20%, based on the preset numerical value. For example, provided that the molar percentage of steroid lipids in the total lipids in a solvent-containing solution is about 40%, it is generally considered that the molar percentage of steroid lipids is 30%-50%.

In the present invention, unless otherwise specified, the terms "include", "involve", "contain", and similar expressions in the description and claims shall be interpreted as "include but are not limited to", openly and inclusively.

In the present invention, when two or more objects are "preferably each independently selected from" something and there are multiple levels of preferred options, the objects are not necessarily selected from the preferred options of the same level but allowed to be any of the following cases: one is selected from a wider range of options while the another is selected from a narrower range of options, one is selected from the maximum range of options while another is selected from any allowable options, or all of the objects are selected from the preferred options of the same level. For example, "R₁ and R₂ are preferably each independently a linear alkyl group, more preferably a C₁₋₂₅ linear alkyl group, more preferably a C₁₋₁₇ linear alkyl group" means R₁ is a C₁₋₂₅ linear alkyl group and R₂ is a C₁₋₁₇ linear alkyl group, or R₁ is a C₁₋₁₇ linear alkyl group and R₂ is a C₁₋₂₅ linear alkyl group, or R₁ and R₂ are both C₁₋₂₅ linear alkyl groups, or R₁ and R₂ are both C₁₋₁₇ linear alkyl groups.

**In** the present invention, a divalent linking group, e.g., a hydrocarbylene group, an alkylene group, an arylene group, an amide bond, and the like, can use either one of its two connecting ends to be connected to another group, unless otherwise specified. For example, when an amide bond is used as a divalent linking group between C-CH₂CH₂- and -CH₂-D, both C-CH₂CH₂-C(=O)NH-CH₂-D and C-CH₂CH₂-NHC(=O)-CH₂-D are allowable.

In the present invention, when a terminal group of a linking group in a structural formula is easily confused with a substituent group of said linking group, " 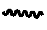 " is used to mark the location where the linking group is connected to other groups. For example, in the structural formulas 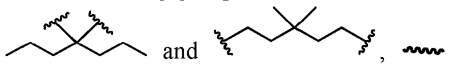 are used to mark the two locations where the divalent linking group is connected to other groups; the two aforementioned structural formulas represent -CH(CH₂CH₂CH₃)₂- and -CH₂CH₂CH(CH₃)₂-CH₂CH₂-, respectively.

In the present invention, the range of the number of carbon atoms in a group is denoted as a subscript of C, representing the number of carbon atoms of the group. For example, C₁₋₁₂ represents a group "having 1 to 12 carbon atoms", and C₁₋₃₀ indicates a group "having 1 to 30 carbon atoms". A "substituted C₁₋₁₂ alkyl group" refers to a compound obtained from one or more hydrogen atoms of a C₁₋₁₂ alkyl group being replaced by substituents. A "C₁₋₁₂ substituted alkyl group" refers to a compound having 1 to 12 carbon atoms which is obtained from one or more hydrogen atoms of an alkyl group being replaced by substituents. As another example, when a group can be selected from C₁₋₁₂ alkylene groups, it can be any alkylene group with the number of carbon atoms in the range indicated by the subscript, that is, the group can be selected from the group consisting of C₁, C₂, C₃, C₄, C₅, C₆, C₇, C₈, C₉, C₁₀, C₁₁ and C₁₂ alkylene groups. In the present invention, a subscript marked in interval form represents any integer within the range unless otherwise specified, and the numerical range includes two endpoints.

In the present invention, heteroatoms are not particularly limited, including but not limited to O, S, N, P, Si, F, Cl, Br, I, B, etc.

In the present invention, the heteroatom used for substitution is referred to as a "substituent atom", and the group used for substitution is referred to as a "substituent group".

In the present invention, the term "substituted" indicates that at least one hydrogen atom of any aforementioned group (e.g., aliphatic hydrocarbon groups, hydrocarbon groups, alkyl groups or alkylene groups) is replaced by a bond connected to a non-hydrogen atom, said non-hydrogen atom including but not limited to a halogen atom (F, Cl, Br, or I), an oxo group (=O), a hydroxyl group (-OH), a hydrocarbyloxy group (-OR_{d}, wherein R_{d} is a C₁₋₁₂ alkyl group), a carboxyl group (-COOH), an amine group (-NR_{c}R_{c}, wherein both R_{c} are each independently H or a C₁₋₁₂ alkyl group), a C₁₋₁₂ alkyl group, and a cycloalkyl group. In some embodiments, said substituent group is a C₁₋₁₂ alkyl group. In another embodiment, said substituent group is a cycloalkyl group. In another embodiment, said substituent group is a halide group, e.g., fluoride. In another embodiment, said substituent group is an oxo group. In another embodiment, said substituent group is a hydroxyl group. In another embodiment, said substituent group is an alkoxy group. In another embodiment, said substituent group is a carboxyl group. In another embodiment, said substituent group is an amine group.

In the present invention, the term "atomic spacing" or "atomic distance" refers to the number of main chain atoms spaced along the main chain, taking into account no pendant groups or side chains. It is usually the shortest atomic distance, which can be used to represent the length of the linking group. For example, the atomic spacing between A and B in A-CO-NH-B is 2, that in A-*p*-Ph-CH₂-B is 5 (wherein *p*-Ph is *p*-phenylene), and that in A-CH(CH₂CH₂CH₂CH₃)-B is 1. The "main-chain atoms" accounted for the atomic spacing can only be non-hydrogen atoms. Wherein, concerning the divalent linking group with a ring structure, its atomic spacing refers to the minimum number of atoms calculated along the ring. For example, the atomic spacing of *p*-phenylene, namely 1,4-phenylene, is 4, the atomic spacing of *m*-phenylene is 3, and the atomic spacing of *o*-phenylene is 2. For another example, the atomic spacings of -CH₂-, -CH(CH₃)-, -C(CH₃)₂-, -CH(CH₂Ph)₂- and -C(CH₂OX)- are all 1.

In the present invention, a "carbon chain linking group" refers to the linking group whose main chain atoms are all carbon atoms, while the pendant chains are allowed to contain heteroatoms or heteroatom-containing groups that replace the hydrogen atoms connected to the main chain carbon atoms. When a "main chain atom" is a heteroatom, it can also be termed a "main chain heteroatom". For example, A-S-CH₂-B, A-O-CH₂-B, and 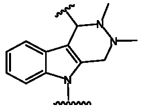 (wherein the atomic spacing is 4) are considered to contain main chain heteroatoms. Carbon chain linking groups can be divided into hydrocarbylene groups and carbon chain linking groups containing heteroatoms in their pendant groups; said pendant groups containing heteroatoms of carbon chain linking groups include but are not limited to an oxo group (=O), a thioxo group (=S), an imino group (connected to the main chain carbon through a carbon-nitrogen double bond), an oxa-hydrocarbon group in the form of an ether bond, a thia-hydrocarbon group in the form of a thioether bond, an aza-hydrocarbon group in the form of a tertiary amino group, etc. The main chain of the "carbon chain linking group" is entirely composed of carbon atoms, and the pendant groups of the carbon chain are allowed to contain heteroatoms, that is, the main chain is constructed by connecting methylene groups or substituted methylene groups. Said substituted methylene group can be substituted by one monovalent substituent, two monovalent substituents, or one divalent substituent (e.g., a divalent oxygen atom, or one that forms a three-membered ring 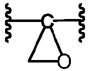 with a divalent methylene group). Said substituted methylene group can be a methylene group with one hydrogen atom being substituted (e.g., -CH(CH₃)-), two hydrogen atoms being substituted respectively (e.g., -(CH₃)C(OCH₃)-), or two hydrogen atoms being substituted at the same time (e.g., a carbonyl group, a thiocarbonyl group, -C(=NH)-, -C(=N⁺H₂)-, and a cyclic pendant group (e.g., 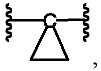 wherein the atomic spacing is 1) ).

In the present invention, the secondary amino bond and the hydrazine bond refer to the "-NH-" capped with hydrocarbylene groups at both ends, e.g., -CH₂-NH-CH₂-; while -C(=O)-NH- is termed an amide bond instead of a secondary amino bond.

In the present invention, a compound, a group, or an atom can be substituted and heterosubstituted at the same time, for example, a hydrogen atom can be replaced by a nitrophenyl group, and -CH₂-CH₂-CH₂- can be replaced by -CH₂-S-CH(CH₃)-.

In the present invention, a "linking bond" is only for connection and contains no atoms. If the definition of a group includes a linking bond, it means the group can be absent.

In the present invention, the expression "at each occurrence, each independently" not only mean that different groups can be each independently selected from the definitions, but also mean that the same group appearing at each different position can also be independently selected from the definition. For example, Z is, at each occurrence, independently selected from the group consisting of -C(=O)-, -OC(=O)-, -C(=O)O-, -OC(=O)O-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NR_{c}C(=O)S-, and 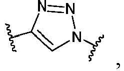 wherein R_{c} is, at each occurrence, independently a hydrogen atom or a C₁₋₁₂ alkyl group; in the group "-NR_{c}C(=O)NR_{c}-", two R_{c} groups can be the same or different, which are each independently a hydrogen atom or a C₁₋₁₂ alkyl group.

In the present invention, the term "group" contains at least 1 atom, referring to the radical formed by a compound losing one or more atoms. Relative to a compound, the group formed by a compound losing part of its groups is also termed a residue. The valence of a group is not particularly limited, and examples include a monovalent group, a divalent group, a trivalent group, a tetravalent group, ..., a hectovalent group, etc. Wherein, groups whose valence is equal to or greater than 2 are collectively defined as linking groups. A linking group can also contain only one atom, such as an oxy group and a thioxy group.

In the present invention, the term "hydrocarbon" refers to a class of compounds that contains only carbon atoms and hydrogen atoms.

In the present invention, hydrocarbons are classified into aliphatic hydrocarbons and aromatic hydrocarbons by category. Hydrocarbons containing neither phenyl rings nor hydrocarbyl-substituted phenyl rings are defined as aliphatic hydrocarbons. Hydrocarbons containing at least one phenyl ring or hydrocarbyl-substituted phenyl ring are defined as aromatic hydrocarbons. An aromatic hydrocarbon can contain aliphatic hydrocarbon groups, such as toluene, diphenylmethane, 2,3-dihydroindene, etc.

In the present invention, hydrocarbons are classified into saturated hydrocarbons and unsaturated hydrocarbons by the degree of saturation. All aromatic hydrocarbons are unsaturated hydrocarbons. Saturated aliphatic hydrocarbons are also termed alkanes. An unsaturated aliphatic hydrocarbon is not particularly limited with respect to the degree of unsaturation. For example, it includes but is not limited to alkenes (containing carbon-carbon double-bonds), alkynes (containing carbon-carbon triple-bonds), dienes (containing two conjugated carbon-carbon double-bonds), and the like. When the aliphatic moieties are saturated in an aromatic hydrocarbon, said aromatic hydrocarbon is also termed aryl alkane, such as toluene.

In the present invention, the structures of hydrocarbons are not particularly limited, including linear structures without pendant groups, branched structures bearing pendant groups, ring-containing structures containing at least one ring, dendritic structures, comb-like structures, hyperbranched structures, etc. Unless otherwise specified, preferable structures include linear structures without pendant groups, branched structures bearing pendant groups and ring-containing structures, which correspond to linear hydrocarbons, branched hydrocarbons and cyclic hydrocarbons, respectively. Wherein, hydrocarbons without cyclic structures are termed open-chain hydrocarbons, including but not limited to linear structures without pendant groups and branched structures bearing pendant groups. Open-chain hydrocarbons fall into the scope of aliphatic hydrocarbons, so linear hydrocarbons are also described as linear aliphatic hydrocarbons and branched hydrocarbons are also described as branched aliphatic hydrocarbons.

In the present invention, hydrocarbons with any carbon atom replaced by a heteroatom are collectively referred to as hetero-hydrocarbons.

In the present invention, an aliphatic hetero-hydrocarbon refers to the hetero-hydrocarbon derived from an aliphatic hydrocarbon, including aliphatic heterocyclic hydrocarbons and aliphatic open-chain hetero-hydrocarbons. Saturated aliphatic hetero-hydrocarbons are also termed heteroalkanes.

In the present invention, "hydrocarbon group" refers to the residue of a hydrocarbon molecule with at least one hydrogen atom removed. According to the number of the lost hydrogen atoms, hydrocarbon groups can be classified into monovalent hydrocarbon groups (with one hydrogen atom lost), divalent hydrocarbon groups (with two hydrogen atoms lost, also referred to as hydrocarbylene groups), trivalent hydrocarbon groups (with three hydrogen atoms lost), and the like. Accordingly, when n hydrogen atoms are lost, the valence of the resulting hydrocarbon group is n. In the present invention, hydrocarbon groups refer specifically to monovalent hydrocarbon groups, unless otherwise specified.

In the present invention, the source of hydrocarbon group is not particularly limited, for example, aliphatic hydrocarbons or aromatic hydrocarbons, saturated hydrocarbons or unsaturated hydrocarbons, linear hydrocarbons, branched hydrocarbons or cyclic hydrocarbons, hydrocarbons or hetero-hydrocarbons, etc. According to the degree of saturation, e.g., the source can be alkanes, alkenes, alkynes, or dienes, etc.; with respect to cyclic hydrocarbons, the source can be, e.g., alicyclic hydrocarbons or aromatic hydrocarbons, monocyclic hydrocarbons or polycyclic hydrocarbons; and, with respect to heterocyclic hydrocarbons, the source can be, e.g., aliphatic heterocyclic hydrocarbons or aromatic heterocyclic hydrocarbons.

In the present invention, the "aliphatic hydrocarbon group" refers to the residue of an aliphatic hydrocarbon molecule with at least one hydrogen atom removed. Aliphatic hydrocarbon groups in the present invention refer specifically to monovalent aliphatic hydrocarbon groups, unless otherwise specified. Aliphatic hydrocarbon groups include saturated aliphatic hydrocarbon groups and unsaturated aliphatic hydrocarbon groups.

In the present invention, the "alkyl group" refers to a hydrocarbon group obtained from an alkane losing a hydrogen atom at any location, unless otherwise specified. Said alkyl group can be linear or branched, substituted or unsubstituted. Specific examples include that a propyl group refers to either a 1-propyl group or an isopropyl group, and a propylene group refers to a 1,3-propylene group, a 1,2-propylene group or an isopropylidene group.

In the present invention, the "unsaturated hydrocarbon group" refers to a hydrocarbon group obtained from an unsaturated hydrocarbon losing hydrogen atoms. The hydrocarbon group obtained from an unsaturated hydrocarbon losing hydrogen atoms bonded to unsaturated carbon atoms, can be an alkenyl group, an alkynyl group, or a dienyl group, etc., e.g., a propenyl group or a propynyl group. According to the difference of unsaturated bonds, the hydrocarbon group obtained from an unsaturated hydrocarbon losing hydrogen atoms bonded to saturated carbon atoms, can be termed, e.g., an alkenyl hydrocarbon group, an alkyne, a dienyl hydrocarbon group, or the like, and specifically, e.g., an allyl group or a propargyl group.

In the present invention, the "alkenyl" or "alkenyl group" refers to a substituted or unsubstituted, linear or branched alkenyl group which contains two or more carbon atoms (e.g., two, three, four, five, six, seven, eight, nine, ten, eleven, twelve, thirteen, fourteen, fifteen, sixteen, seventeen eighteen, nineteen, twenty or more carbon atoms) and at least one carbon-carbon double bond. The term "C₂₋₁₅ alkenyl group" refers to a substituted or unsubstituted, linear or branched alkenyl group which contains 2-15 carbon atoms and at least one carbon-carbon double bond, that is, an alkenyl group can contain one, two, three, four, or more carbon-carbon double bonds. Alkenyl groups in the present invention include substituted and unsubstituted alkenyl groups, unless otherwise specified.

In the present invention, the "alkynyl" or "alkynyl group" refers to a linear or branched, optionally substituted hydrocarbon which contains two or more carbon atoms (e.g., two, three, four, five, six, seven, eight, nine, ten, eleven, twelve, thirteen, fourteen, fifteen, sixteen, seventeen eighteen, nineteen, twenty or more carbon atoms) and at least one carbon-carbon triple bond. The term "C₂₋₁₅ alkynyl group" refers to a substituted or unsubstituted, linear or branched alkynyl group which contains 2-15 carbon atoms and at least one carbon-carbon triple bond, that is, an alkynyl group can contain one, two, three, four, or more carbon-carbon triple bonds. Alkynyl groups in the present invention include substituted and unsubstituted alkynyl groups, unless otherwise specified.

In the present invention, the term "molecular weight" represents the mass of a compound, and the term "average molecular weight" represents the mass of a compound component of a general formula in macroscopic matter. Unless otherwise specified, the "average molecular weight" refers to the "number-average molecular weight" (Mₙ). The number-average molecular weight refers to the molecular weight of polydisperse blocks or substances, or that of monodisperse blocks or substances. Unless otherwise specified, the unit of measurement of "molecular weight" and "average molecular weight" is dalton, Da. The molecular weight of a polyethylene glycol chain can also be measured by "degree of polymerization" which specifically refers to the number of repeating units (oxyethylene units, EO-units) in a compound molecule. Accordingly, "average degree of polymerization", "number-average degree of polymerization" or "number of EO units" represents the average value or the number average value of the number of repeating units.

In the present invention, for polydisperse cases, when a term such as "equal", "same", "equivalent", or "approximately equal" (including other forms of equivalent expression) is used to describe the molecular weight or the degree of polymerization of a single compound and the number-average molecular weight or the number-average degree of polymerization of a compound component in macroscopic matter, unless otherwise specified, the term does not impose a strict numerical equality but indicates an approximation or an approximate equality in value; said approximation or approximate equality preferably refers to a deviation within ±10%, more preferably a deviation within ±5%, generally based on the preset value. The terms "about" and "approximately" generally indicate a numerical range of ±10% which, in some cases, can be amplified to ±15%, not exceeding ±20%. For example, the deviations of 10 kDa from 11 kDa and 12 kDa are 10% and 20%, respectively. As another example, when the molecular weight of a certain PEG component in the specified general formula is 5 kDa, it is allowed that the corresponding molecular weight or number-average molecular weight is 5 kDa±10%, that is, variable within the range of 4500~5500 Da. However, for monodisperse cases, the sameness or equality with respect to the number of oxyethylene units in a single compound molecule or general formula thereof indicates the strict numerical equality; for example, if the number of EO units of a certain PEG component is set to 11, then a value being 12 is beyond the scope; whereas, in order to obtain the compound component with the desired number of EO units, the products in macroscopic matter obtained by certain preparation methods may also contain impurities with other numbers of EO units besides the component with the target number of EO units because of the limitation of preparation methods and purification methods; in this case, if the deviation of the average number of EO units from the preset number of EO units is within ±5% (preset value ≥10) or within ±0.5 (preset value <10), it can be considered to have obtained the monodisperse product in macroscopic matter with the target component; moreover, when the content of the component with the number of EO units as desired or within the range of average number reaches certain percentages (preferably ≥90%, more preferably >95%, more preferably >96%, more preferably >98%, more preferably 99% to 100%), the obtained product in macroscopic matter falls within the protected scope of the present invention; even if the above mentioned content percentages are not met, the resulting products of insufficient contents and the components in the form of co-products or by-products, as long as prepared by the preparation methods of the present invention or by similar methods using basically the same preparation ideas, whether separation and purification are performed or not, all fall within the protected scope of the present invention.

In the present invention, when Da, kDa, repeating unit, and number of EO units are used to describe the molecular weight of a compound of a general formula containing polydisperse components, for a single molecule, the value falls within a certain range covering the specified value (including endpoints, preferably within the range of ±10%); when the number of oxyethylene units is used to describe the preset molecular weight of a compound of a general formula containing monodisperse components, the value is not variable in any range but a discrete point; however, the average number of the EO units might be variable within a certain range (not exceeding ±10% or ±1, preferably not exceeding ±5% or ±0.5) because of heterogeneity in molecular weights of the prepared product. For example, the molecular weight of mPEG is 5 kDa, it means the molecular weight of a single molecule of the general formula is between 4500~5500 Da, and the average molecular weight of the corresponding component of the prepared product is 5 kDa, that is, the product with the average molecular weight between 4500~5500 Da is regarded as the target product, and only the components whose molecular weights fall within the scope contribute to the content of the target component; for another example, if the designed mPEG contains 22 oxyethylene units, then the numbers of EO units of all compound molecules of the general formula should be strictly 22, while the prepared product could be a mixture of compounds with the numbers of EO units being 20, 21, 22, 23, and 24, and in this case, when the average number of EO units is within the range of 22±2.2 (preferably in the range of 22±1.1), it is considered to have obtained the target component, meanwhile the components whose molecular weights fall within the numerical range can be regarded as the target component and used to calculate the purity. In the present invention, the product with PDI<1.005 can be considered to be monodisperse with PDI=1.

In the present invention, concerning the polydispersity index PDI in the present invention, different batches of materials can be regarded as the same material without significant difference, as long as the PDI values do not exceed the preset value while the other parameters are the same or considered to be the same.

In the present invention, concerning the percentage, "about" and "approximately" generally refer to a tolerance of ±0.5%.

In the present invention, the terms "stable" and "degradable" regarding groups are a pair of opposing concepts.

In the present invention, the term "degradable" ("be degradable" or "can be degraded") means that a chemical bond can be cleaved into at least two independent residues. If a linking group remains whole, but with its structure altered after chemical changes, then it still belongs to the scope of "being stable". The conditions to be "degradable" are not particularly limited, which can be physiological conditions in vivo, simulated physiological environments in vitro, or other conditions, preferably physiological conditions in vivo and simulated physiological environments in vitro. Said physiological condition is not particularly limited, including but not limited to physiological environments of serum, heart, liver, spleen, lung, kidney, bone, muscle, fat, brain, lymph node, small intestine, gonad, etc., which are intracellular or in the extracellular matrix, in normal tissues or in pathologic tissues (e.g., tumor, inflammation, etc.). Said simulated physiological environment in vitro is not particularly limited, including but not limited to physiological saline, buffer, culture medium, and the like. The degradation is not particularly limited with respect to the rate, e.g., rapid degradation via enzymolysis, slow hydrolysis under physiological conditions, etc. Said physiological conditions in vivo include physiological conditions during treatment such as ultraviolet radiation, thermal therapy, etc. The conditions for degradation include but are not limited to light, heat, low temperature, enzymatic condition, oxidation-reduction condition, acidic condition, basic condition, physiological condition, simulated physiological environment in vitro, etc., preferably light, heat, enzymatic condition, oxidation-reduction condition, acidic condition, basic condition, etc. Said "degradable" means that the degradation can occur under any stimulation from the above conditions. Said light condition includes but is not limited to visible light, ultraviolet light, infrared light, near-infrared light, mid-infrared light, etc. Said heat condition refers to the temperature higher than the normal physiological temperature, normally the temperature higher than 37°C and below 45°C, and preferably below 42°C. Said low temperature refers to the temperature below the human physiological temperature, preferably below 25°C, more preferably ≤10°C, with specific examples such as refrigeration temperature, freezer temperature, temperature for liquid nitrogen treatment, 2~10°C, 4~8°C, 4°C, 0°C, -20±5°C, etc. Said enzymatic condition is not particularly limited, and all enzymes that can be physiologically generated are included, e.g., peptidases, proteases, lyases, etc. Said oxidation-reduction condition is not particularly limited, e.g., redox transformation or hydrogenation-reduction transformation between a mercapto group and a disulfide bond. Said acidic condition and basic condition mainly refer to the pH conditions of internal body parts such as normal tissues, pathologic tissues, and organs or tissues in treatment; for example, the stomach is under acidic condition, and a tumor site is usually under acidic condition as well. Said "degradable" means that the degradation can be realized through metabolism in vivo (e.g., physiological effect, enzymatic reaction, oxidation-reduction, etc.), microenvironment stimulation in specific areas inside the body (e.g., acidic condition and basic condition), clinical therapeutic stimulation (e.g., light, heat, and low temperature), etc. What should be noted is that, for bond cleavage, some conditions in organic chemistry that are extreme for organisms, e.g., strong acid, strong base, high temperature (e.g., above 100°C), etc., are not included in the scope of the conditions for degradation of degradable bonds in the present invention. For example, although an ether bond can be cleaved under strong acid conditions (e.g., hydrobromic acid), it is always classified as a stable linking group in the present invention.

In the present invention, as long as a linking group can keep as a whole linking group (i.e., a linking group which can keep covalently linking with the adjacent groups), it would be defined as "stable" ("be stable" or "can remain stable"), wherein chemical changes without breaking the wholeness of the linking group are allowed. Said chemical changes are not particularly limited, including but not limited to isomerization, oxidation reaction, reduction reaction, ionization, protonation, deprotonation, substitution reaction, etc. The conditions to be "stable" are not particularly limited, including but not limited to light, heat, low temperature, enzymatic condition, oxidation-reduction, neutral condition, acidic condition, basic condition, physiological condition, simulated physiological environment in vitro, etc., preferably light, heat, enzymatic condition, oxidation-reduction, acidic condition, basic condition, etc. A group or compound being "stable" indicates that the components can remain stably connected in the metabolic cycle in vivo and the molecular weight will not be reduced due to chain cleavage as long as the group or compound maintains integrity without particular stimulation (e.g., pH condition in specific areas and light, heat, and low temperature in treatment).

In the present invention, for a specific linking group, "stable" is not an absolute concept. For example, an amide bond is much more stable than an ester bond under acidic or basic condition, and in the present invention, "stable" linking groups include amide bonds. However, the peptide bond, for example, is a special kind of amide bond formed via the dehydration condensation of two amino acids that respectively provide an α-carboxyl group and an α-amino group for the reaction, could also be cleaved when encountering specific enzymatic interactions, and therefore it is also within the scope of "degradable" linking groups. Similarly, a urethane group, a thiourethane group, or the like could be either a "stable" linking group or a "degradable" linking group. More commonly, a urethane group, a thiourethane group, and the like tend to degrade slowly, while an amide bond in the form of a non-peptide bond can remain stable in the systemic circulation in vivo. As another example, common ester bonds will degrade under acidic or basic condition, while an ester bond contained in a special structure could also degrade under UV exposure. As another example, even though some chemical bonds will degrade under specific enzymatic conditions, they can still be regarded as stable if their circulation in clinical use (e.g., point-of-site administration) does not or basically not go through said enzymatic conditions.

In the present invention, in order to define more clearly the degradability of a compound structure, provided herein is a criterion for reference which is, a threshold being a specific percentage (e.g., 90%) of the examined chemical bond maintained within a limited time interval. Taking 90% as an example, it usually takes the pharmacokinetic curve of functionalized PEGylated products as a reference, and is based on the dose percentage that meets the clinical evaluation criteria. For example, for intravenously administered PEGylated drugs, when the plasma concentration (calculated in terms of effective drug ingredients including PEGylated drugs and non-PEGylated components after degradation) is lower than 15% of the initial concentration (or another percentage more consistent with the clinical evaluation of the drug), the remaining 85% is taken as the base value; if the proportion of a linking group with its chemical bonds maintained exceeds 90%, then the group is regarded as stable in the present invention; on the contrary, if said 90% is not met, then the linking group belongs to degradable groups. The hydrolytic stabilization and enzymatic degradation reported in the published literature are also included in the present invention. Taking hydrolytic stabilization as an example, the hydrolysis rate in the process of hydrolytic stabilization reported in the published literature is also included herein, preferably referring to the hydrolysis rate less than 1-2% per day (generally 2%) under physiological conditions, in mass or molar weight. The hydrolysis rate of typical chemical bonds can be found in most standard handbooks of chemistry.

In the present invention, the term "hydroxyl protecting group" includes all the groups which can be used as common hydroxyl protecting groups. A hydroxyl protecting group is preferably selected from the group consisting of an alkanoyl group (e.g., an acetyl group and a butyryl group), an aromatic alkanoyl group (e.g., a benzoyl group), a benzyl group, a triphenylmethyl group, a trimethylsilyl group, a t-butyldimethylsilyl group, an allyl group, an acetal group, and a ketal group. The removal of an acetyl group is generally carried out under basic condition, most commonly by the ammonolysis with NH₃/MeOH or by the methanolysis catalyzed by methanol anions. The benzyl group is easy to be removed via palladium-catalyzed hydrogenolysis in a neutral solution at room temperature, or via the reduction reaction with metallic sodium in ethanol or liquid ammonia. The triphenylmethyl group is usually removed by catalytic hydrogenolysis. The trimethylsilyl group is usually removed with reagents containing fluoride ions (e.g., tetrabutylammonium fluoride/anhydrous THF, etc.). The t-butyldimethylsilyl ether is relatively stable, which can withstand the ester hydrolysis with alcoholic potassium hydroxide and mild reduction conditions (e.g., Zn/CH₃OH and the like), and can be removed by fluoride ions (e.g., Bu₄N⁺F⁻) in THF solution or by aqueous acetic acid at room temperature.

In the present invention, the "carboxyl protecting group" refers to the protecting group which can be transformed into a carboxyl group through hydrolysis or the deprotection reaction of the carboxyl protecting group itself. A carboxyl protecting group is preferably selected from the group consisting of an alkyl group (e.g., a methyl group, an ethyl group and a butyl group) and an aralkyl group (e.g., a benzyl group), and more preferably selected from the group consisting of a butyl group (tBu), a methyl group (Me), and an ethyl group (Et). In the present invention, the "protected carboxyl group" refers to the group obtained from a carboxyl group being protected by an appropriate carboxyl protecting group, preferably selected from the group consisting of a methoxycarbonyl group, an ethoxycarbonyl group, a t-butyloxycarbonyl group, and a benzyloxycarbonyl group. Said carboxyl protecting group can be removed through hydrolysis catalyzed by acids or alkalis, or through pyrolysis reaction occasionally; for example, *t*-butyl groups can be removed under mild acidic condition, and benzyl groups can be removed by hydrogenolysis. The reagent used for removing carboxyl protecting groups is selected from the group consisting of TFA, H₂O, LiOH, NaOH, KOH, MeOH, EtOH and combinations thereof, preferably selected from the combination of TFA and H₂O, the combination of LiOH and MeOH, and the combination of LiOH and EtOH. The deprotection of a protected carboxyl group can produce the corresponding free acid, which can be carried out in the presence of an alkali that forms pharmaceutically acceptable salts with said free acid generated during said deprotection.

In the present invention, the term "amino protecting group" includes all the groups which can be used as common amino protecting groups, such as an aryl C₁₋₆ alkyl group, a C₁₋₆ alkoxy C₁₋₆ alkyl group, a C₁₋₆ alkoxycarbonyl group, an aryloxycarbonyl group, C₁₋₆ alkylsulfonyl group, an arylsulfonyl group, a silyl group, etc. An amino protecting group is preferably selected from the group consisting of a *t*-butoxycarbonyl group (Boc), a *p*-methoxybenzyloxycarbonyl group (Moz) and a 9-fluorenylmethoxycarbonyl group (Fmoc). The reagent used for removing amino protecting groups is selected from the group consisting of TFA, H₂O, LiOH, MeOH, EtOH and combinations thereof, preferably selected from the combination of TFA and H₂O, the combination of LiOH and MeOH, and the combination of LiOH and EtOH. The reagent used for removing the Boc protecting group is TFA or HCl/EA, preferably TFA. The reagent used for removing the Fmoc protecting group is the *N,N*-dimethylformamide (DMF) solution containing 20% piperidine.

In the present invention, the "activation of a carboxyl group" refers to the activation of a carboxyl group with carboxyl activating agents. The activated carboxyl group can promote the condensation reactions, for example, by inhibiting the generation of racemic impurities, by accelerating the reaction through catalysis, etc. The "carboxyl activating group" refers to the residue of a carboxyl activating agent. Said carboxyl activating agent is selected from the group consisting of *N*-hydroxysuccinimide (NHS), 1-ethyl-3-(3-dimethylaminopropyl)carbodiimide hydrochloride (EDCI), *N*-hydroxy-5-norbornene-2,3-dicarboximide (HONb), and *N,N'-d*icyclohexylcarbodiimide (DCC), and combinations thereof, preferably selected from the combination of NHS/EDCI, the combination of NHS/DCC, and the combination of HONb/DCC, and more preferably the combination of NHS/EDCI.

In the present invention, the term "cation" refers to the corresponding structure bearing a positive charge, either permanently, or non-permanently but in response to certain conditions (such as pH). Therefore, the cations include permanent cations and those cationisable compounds, groups, or atoms. Permanent cations refer to the corresponding compounds, groups, or atoms that bear positive charges under conditions of any pH value or hydrogen ion activity of their environment. Typically, a positive charge is generated by the presence of quaternary nitrogen atom. When a compound carries multiple such positive charges, it can be called a permanent cation. A cationisable substance refers to a compound, group or atom that is positively charged at a lower pH and uncharged at a higher pH of its environment. On the other hand, in non-aqueous environments where the pH cannot be determined, a cationisable compound, group, or atom is positively charged at a high hydrogen ion concentration and uncharged at a low concentration or activity of hydrogen ions. It depends on the individual properties of the cationisable or polycationisable compound, especially the pKa of the respective cationisable group or atom, at which pH or hydrogen ion concentration said compound is charged or uncharged. In the diluted aqueous environment, the so-called Henderson-Hasselbalch equation can be used to estimate the fraction of positively charged cationisable compounds, groups or atoms, which is well-known to those skilled in the art. For example, in some embodiments, if a compound or moiety is cationisable, it is preferred that it is positively charged at a pH value of about 1 to 9, preferably 4 to 9, 5 to 8 or even 6 to 8, more preferably at a pH value of or below 9, of or below 8, of or below 7, and most preferably at physiological pH values (e.g., about 7.3 to 7.4), i.e., under physiological conditions, particularly under physiological salt conditions of the cell in vivo. In other embodiments, it is preferred that the cationisable compound or moiety is predominantly neutral at physiological pH values (e.g., about 7.0-7.4), but becomes positively charged at lower pH values. In some embodiments, the preferred range of pKa for the cationisable compound or moiety is about 5 to about 7.

In the present invention, the "cationic component/compound" typically refers to a charged molecule, which is positively charged (cationic) at a pH value of typically about 1 to 9. In some embodiments, the cationic component/compound is preferably charged at a pH value of or below 9 (e.g., 5 to 9), of or below 8 (e.g., 5 to 8), of or below 7 (e.g., 5 to 7), and most preferably at physiological pH values (e.g., about 7.3 to 7.4). Therefore, a cationic peptide, protein, polysaccharide, lipid or polymer according to one embodiment of the present invention is positively charged under physiological conditions, particularly under physiological salt conditions of the cell in vivo.

In the present invention, a lipid nanoparticle, cationic peptide, protein, polysaccharide, lipid, or polymer is uncharged, having a neutral charge or being respectively electrically neutral under physiological conditions, particularly under the physiological salt conditions of the cell in vivo. A cationic peptide or protein preferably contains a larger amount of cationic amino acids, e.g., a greater number of Arg, His, Lys or Orn than other amino acid residues (especially more cationic amino acids than anionic amino acid residues like Asp or Glu), or contains blocks predominantly formed by cationic amino acid residues. The expression "cation" may also refer to "polycationic" components/compounds. The cationic component/compound may also refer to a cationic lipid capable of being positively charged. For example, cationic lipids contain one or more amine groups bearing positive charges, and the preferred cationic lipids are ionizable so that they can exist in a positively charged or neutral form depending on pH. The ionization of the cationic lipid affects the surface charge of a lipid nanoparticle (LNP) under different pH conditions. This charge state can influence plasma protein absorption, blood clearance and tissue distribution as well as the ability to form non-bilayer structures critical to the intracellular delivery of nucleic acids.

In the present invention, the "PEGylated lipid " refers to a molecule containing lipid and polyethylene glycol moieties. In addition to those represented by the general formula (2) in the present invention, PEGylated lipids also include but are not limited to 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-cholesterol, PEG-diacylglycamide (PEG-DAG), PEG-dialkyloxypropyl (PEG-DAA), and specifically PEG500-dipalmitoylphosphatidylcholine, PEG2000-dipalmitoylphosphatidylcholine, PEG500-stearylphosphatidylethanolamine PEG2000-distearylphosphatidylethanolamine, PEG500-1,2-oleoylphosphatidylethanolamine, PEG2000-1,2-oleoylphosphatidylethanolamine, PEG2000-2,3-distearoylglycerol (PEG-DMG), and the like.

In the present invention, the "neutral lipid" refers to any lipid substance which is uncharged or exists in the form of neutral zwitterion at the chosen pH, preferably phospholipid. This kind of lipid includes but is not limited to 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanola mine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dioleoylphosphatidylserine (DOPS), dipalmitoylphosphatidylglycerol (DPPG), palmitoyloleoylphosphatidylethanolamine (POPE), distearoylphosphatidylethanolamine (DSPE), dipalmitoylphosphatidylethanolamine (DPPE), dimyristoylphosphoethanolamine (DMPE), 1-stearoyl-2-oleoyl-phosphatidyethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoylphosphatidylcholine, lysophosphatidylcholine, lysophosphatidylethanolamine (LPE), and combinations thereof. The neutral lipid can be synthetic or natural.

In the present invention, a "steroid lipid" is selected from the group consisting of a cholesterol, a coprostanol, sitosterol, an ergosterol, a campesterol, a stigmasterol, a brassicasterol tomatidine, an ursolic acid, a α-tocopherol, and mixtures thereof.

In the present invention, an "amino acid residue" is an amino acid from which, formally, a hydrogen atom has been removed from an amino group and/or from which, formally, a hydroxy group has been removed from a carboxy group and/or from which, formally, a hydrogen atom has been removed from a sulfhydryl group and/or with a protected amino group and/or with a protected carboxyl group and/or with a protected sulfhydryl group. Imprecisely, an amino acid residue can be described as an amino acid. The source of the amino acid in the present invention is not particularly limited unless otherwise specified, which can be either natural or unnatural, or a mixture of both. The configuration of the amino acid in the present invention is not particularly limited unless otherwise specified, which can be either L-type or D-type, or a mixture of both. In one embodiment of the invention, the amino acid is a hydrophobic amino acid, selected from the group consisting of tryptophan (Trp), phenylalanine (Phe), valine (Val), isoleucine (Ile), leucine (Leu), and tyrosine (Tyr). In another embodiment of the invention, the amino acid is a hydrophilic amino acid, selected from the group consisting of glutamic acid (Glu), aspartic acid (Asp), histidine (His), glutamine (Gln), asparagine (Asn), serine (Ser), threonine (Thr), proline (Pro), glycine (Gly), lysine (Lys), and arginine (Arg), preferably selected from glycine and lysine, and more preferably lysine.

In the present invention, the term "functional group source" refers to those having reaction activity or potential reaction activity, photosensitivity or potential photosensitivity, targeting properties or potential targeting properties. Said "potential" means that the functional group source can be converted into a reactive group through chemical processes including but not limited to functional modification (e.g., grafting, substitution, etc.), deprotection, salt complexation and decomplexation, ionization, protonation, deprotonation, leaving group transformation, etc., and can exhibit luminescence or targeting properties through external stimuli such as light, heat, enzymes, specific binding molecules, microenvironment in vivo, etc. Said luminescence is not particularly limited, including but not limited to visible light, fluorescence, phosphorescence, etc.

In the present invention, a variant form refers to a structural form that can be transformed into the target reactive group after any process of chemical change selected from the group consisting of oxidation, reduction, hydration, dehydration, electronic rearrangement, structural rearrangement, salt complexation and decomplexation, ionization, protonation, deprotonation, substitution, deprotection, leaving group transformation, etc.

In the present invention, a "variant form of a reactive group" refers to a form which still has reactivity after the reactive group undergoes at least one process of chemical change selected from oxidation, reduction, hydration, dehydration, electronic rearrangement, structural rearrangement, salt complexation and decomplexation, ionization, protonation, deprotonation, substitution, deprotection, leaving group transformation, etc., or a non-reactive form that has been protected.

In the present invention, the "micro-modification" refers to a chemical modification process that can be completed through simple chemical reaction processes. Said simple chemical reaction processes mainly refer to deprotection, salt complexation and decomplexation, ionization, protonation, deprotonation, leaving group transformation, etc.

The "micro variant form" corresponds to the "micro-modification", referring to the structural form that can be transformed into the target reactive group after simple chemical reaction processes such as deprotection, salt complexation and decomplexation, ionization, protonation, deprotonation, leaving group transformation, etc. Said leaving group transformation includes the transformation from an ester form to an acyl chloride form.

The description "any appropriate" in expressions such as "any appropriate linking group", "any appropriate reactive group", etc., indicates that the structure accords with the fundamental principle of chemical structure and can enable the preparation method in the present invention to be implemented successfully. A chemical structure described in this way can be considered to have a clear and determined scope.

When at least two structural types are listed, the "any combination" of the listed structural types refers to a combination of any two or more afore-listed types of relevant structures; the number of a structural unit is not limited, which means, the number of any structural unit can be 0, 1, or greater than 1; and, when the number of structural units of the same type is greater than 1, the structural units could be from the same or different chemical structures, wherein the total number of said structural units is at least 2. For example, an arbitrary combination of alkylene groups, divalent cycloalkyl groups, divalent cycloalkenyl groups, divalent cycloalkynyl groups, divalent cyclodienyl groups, arylene groups, carbon-carbon double bonds, carbon-carbon triple bonds, conjugated carbon-carbon double bonds, divalent aliphatic heterocyclic linking groups, divalent aromatic heterocyclic linking groups, and carbon chain linking groups with heteroatom-containing pendant groups, can be, e.g., -Ph-CH₂-Ph-(arylene-alkylene-arylene), -CH₂-Ph-CH₂CH₂-(alkylene-arylene-alkylene; wherein, the alkylene group has a quantity of 2, with different chemical structures), or a structure obtained from the phenyl ring of any aforementioned example being replaced with the ring of a hexane, diazepine, or 1-(2-pyridinyl)hexahydro-1H-1,4-diazepine. As another example, cycloalkenyl hydrocarbon group = cycloalkenyl group + hydrocarbylene group = hydrocarbon group substituted by a cycloalkenyl group, and cyclodienyl hydrocarbon group = hydrocarbon group substituted by a cyclodienyl group. In the present invention, the alkylene group (i.e., divalent alkyl group) includes the open-chain alkylene group and the divalent cycloalkyl group; the open-chain alkylene group refers to the divalent alkyl group containing no ring structures, and the divalent cycloalkyl group refers to the ring-containing divalent alkyl group.

In the present invention, the "adjuvant" or "adjuvant component" is typically a (e.g., pharmacological or immunological) agent or composition that may modify (e.g., enhance) the efficacy of other agents (e.g., drugs or vaccines). Conventionally, the term refers in the context of the invention to a compound or composition that serves as a carrier or auxiliary substance for immunogens and/or other pharmaceutically active compounds. It is to be interpreted in a broad sense and refers to a broad spectrum of substances that are able to increase the immunogenicity of antigens incorporated into or co-administered with an adjuvant in question. In the present invention, an adjuvant will preferably enhance the specific immunogenic effect of the active agents of the present invention. Typically, "adjuvant" or "adjuvant component" has the same meaning and can be used mutually. Adjuvants may be divided, e.g., into immuno potentiators, antigenic delivery systems or even combinations thereof.

In the present invention, the "N/P ratio" refers to molar ratio of nitrogen atoms in cationic lipids to phosphoric acid groups in nucleic acids.

In the present invention, the "nucleic acid" refers to DNA, RNA or modified form thereof, including purines and pyrimidine bases in DNA (adenine "A", cytosine "C", guanine "G", thymine "T"), or purines and pyrimidine bases in RNA (adenine "A", cytosine "C", guanine "G", uracil "U").

In the present invention, the term "RNA" refers to a ribonucleic acid that may be naturally or non-naturally occurring. For example, an RNA may include modified and/or non-naturally occurring components such as one or more nucleobases, nucleosides, nucleotides, or linkers. An RNA may include a cap structure, a chain terminating nucleoside, a stem loop, a polyA sequence, and/or a polyadenylation signal. An RNA may have a nucleotide sequence encoding a polypeptide of interest. For example, an RNA may be a messenger RNA (mRNA). The translation of an mRNA encoding a particular polypeptide, for example, the in vivo translation of an mRNA inside a mammalian cell, may produce the encoded polypeptide. An RNA may be selected from the non-limiting group consisting of small interfering RNA (siRNA), asymmetrical interfering RNA (aiRNA), microRNA (miRNA), Dicer-substrate RNA (dsRNA), small hairpin RNA (shRNA), mRNA, single guide RNA (sgRNA), cas9 mRNA and mixtures thereof.

In the present invention, an antisense oligonucleotide or small interfering RNA (siRNA) can inhibit the expression of the target gene and the target protein in vitro or in vivo.

In the present invention, the Fluc mRNA can express the luciferase protein which emits bioluminescence in the presence of fluorescein substrates, so Fluc is commonly used in mammalian cell culture to measure gene expression and cell activity.

In the present invention, the term "inhibiting the expression of a target gene" refers to the ability of nucleic acids to silence, reduce or inhibit the expression of a target gene. To examine the extent of gene silencing, a test sample (e.g., a sample of cells in culture expressing the target gene) is contacted with nucleic acids that inhibit the expression of the target gene. The expression of the target gene in the test sample or test animal is compared to the expression of the target gene in a control sample (e.g., a sample of cells in culture expressing the target gene) which is not contacted with or administered the nucleic acids. The expression of the target gene in the control sample may be assigned a value of 100%. In particular embodiments, inhibition of expression of a target gene is achieved when the level of target gene expression in the test sample or the test mammal relative to the level of target gene expression in the control sample or the control mammal is about 95%, 90%, 85%, 80%, 75%, 70%, 65%, 60%, 55%, 50%, 45%, 40%, 35%, 30%, 25%, 20%, 15%, 10%, 5%, or 0%.

In the present invention, assays for determining the level of target gene expression include but are not limited to dot blots, northern blots, in situ hybridization, ELISA, immunoprecipitation, enzyme function, and phenotypic assays.

In the present invention, the term "transfection" refers to the introduction of a species (e.g., RNA) into a cell. Transfection may occur, for example, in vitro, ex vivo, or in vivo.

In the present invention, the term "antigen" typically refers to a substance that can be recognized by the immune system, preferably recognized by the adaptive immune system, and triggers an antigen-specific immune response, for example, forming antibodies and/or antigen-specific T cells as a part of the adaptive immune response. Typically, the antigen may be or may contain a peptide or a protein that can be presented to T cells by MHC. In the present invention, the antigen may be a translation product of the provided nucleic acid molecule (preferably mRNA as defined herein). In this context, fragments, variants, and derivatives of peptides and proteins containing at least one epitope are also recognized as antigens.

In the present invention, the term "delivery" refers to providing an entity to the target, for example, delivering drugs and/or therapeutic agents and/or prophylactic agents to subjects, said subjects being tissues and/or cells of human and/or other animals.

In the present invention, the "pharmaceutically acceptable carrier" refers to a diluent, adjuvant, excipient, or vehicle administered together with the therapeutic agent, which is, in a reasonable medical judgment range, suitable for the contact of the human and/or other animal without excessive toxicity, stimulation, hypersensitive response, other problems or complications corresponding to a reasonable benefit/risk ratio. Pharmaceutically acceptable carriers that can be used in the pharmaceutical composition in the present invention include but are not limited to sterile liquids, e.g., water and oil, including the oil from petroleum, animal, plant, or synthetic origin, e.g., peanut oil, soybean oil, mineral oil, sesame oil, etc. When said pharmaceutical composition is administered intravenously, water is an exemplary carrier. Physiological saline, glucose and aqueous glycerol solution can also be used as liquid carriers, and are especially used for injection. Suitable pharmaceutical excipients include starch, glucose, lactose, sucrose, gelatin, maltose, chalk, silica gel, sodium stearate, glyceryl monostearate, talc, sodium chloride, defatted milk powder, glycerol, propylene glycol, water, ethanol, etc. Said composition can also contain a small amount of humectant, emulsifier or pH buffer as needed. Oral preparations can contain standard carriers, e.g., pharmaceutical grade mannitol, lactose, starch, magnesium stearate, sodium saccharin, cellulose, magnesium carbonate, etc. Specifically, excipients include but are not limited to anti-adherents, antioxidants, binders, coatings, compression aids, disintegrants, dyes (pigments), emollients, emulsifiers, fillers (diluents), film formers or coatings, flavors, fragrances, glidants (flow enhancers), lubricants, preservatives, printing inks, sorbents, suspending or dispersing agents, sweeteners, and waters for hydration. More specifically, excipients include but are not limited to butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, phenyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E (α-tocopherol), vitamin C, and xylitol.

In the present invention, pharmaceutical compositions can act systemically and/or locally. For this purpose, they can be administered by appropriate routes such as injection (e.g., intravenous, intraarterial, subcutaneous, intraperitoneal, or intramuscular injection, including instillation) or transdermal administration, or administered by oral, buccal, transnasal, transmucosal or topical routes or in the form of ophthalmic preparation or by inhalation. Regarding these administration routes, the pharmaceutical compositions of the present invention can be administered in a suitable dosage form. The dosage forms include but are not limited to tablets, capsules, lozenges, hard sugar agents, powders, sprays, creams, ointments, suppositories, gels, pastes, emulsions, ointments, aqueous suspensions, injectable solutions, elixirs, and syrups.

In the present invention, vaccines are preventive or therapeutic materials that provide at least one antigen or antigenic function. An antigen or antigenic function can stimulate the body's adaptive immune system to provide an adaptive immune response.

In the present invention, treatment refers to the management and care of patients to resist diseases, obstacles or symptoms, which is intended to delay the development of diseases, obstacles or symptoms, reduce or alleviate symptoms and complications, and/or cure or eliminate diseases, obstacles or symptoms. The patients to be treated are preferably mammals, especially humans.

1.1. A PEGylated lipid having the structure represented by the general formula (2): 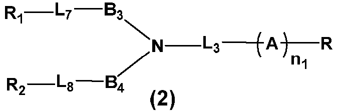
or a pharmaceutically acceptable salt, tautomer, or stereoisomer thereof,
wherein, one of L₇ and L₈ is -OC(=O)O-, -C(=O)-, -O-, -O(CR_{c}R_{c})ₛO-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, or -NR_{c}C(=O)S, and the other is a linking bond, -OC(=O), -C(=O)O-, -OC(=O)O-, -C(=O)-, -O-, -O(CR_{c}R_{c})ₛO-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, or -NR_{c}C(=O)S-, wherein, R_{c} is independently, at each occurrence, a hydrogen atom or an alkyl group, s is 2, 3, or 4.
L₃ is a linking bond, -L₄-, -Z-L₄-, -L₄-Z-, -Z-L₄-Z-, -L₄-Z-L₅-, -Z-L₄-Z-L₅-, -L₄-Z-L₅-Z-, -Z-L₄-Z-L₅-Z-, or -L₄-Z-L₄-Z-L₅-Z-; said L₄ and L₅ are carbon-chain linking groups, which are each independently represented by -(CRₐR_{b})ₜ-(CRₐR_{b})ₒ-(CRₐR_{b})ₚ-, wherein, t, p, and q are each independently an integer from 0 to 12, and t, o, and p are not simultaneously 0; Rₐ and R_{b} are, at each occurrence, each independently a hydrogen atom or a C₁₋₁₂ alkyl group; said Z is, at each occurrence, independently selected from the group consisting of -C(=O)-, -OC(=O)-, -C(=O)O-, -OC(=O)O-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NR_{c}C(=O)S-, and 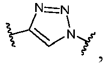 wherein, R_{c} is, at each occurrence, independently a hydrogen atom or a C₁₋₁₂ alkyl group;
B₃ and B₄ are each independently a linking bond or a C₁₋₃₀ alkylene group;
R₁ and R₂ are each independently a C₁₋₃₀ aliphatic hydrocarbon group;
R contains any atom or group selected from the group consisting of a hydrogen atom, an alkyl group, an alkoxy group, an alcoholic hydroxyl group, a protected alcoholic hydroxyl group, a thiol group, a protected thiol group, a carboxyl group, a protected carboxyl group, an amino group, a protected amino group, an aldehyde group, a protected aldehyde group, an ester group, a carbonate group, a urethane group, a succinimidyl group, a maleimide group, a protected maleimide group, a dimethylamino group, an alkenyl group, an alkenoate group, an azido group, an alkynyl group, a folate group, a rhodamine group, a biotinyl group, a monosaccharide group, and a polysaccharide group;
A is -(CRₐR_{b})ₛO- or -O(CRₐR_{b})ₛ-, wherein s is 2, 3, or 4;
n₁ is an integer approximately from 20 to 250;
said alkyl group, alkylene group, alkoxy group, and aliphatic hydrocarbon group are each independently substituted or unsubstituted.

### 1.1.1. Divalent linking group L₃, L₄, L₅, L₇, L₈, Z, Z₁, Z₂

In the present invention, the structures of L₃, L₄, L₅, L₇, L₈, Z, Z₁, and Z₂ are not particularly limited, each independently including but not limited to linear structures, branched structures or ring-containing structures.

In the present invention, the number of non-hydrogen atoms of L₃, L₄, L₅, L₇, L₈, Z, Z₁, and Z₂ are not particularly limited, each preferably ranging independently from 1 to 50, more preferably from 1 to 20, and more preferably from 1 to 10. Said non-hydrogen atom is a carbon atom or a heteroatom. Said heteroatom includes but is not limited to O, S, N, P, Si, B, etc. When the number of non-hydrogen atoms is 1, the non-hydrogen atom can be a carbon atom or a heteroatom. When the number of non-hydrogen atoms is greater than 1, the species of non-hydrogen atoms are not particularly limited, which can be 1, 2 or more than 2 species; when the number of non-hydrogen atoms is greater than 1, they can be any combination of carbon atoms and carbon atoms, carbon atoms and heteroatoms, or heteroatoms and heteroatoms.

In the present invention, two identical or different reactive groups may form a divalent linking group after reaction. The reaction conditions are related to the types of the resulting divalent linking groups, and the prior art can be introduced herein. For example, amino groups can react with active esters, active formates, sulfonate esters, aldehydes, α,β-unsaturated bonds, carboxylic groups, epoxides, isocyanates, and isothiocyanates, respectively, to obtain divalent linking groups such as amide groups, urethane groups, amino groups, imide groups (which can be further reduced to secondary amino groups), amino groups, amide groups, amino alcohols, urea bonds, thiourea bonds, etc.; mercapto groups can react with active esters, active formates, sulfonate groups, mercapto groups, maleimide groups, aldehyde groups, α,β-unsaturated bonds, carboxyl groups, iodoacetamide groups, and anhydride groups, respectively, to obtain divalent linking groups such as thioester groups, thiocarbonate groups, thioether groups, disulfide groups, thioether groups, thiohemiacetal linking groups, thioether groups, thioester groups, thioether groups, imide groups, etc.; unsaturated bonds can react with mercapto groups to obtain thioether groups; carboxyl groups or acyl halides can respectively react with mercapto groups and amino groups to obtain groups such as thioester bonds, amide bonds, etc.; hydroxyl groups can react with carboxyl groups, isocyanates, epoxides, and chlorocarbonyloxy groups to obtain divalent linking groups such as ester groups, carbamate groups, ether bonds, carbonate groups, etc.; carbonyl groups or aldehyde groups can react with amino groups, hydrazines and hydrazides to obtain divalent linking groups such as imine groups, hydrazone groups, acylhydrazone groups, etc.; reactive groups such as azido groups, alkynyl groups, alkenyl groups, mercapto groups, azido groups, dienyl groups, maleimide groups, 1,2,4-triazoline-3,5-dione groups, dithioester groups, hydroxylamine groups, hydrazide groups, acrylate groups, allyloxy groups, isocyanate groups, tetrazole groups and the like can undergo click reactions to form various divalent linking groups including but not limited to the structures of triazoles, isoxazoles, thioether bonds, and the like.

The stability of divalent linking groups L₃, L₄, L₅, L₇, L₈, Z, Z₁, and Z₂ are not particularly limited, wherein any divalent linking group or a divalent linking group consisting of any aforementioned divalent linking group and adjacent heterosubstituted groups thereof is independently a stable linking group STAG or a degradable linking group DEGG.

### 1.1.1.1. Divalent linking group L₇, L₈

In the present invention, wherein, one of L₇ and L₈ is -OC(=O)O-, -C(=O)-, -O-, -O(CR_{c}R_{c})ₛO-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, or -NR_{c}C(=O)S-, and the other is a linking bond, -OC(=O), -C(=O)O-, -OC(=O)O-, -C(=O)-, -O-, -O(CR_{c}R_{c})ₛO-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, or -NR_{c}C(=O)S-, wherein, R_{c} is independently, at each occurrence, a hydrogen atom or an alkyl group, s is 2, 3, or 4.

In one specific embodiment of the present invention, it is preferred that one of L₇ and L₈ is -OC(=O)O-, -C(=O)-, -O-, -O(CH₂)ₛO-, -S-, -C(=O)S-, -SC(=O)-, -NHC(=O)-, -C(=O)NH-, -NHC(=O)NH-, -OC(=O)NH-, -NHC(=O)O-, -SC(=O)NH-, or -NHC(=O)S-, and the other is a linking bond, -OC(=O)-, -C(=O)O-, -OC(=O)O-, -C(=O)-, -O-, -O(CH₂)ₛO-, -S-, -C(=O)S-, -SC(=O)-, -NHC(=O)-, -C(=O)NH-, -NHC(=O)NH-, -OC(=O)NH-, -NHC(=O)O-, -SC(=O)NH-, or -NHC(=O)S-.

In one specific embodiment of the present invention, it is more preferred that one of the aforementioned L₇ and L₈ is -OC(=O)O-, -C(=O)-, -O-, -O(CH₂)ₛO-, -S-, -C(=O)S-, -SC(=O)-, -NHC(=O)-, -C(=O)NH-, -NHC(=O)NH-, -OC(=O)NH-, -NHC(=O)O-, -SC(=O)NH-, or -NHC(=O)S-, and the other is a linking bond, -O-, -OC(=O)-, or -C(=O)O-.

In one specific embodiment of the present invention, said L₇ and L₈ are preferably selected from the following situations:
(1) one of L₇ and L₈ is -C(=O)-, and the other is a linking bond;
(2) one of L₇ and L₈ is -C(=O)-, and the other is -OC(=O)- or -C(=O)O-;
(3) L₇ and L₈ are both -O-.

### 1.1.1.2. Divalent linking group L₃

In the present invention, L₃ is a linking bond, -L₄-, -Z-L₄-, -L₄-Z-, -Z-L₄-Z-, -L₄-Z-L₅-, -Z-L₄-Z-L₅-, -L₄-Z-L₅-Z-, -Z-L₄-Z-L₅-Z-, or -L₄-Z-L₄-Z-L₅-Z-; said L₄ and L₅ are carbon-chain linking groups, which are each independently represented by -(CRₐR_{b})ₜ-(CRₐR_{b})ₒ-(CRₐR_{b})ₚ-, wherein, t, p, and q are each independently an integer from 0 to 12, and t, o, and p are not simultaneously 0; Rₐ and R_{b} are, at each occurrence, each independently a hydrogen atom or a C₁₋₁₂ alkyl group; said Z is, at each occurrence, independently selected from the group consisting of -C(=O)-, -OC(=O)-, -C(=O)O-, -OC(=O)O-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NR_{c}C(=O)S-, and 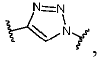 wherein, R_{c} is, at each occurrence, independently a hydrogen atom or a C₁₋₁₂ alkyl group;

In one specific embodiment of the present invention, R_{c} is preferably a hydrogen atom.

In one specific embodiment of the present invention, L₃ contains degradable groups, and said degradable group refers to the group which can degrade under any condition selected from the group consisting of light, heat, low temperature, enzymatic condition, oxidation-reduction condition, acidic condition, basic condition, physiological condition, and simulated physiological environment in vitro; the L₄ and L₅ contained in said L₃ are preferably each independently -(CH₂)ₜ-; L₃ is -(CH₂)ₜ-, -(CH₂)ₜZ-, -Z(CH₂)ₜ-, -(CH₂)ₜZ(CH₂)ₜ-, -Z(CH₂)ₜZ-, -(CH₂)ₜZ(CH₂)ₜZ-, -Z(CH₂)ₜZ(CH₂)ₜ-, or -Z(CH₂)ₜZ(CH₂)ₜZ-; wherein, t is an integer from 1 to 12, and Z is, at each occurrence, independently -C(=O)-, -OC(=O)-, -C(=O)O-, -OC(=O)O-, -O-, -S-, -C(=O)S-, -SC(=O)-, -NHC(=O)-, -C(=O)NH-, -NHC(=O)NH-, -OC(=O)NH-, -NHC(=O)O-, -SC(=O)NH-, or -NHC(=O)S-; L₃ is preferably -(CH₂)ₜ-, -(CH₂)ₜO-, -(CH₂)ₜC(=O)-, -(CH₂)ₜC(=O)O-, -(CH₂)ₜOC(=O)-, -(CH₂)ₜC(=O)NH-, -(CH₂)ₜNHC(=O)-, -(CH₂)ₜOC(=O)O-, -(CH₂)ₜNHC(=O)O-, -(CH₂)ₜOC(=O)NH-, -(CH₂)ₜNHC(=O)NH-, -O(CH₂)ₜ-, -C(=O)(CH₂)ₜ-, -C(=O)O(CH₂)ₜ-, -OC(=O)(CH₂)ₜ-, -C(=O)NH(CH₂)ₜ-, -NHC(=O)(CH₂)ₜ-, -OC(=O)O(CH₂)ₜ-, -NHC(=O)O(CH₂)ₜ-, -OC(=O)NH(CH₂)ₜ-, -NHC(=O)NH(CH₂)ₜ-, -(CH₂)ₜO(CH₂)ₜ-, -(CH₂)ₜC(=O)(CH₂)ₜ-, -(CH₂)ₜC(=O)O(CH₂)ₜ-, -(CH₂)ₜOC(=O)(CH₂)ₜ-, -(CH₂)ₜC(=O)NH(CH₂)ₜ-, -(CH₂)ₜNHC(=O)(CH₂)ₜ-, -(CH₂)ₜOC(=O)O(CH₂)ₜ-, -(CH₂)ₜNHC(=O)O(CH₂)ₜ-, -(CH₂)ₜOC(=O)NH(CH₂)ₜ-, -(CH₂)ₜNHC(=O)NH(CH₂)ₜ-, -O(CH₂)ₜO-, -C(=O)(CH₂)ₜC(=O)-, -C(=O)O(CH₂)ₜC(=O)O-, -OC(=O)(CH₂)ₜOC(=O)-, -C(=O)O(CH₂)ₜOC(=O)-, -OC(=O)(CH₂)ₜC(=O)O-, -OC(=O)O(CH₂)ₜOC(=O)O-, -C(=O)NH(CH₂)ₜC(=O)NH-, -NHC(=O)(CH₂)ₜNHC(=O)-, -NHC(=O)(CH₂)ₜC(=O)NH-, -C(=O)NH(CH₂)ₜNHC(=O)-, -NHC(=O)O(CH₂)ₜNHC(=O)O-, -OC(=O)NH(CH₂)ₜOC(=O)NH-, -NHC(=O)O(CH₂)ₜOC(=O)NH-, -OC(=O)NH(CH₂)ₜNHC(=O)O-, -NHC(=O)NH(CH₂)ₜNHC(=O)NH-, -C(=O)(CH₂)ₜO-, -C(=O)(CH₂)ₜC(=O)O-, -C(=O)(CH₂)ₜOC(=O)-, -C(=O)(CH₂)ₜOC(=O)O-, -C(=O)(CH₂)ₜNHC(=O)O-, -C(=O)(CH₂)ₜOC(=O)NH-, -C(=O)(CH₂)ₜNHC(=O)NH-, -C(=O)(CH₂)ₜC(=O)O(CH₂)ₜ-, -C(=O)(CH₂)ₜOC(=O)(CH₂)ₜ-, -C(=O)(CH₂)ₜOC(=O)O(CH₂)ₜ-, -C(=O)(CH₂)ₜNHC(=O)O(CH₂)ₜ-, -C(=O)(CH₂)ₜOC(=O)NH(CH₂)ₜ-, -C(=O)(CH₂)ₜNHC(=O)NH(CH₂)ₜ-, or -C(=O)(CH₂)ₜC(=O)(CH₂)ₜNHC(=O)O-, wherein t is an integer from 2 to 12; most preferably, L₃ is -C(=O)O-, -(CH₂)ₜO-, -(CH₂)ₜC(=O)O-, -(CH₂)ₜOC(=O)-, -(CH₂)ₜO-, -C(=O)(CH₂)ₜO-, -C(=O)(CH₂)ₜOC(=O)-, -C(=O)(CH₂)ₜC(=O)O-, -C(=O)(CH₂)ₜC(=O)NH-, -C(=O)(CH₂)ₜOC(=O)NH-, -C(=O)(CH₂)ₜNHC(=O)O-, -C(=O)(CH₂)ₜOC(=O)NH(CH₂)ₜ-, or -C(=O)(CH₂)ₜC(=O)(CH₂)ₜNHC(=O)O-.

### 1.1.2 Description of stable and degradable groups

In the present invention, a stable linking group STAG or a degradable linking group DEGG can exist within any of the divalent linking groups L₃, L₄, L₅, L₇, L₈, Z, Z₁, and Z₂ mentioned above, or within a divalent linking group consisting of any aforementioned divalent linking group and adjacent heterosubstituted groups thereof.

### 1.1.2.1. Stable divalent linking groups STAG in the present invention

The condition of being stable for a stable divalent linking group STAG is not particularly limited, including but not limited to any condition selected from light, heat, low temperature, enzymatic condition, oxidation-reduction, acidic condition, basic condition, physiological condition, simulated physiological environment in vitro, etc., preferably light, heat, enzymatic condition, oxidation-reduction, acidic condition, basic condition, etc.

The type of stable divalent linking group STAG is not particularly limited, including but not limited to an alkylene group, a divalent heteroalkyl group, a carbon-carbon double bond, a carbon-carbon triple bond, a divalent dienyl group, a divalent cycloalkyl group, a divalent cycloalkenyl group, a divalent cycloalkenyl hydrocarbon group, a divalent cycloalkynyl hydrocarbon group, an aromatic ring, an aliphatic heterocyclic group, a heterophenylene group, an aromatic-fused heterocyclic group, a hetero-fused heterocyclic group, a substituted alkylene group, a substituted heteroalkyl group, a substituted divalent heteroalkyl group, a substituted double bond, a substituted triple bond, a substituted dienyl group, a substituted divalent cycloalkyl group, a substituted divalent cycloalkenyl group, a substituted divalent cycloalkenyl hydrocarbon group, a substituted divalent cycloalkynyl hydrocarbon group, a substituted aromatic ring, a substituted aliphatic heterocyclic group, a substituted heterophenylene group, a substituted aromatic-fused heterocyclic group, a substituted hetero-fused heterocyclic group, an ether bond, a thioether bond, a urea bond, a thiourea bond, a carbamate group, a thiocarbamate group, -P(=O)-, a divalent silyl group without active hydrogen atoms, a divalent boron-containing linking group, a secondary amino group, a tertiary amino group, a carbonyl group, a thiocarbonyl group, an amide group, a thioamide group, a sulfonamide group, an enamino group, a triazole group, a 4,5-dihydroisoxazole group, a skeleton of an amino acid or its derivative, and a stable divalent linking group composed of any two or more groups thereof.

Specifically, a stable divalent linking group STAG includes but is not limited to the structures described or listed in the references CN104530413A, CN104530415A and CN104530417A. Taking CN104530417A as an example, the corresponding paragraphs are from [0627] to [0704]. The way in which a STAG is composed of two or more stable divalent linking groups is not particularly limited, which includes but is not limited to those described in the paragraph [0704] of CN104530417A.

### 1.1.2.2. Degradable divalent linking group DEGG in the present invention

The condition of degradable divalent linking groups DEGG to be degradable is not particularly limited, including but not limited to light, heat, low temperature, enzymatic condition, oxidation-reduction, acidic condition, basic condition, physiological condition, simulated physiological environment in vitro, etc., preferably light, heat, enzymatic condition, oxidation-reduction, acidic condition, basic condition, etc.

The divalent linking group formed by the combination of any degradable divalent linking group DEGG and any stable divalent linking group STAG is still a degradable linking group. As for an aromatic ring-containing degradable divalent linking group, it can also be formed by the combination of aromatic rings and degradable divalent linking groups.

The type of degradable divalent linking group DEGG is not particularly limited, including but not limited to those containing any divalent linking group selected from the group consisting of a disulfide bond, a vinylether bond, an ester group, a thioate group, a thioester group, a dithioester group, a carbonate group, a thiocarbonate group, a dithiocarbonate group, a trithiocarbonate group, a carbamate group, a thiocarbamate group, a dithiocarbamate group, an acetal group, a cycloacetal group, a thioacetal group, an azaacetal group, an azacycloacetal group, an azathiaacetal group, a dithioacetal group, a hemiacetal group, a thiohemiacetal group, an azahemiacetal group, a ketal group, a thioketal group, an azaketal group, an azacycloketal group, an azathiaketal group, an imine bond, a hydrazone bond, an acylhydrazone bond, an oxime bond, a thiooxime ether group, a semicarbazone bond, a thiosemicarbazone bond, a hydrazino group, a hydrazide group, a thiocarbohydrazide group, an azocarbohydrazide group, an azothiocarbohydrazide group, a hydrazino formate group, a hydrazino thioformate group, a carbohydrazide group, a thiocarbohydrazide group, an azo group, an isourea group, an isothiourea group, an allophanate group, a thioallophanate group, a guanidino group, an amidino group, an aminoguanidinyl group, a carbamimidamido group, an imino acid group, a thioimidate group, a sulfonate group, a sulfinate group, a sulfonyl hydrazide group, a sulfonyl ureido group, a maleimide group, an orthoester group, a phosphate group, a phosphirate group, a phosphinate group, a phosphonate group, a phosphosilicate group, a silicate group, an amide group, a thioamide group, a sulfonamide group, a polyamide group, a phosphamide group, a phosphoramidite group, a pyrophosphamide group, a cyclophosphamide group, an ifosfamide group, a thiophosphamide group, an aconityl group, a peptide fragment, a skeleton of a nucleotide or its derivative, and a skeleton of a deoxynucleotide or its derivative, and those containing the combination of any two or more said divalent linking groups.

Herein, said carbamate group, thiocarbamate group, amide group, phosphamide group, and the like, can be regarded as either a stable linking group or a degradable linking group, depending on the environmental characteristics of its use.

Specifically, a degradable divalent linking group DEGG includes but is not limited to the structures described or listed in the references CN104530413A, CN104530415A and CN104530417A. Taking CN104530417A as an example, the corresponding paragraphs are from [0705] to [0725].

### 1.1.3. Alkylene group B₃ and B₄

In the present invention, B₃ and B₄ are each independently a linking bond or a C₁₋₃₀ alkylene group, more preferably a linking bond or a C₁₋₂₀ alkylene group.

In one specific embodiment of the present invention, B₃ and B₄ are both linking bonds.

In one specific embodiment of the present invention, one of B₃ and B₄ is a linking bond, and the other is a C₁₋₂₀ alkylene group.

In one specific embodiment of the present invention, B₃ and B₄ are each independently a C₁₋₂₀ alkylene group, and specifically, each independently selected from the group consisting of a methylene group, an ethylene group, a propylene group, a butylene group, a pentylene group, a hexylene group, a heptylene group, an octylene group, a nonylene group, a decylene group, an undecylene group, a dodecylene group, a tridecylene group, a tetradecylene group, a pentadecylene group, a hexadecylene group, a heptadecylene group, an octadecylene group, a nonadecylene group, and an eicosylene group.

### 1.1.4. Aliphatic hydrocarbon groups R₁, R₂

In the present invention, R₁ and R₂ are each independently a C₁₋₃₀ aliphatic hydrocarbon group.

In one specific embodiment of the present invention, R₁ and R₂ are preferably each independently a C₁₋₂₀ aliphatic hydrocarbon group.

In one specific embodiment of the present invention, R₁ and R₂ are preferably each independently a linear alkyl group, a branched alkyl group, a linear alkenyl group, a branched alkenyl group, a linear alkynyl group, or a branched alkynyl group, preferably a linear aliphatic hydrocarbon group, more preferably a C₁₋₂₅ linear aliphatic hydrocarbon group, more preferably a C₁₋₂₀ linear aliphatic hydrocarbon group; most preferably selected from the group consisting of a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an eicosyl group, a (Z)-tridec-8-enyl group, a (Z)-tetradec-9-enyl group, a (Z)-pentadec-8-enyl group, a (Z)-hexadec-9-enyl group, a (Z)-heptadec-5-enyl group, a (Z)-heptadec-8-enyl group, a (E)-heptadec-8-enyl group, a (Z)-heptadec-10-enyl group, a (8Z, 11Z)-heptadec-8,11-dienyl group, a (Z)-octodec-6-enyl group, a (Z)-octodec-9-enyl group, a (E)-octodec-9-enyl group, a (*Z*)-octodec-11-enyl group, a (9*Z*, 12*Z*)-octodec-9,12-dienyl group, a (9*Z*, 12*Z*, 15*Z*)-octodec-9,12,15-trienyl group, a (8*Z*, 11*Z*, 14*Z*)-octodec-8,11,14-trienyl group, a (*Z*)-eicos-11-enyl group, a (11*Z,* 14*Z*)-eicos-11,14-dienyl group, a (*Z*)-nonadec-10-enyl group, a (10*Z*, 13*Z*)-nonadec-10,13-dienyl group, a 2,6,10-trimethylundec-1,5,9-trienyl group, a 3,7,11-trimethyldodec-2,6,10-trienyl group, and a 3,7,11,15-tetramethylhexadec-2-enyltridecyl group.

In one specific embodiment of the present invention, it is preferred that the aforementioned R₁ and R₂ are both methyl groups.

In one specific embodiment of the aforementioned PEGylated lipid of the present invention, R₁ and R₂ are each independently a branched alkyl group, a branched alkenyl group, or a branched alkynyl group, and each independently represented as 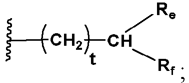 wherein, Rₑ and R_{f} are each independently selected from the group consisting of a C₁₋₁₅ alkyl group, a C₂₋₁₅ alkenyl group, and a C₂₋₁₅ alkynyl group, more preferably the group consisting of a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a vinyl group, a propenyl group, an allyl group, a butenyl group, an enyl butyl group, a pentenyl group, an enyl pentyl group, a hexenyl group, an enyl hexyl group, a heptenyl group, an enyl heptyl group, an octenyl group, an enyl octyl group, a nonenyl group, an enyl nonyl group, a decenyl group, an enyl decyl group, an ethynyl group, a propynyl group, a propargyl group, a butynyl group, an ynyl butyl group, a pentynyl group, an ynyl pentyl group, a hexynyl group, an ynyl hexyl group, a heptynyl group, an ynyl heptyl group, an octynyl group, an ynyl octyl group, a nonynyl group, an ynyl nonyl group, a decynyl group, and an ynyl decyl group, and more preferably the group consisting of a methyl group, an ethyl group, a propyl group, a butyl group, a pentyl group, a hexyl group, a heptyl group, an octyl group, a nonyl group, and a decyl group. R₁ and R₂ are further preferably each independently selected from the group consisting of the following structures: 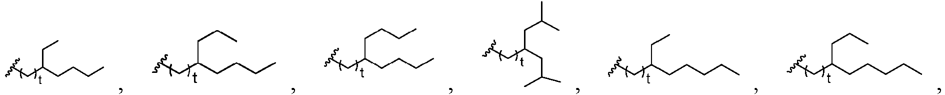 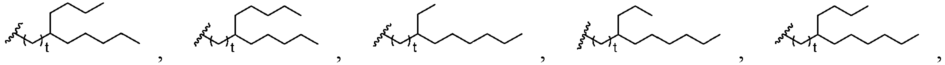 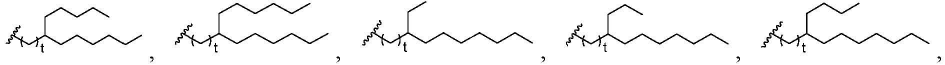 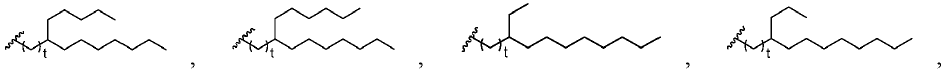 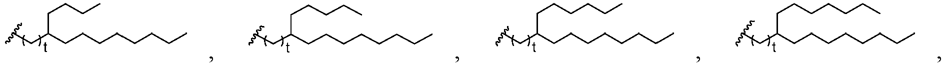 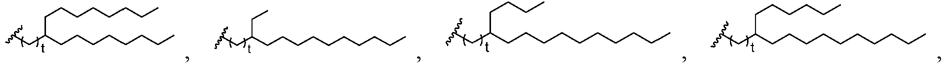 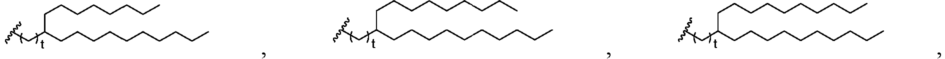 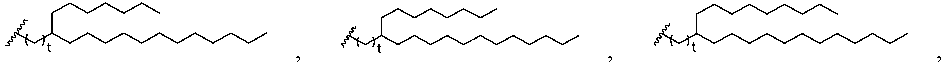 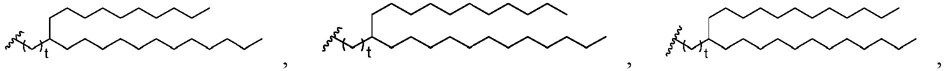 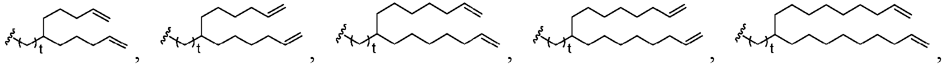 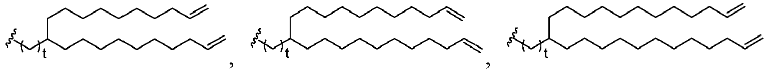 wherein t is an integer from 0 to 12.

### 1.1.5. R

In the present claimed invention, R contains any atom or group selected from the group consisting of a hydrogen atom, an alkyl group, an alkoxy group, an alcoholic hydroxyl group, a protected alcoholic hydroxyl group, a thiol group, a protected thiol group, a carboxyl group, a protected carboxyl group, an amino group, a protected amino group, an aldehyde group, a protected aldehyde group, an ester group, a carbonate group, a urethane group, a succinimidyl group, a maleimide group, a protected maleimide group, a dimethylamino group, an alkenyl group, an alkenoate group, an azido group, an alkynyl group, a folate group, a rhodamine group, a biotinyl group, a monosaccharide group, and a polysaccharide group.

### 1.1.5.1. R_{d}

In the present invention, R_{d} is, at each occurrence, independently a C₁₋₁₂ alkyl group.

In one specific embodiment of the present invention, R_{d} is preferably a C₁₋₈ alkyl group; specifically, R_{d} is preferably a methyl group, an ethyl group, a propyl group, a butyl group, a pentyl group, a hexyl group, a heptyl group, or an octyl group.

### 1.1.5.2. G₁

In the present invention, j is 0 or 1; when j is 0, G₁ is absent; when j is 1, G₁ exists as a (k+1)-valent terminal branching group and leads to k F containing functional groups, wherein k is an integer from 2 to 8, preferably 2, 3 or 4.

In one specific embodiment of the present invention, when G₁ is preferably a branching group with a valence of three or more, G₁ is preferably a trivalent or tetravalent branching group; G₁ is preferably a trivalent branching group, more preferably a trivalent branching group as a residue of glycerol or amino acid.

In the present invention, when a terminal bifunctionalization is performed, G₁ is preferably derived from an alcohol, thiol, primary amine, secondary amine, sulfonate, or halide compound which contains two free or protected hydroxyl groups (e.g., triethanolamine p-toluenesulfonate, glycerol monomercaptoacetate, 3,4-dihydroxy-2'-chloro-acetophenone, and hydroxyl-protected forms thereof), or form an alcohol, thiol, primary amine, secondary amine, sulfonate, or halide compound which contains two free or protected mercapto groups (e.g., dimercaprol and mercapto-protected forms thereof), or from an alcohol, thiol, primary amine, secondary amine, sulfonate, and halide compound which contains two primary amino groups, two secondary amino groups, two protected primary amino groups, or two protected secondary amino groups, etc. Wherein, an exemplary alcohol containing two primary amino groups is 1,3-diamino-2-propanol. The terminal bifunctionalization can be carried out using an aldehyde containing 1 epoxy group, an alcohol containing 1 epoxy group (e.g., 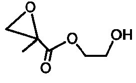 ), a sulfonate containing 1 epoxy group, a halide containing 1 epoxy group, or a compound containing one epoxy group and 1 other type of reactive group. The terminal bifunctionalization can also be achieved by the Michael addition reaction of a primary amine and 2 molecules of acrylic acid ester. It is also possible to reduce the disulfide bond after capping with lipoic acid and then proceed to ring-opening, therefore obtaining two mercapto groups at the ends.

In the present invention, when a terminal trifunctionalization is performed, G₁ is preferably derived from a tetrafunctionalized small molecule (htetraSM) which contains three hydroxyl groups and one different kind of reactive group, including but not limited to N-tris(hydroxymethyl)methyl-2-aminoethanesulfonic acid, 3-[tris(hydroxymethyl)methylamino]-1-propanesulfonic acid, methyl 6-O-tosyl-α-D-glucopyranoside, 2-(bromomethyl)-2-(hydroxymethyl)-1,3-propanediol, tris(hydroxymethyl)aminomethane, 2-amino-1,3,4-octadecanetriol, 3-aminopropylsilanetriol, 4-(2-amino-1-hydroxylethyl)-1,2-benzenediol, 4-[1-hydroxy-2-(isopropylamino)ethyl]-1,2-benzenediol, 3,4-dihydroxy-α-(methylaminomethyl)benzyl alcohol, 2,5-anhydro-1-azido-1-deoxy-D-glucitol, 2,3,4-trihydroxybutanal (L-erythrose, D-erythrose, L-(+)-threose and D-(+)-threose), 2,3,4-trihydroxybenzaldehyde, 3,4,5-trihydroxybenzaldehyde, *N-*[tris(hydroxymethyl)methyl]glycine, 2,3,4-trihydroxybutyric acid (including but not limited to erythorbic acid and threonic acid), 2,4,6-trihydroxybenzoic acid, shikimic acid, 3,4,5-trihydroxybenzoic acid, 2,3,4-trihydroxybenzoic acid, arjunolic acid, 1,4,7-tris(tert-butoxycarbonyl)-1,4,7,10-tetraazacyclododecane, tri-(t-butoxycarbonyl)spermine, 1,4,7-tris(tert-butoxycarbonyl)-1,4,7,10-tetraazacyclododecane, etc., and any hydroxyl-protected form thereof. Also included herein is a group consisting of citric acid, laricic acid, *N*-(2-hydroxyethyl)ethylenediamine-triacetic acid, pentaerythritol triacrylate, aminomethanetrispropionic acid, tri(tert-butyl) aminomethanetrispropionate, etc. Also included herein is the tetravalent silicon-atom branching center obtained via the terminal branching reaction based on alkenyl, trichlorosilane and allylmagnesium chloride, referring to the literature "Macromolecules, Vol. 33, No. 12, 2000". Also included herein are trifunctionalized small molecules such as 1,4,7-tris(t-butoxycarbonylmethyl)-1,4,7,10-azacyclotetradecane (NOTA), which require an excess amount in the reaction.

### 1.1.5.3. F containing functional groups

In one specific embodiment of the present invention, F is preferably -(Z₂)_{q}-(Z₁)_{q1}-R₀₁, wherein, q and q1 are each independently 0 or 1; Z₁ and Z₂ are each independently a divalent linking group; R₀₁ is a functional group capable of interreacting with bio-related substances.

In one specific embodiment of the present invention, Z₁ and Z₂ are more preferably each independently selected from the group consisting of -L₄-, -L₄-Z-, -Z-L₄-, -Z-L₄-Z-, -L₄-Z-L₅-, -Z-L₄-Z-L₅-, and -L₄-Z-L₅-Z-, wherein, t is an integer from 1 to 12.

In a more specific embodiment of the present invention, R₀₁ is preferably selected from the group consisting of a functional group, a variant form of a functional group, a therapeutic targeting functional group, and a fluorescent functional group; wherein, said variant form includes precursors of functional groups, active forms with functional groups as precursors, substituted active forms, protected forms, and deprotected forms; wherein, said precursors of functional groups refer to structures which can be transformed into the functional group through at least one process selected from the group consisting of oxidation, reduction, hydration, dehydration, electronic rearrangement, structural rearrangement, salt complexation and decomplexation, ionization, protonation, and deprotonation; wherein, the variant forms of functional groups refer to the reactive forms of functional groups after at least one chemical change process selected from the group consisting of oxidation, reduction, hydration, dehydration, electronic rearrangement, structural rearrangement, salt complexation and decomplexation, ionization, protonation, deprotonation, substitution, and deprotection, or refer to the non-reactive forms after protection; said R₀₁ is more preferably a functional group selected from the group consisting of the functional groups in the following Classes A to H and variant forms thereof, or from the group consisting of the functional groups in the following Classes I to J:
Class A: active ester groups and analogs thereof; wherein, an active ester group includes a succinimidyl active ester group, a p-nitrophenyl active ester group, an o-nitrophenyl active ester group, a benzotriazole active ester group, a 1,3,5-trichlorophenyl active ester group, a 1,3,5-trifluorophenyl active ester group, a pentafluorophenyl active ester group, and an iminazole active ester group; wherein, analogs of active ester groups include a 2-thione-3-thiazolidine-formate group, a 2-thioxo-thiazolidine-3-carboxylate group, a 2-thione-pyrrolidine-*N*-carboxylate group, a 2-thione-pyrrolidine-*N*-formate group, a 2-thione-benzothiazole-*N*-formate group, and a 1-oxo-3-thioxoisoindoline-*N*-formate group;
Class B: a sulfonate group, a sulfinate group, a sulfonyl group, a sulfoxide group, a 1,3-disulfonyl-2-propylcarbonylphenyl group, and a (2-sulfonylmethyl)acryl group;
Class C: a hydroxylamine group, a mercapto group, a primary amino group, a secondary amino group, a halogen atom, a haloacetamide group, a tetramethylpiperidinyloxy group, a dioxapiperidinyloxy group, an ammonium salt group, a hydrazino group, a disulfide group, an ester group, thioate group, a thioester group, a carbonate group, a thiocarbonate group, a dithiocarbonate group, a trithiocarbonate group, a xanthate group, a perthiocarbonate group, a tetrathiodiester group, an *O*-carbonyl hydroxylamine group, an amide group, an imide group, a hydrazide group, a sulfonyl hydrazide group, a hydrazone group, an imine group, an enamino group, an alkynyl amine group, a urethane group, a thiourethane group, a dithiourethane group, and a protected amino group;
Class D: a carboxyl group, a sulfonic acid group, a sulfenic acid group, a hydroxamic acid group, a thiohydroxamic acid group, a xanthogenic acid group, an acyl halide group, a sulfonyl chloride group, an aldehyde group, a glyoxal group, an acetal group, a hemiacetal group, a hydrated aldehyde group, a ketone group, a ketal group, a hemiketal group, a hemiketal group, a ketal group, a hydrated ketone group, an orthoacid group, an orthoester group, a cyanate group, a thiocyanate group, an isocyanato group, an isothiocyanate group, an ester group, an oxycarbonyl halide group, an oxazolinyl group, an isoxazolinyl group, a thioaldehyde group, a thione group, a thioacetal group, a thione hydrate group, a thioketal group, a thioate group, a thioester group, a dithioester group, a thiohemiacetal group, a monothiohydrate group, a dithiohydrate group, a thiolhydrate group, a monothiocarboxylic acid group containing a thiocarbonyl group, a monothiocarboxylic acid group containing a thiol group, a dithiocarboxylic acid group, a ureido group, a thioureido group, a guanidino group and its protonated form, an amidino group and its protonated form, an anhydride group, a squaric acid group, a squarate group, a semi-squaric acid group, a semi-squarate group, a *N*-carbamoyl-3-imidazole group, a *N*-carbamoyl-3-methylimidazolium iodide group, an imino acid group, an imidate group, a nitrone group, an oxime group, and a pseudourea group;
Class E: a maleimide group, an acrylate group, a *N*-acrylamide group, a methacrylate group, a N-methacrylamide group, a protected maleimide group, a maleamic acid group, a 1,2,4-triazoline-3,5-dione group, a linear azo compound group, a cyclic azo compound group, a cycloalkenyl group; wherein, the cycloalkenyl group includes a cyclooctenyl group, a norbornenyl group, a 7-oxa-bicyclo[2.2.1]hept-5-en-2-yl, a bicycloheptadienyl group, and a 7-oxa-bicycloheptadienyl group;
Class F: an epoxy group, an ethenyl group, a propenyl group, an alkenyl hydrocarbon group, an alkynyl group, and an alkynyl hydrocarbon group.
Class G,
Class Ga: a cycloalkynyl group, a cycloalkynyl heterohydrocarbon group, a linear conjugated dienyl group, a cyclic conjugated dienyl group, a heterosubstituted cyclic conjugated dienyl group, and a 1,2,4,5-tetrazinyl group;
Class Gb: an azide group, a nitrile oxide group, a cyano group, an isocyano group, an aldoxime group, a diazo group, a diazonium group, an azo oxide group, a nitrilimine group, a N-aldimine oxide group, a tetrazole group, a 4-acetyl-2-methoxy-5-nitrophenoxy group and its diazo form, and other functional groups which can undergo 1,3-dipolar cycloaddition reactions;
Class H: a hydroxyl group, a protected hydroxyl group, a siloxy group, a protected dihydroxyl group, a trihydroxysilyl group, a protected trihydroxysilyl group; wherein, the hydroxyl group includes an alcoholic hydroxyl group, a phenolic hydroxyl group, an enolic hydroxyl group, and a hemiacetal hydroxyl group;
Class I: targeting groups and pharmaceutically acceptable salts thereof;
Class J: fluorescent groups, including a fluorescein group, a rhodamine group, an anthracenyl group, a pyrenyl group, a coumarin group, a fluorescent yellow 3G group, a carbazole group, an imidazole group, an indole group, an alizarin violet group, and residues of functional derivatives thereof.

Further, R₀₁ is preferably selected from the group consisting of the functional groups in the following Classes A to H and variant forms thereof, and the functional derivatives in Classes I to J; said variant form is a precursor of functional group, an active form with functional group as precursor, a substituted active form, a protected form, or an unprotected form:
Class A: 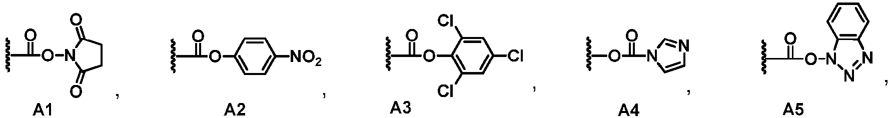 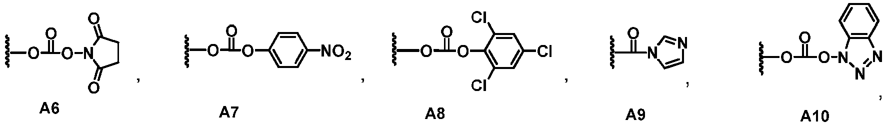 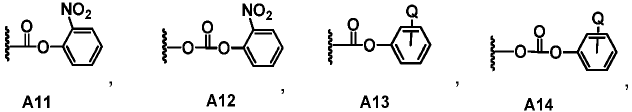 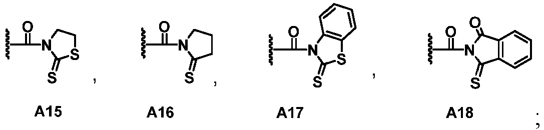 or Class B: 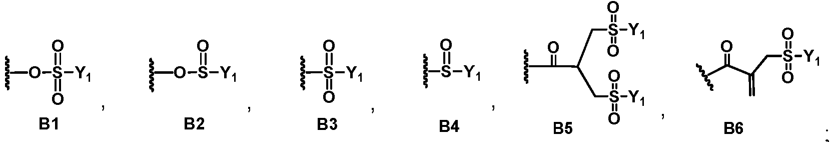
or Group C: 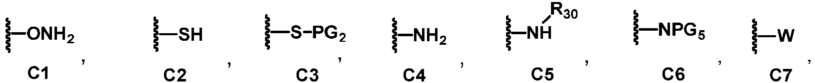 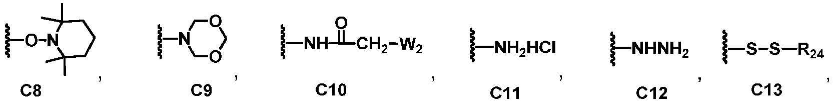 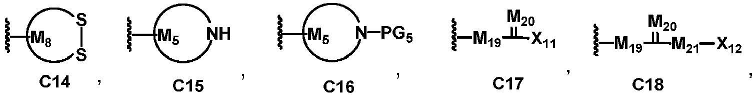 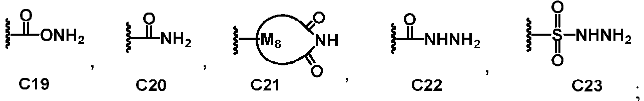 or Class D: 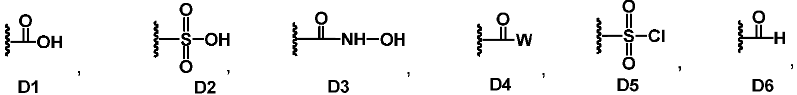 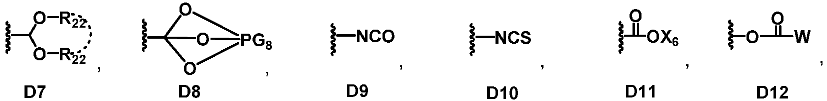 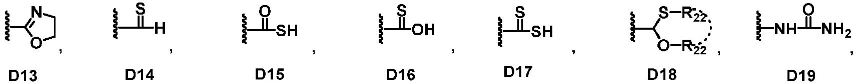 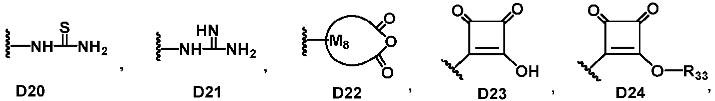 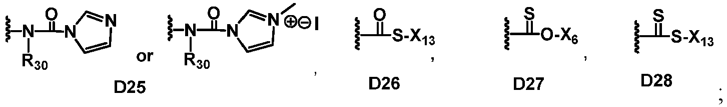
or Class E: 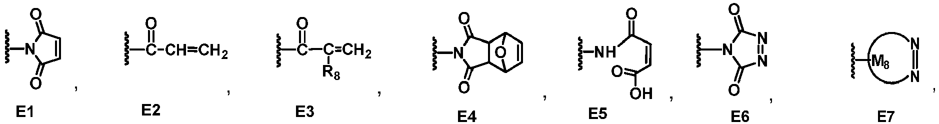 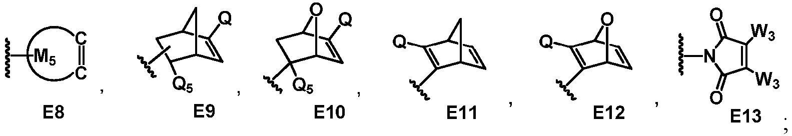 or Class F: 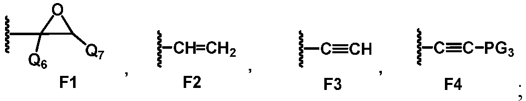
or Class G:
Group Ga: 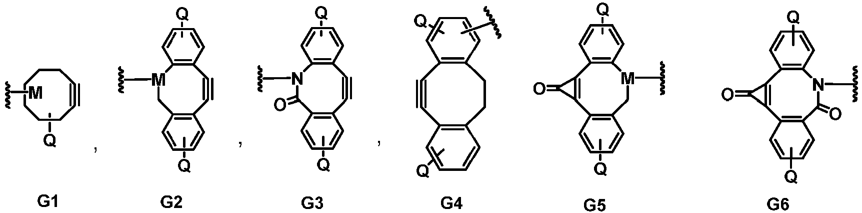 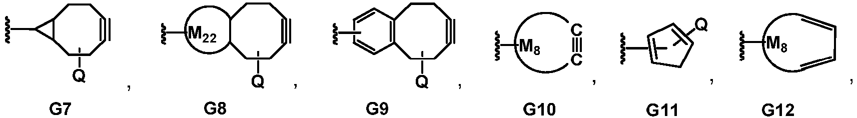 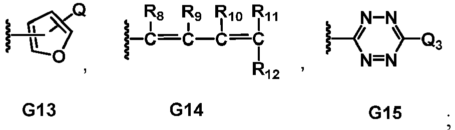
or Class Gb: 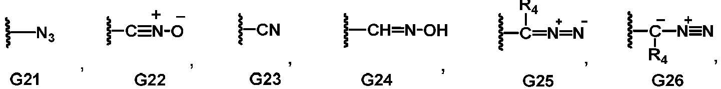 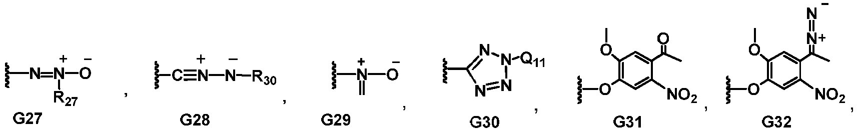 or Class H: 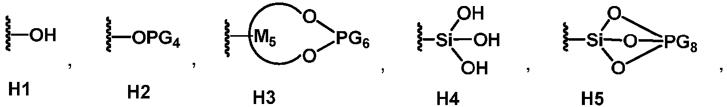 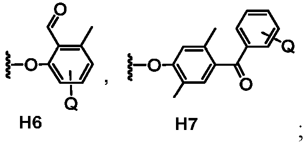
or Class I: 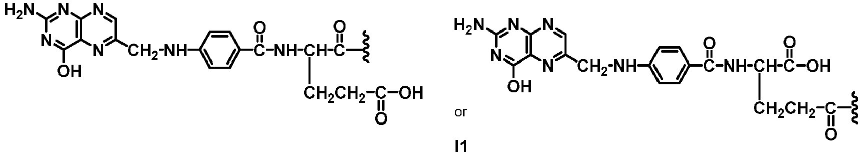 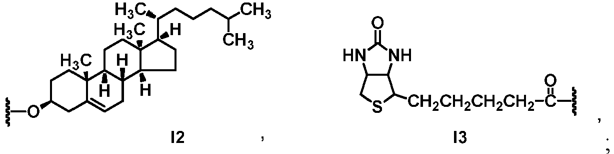 or Class J: 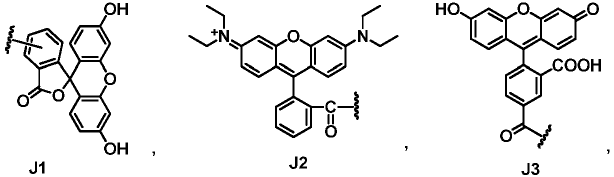 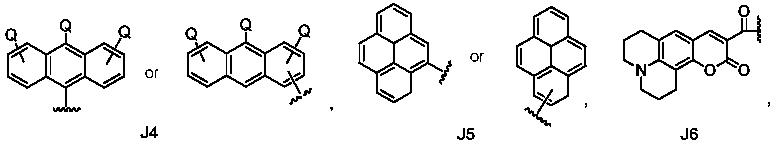 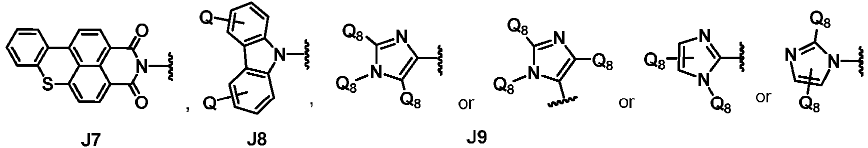 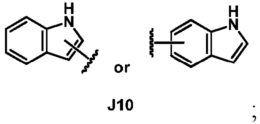
wherein, M₅ is a ring-constituting atom, selected from the group consisting of a carbon atom, a nitrogen atom, a phosphorus atom, and a silicon atom; the cyclic structure containing M₅ is a 3- to 50-membered ring, preferably a 3- to 32-membered ring, more preferably a 3- to 18-membered ring, and more preferably a 5- to 18-membered ring; said cyclic structure is preferably selected from the group consisting of cyclohexane, furanose ring, pyranose ring, benzene, tetrahydrofuran, pyrrolidine, thiazolidine, cyclohexene, tetrahydropyran, piperidine, 1,4-dioxane, pyridine, pyridazine, pyrimidine, pyrazine, 1,3,5-triazine, 1,4,7-triazacyclononane, cyclotripeptide, indene, indane, indole, isoindole, purine, naphthalene, dihydroanthracene, xanthene, thioxanthene, dihydrophenanthrene, 10,11-dihydro-5H-dibenzo[a,d]cycloheptane, dibenzocycloheptene, 5-dibenzosuberenone, quinoline, isoquinoline, fluorene, carbazole, iminodibenzyl, acenaphthene, dibenzocyclooctyne, aza-dibenzocyclooctyne, substituted forms thereof, and heterosubstituted forms thereof;
wherein, Y₁ is a leaving group connected to a sulfonyl group, a sulfinyl group, an oxysulfonyl group, or an oxysulfinyl group, which is selected from the group consisting of a methyl group, an ethyl group, an *n*-propyl group, an isopropyl group, a butyl group, a pentyl group, a hexyl group, a heptyl group, an octyl group, a nonyl group, a decyl group, a vinyl group, a phenyl group, a benzyl group, a *p*-methylphenyl group, a 4-(trifluoromethoxy)phenyl group, a trifluoromethyl group, and a 2,2,2-trifluoroethyl group;
wherein, W is F, Cl, Br, or I;
wherein, W₂ is F, Cl, Br, or I;
wherein, W₃ is a leaving group, selected from the group consisting of F, Cl, Br, I and PhS;
wherein, 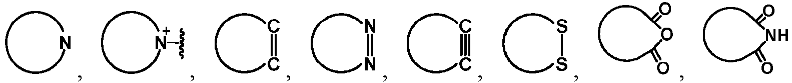 and 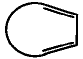 are cyclic structures, of which the skeletons contain a nitrogen atom, a nitrogenium ion, a double bond, an azo bond, a triple bond, a disulfide bond, an anhydride group, an imide group, and a dienylene, respectively; said cyclic structures are selected from the group consisting of a carbocycle, a heterocycle, a benzoheterocycle, a substituted carbocycle, a substituted heterocycle, and a substituted benzoheterocycle;
wherein, M is a carbon atom, a nitrogen atom, a phosphorus atom, or a silicon atom of the ring skeleton;
wherein, M₈ is a carbon atom, a nitrogen atom, a phosphorus atom, or a silicon atom of the ring skeleton; the number of ring-constituting atoms of the cyclic structure containing M₈ is from 4 to 50, preferably from 4 to 32, and more preferably from 5 to 32;
wherein, M₂₂ is a carbon atom, a nitrogen atom, a phosphorus atom, or a silicon atom of an alicycle or heteroalicycle; the number of ring-constituting atoms of the cyclic structure containing M₂₂ is 4, 5, 6, 7, or 8;
wherein, R₂₂ is a terminal group or a divalent linking group connected to an oxygen atom or a sulfur atom, which is any atom or group selected from the group consisting of hydrogen atom, R₂₁, and R₃₃;
wherein, R₂₁ is a divalent linking group and participates in forming a ring; R₂₁ is selected from the group consisting of a C₁₋₂₀ hydrocarbylene group, a divalent C₁₋₂₀ heterohydrocarbon group, a substituted C₁₋₂₀ hydrocarbylene group, a substituted divalent C₁₋₂₀ heterohydrocarbon group, and combinations of any two or three thereof; R₂₁ is preferably selected from the group consisting of a methylene group, an ethylene group, a propylene group, a butylene group, a pentylene group, a hexylene group, a heptylene group, an octylene group, a nonylene group, a decylene group, a 1,2-phenylene group, a benzylene group, a C₁₋₂₀ oxa-alkylene group, a C₁₋₂₀ thia-alkylene group, a C₁₋₂₀ aza-alkylene group, an aromatic aza-hydrocarbyl group, substituted forms thereof, and combinations of any two or more identical, different, or substituted forms thereof;
wherein, R₃₃ is a terminal group connected to an oxy group or a thioxy group, which is selected from the group consisting of a C₁₋₂₀ hydrocarbon group, a C₁₋₂₀ heterohydrocarbon group, a C₁₋₂₀ substituted hydrocarbon group, and a C₁₋₂₀ substituted heterohydrocarbon group, and preferably the group consisting of a methyl group, an ethyl group, an n-propyl group, an isopropyl group, a butyl group, a pentyl group, a hexyl group, a heptyl group, an octyl group, a nonyl group, a decyl group, a benzyl group, an allyl group, and substituted forms thereof;
wherein, R₄ is a hydrogen atom, substituent atom, or substituent group connected to the carbon atom of a structure with the formula of -(R₄)C=N⁺=N⁻ or -(R₄)C⁻-N⁺≡N, and is preferably any atom or group selected from the group consisting of a hydrogen atom, a methyl group, an ethyl group, an *n*-propyl group, an isopropyl group, a butyl group, a pentyl group, a hexyl group, a heptyl group, an octyl group, a nonyl group, a decyl group, an allyl group, a propenyl group, an ethenyl group, a phenyl group, a methylphenyl group, a butylphenyl group, and a benzyl group;
wherein, R₈, R₉, R₁₀, R₁₁ and R₁₂ are each independently a hydrogen atom, a substituent atom, or a substituent group connected to a double bond (-C=C-); R₈, R₉, R₁₀, R₁₁, and R₁₂ can be the same or different in one molecule; R₈, R₉, R₁₀, R₁₁ and R₁₂ are each independently selected from the group consisting of a hydrogen atom, a fluorine atom, and a methyl group; in the class E3, R₈ is preferably a methyl group;
wherein, R₂₄ is a terminal group connected to a disulfide bond, which is selected from the group consisting of a C₁₋₂₀ alkyl group, an aryl group, and a heterosubstituted phenyl group;
wherein, R₂₇ is a substituent connected to an azo group, which is selected from the group consisting of a phenyl group, a substituted phenyl group and a heterosubstituted phenyl group;
wherein, R₃₀ is a hydrocarbon group, selected from the group consisting of a C₁₋₂₀ alkyl group, a benzyl group, and a benzyl group with hydrogen atoms at the benzene ring replaced by C₁₋₂₀ hydrocarbon groups;
wherein, M₁₉, M₂₀, and M₂₁ are each independently an oxygen atom or a sulfur atom, and in one molecule, M₁₉, M₂₀, and M₂₁ can be the same or different;
wherein, X₆ is a terminal group connected to an oxygen atom of an ester group, which is a hydroxyl protecting group or the group LG₄; LG₄ is selected from the group consisting of a C₁₋₂₀ alkyl group, an aryl group, an aralkyl group, a C₁₋₂₀ heteroalkyl group, a heteroaryl group, a heteroaralkyl group, a C₁₋₂₀ alkylcarbonyl group, an arylcarbonyl group, an arylalkylcarbonyl group, a C₁₋₂₀ heteroalkylcarbonyl group, a heteroarylcarbonyl group, a heteroarylalkylcarbonyl group, a C₁₋₂₀ alkoxycarbonyl group, an aryloxycarbonyl group, an arylalkoxycarbonyl group, a C₁₋₂₀ (alkylthio)carbonyl group, an (arylthio)carbonyl group, an (arylalkylthio)carbonyl group, a C₁₋₂₀ alkylaminocarbonyl group, an arylaminocarbonyl group, an arylalkylaminocarbonyl group, a C₁₋₂₀ heteroalkoxycarbonyl group, a heteroaryloxycarbonyl group, a heteroarylalkoxycarbonyl group, a C₁₋₂₀ hetero(alkylthio)carbonyl group, a hetero(arylthio)carbonyl group, a hetero(arylalkylthio)carbonyl group, a C₁₋₂₀ heteroalkylaminocarbonyl group, a heteroarylaminocarbonyl group, a heteroarylalkylaminocarbonyl group, a C₁₋₂₀ (alkyl)thiocarbonyl group, an (aryl)thiocarbonyl group, an (arylalkyl)thiocarbonyl group, a C₁₋₂₀ hetero(alkyl)thiocarbonyl group, a hetero(aryl)thiocarbonyl group, a hetero(arylalkyl)thiocarbonyl group, a C₁₋₂₀ alkoxy-thiocarbonyl group, an aryloxy-thiocarbonyl group, an arylalkoxy-thiocarbonyl group, a C₁₋₂₀ (alkylthio)thiocarbonyl group, an (arylthio)thiocarbonyl group, an (arylalkylthio)thiocarbonyl group, a C₁₋₂₀ alkylaminothiocarbonyl group, an arylaminothiocarbonyl group, an arylalkylaminothiocarbonyl group, a C₁₋₂₀ heteroalkyloxy-thiocarbonyl group, a heteroaryloxy-thiocarbonyl group, a heteroarylalkoxy-thiocarbonyl group, a C₁₋₂₀ hetero(alkylthio)thiocarbonyl group, a hetero(arylthio)thiocarbonyl group, a hetero(arylalkylthio)thiocarbonyl group, a C₁₋₂₀ heteroalkylaminothiocarbonyl group, a heteroarylaminothiocarbonyl group, a heteroarylalkylaminothiocarbonyl group, and substituted forms thereof; wherein, the substituent atom or substituent group is a fluorine atom, an alkoxy group, or a nitro group;
wherein, X₁₁ is a terminal group connected to a carbonyl group or a thiocarbonyl group, selected from C₁₋₂₀ alkyl groups;
wherein, X₁₂ is a terminal group connected to a carbonate group or a thiocarbonate group, selected from C₁₋₂₀ hydrocarbon groups;
wherein, X₁₃ is a terminal group connected to a thioxy group, selected from the group consisting of a mercapto protecting group and the group LG₂;
wherein, LG₂ is selected from the group consisting of a methyl group, an ethyl group, an n-propyl group, an isopropyl group, a t-butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an eicosyl group, an allyl group, a trityl group, a phenyl group, a benzyl group, a methylbenzyl group, a nitrobenzyl group, a *t*-butylthio group, a benzylthio group, a 2-pyridylthio group, an acetyl group, a benzoyl group, a methoxycarbonyl group, an ethoxycarbonyl group, a *t*-butoxycarbonyl group, a phenoxycarbonyl group, a benzyloxycarbonyl group, a (methylthio)carbonyl group, an (ethylthio)carbonyl group, a (*t*-butylthio)carbonyl group, a (phenylthio)carbonyl group, a (benzylthio)carbonyl group, a 2-pyridylcarbonyl group, a methylaminocarbonyl group, an ethylaminocarbonyl group, a *t*-butylaminocarbonyl group, a benzylaminocarbonyl group, an ethyl-thiocarbonyl group, a phenyl-methylthiocarbonyl group, a methoxy-thiocarbonyl group, an ethoxy-thiocarbonyl group, a *t*-butyloxy-thiocarbonyl group, a phenoxy-thiocarbonyl group, a benzyloxy-thiocarbonyl group, a (methylthio)thiocarbonyl group, an (ethylthio)thiocarbonyl group, a (*t*-butylthio)thiocarbonyl group, a (phenylthio)thiocarbonyl group, a (benzylthio)thiocarbonyl group, a methylaminothiocarbonyl group, an ethylaminothiocarbonyl group, a *t*-butylaminothiocarbonyl group, a benzylaminothiocarbonyl group, a C₁₋₁₀ halohydrocarbon group, a trifluoroacetyl group, a nitrophenyl group, and substituted forms thereof; wherein, the substituent atom or substituent group is a fluorine atom, an alkoxy group or a nitro group;
wherein, Q is an atom or a substituent that can promote the inductive or conjugate effect of electrons of unsaturated bonds; when Q is connected to the ring, the number of Q can be one or greater than one; when the number of Q is greater than one, they can be of the same structure or a combination of two or more different structures; when Q is a substituent group, it can have a linear structure, a branched structure bearing pendant groups, or a ring-containing structure;
wherein, Q₃ is a hydrogen atom or a group that can promote the inductive or conjugate effect of electrons of unsaturated bonds, which can be any atom or group selected from the group consisting of a hydrogen atom, a fluorine atom, a chlorine atom, a bromine atom, an iodine atom, a methyl group, an ethyl group, an n-propyl group, an isopropyl group, a butyl group, a pentyl group, a hexyl group, a heptyl group, an octyl group, a nonyl group, a decyl group, an ethenyl group, a propenyl group, an allyl group, a propynyl group, a propargyl group, a cyclopropyl group, a cyclopropenyl group, a phenyl group, a benzyl group, a butylphenyl group, a *p*-methylphenyl group, a *p*-nitrophenyl group, an *o*-nitrophenyl group, a *p*-methoxyphenyl group, an azaphenyl group, a methoxy group, an ethoxy group, a phenoxy group, a benzyloxy group, a methylthio group, an ethylthio group, a phenylthio group, a benzylthio group, a trifluoromethyl group, a 2,2,2-trifluoroethyl group, and substituted forms thereof;
wherein, Q₅ is a hydrogen atom, a methyl group, an ethyl group, or a propyl group; when Q₅ is connected to the ring, the number of Q can be one or greater than one; when the number of Q is greater than one, they can have the same structure, or be a combination of two or more different structures;
wherein, Q₆ is a hydrogen atom or a methyl group; Q₇ is a hydrogen atom, a methyl group, a phenyl group, or a substituted phenyl group; in one molecule, Q₆ and Q₇ can be identical or different from each other;
wherein, Q₈ is a substituent atom or substituent group of an imidazole group, selected from the group consisting of a hydrogen atom, a methyl group, an ethyl group, a propyl group, a butyl group, and a phenyl group; the number of Q₈ can be one or greater than one; when the number of Q₈ is greater than one, they can have the same structure, or be a combination of two or more different structures;
wherein, Q₁₁ is a substituent group connected to a nitrogen atom of a tetrazole group, selected from the group consisting of a phenyl group, a substituted phenyl group, and an azaphenyl group;
wherein, PG₂ is a mercapto protecting group, and the protected mercapto group is represented as SPG₂ and preferably selected from the group consisting of a thioether, a disulfide, a silyl thioether, and a thiocarboxylate;
wherein, PG₃ is an alkynyl protecting group, preferably a silyl group;
wherein, PG₄ is a hydroxyl protecting group, and the protected hydroxyl group is represented as OPG₄ and preferably selected from the group consisting of an ether, a silyl ether, an ester, a carbonate, and a sulfonate;
wherein, PG₅ is an amino protecting group, and the protected amino group is represented as NPG₅ and preferably selected from the group consisting of a carbamate, an amide, an imide, an N-alkyl amine, an N-aryl amine, an imine, an enamine, an imidazole, a pyrrole, and an indole;
wherein, PG₆ is a dihydroxyl protecting group, forming a five- or six-membered cyclic acetal structure with two oxygen atoms; PG₆ is a methylene group or a substituted methylene group; wherein, the substituent of PG₆ is a hydrocarbyl substituent or a heteroatom-containing substituent, and PG₆ is selected from the group consisting of a methylene group, a 1-methylmethylene group, a 1,1-dimethylmethylene group, a 1,1-cyclopentylene group, a 1,1-cyclohexylene group, a 1-phenylmethylene group, and a 3,4-dimethylphenylmethylene group;
wherein, PG₈ is a protecting group of orthocarbonic acid or orthosilicic acid.

### 1.1.5.4. Specific examples of R

In one aspect of the claimed invention, R contains any atom or group selected from the group consisting of a hydrogen atom, an alkyl group, an alkoxy group, an alcoholic hydroxyl group, a protected alcoholic hydroxyl group, a thiol group, a protected thiol group, a carboxyl group, a protected carboxyl group, an amino group, a protected amino group, an aldehyde group, a protected aldehyde group, an ester group, a carbonate group, a urethane group, a succinimidyl group, a maleimide group, a protected maleimide group, a dimethylamino group, an alkenyl group, an alkenoate group, an azido group, an alkynyl group, a folate group, a rhodamine group, a biotinyl group, a monosaccharide group, and a polysaccharide group. In a preferred embodiment according to the claimed invention R is selected from the group consisting of H, -CH₃, -CH₂CH₃, -(CH₂)ₜOH, -(CH₂)ₜSH, -OCH₃, -OCH₂CH₃, -(CH₂)ₜNH₂, -(CH₂)ₜC(=O)OH, -C(=O)(CH₂)ₜC(=O)OH, -C(=O)CH₃, -(CH₂)ₜN₃, -C(=O)CH₂CH₃, -C(=O)OCH₃, -OC(=O)OCH₃, -C(=O)OCH₂CH₃, -OC(=O)OCH₂CH₃, -(CH₂)ₜN(CH₃)₂, -(CH₂)ₜN(CH₂CH₃)₂, -(CH₂)ₜCHO, 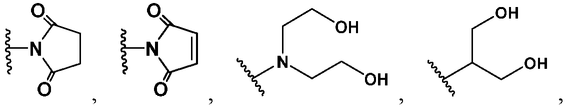 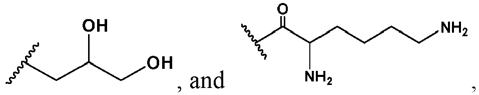 wherein, t is an integer from 0 to 12.

### 1.1.6. A

In the present invention, A is -(CRₐR_{b})ₛO- or -O(CRₐR_{b})ₛ-, wherein, s is 2, 3, or 4, Rₐ and R_{b} are each independently a hydrogen atom or a C₁₋₁₂ alkyl group.

In one specific embodiment of the present invention, Rₐ and R_{b} are both hydrogen atoms, and s is 2, specifically corresponding to A being CH₂CH₂O- or -OCH₂CH₂-.

In the present invention, the number-average molecular weight of the polyethylene glycol chains of the PEGylated lipids is 900, 1000, 1500, 2000, 2500, 3000, 3350, 3500, 4000, 5000, 5500, 6000, 6500, 7000, 7500, 8000, 8500, 9000, 9500, 10000, or 11000.

In one specific embodiment of the present invention, the polyethylene glycol chains of the PEGylated lipids can be monodisperse or polydisperse.

In one specific embodiment of the present invention, the polyethylene glycol chains are preferably polydisperse, and the number-average degree of polymerization (n₁) is preferably an integer from approximately 20 to 100, more preferably an integer from approximately 20 to 60, more preferably an integer from approximately 40 to 60, and more preferably 44, 45, 46, 48, 50, 52, 54, 56, 58, or 60.

In one specific embodiment of the present invention, the polyethylene glycol chains are preferably monodisperse, and the number of EO-unit is preferably from 20 to 70, more preferably from 20 to 60, and more preferably 20, 22, 24, 26, 27, 28, 29, 30, 32, 34, 36, 38, 40, 42, 44, 45, 46, 48, 50, 52, 54, 56, 58, or 60.

### 1.1.7. Examples of specific general formulas

In one specific embodiment of the present invention, the structure of PEGylated lipid of the present invention is preferably selected from the group consisting of the following structural formulas:

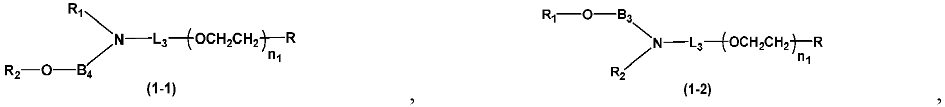

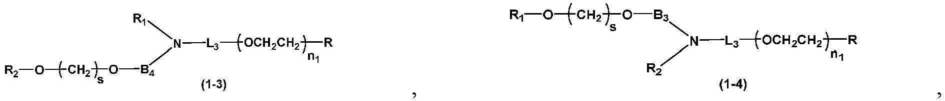

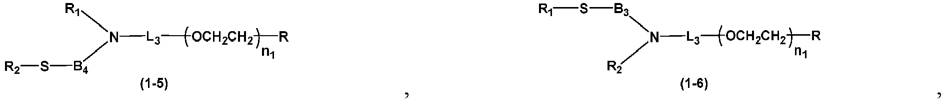

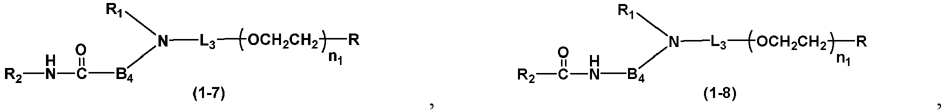

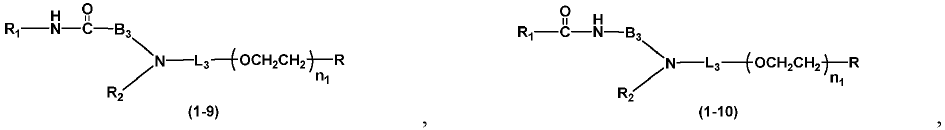

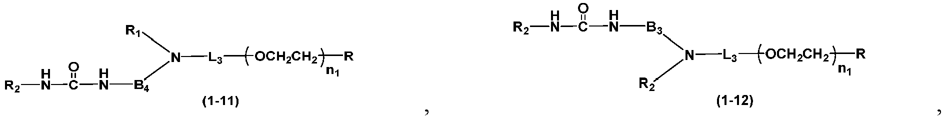

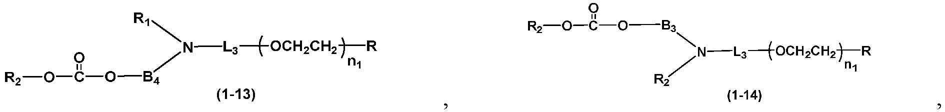

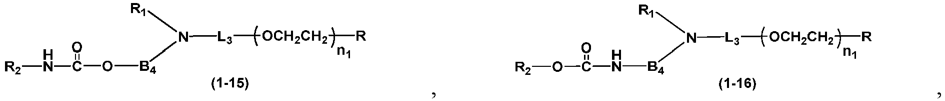

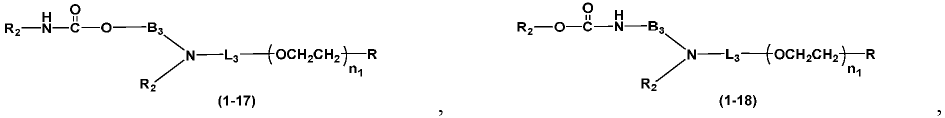

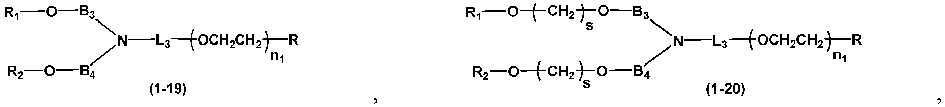

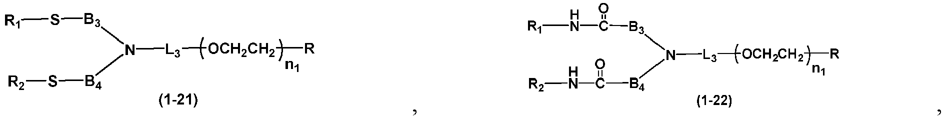

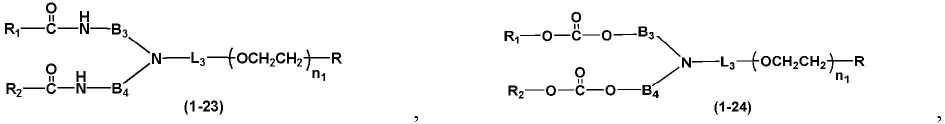

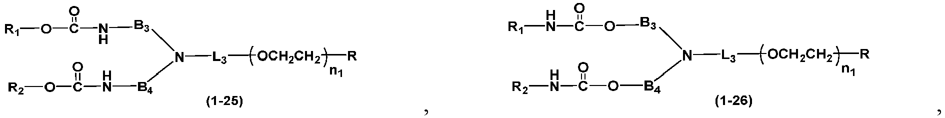

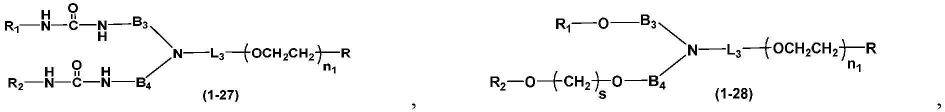

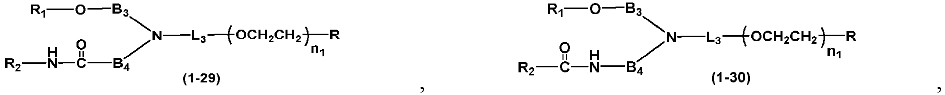

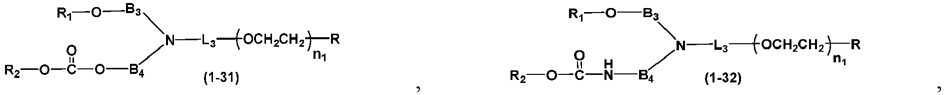

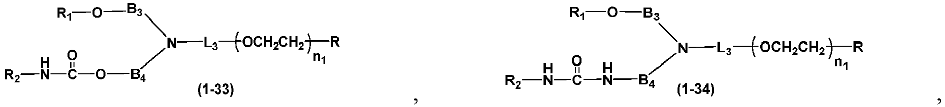

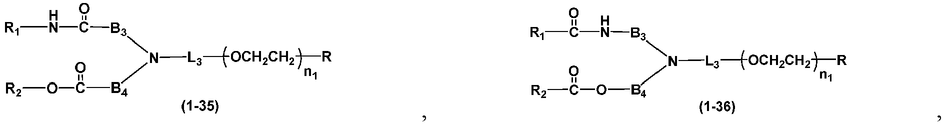

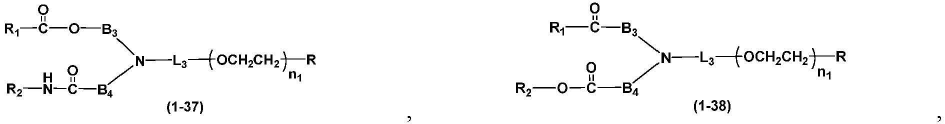

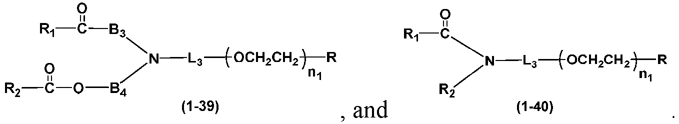

### 1.1.8. Specific structural examples of PEGylated lipids

In some specific embodiments of the present invention, PEGylated lipids with the following structures are finally obtained, which include but are not limited to the following structures: 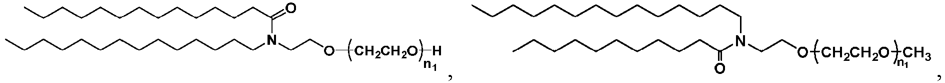 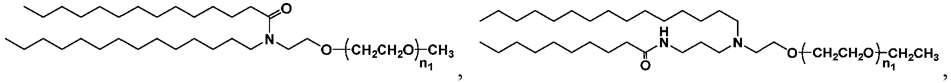 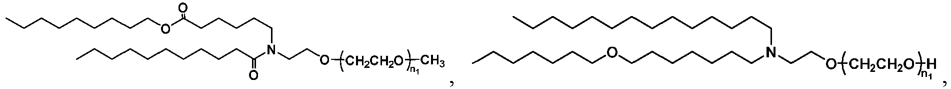 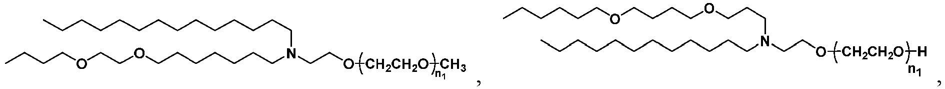 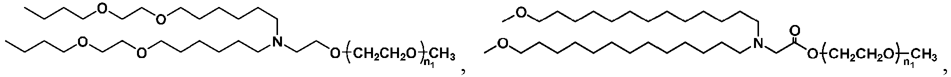 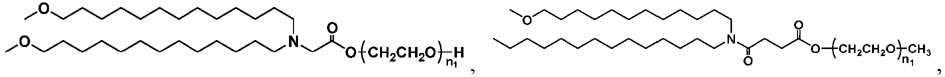 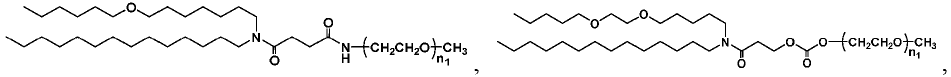 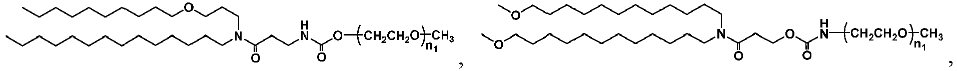 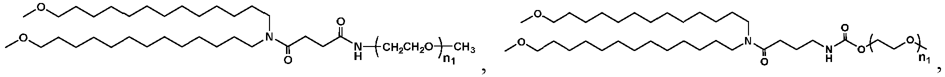 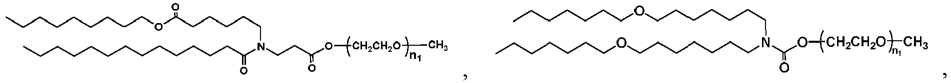 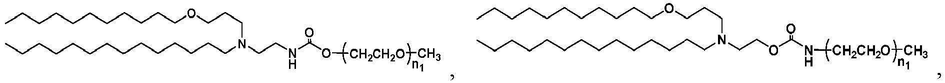 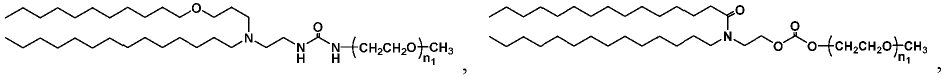 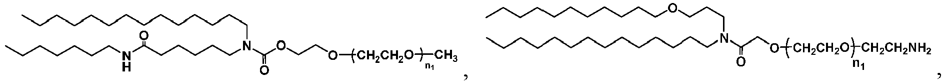 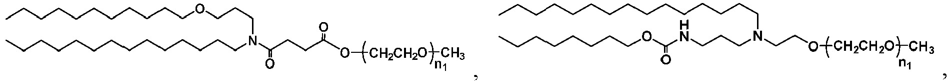 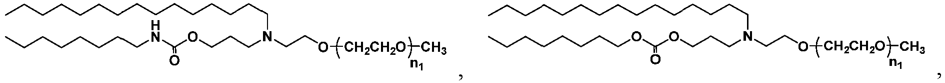 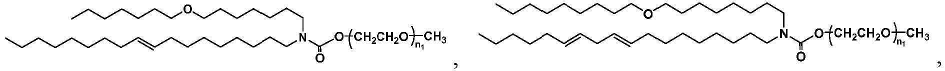 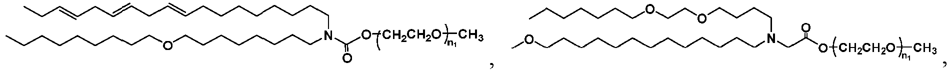 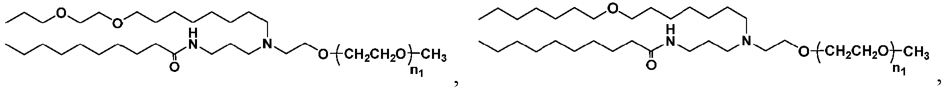 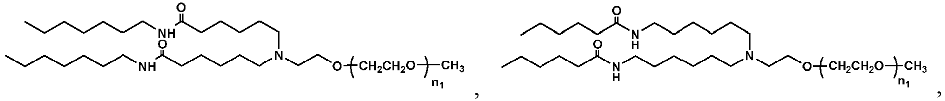 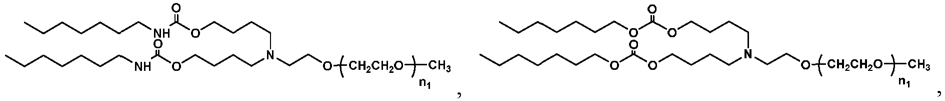 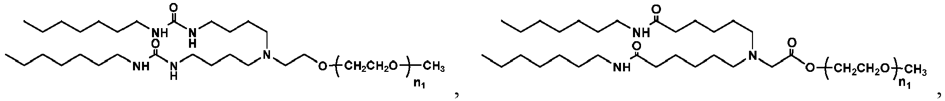 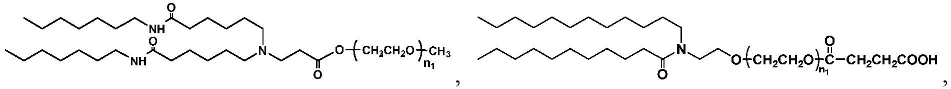 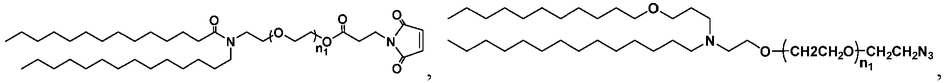 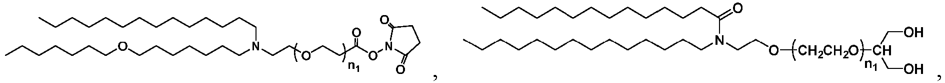 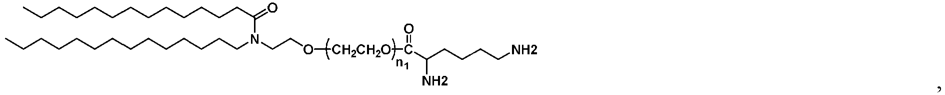 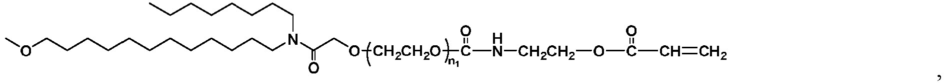 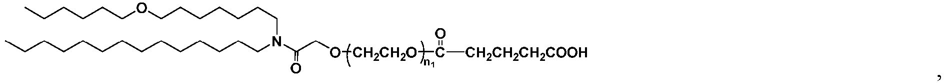 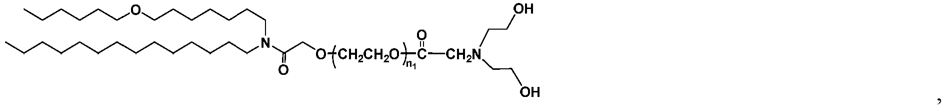 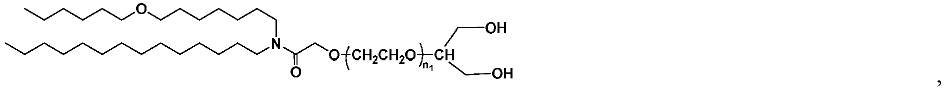 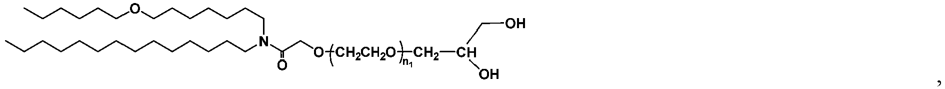 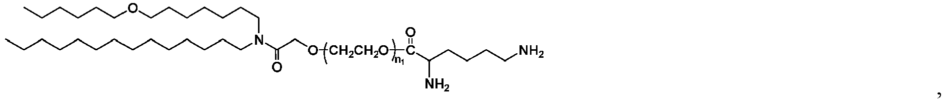 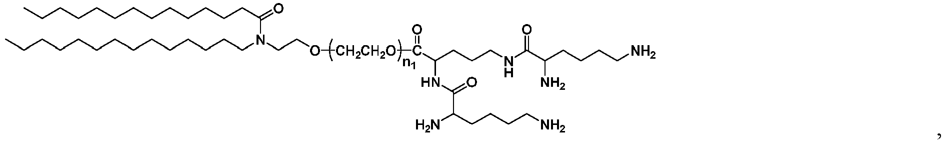 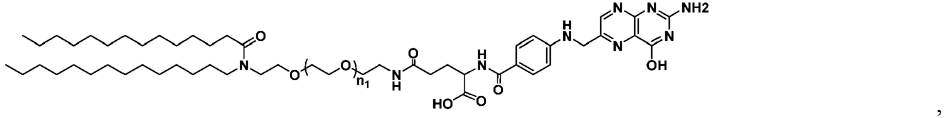 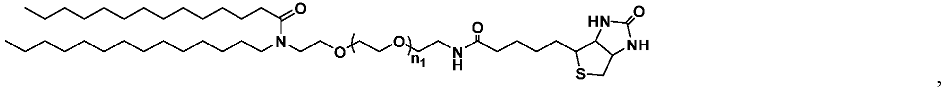 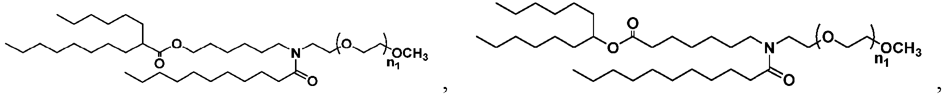 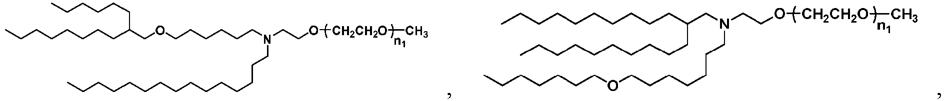 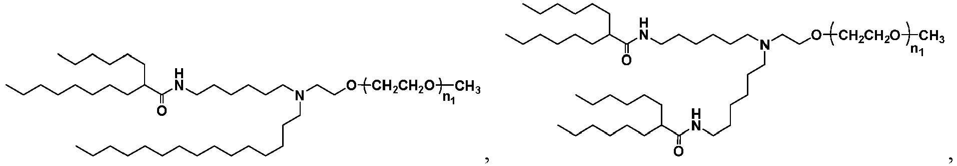 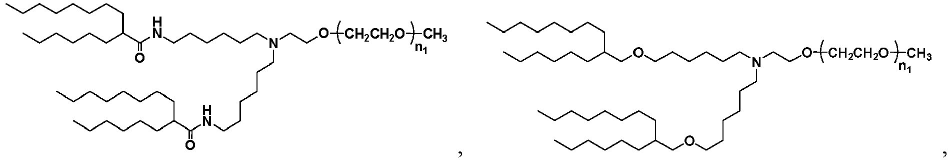 and 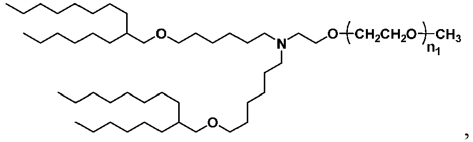 wherein, n₁ is an integer from 20 to 250.

### 2. Preparation of PEGylated lipids

In the present invention, any of the above-mentioned PEGylated lipids can be prepared by methods including but not limited to the following:

### 2.1. Method-1:

Step 1: Activating the carboxyl terminus of the acid A-1 or A-1' containing an unprotected carboxyl group with carboxyl activating agents to obtain the ester A-2 or A-2' containing an activated carboxyl terminus, wherein B₃' and B₄' are each independently a linking bond or an alkylene group having one less methylene group than B₃ and B₄; R₁' and R₂' are each independently R₁, R₂ or an aliphatic hydrocarbon group having one less methylene group than R₁ and R₂; R₇ is a carboxyl-activating group; and, when either B₃' or L₇ is not a linking bond, R₁' is R₁; when both B₃' and L₇ are linking bonds; R₁' is an aliphatic hydrocarbon group having one less methylene group than R₁; when any of B₄' and L₈ is not a linking bond, R₂' is R₂; when both B₄' and L₈ are linking bonds, R₂' is an aliphatic hydrocarbon group having one less methylene group than R₂;

Step 2: Carrying out the condensation reaction between the ester A-2 or A-2' containing an activated carboxyl terminus and the primary amine derivative A-3 or A-3' containing a nitrogen-source terminal group to obtain the amide intermediate A-4 or A-4';

Step 3: Using reducing agents to reduce the amide intermediate A-4 or A-4' to the secondary amine intermediate A-5 or A-5';

Step 4: Carrying out the coupling reaction between the secondary amine intermediate and the dual end-functionalized PEG derivative which is, the biLPEG molecule A-6, to obtain the PEGylated lipid derivative A-7 or A-7'; wherein, the biLPEG can be monodisperse or polydisperse; wherein, the two terminal functional groups of biLPEG can be the same or different; wherein, the R' end of biLPEG contains the reactive group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation; wherein, the F₁ contains active functional groups, which can react with the secondary amino group of the secondary amine intermediate A-5 or A-5' to obtain the nitrogen-atom branching center and the divalent linking group L₃;
when R' is R, the structure of A-7 or A-7' is represented by the general formula (2);
when R' is not R, A-8 or A-8' represented by the general formula (2) is obtained via terminal micro-modification of A-7 or A-7'; said terminal micro-modification is selected from chemical reactions including deprotection, salt complexation and decomplexation, ionization, protonation, deprotonation, and leaving group transformation.

Step 1: 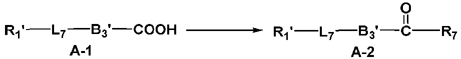 or

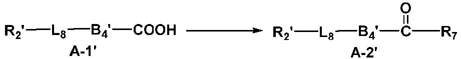

Step 2: 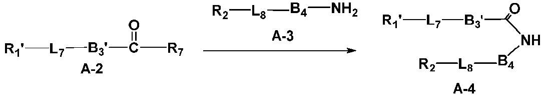 or

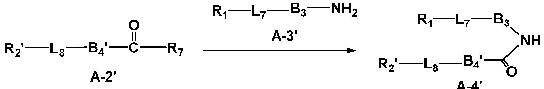

Step 3: 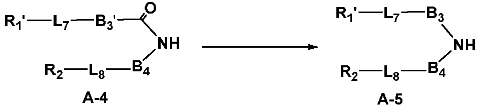 or or

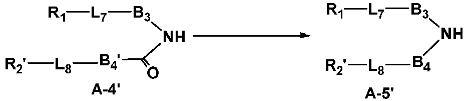

Step 4: 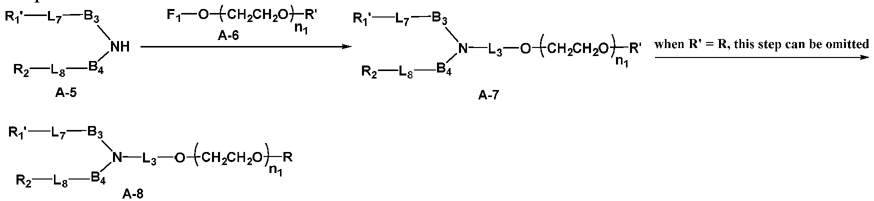 or

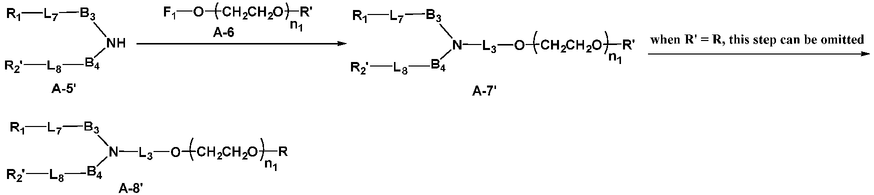

Wherein, the descriptions of L₃, L₇, L₈, B₃, B₄, R, R₁, R₂, and n₁ are in line with those of the general formula (2), which are not repeated here.

In one specific embodiment of the present invention, in the foregoing Method-1, said A-1 is preferably R₁'-COOH or R₂'-COOH, said A-3 is preferably R₁-NH₂ or R₂-NH₂, said carboxyl activating agent is preferably selected from the group consisting of N-hydroxysuccinimide (NHS), 1-ethyl-3-(3-dimethylaminopropyl)carbodiimide hydrochloride (EDCI), N-hydroxy-5-norbornene-2,3-dicarboximide (HONb), and N,N'-dicyclohexylcarbodiimide (DCC), and said PEGylated lipid is preferably prepared by the following routes:

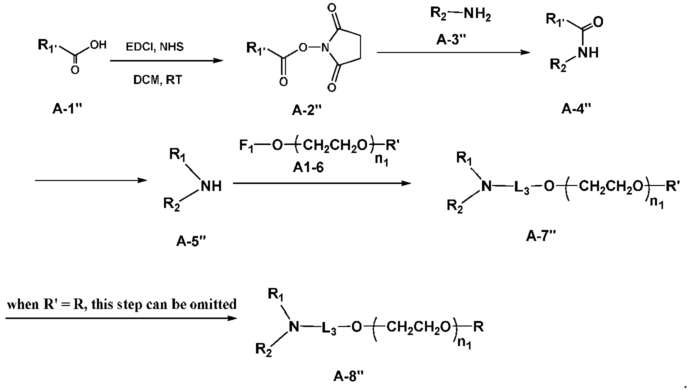

### 2.2 Method-2:

Step 1: Carrying out the coupling reaction between the bifunctional PEG derivative biLPEG molecule B-1, and the primary amine derivative B-2 or B-2' containing a nitrogen-source terminal group to obtain the PEGylated secondary amine derivative B-3 or B-3'; wherein, biLPEG can be monodisperse or polydisperse, the two terminal functional groups of biLPEG can be the same or different, and the R' end of biLPEG contains the reactive group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation; wherein, the F₁ contains active functional groups, which can react with the amino group of the primary amine B-2 or B-2' to obtain the secondary amine derivative B-3 or B-3' containing the divalent linking group L₃;
Step 2: Carrying out the alkylation reaction between the secondary amine derivative B-3 or B-3' and the compound B-4 or B-4' with the reactive group F_{N} to obtain the PEGylated lipid derivative B-5 or B-5'; said F_{N} is a reactive group which can react with amino group or secondary amino group, preferably -OMs, -OTs, -CHO, -F, -Cl, or -Br;
when R' is R, the structure of B-5 or B-5' is represented by the general formula (2);
when R' is not R, B-6 or B-6' represented by the general formula (2) is obtained via terminal micro-modification of B-5 or B-5'; said terminal micro-modification is selected from chemical reactions including deprotection, salt complexation and decomplexation, ionization, protonation, deprotonation, and leaving group transformation.
Step 1: 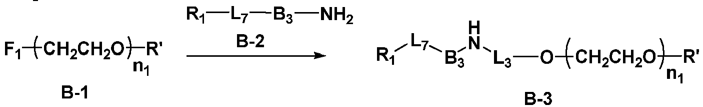 or 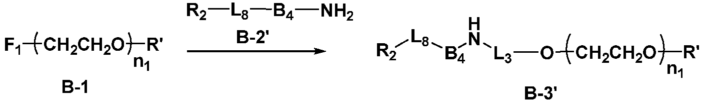
Step 2: 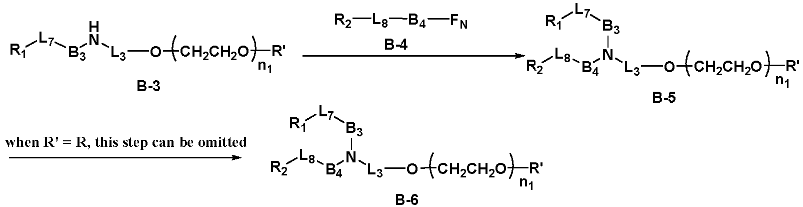 or

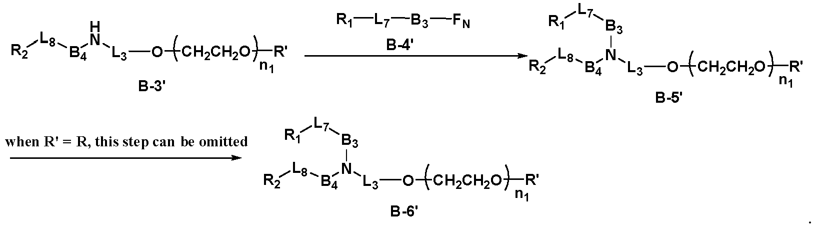

Wherein, the descriptions of L₃, L₇, L₈, B₃, B₄, R, R₁, R₂, and n₁ are in line with those of the general formula (2), and no more repeated here.

In one specific embodiment of the present invention, in the foregoing Method-2, said B-3 or B-3' is R₁-NH₂ or R₂-NH₂, said B-4 or B-4' is R₂-L₈-B₄-Br or R₁-L₇-B₃-Br, and said F₁ contains the -OMs group.

### 2.3. Method-3:

Step 1: Carrying out the reaction between the small molecule C-1 and the small molecule C-2 to obtain the small molecule intermediate C-3 which contains the divalent linking group L₇, the reactive group F_{N} at one end, and an aliphatic hydrocarbon group R₁ at the other end; wherein, F₃ and F₄ are each independently a reactive group, which can form the divalent linking group L₇ via reaction; wherein, C-2 contains a pair of heterofunctional groups F₃ and F_{N}, and said F_{N} is the reactive group which can react with amino group or secondary amino group, preferably -OMs, -OTs, -CHO, -F, -Cl or -Br;

Step 2: Carrying out the alkylation reaction between two molecules of the small molecule intermediate C-3 and the PEGylated primary amine derivative C-4 containing a nitrogen-source terminal group to obtain the PEGylated lipid C-5, wherein, the R' end contains the reactive group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation;
when R' is R, the structure of C-5 is represented by the general formula (2);
when R' is not R, C-6 represented by the general formula (2) is obtained via terminal micro-modification of C-5; said terminal micro-modification is selected from chemical reactions including deprotection, salt complexation and decomplexation, ionization, protonation, deprotonation, and leaving group transformation; wherein, R₁ is the same as R₂, B₃ is the same as B₄, and L₇ is the same as L₈.

Wherein, the descriptions of L₃, L₇, L₈, B₃, B₄, R, R₁, R₂, and n₁ are in line with those of the general formula (2), and no more repeated here.

### Step 1:

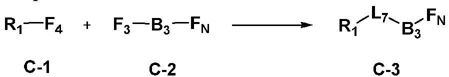

### Step 2:

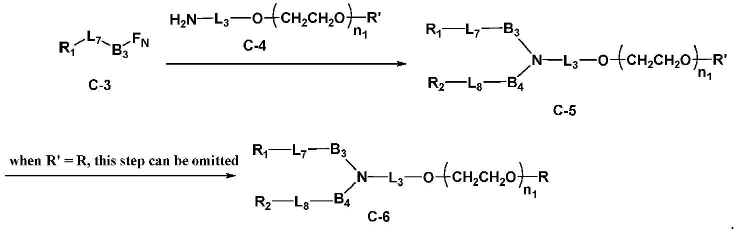

### 2.4. Method-4:

Step 1: Carrying out the reaction between the small molecule D-1 and the small molecule D-2 to obtain the small molecule intermediate D-3 which contains the divalent linking group L₇, a hydroxyl group at one end, and an aliphatic hydrocarbon group R₁ at the other end; wherein, F₃ and F₄ are each independently a reactive group, which can form the divalent linking group L₇ via reaction; wherein, D-2 contains a pair of heterofunctional groups F₃ and a hydroxyl group;

Step 2: Carrying out the reaction in which the hydroxyl group of the small molecule intermediate D-3 is oxidized to an aldehyde group to obtain the small molecule intermediate D-4 containing an aldehyde group, wherein B₃' is an alkylene group having one less methylene group than B₃;

Step 3: Carrying out the addition reaction between two molecules of the small molecule intermediate D-4 containing an aldehyde group and the PEGylated primary amine derivative D-5 containing a nitrogen-source terminal group to obtain the PEGylated lipid D-6, wherein, the R' end contains the reactive group R₀₁ or a micro variant form of R₀₁; said micro variant form refers to the group which can be transformed into R₀₁ through any chemical process selected from deprotection, salt complexation and decomplexation, ionization, protonation, deprotonation, and leaving group transformation;
when R' is R, the structure of D-6 is represented by the general formula (2);
when R' is not R, D-7 represented by the general formula (2) is obtained via terminal micro-modification of D-6; said terminal micro-modification is selected from chemical reactions including deprotection, salt complexation and decomplexation, ionization, protonation, deprotonation and leaving group transformation; wherein, R₁ is the same as R₂, B₃ is the same as B₄, and L₇ is the same as L₈.

### Step 1:

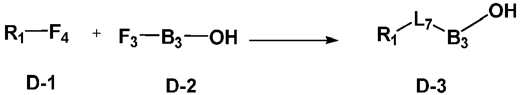

### Step 2:

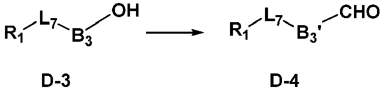

### Step 3:

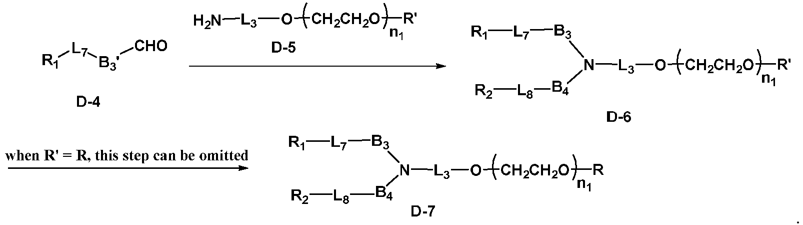

Wherein, the descriptions of L₃, L₇, L₈, B₃, B₄, R, R₁, R₂, and n₁ are in line with those of the general formula (2), and no more repeated here.

### 2.5. Description of relevant starting materials and/or steps in the preparation process

### 2.5.1. Carboxyl activating agent, condensing agent, oxidizing agent, and reducing agent

In the present invention, the "activation of a carboxyl group" refers to the activation of a carboxyl group with carboxyl activating agents. The activated carboxyl group can promote the condensation reactions, for example, by inhibiting the generation of racemic impurities, by accelerating the reaction through catalysis, etc. The "carboxyl activating group" refers to the residue of a carboxyl activating agent. Said carboxyl activating agent is selected from the group consisting of *N*-hydroxysuccinimide (NHS), 1-ethyl-3-(3-dimethylaminopropyl)carbodiimide hydrochloride (EDCI), *N*-hydroxy-5-norbornene-2,3-dicarboximide (HONb), and *N,N'*-dicyclohexylcarbodiimide (DCC), and combinations thereof, preferably the combination of NHS/EDCI, NHS/DCC, or HONb/DCC, and more preferably the combination of NHS/EDCI.

In the present invention, the condensing agent used in reactions is not particularly limited, but preferably selected from the group consisting of *N,N*'-dicyclohexylcarbodiimide (DCC), 1-ethyl-3-(3-dimethylaminopropyl)carbodiimide hydrochloride (EDC·HCl), 2-(7-azobenzotriazole-*N,N,N',N'*-tetramethyluronium hexafluorophosphate (HATU) and (benzotriazol-1-yl)-*N,N,N',N'*-tetramethyluronium hexafluorophosphate (HBTU), and most preferably DDC. Generally, the amount of the condensing agent used is 1 to 20 folds of the molar equivalent of the carboxylic acid, preferably 5-10 folds, and a suitable catalyst (such as 4-dimethylaminopyridine) can be added to the reaction.

In the present invention, the oxidizing agent used in reactions is not particularly limited, as long as it is a compound or a combination of compounds that can increase the valence of the substrate; said oxidizing agent is preferably selected from the group consisting of phenyliodine(III)bis(trifluoroacetate), 1,4-benzoquinone, benzyl trimethyl ammonium tribromide, pyridinium dichromate, potassium dichromate, ozone, oxygen, hydrofluoric acid, sodium hypochlorite, cobaltic acetate, cobalt acetate, manganous acetate, palladium(II) acetate, cupric acetate, monoperoxyphthalic acid, iodine, *N*-iodosuccinimide, iodylbenzene, 2-iodylbenzoic acid, dimethyldioxirane, dimethyl sulfoxide-oxalyl chloride, DDQ, dichlorotris(triphenylphosphine)ruthenium, manganese dioxide, (diacetoxyiodo)benzene, periodic acid, sodium periodate, sodium periodate-osmium tetraoxide, potassium permanganate, sodium perborate, perbenzoic acid, dibenzoyl peroxide, nickel peroxide, hydrogen peroxide, cumyl hydroperoxide, *t*-butyl hydroperoxide, peracetic acid, *m*-chloroperbenzoic acid, *N*-chlorosuccinimide, pyridinium chlorochromate, palladium chloride-cupric chloride, urea hydrogen peroxide adduct, triphenylcarbenium tetrafluoroborate, tributyltin oxide, cobalt trifluoride, vanadium oxytrifluoride, chromium trioxide, manganese triacetate, TEMPO, diammonium cerium nitrate, bromine, pyridine *N*-oxide, silver oxide, *O*-ethylperoxycarbonic acid, manganese acetylacetonate, vanadyl acetylacetonate, aluminium isopropoxide, potassium peroxymonosulfate, dichloroiodobenzene, etc., and combinations thereof, and more preferably selected from the group consisting of oxygen, sodium hypochlorite, hydrogen peroxide, dichloroiodobenzene, potassium peroxymonosulfate, etc., and combinations thereof. The molar equivalent of the oxidizing agent used is 1 to 50 folds of that of the hydroxyl groups of intermediate compounds, preferably 1 to 20 folds, and more preferably 5 to 10 folds.

In the present invention, the reducing agents used in reactions are not particularly limited, as long as the Schiff bases formed via the reaction between amines and aldehydes or ketones can be reduced to amine groups; said reducing agents are preferably selected from the group consisting of sodium borohydride, sodium cyanoborohydride, lithium aluminum hydride, borane, diborane, diisobutylaluminum hydride, diisopinocampheylborane, lithium borohydride, zinc borohydride, borane-pyridine complex, borane-methyl sulfide complex, borane-tetrahydrofuran complex, etc., and combinations thereof, and more preferably sodium cyanoborohydride. The molar equivalent of the reducing agent used is 1 to 50 folds of that of the amino groups to be modified, preferably 1 to 20 folds, and more preferably 5 to 10 folds.

In the present invention, the reaction temperature is 0 to 200°C, preferably 0 to 100°C, and more preferably 0 to 25°C. The reaction time is preferably 10 min to 48 h, and more preferably 30 min to 24 h. The obtained product can be purified by a purification means such as extraction, recrystallization, adsorption treatment, precipitation, reverse precipitation, membrane dialysis, supercritical extraction, etc.

In the present invention, the reaction solvent can be absent or an aprotic solvent; said aprotic solvent includes toluene, benzene, xylene, acetonitrile, ethyl acetate, diethyl ether, tert-butyl methyl ether, tetrahydrofuran, chloroform, dichloromethane, dimethylsulfoxide, dimethylformamide, and dimethylacetamide, and is preferably tetrahydrofuran, dichloromethane, dimethylsulfoxide, or dimethylformamide.

In the present invention, the bases used in reactions can be organic bases (e.g., triethylamine, pyridine, 4-dimethylaminopyridine, imidazole, or *N,N-*diisopropylethylamine), preferably triethylamine or pyridine. The molar equivalent of the base used is 1 to 50 folds of that of carboxylic acids, preferably 1 to 10 folds, and more preferably 2 to 3 folds.

### 2.5.2. "Protection" and "deprotection" of relevant groups involved in the reaction process

In the present invention, the reaction process also involves the "protection" and "deprotection" processes of relevant groups. In order to prevent a functional group from affecting the reaction, the functional group is usually protected. In addition, when there are two or more functional groups, selectively only the target functional group undergoes the reaction, so the other functional groups should be protected. The protecting group not only provides stable protection to the target functional group, but is also expected to be removed easily as needed. Therefore, in organic synthesis, it is important to remove only the protecting group bonded to the specified functional group under appropriate conditions.

In the present invention, a "carboxyl protecting group" refers to the protecting group which can be transformed into a carboxyl group via hydrolysis or deprotection. A carboxyl protecting group is preferably an alkyl group (e.g., methyl, ethyl, tert-butyl) or an aralkyl group (e.g., benzyl), and more preferably a tert-butyl group (tBu), a methyl group (Me), or an ethyl group (Et). In the present invention, a "protected carboxyl group" refers to the group formed via the protection of a carboxyl group with an appropriate carboxyl protecting group, and is preferably a methoxycarbonyl group, an ethoxycarbonyl group, a *t*-butoxycarbonyl group, or a benzyloxycarbonyl group. Said carboxyl protecting group can be removed through hydrolysis catalyzed by acids or alkalis, or through pyrolysis reactions occasionally; for example, *t*-butyl groups can be removed under mild acidic conditions, and benzyl groups can be removed by hydrogenolysis. The reagents used for the removal of carboxyl protecting groups are selected from the group consisting of TFA, H₂O, LiOH, NaOH, KOH, MeOH, EtOH, and combinations thereof, preferably the combination of TFA and H₂O, the combination of LiOH and MeOH, or the combination of LiOH and EtOH. A protected carboxyl group can undergo deprotection and then produce the corresponding free acid; said deprotection can be conducted in the presence of an alkali, and said alkali forms pharmaceutically acceptable salts with said free acid obtained from said deprotection.

In the present invention, the "amino protecting group" includes all the groups which can be used as common amino protecting groups, such as an aryl C₁₋₆ alkyl group, a C₁₋₆ alkoxy C₁₋₆ alkyl group, a C₁₋₆ alkoxycarbonyl group, an aryloxycarbonyl group, C₁₋₆ alkylsulfonyl group, an arylsulfonyl group, a silyl group, etc. An amino protecting group is preferably selected from the group consisting of a *t*-butoxycarbonyl group (Boc), a *p*-methoxybenzyloxycarbonyl group (Moz) and a 9-fluorenylmethoxycarbonyl group (Fmoc). The reagent used for removing amino protecting groups is selected from the group consisting of TFA, H₂O, LiOH, MeOH, EtOH and combinations thereof, preferably selected from the combination of TFA and H₂O, the combination of LiOH and MeOH, and the combination of LiOH and EtOH. The reagent used for removing the Boc protecting group is TFA or HCl/EA, preferably TFA. The reagent used for removing the Fmoc protecting group is the *N,N*-dimethylformamide (DMF) solution containing 20% piperidine.

In the present invention, said hydroxyl group protected by a hydroxyl protecting group is not particularly limited, e.g., an alcoholic hydroxyl group, a phenolic hydroxyl group, and the like. Wherein, said amino group protected by an amino protecting group is not particularly limited, selected from the group consisting of a primary amine, a secondary amine, a hydrazine, an amide, and the like. In the present invention, amino groups are not particularly limited, including but not limited to primary amino groups, secondary amino groups, tertiary amino groups, and quaternary ammonium ions.

In the present invention, the deprotection of protected hydroxyl groups is related to the type of hydroxyl protecting group. Said type of hydroxyl protecting group is not particularly limited; for example, benzyl groups, silyl ethers, acetals, or tert-butyl groups can be used to protect terminal hydroxyl groups, and the corresponding deprotection methods include the follows:
A: Deprotection of benzyl groups
   The deprotection of benzyl groups can be achieved via hydrogenation using a hydrogenation reduction catalyst and a hydrogen donor. As used herein, the water content should be less than 1% in order to facilitate the reaction. When the water content is more than 1%, the cleavage of polyethylene glycol chains will occur and generate low-molecular-weight polyethylene glycol with hydroxyl groups, which can participate in the subsequent polymerization reaction or functionalization reaction to introduce impurities into the target product and may even react with bio-related substances to change the properties of formulations.
   The catalyst for hydrogenation reduction is not particularly limited, preferably palladium or nickel, and its carrier is not particularly limited, preferably alumina or carbon, more preferably carbon. The amount of palladium used is 1 to 100 wt% of that of compounds containing protected hydroxyl groups, preferably 1 to 20 wt%. When the amount of palladium used is less than 1 wt%, both the rate of deprotection and the rate of conversion will decrease. The moieties that have not been deprotected are unable to participate in the subsequent polymerization or functionalization, resulting in a low ratio of functionalization of the final product. However, when the amount of palladium used exceeds 100 wt%, it is easy to cause the polyethylene glycol chains to be cleaved.
   The reaction solvent is not particularly limited, as long as both the starting materials and the products can be dissolved, but is preferably methanol, ethanol, ethyl acetate, tetrahydrofuran, or acetic acid, more preferably methanol. The hydrogen donor is not particularly limited, but is preferably hydrogen, cyclohexene, 2-propanol, or ammonium formate, etc. The reaction temperature is preferably in the range of 25 to 40°C. When the temperature is higher than 40 °C, the cleavage of polyethylene glycol chains is easy to occur. The reaction time is not particularly limited, which is negatively correlated with the amount of catalyst used, preferably 1 to 5 hours; when the reaction time is shorter than 1 hour, the conversion rate is relatively low; when the reaction time is longer than 5 hours, the cleavage of polyethylene glycol chains is easy to occur.
B: Deprotection of acetals and ketals
   The compounds used for protecting hydroxyl groups in the forms of acetals or ketals are preferably ethyl vinyl ether, tetrahydropyran, acetone, 2,2-dimethoxypropane, or benzaldehyde, etc. The deprotection of such acetals or ketals can be realized under an acidic condition wherein the pH of the solution is preferably 0 to 4. When the pH is higher than 4, the acidity is too weak for the protecting group to be completely removed. When the pH is lower than 0, the acidity is too strong so that the polyethylene glycol chain tends to be cleaved. The acid is not particularly limited, but preferably acetic acid, phosphoric acid, sulfuric acid, hydrochloric acid, or nitric acid, more preferably hydrochloric acid. The reaction solvent is not particularly limited as long as it allows the starting materials and the products to be dissolved, preferably water. The reaction temperature is preferably 0 to 30°C. When the temperature is lower than 0°C, the reaction rate is relatively slow, and the protecting group cannot be completely removed; when the temperature is higher than 30°C, the cleavage of polyethylene glycol chains is easy to occur under an acidic condition.
C: Deprotection of silyl ethers
   The protected hydroxyl groups in the forms of silyl ethers include trimethylsilyl ether, triethylsilyl ether, tert-butyldimethylsilyl ether, tert-butyldiphenylsilyl ether, etc. The deprotection of such silyl ethers uses compounds containing fluoride ions; wherein, said compounds are preferably selected from the group consisting of tetrabutylammonium fluoride, tetraethylammonium fluoride, hydrofluoric acid, and potassium fluoride, and more preferably selected from tetrabutylammonium fluoride and potassium fluoride. The molar equivalent of the fluorine-containing reagent used is 5 to 20 folds of that of the protected hydroxyl group, preferably 8 to 15 folds of that of the initiator. When the molar equivalent of the fluorine-containing reagent used is less than 5 folds of that of the protected hydroxyl group, the deprotonation might be incomplete. When the molar equivalent of the deprotection reagent used exceeds 20 folds of that of the protected hydroxyl group, the excess reagents or compounds will bring difficulty in the purification and possibly cause side reactions in subsequent steps. The reaction solvent is not particularly limited, as long as the reactants and the products can be dissolved, but is preferably an aprotic solvent and more preferably tetrahydrofuran or dichloromethane. The reaction temperature is preferably 0 to 30°C; when the temperature is lower than 0°C, the reaction rate is relatively slow, and the protecting group cannot be completely removed.
D: Deprotection of tert-butyl groups
   The deprotection of tert-butyl groups is carried out under an acidic condition wherein the pH of the solution is preferably 0 to 4. When the pH is higher than 4, the acidity is too weak for the protecting group to be completely removed; when the pH is lower than 0, the acidity is too strong, and the cleavage of polyethylene glycol chains is easy to occur. The acid is not particularly limited, preferably acetic acid, phosphoric acid, sulfuric acid, hydrochloric acid, or nitric acid, more preferably hydrochloric acid. The reaction solvent is not particularly limited as long as the starting materials and the products can be dissolved, preferably water. The reaction temperature is preferably 0°C to 30°C. When the temperature is lower than 0°C, the reaction rate is relatively slow, and the protective group cannot be completely removed; when the temperature is higher than 30°C, the cleavage of polyethylene glycol chains is easy to occur.

In the following preparation methods for linear functionalization of terminal hydroxyl groups of polyethylene glycol chains, it is preferred that q=0, q₁=1, and Z₁ is a 1,2-methylene group. When q is not 0, for example, when a linking group such as an amino acid group, a succinyl group, etc., is present between PEG and R₀₁, the prior art capable of generating Z₂ or Z₁ (including but not limited to alkylation, condensation, click reactions, etc.) can be used, and the preparation process can be carried out referring to the following linear functionalization methods.

### 2.5.3. Alkylation reaction

In the present invention, the alkylation reaction is preferably based on hydroxyl groups, mercapto groups, or amino groups, corresponding to the formation of ether bonds, thioether bonds, secondary amino groups, and tertiary amino groups, respectively. Examples are as follows:

### 2.5.3.1. Alkylation reaction between an alcohol substrate and a sulfonate or halide

An amine intermediate can be obtained via the nucleophilic substitution with a sulfonate or halide on an alcohol substrate under basic conditions. Wherein, the molar equivalent of the sulfonate or halide is 1 to 50 folds of that of the alcohol substrate, preferably 1 to 5 folds. When the molar equivalent of the sulfonate or halide is less than 1 fold of that of the alcohol substrate, the substitution might be incomplete and cause difficulty in the purification process. When the molar equivalent of the sulfonate or halide exceeds 50 folds of that of the alcohol substrate, the excess reagent tends to bring difficulty in the purification process, and might be brought into the subsequent step and therefore cause increased side reactions which further increase the difficulty of purification.

The resulting product is a mixture of ether intermediate, excess sulfonate, and excess halide, and can be purified by means such as anion exchange resin, osmosis, ultrafiltration, etc. Wherein, the anion exchange resin is not particularly limited, as long as the target product can undergo ion-exchange and adsorption in the resin, and is preferably the ion exchange resin of tertiary amines or quaternary ammonia salts, with the matrix being dextran, agarose, polyacrylate, polystyrene, or poly(diphenylethylene), etc. The solvents used for osmosis or ultrafiltration are not limited, generally water or an organic solvent. Said organic solvent is not particularly limited, as long as the product can be dissolved within, but is preferably dichloromethane, chloroform, or the like.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base used can be an organic base (e.g., triethylamine, pyridine, 4-dimethylaminopyridine, imidazole, diisopropylethylamine) or an inorganic base (e.g., sodium carbonate, sodium hydroxide, sodium bicarbonate, sodium acetate, potassium carbonate, potassium hydroxide), preferably an organic base, and more preferably triethylamine or pyridine. The molar equivalent of the base used is 1 to 50, preferably 1 to 10, and more preferably 3 to 5 folds of that of the sulfonate or halide.

### 2.5.3.2. Alkylation reaction between an amine substrate and a sulfonate or halide

An amine intermediate can be obtained via the nucleophilic substitution between an amine substrate and a sulfonate or halide under a basic condition. Wherein, the amount of the sulfonate or halide is 1 to 50 folds of that of the amine substrate, preferably 1 to 5 folds. When the amount of the sulfonate or halide is less than 1-fold of that of the amine substrate, the substitution may not sufficiently proceed and the purification tends to be difficult. When the amount of the sulfonate or halide exceeds 50 folds of that of the amine substrate, the excess reagent tends to cause difficulty in the purification process and might be brought to the subsequent steps to result in increased side reactions which further increase the difficulty of purification.

The resulting product is a mixture of amine intermediate, excess sulfonate, and excess halide, the mixture can be purified by purification means such as anion exchange resin, osmosis treatment, ultrafiltration treatment or the like. Wherein, the anion exchange resin is not particularly limited as long as the target product can undergo ion-exchange and adsorption in the resin, and is preferably the ion exchange resin of tertiary amines or quaternary ammonia salts, with the matrix being dextran, agarose, polyacrylate, polystyrene, or poly(diphenylethylene), etc. The solvents used for osmosis or ultrafiltration are not limited, generally water or an organic solvent. Said organic solvent is not particularly limited, as long as the product can be dissolved within, but is preferably dichloromethane, chloroform, or the like.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base used can be an organic base (e.g., triethylamine, pyridine, 4-dimethylaminopyridine, imidazole, diisopropylethylamine) or an inorganic base (e.g., sodium carbonate, sodium hydroxide, sodium bicarbonate, sodium acetate, potassium carbonate, potassium hydroxide), preferably an organic base, and more preferably triethylamine or pyridine. The molar equivalent of the base used is 1 to 50, preferably 1 to 10, and more preferably 3 to 5 folds of that of the sulfonate or halide.

### 2.5.3.3. Alkylation reaction between an amine substrate and an aldehyde derivative

The amine substrate reacts with an aldehyde derivative to obtain an imine intermediate, which is followed by obtaining an intermediate using reducing agents. Wherein, the molar equivalent of the aldehyde derivative is 1 to 20, preferably 1 to 2, and more preferably 1 to 1.5 folds of that of the amine substrate. When the molar equivalent of the aldehyde derivative exceeds 20 folds of that of the amine substrate, the excess reagent tends to cause difficulty in the purification process, and might be brought into the subsequent step and therefore increase difficulty in the purification. When the molar equivalent of the aldehyde derivative is less than 1 fold of that of the amine substrate, the reaction might be incomplete, causing further difficulty in the purification. Wherein, the resulting product can be obtained after purification by means such as cation exchange resin, osmosis, ultrafiltration, etc. Said cation exchange resin is not particularly limited, as long as it can undergo ion-exchange with quaternary ammonium cations and realize the isolation. The solvents used for osmosis or ultrafiltration treatment are not limited, generally water or an organic solvent. Said organic solvent is not particularly limited, as long as the product can be dissolved within, but is preferably dichloromethane, chloroform, or the like.

The reaction solvent is not limited, preferably an organic solvent such as methanol, ethanol, water, toluene, benzene, xylene, acetonitrile, ethyl acetate, tetrahydrofuran, chloroform, dichloromethane, dimethylsulfoxide, dimethylformamide, dimethylacetamide, etc., and more preferably water or methanol.

The reducing agent is not particularly limited, as long as the imine can be reduced to an amine, but is preferably sodium borohydride, lithium aluminum hydride, sodium cyanoborohydride, or Zn/AcOH, etc., and more preferably sodium cyanoborohydride. The amount of the reducing agent used is generally 0.5 to 50 folds and preferably 1 to 10 folds of that of the aldehyde derivative.

### 2.5.4. Linear dual end-functionalized PEG derivative biLPEG

Said biLPEG contains a functional group at one end, which can react with amino groups (primary amino groups or secondary amino groups), and forms the divalent linking group L₃ via coupling reaction.

The other end of said biLPEG has a structure identical or different from the target structure R, and the corresponding functional group is the same as or different from the target functional group. The functional group on this end of the biLPEG includes but is not limited to the functional groups in the aforementioned classes A~J, including the precursors of any aforementioned functional groups, variant forms with the functional groups as precursors, substituted forms, protected forms, deprotected forms, and the like.

**The functional groups at both ends of said biLPEG may be the same or different,** and said biLPEG is preferably a linear heterofunctional PEG derivative (biheteroLPEG) with two different functional groups at both ends. Wherein, in the present invention, a pair of heterofunctional groups which can exist simultaneously includes but is not limited to the group consisting of a hydroxyl group paired with a protected hydroxyl group, a hydroxyl group or a protected hydroxyl group with a non-hydroxyl reactive group belonging to classes A~H (e.g., an amino group, a protected amino group, an ammonium salt group, an aldehyde group, an active ester group, a maleimide group, a carboxyl group, a protected carboxyl group, an alkynyl group, a protected alkynyl group, an azido group, an alkenyl group, an acrylic acid group, an acrylate group, a methacrylate group, an epoxy group, an isocyanato group, etc.), a hydroxyl group or a protected hydroxyl group paired with a functional group or derivative thereof belonging to the classes I-J (such as a targeting group, a photosensitive group, etc.), an active ester group paired with a maleimide group, an active ester group paired with an aldehyde group, an active ester group paired with an azido group, an active ester group paired with an alkynyl group or a protected alkynyl group, an active ester group paired with an acrylate group, an active ester group paired with a methacrylate group, an active ester group paired with an acrylic acid group, a maleimide group paired with an azido group, a maleimide group paired with an alkynyl group or a protected alkynyl group, a maleimide group paired with an acrylate group, a maleimide group paired with a methacrylate group, a maleimide group paired with an acrylic acid group, a maleimide group paired with a carboxyl group, a maleimide group paired with an amino group or a protected amino group or an ammonium salt group, a maleimide group paired with an isocyanato group, a maleimide group paired with a protected mercapto group, an aldehyde group paired with an azido group, an aldehyde group paired with an acrylate group, an aldehyde group paired with a methacrylate group, an aldehyde group paired with an acrylic acid group, an aldehyde group paired with an epoxy group, an aldehyde group paired with a carboxyl group, an aldehyde group paired with an alkynyl group or a protected alkynyl group, an azido group paired with a mercapto group or a protected mercapto group, an azido group paired with an amino group or a protected amino group or an ammonium salt group, an azido group paired with an acrylate group, an azido group paired with a methacrylate group, an azido group paired with an acrylic acid group, an azido group paired with a carboxyl group, an acrylate group paired with an amino group or a protected amino group or an ammonium salt group, an acrylate group paired with an isocyanato group, an acrylate group paired with an epoxy group, an acrylate group paired with a methacrylate group, an acrylate group paired with a carboxyl group, a methacrylate group paired with a carboxyl group, a methacrylate group paired with an amino group or a protected amino group or an ammonium salt group, a methacrylate group paired with an isocyanato group, a methacrylate group paired with an epoxy group, an alkynyl group or a protected alkynyl group paired with an amino or a protected amino group or an ammonium salt group, an alkynyl group or a protected alkynyl group paired with an isocyanato group, an alkynyl group or a protected alkynyl group paired with an acrylate group, an alkynyl group or a protected alkynyl group paired with a methacrylate group, an alkynyl group or a protected alkynyl group paired with acrylic acid group, an alkynyl group or a protected alkynyl group paired with an epoxy group, an alkynyl group or a protected alkynyl group paired with a carboxyl group, a protected alkynyl group paired with an azido group, an acrylic acid group paired with an isocyanato group, an acrylic acid group paired with an acrylate group, an acrylic acid group paired with an epoxy group, an acrylic acid group paired with a carboxyl group, a carboxyl group paired with a mercapto group or a protected mercapto group, a carboxyl group paired with an amino group or a protected amino group or an ammonium salt group, a carboxyl group paired with an isocyanato group, a carboxyl group paired with an epoxy group, an amino group or a protected amino group or an ammonium salt group paired with a mercapto or a protected mercapto group, a targeting group paired with a non-hydroxyl reactive group, a photosensitive group paired with a non-hydroxyl reactive group, and the like. Wherein, said active ester groups include but are not limited to any succinimidyl active ester groups (e.g., a succinimidyl carbonate group), p-nitrophenyl active ester groups, o-nitrophenyl active ester groups, benzotriazole active ester groups, 1,3,5-trichlorophenyl active ester groups, 1,3,5-trifluorophenyl active ester groups, pentafluorophenyl active ester groups, imidazole active ester groups, 2-thioxo-thiazolidine-3-carboxylate groups, and 2-thione-pyrrolidine-1-carboxylate groups, etc., in the present invention; said amino groups include primary amino groups and secondary amino groups. Said ammonium salt is preferably a hydrochloride form of an amino group, such as NH₂HCl.

### Said biLPEG can be polydisperse or monodisperse.

When a biLPEG is polydisperse, the polydispersity index is not particularly limited, while the polydispersity index of the starting material is preferably below 1.15, more preferably below 1.10, more preferably below 1.08, and more preferably below 1.05. The lower the PDI is, the more uniform the molecular weight and the narrower the molecular weight distribution, which leads to a higher quality of modified products when applied in the modification of molecules such as drugs and thus meets the practical needs to a greater extent.

When a biLPEG is monodisperse, PDI=1 and a PEGylated lipid with a defined molecular structure and molecular weight can be obtained. The products prepared with monodisperse starting materials have a more uniform molecular weight distribution; however, due to the limitation of preparation methods, the molecular weight is mostly limited, and the steps are lengthy. The advantage of using polydisperse starting materials is to provide a simple route and a larger range for molecule-weight adjustment.

The methods for preparing monodisperse polyethylene glycol chains can employ techniques in the prior art, including but not limited to the following literatures "J. Org. Chem. 2006, 71, 9884-9886" and cited references therein, "Angew. Chem. 2009, 121, 1274-1278" and cited references therein, "Expert Rev. Mol. Diagn. 2013, 13(4), 315-319" and cited references therein, "Angew. Chem. Int. Ed. 2014, 53, 6411-6413" and cited references therein, "Bioorganic & Medicinal Chemistry Letters, 2015, 25:38-42" and cited references therein, "Angew. Chem. Int. Ed., 2015, 54:3763-3767" and cited references therein, etc.

### 2.5.5. Linear end-functionalization of polyethylene glycol chain

The method for linear functionalization of the terminal of a polyethylene glycol chain is not particularly limited, but is relevant to the type of the final functional group or its protected form. It can be the linear functionalization of the terminal hydroxyl group of a polyethylene glycol chain, or the conversion of a reactive group into the target functional group or its protected form, or the combination of both.

The method for linear functionalization of the terminal hydroxyl group of a polyethylene glycol chain, starting from the terminal hydroxyl group of the polyethylene glycol chain, obtains a functional group of classes A~J or its protected form after functionalization. The specific preparation method is as documented in the paragraphs from [0960] to [1205] of the document CN104530417A. The general formula of the reaction is as follows: 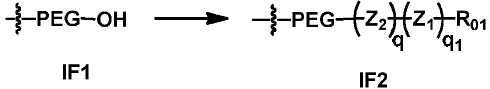 wherein, the structure of -PEG-OH is -(CH₂CH₂O)ₙCH₂CH₂OH, and n is n₁-1; the definitions of q, Z₂, q₁, Z₁, and R₀₁ are the same as those described above.

**The conversion of reactive groups into the target functional groups or protected forms thereof,** can be achieved by any of the following approaches:
Approach 1: Direct modification. The target functional group or its protected form can be obtained via direct modification of a reactive group. Examples include, the conversion of a carboxyl group to an acyl halide group, a hydrazide group, an ester group, a thioester group, or a dithioester group, the conversion of a hydroxyl group, a mercapto group, an alkynyl group, an amino group, a carboxyl group, or the like to the corresponding protected form, and so on. Another example is, the modification of a hydroxyl group, an amino group, or the like with an anhydride.

Approach 2: Coupling reaction between two reactive groups. Said coupling reaction uses a heterofunctional reagent as the starting material containing 1 type of reactive group and the target functional group or its protected form, to introduce the target functional group or its protected form via the reaction between said reactive group and the terminal reactive group of the polyethylene glycol chain. The reaction between two reactive groups is not particularly limited with respect to the manner and method of the reaction; wherein, the reaction conditions are related to the type of the divalent linking group formed in the reaction, and the prior art can be employed, for example, alkylation, addition reaction of alkenes, addition reaction of alkynes, Schiff base reaction-reduction reaction, condensation reaction, etc. Wherein, the alkylation reaction is preferably selected from those based on a mercapto group or an amino group, corresponding to the formation of a thioether bond, a secondary amino group or a tertiary amino group, respectively. Wherein, the condensation reaction includes but is not limited to those forming an ester group, a thioester group, an amide group, an imine bond, a hydrazone bond, a carbamate group, and the like. Examples also include, introducing the target functional group or its protected form via click reactions by using heterofunctional reagents as the starting materials containing not only an azido group, an alkynyl group, an alkenyl group, a trithioester group, a mercapto group, a dienyl group, a furyl group, a 1,2,4,5-tetrazinyl group, a cyanate group, or the like, but also the target functional group or its protected form. The reaction between two reactive groups brings the formation of new bonds. The representative newly formed divalent linking groups include an amide bond, a urethane bond, an ester bond, a secondary amino bond, a thioether bond, a triazole group, and the like.

Approach 3: The target functional group or its protected form can be obtained via the combination of direct modifications and coupling reactions.

In the present invention, starting materials used in every preparation method can be obtained by purchase or synthetic means.

The intermediates and end-products prepared in the present invention can all be purified by purification methods including but not limited to extraction, recrystallization, adsorption treatment, precipitation, reverse precipitation, membrane dialysis, supercritical extraction and the like. The characterization of the structure and molecular weight of the end-products can use characterization methods including but not limited to NMR, electrophoresis, UV-visible spectrophotometer, FTIR, AFM, GPC, HPLC, MALDI-TOF, circular dichroism spectroscopy, and the like.

### 3. Cationic liposomes

### 3.1. Cationic liposomes

In the present invention, provided herein is a cationic liposome containing any foregoing PEGylated lipid whose structure is represented by the general formula (2).

In one specific embodiment of the present invention, the cationic liposome preferably contains not only the PEGylated lipid whose structure is represented by the general formula (2), but also one or more types of lipids selected from the group consisting of neutral lipid, steroid lipid, and cationic lipid; more preferably simultaneously contains three types of lipids as neutral lipid, steroid lipid, and cationic lipid.

In one specific embodiment of the present invention, neutral lipids in the cationic liposomes preferably include but are not limited to 1,2-dilinoleoyl-sn-glycero-3-phosphocholines (DLPC), 1,2-dimyristoleoyl-sn-glycero-3-phosphocholines (DMPC), 1,2-dioleoyl-sn-glycero-3-phosphocholines (DOPC), 1,2-dipalmitoyl-sn-glycero-3-phosphocholines (DPPC), 1,2-distearoyl-sn-glycero-3-phosphatidylcholines (DSPC), 1,2-diundecanoyl-sn-glycero-3-phosphatidylcholines (DUPC), 1-plamitoyl-2-oleoyl-sn-glycero-3-phosphocholines (POPC), 1,2-di-*O*-octadecenyl-sn-glycero-3-phosphatidylcholines (18:0 Diether PC), 1-oleoyl-2-cholesterylhemisuccinyl-sn-glycero-3-phosphocholines (OChemsPC), 1-*O*-hexadecyl-sn-glycero-3-phosphatidylcholines (C16 Lyso PC), 1,2-dilinolenoyl-sn-glycero-3-phosphatidylcholines, 1,2-diarachidonoyl-sn-glycero-3-phosphatidylcholines, 1,2-didecosahexaenoyl-sn-glycero-3-phosphocholines, 1,2-dioleoyl-sn-glycero-3-phosphoethanolamines (DOPE), 1,2-diphytanyl-sn-glycero-3-phosphoethanolamines (ME 16.0 PE), 1,2-distearoyl-sn-glycero-3-phosphoethanolamines, 1,2-dilinoleoyl-sn-glycero-3-phosphoethanolamines, 1,2-dilinolenoyl-sn-glycero-3-phosphoethanolamines, 1,2-diarachidonoyl-sn-glycero-3-phosphoethanolamines, 1,2-didecosahexaenoyl-sn-glycero-3-phosphoethanolamines, 1,2-dioleoyl-sn-glycero-3-phospho-rac-(1-glycerol) sodium salts (DOPG), dioleoyl phosphatidylserines (DOPS), dipalmitoylphosphatidylglycerols (DPPG), palmitoyloleoyl phosphatidylethanolamines (POPE), distearoyl phosphatidylethanolamines (DSPE), dipalmitoyl phosphatidylethanolamines (DPPE), dimyristoleoyl phosphoethanolamines (DMPE), 1-stearoyl-2-oleoyl-stearoylethanolamines (SOPE), 1-stearoyl-2-oleoyl-phosphatidylcholines (SOPC), sphingomyelins, phosphatidylcholines, phosphatidylethnolamines, phosphatidylserines, phosphatidylinositols, phosphatidic acids, palmitoyloleoyl phosphatidylcholines, lysophosphatidylcholines, lysophosphatidylethanolamines (LPE), and combinations thereof.

In one specific embodiment of the present invention, PEGylated lipids in the cationic liposomes are, apart from the PEGylated lipids represented by the general formula (2), preferably selected from the group consisting of PEG500-dipalmitoylphosphatidylcholine, PEG2000-dipalmitoylphosphatidylcholine, PEG500-distearylphosphatidylethanolamine, PEG2000-distearylphosphatidylethanolamine, PEG500-1,2-oleoylphosphatidylethanolamine, PEG2000-1,2-oleoylphosphatidylethanolamine and PEG2000-2,3-distearoylglycerol, preferably selected from the group consisting of PEG2000-dipalmitoylphosphatidylcholine, PEG2000-distearylphosphatidylethanolamine, and PEG2000-1,2-acylphosphatidylethanolamine, more preferably PEG2000-distearylphosphatidylethanolamine.

In one specific embodiment of the present invention, steroid lipids in the cationic liposomes are preferably selected from the group consisting of cholesterols, coprostanols, sitosterols, ergosterols, campesterols, stigmasterols, brassicasterols, tomatidines, ursolic acids, α-tocopherols, and mixtures thereof.

In one specific embodiment according to the claimed invention, the structure of a cationic lipid in a cationic liposome is preferably represented by the following general formula (1): 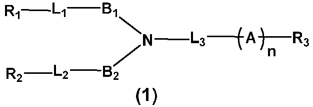
wherein, N is the nitrogen branching center;
L₁ and L₂ are divalent linking groups, each independently selected from the group consisting of -OC(=O)-, -C(=O)O-, -OC(=O)O-, -C(=O)-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; wherein, R_{c} is a hydrogen atom or a C₁₋₁₂ alkyl group;
L₃ is a linking bond or -L₄-Z-L₅-; said L₄ and L₅ are carbon chain linking groups, each independently -(CRₐR_{b})ₜ- or -(CRₐR_{b})ₜ-(CRₐR_{b})ₚ-(CRₐR_{b})_{q}-; wherein, t, p and q are each independently an integer from 0 to 12, Rₐ and R_{b} are each independently a hydrogen atom or a C₁₋₁₂ alkyl group; said Z is a linking bond, or a divalent linking group selected from the group consisting of -OC(=O)-, -C(=O)O-, -OC(=O)O-, -C(=O)-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-;
B₁ and B₂ are each independently a C₁₋₁₂ alkylene group;
R₁ and R₂ are each independently a C₂₋₃₀ aliphatic hydrocarbon group;
R₃ is a hydrogen atom, an alkyl group, an alkoxy group, -C(=O)R_{d}, -C(=O)ORₐ, -OC(=O)Rₐ, or -OC(=O)OR_{d},; wherein, R_{d} is a C₁₋₁₂ alkyl group,;
A is selected from the group consisting of -(CRₐR_{b})ₛO-, -O(CRₐR_{b})ₛ-, -(CRₐR_{b})ₛS-, -S(CRₐR_{b})ₛ-, -(CRₐR_{b})ₛO(CRₐR_{b})ₛS-, -(CRₐR_{b})ₛS(CRₐR_{b})ₛO-, -(CRₐR_{b})ₛNR_{c}(CRₐR_{b})ₛS-, -(CRₐR_{b})ₛS(CRₐR_{b})ₛNR_{c}-, -(CRₐR_{b})ₛNR_{c}(CRₐR_{b})ₛO-, and -(CRₐR_{b})ₛO(CRₐR_{b})ₛNR_{c}-; wherein, s is 2, 3 or 4, Rₐ and R_{b} are each independently a hydrogen atom or a C₁₋₁₂ alkyl group;
when A is -(CRₐR_{b})ₛO- or -O(CRₐR_{b})ₛ-, n is an integer from 2 to 6; when A is not -(CRₐR_{b})ₛO- or -O(CRₐR_{b})ₛ-, n is an integer from 1 to 6;
said alkyl group, alkylene group, alkoxy group, and aliphatic hydrocarbon group are each independently substituted or unsubstituted.

In one specific embodiment of the present invention, the structure of a cationic lipid in a cationic liposome is represented by the general formula (1), and preferably selected from the group consisting of the following structures:

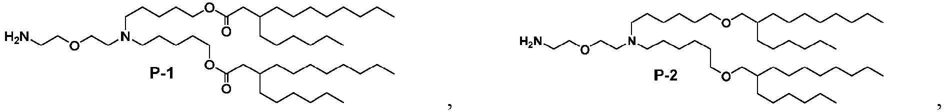

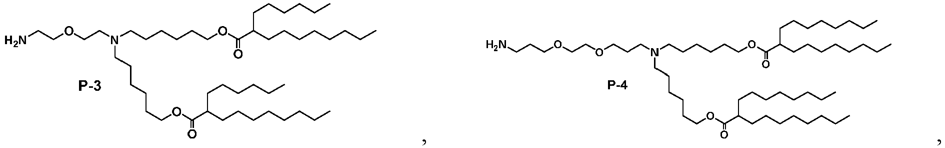

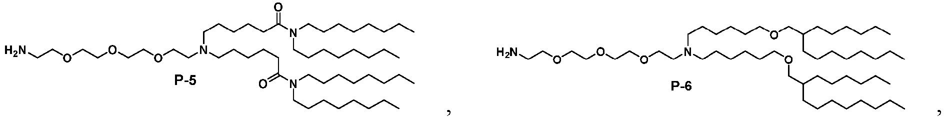

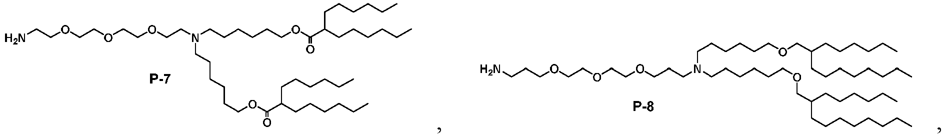

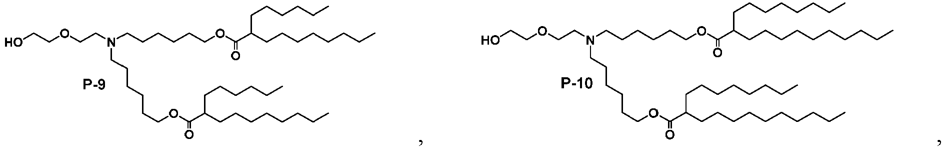

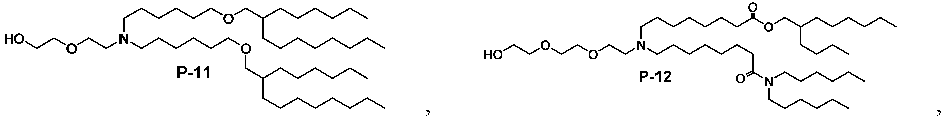

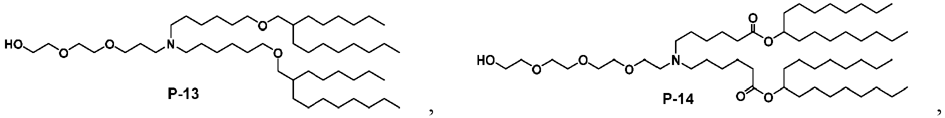

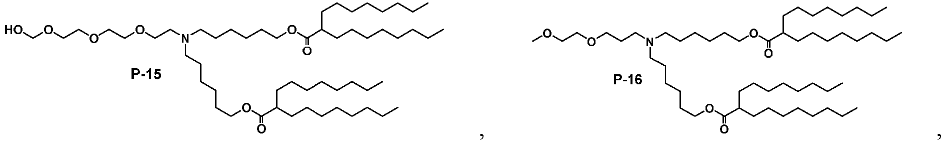

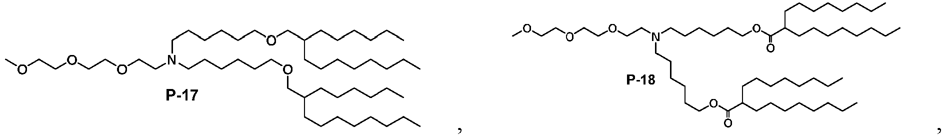

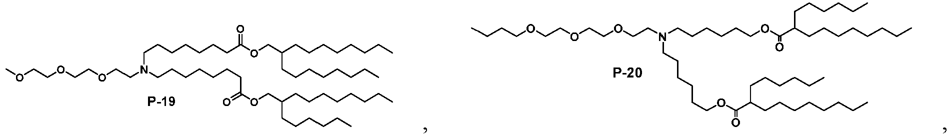

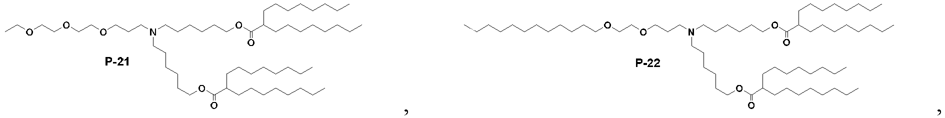

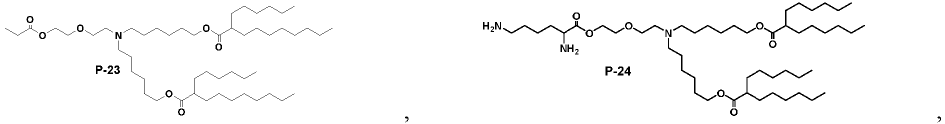

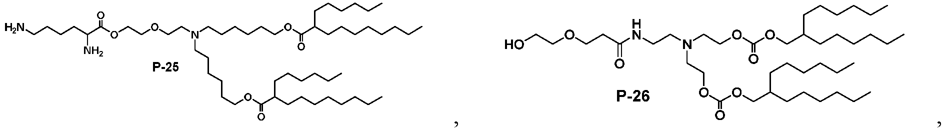

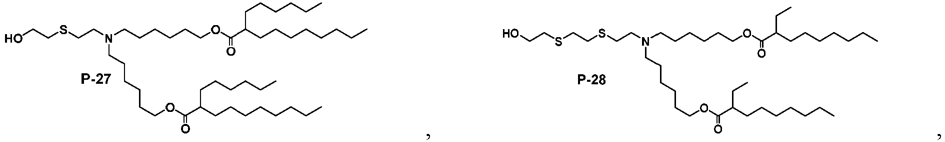

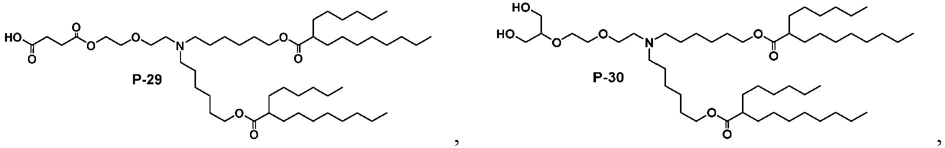

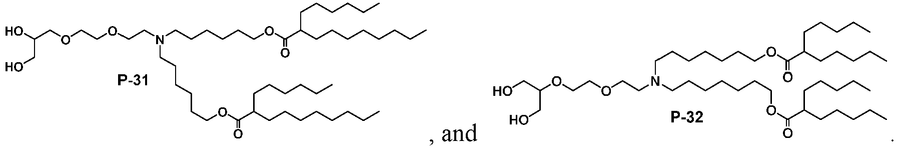

In one specific embodiment of the present invention, the cationic lipids in the cationic liposomes can be selected from not only the cationic lipids represented by the general formula (1), but also the group consisting of *N,N*-dioleyl-*N,N*-dimethylammonium chloride (DODAC), *N,N*-distearyl-*N,N*-dimethylammonium bromide (DDAB), 1,2-dioleoyl-3-trimethylammonium-propane (DOTAP), *N*-[1-(2,3-dioleoyloxy)propyl]-*N,N,N*-trimethylammonium chloride (DOTMA) 1,2-dioleyloxy-*N,N*-dimethylaminopropane (DODMA), 3-(didodecylamino)-*N1,N1*,4-tridodecyl-1-piperazineethanamine (KL10), *N1*-[2-(didodecylamino)ethyl]-*N1,N4,N4*-tridodecyl-1,4-piperazinediethanamine (KL22), 14,25-ditridecyl-15, 18,21,24-tetraaza-octatriacontane (KL25), 1,2-dilinoleyloxy-*N,N*-dimethylaminopropane (DLin-DMA), 2,2-dilinoleyl-4-dimethylaminomethyl-[1,3]-dioxolane (DLin-K-DMA), heptatriaconta-6,9,28,31-tetraen-19-yl 4-(dimethylamino)butanoate (DLin-MC3-DMA), 2,2-dilinoleyl-4-(2-dimethylaminoethyl)-[1,3]-dioxolane (DLin-KC2-DMA), and mixtures thereof.

In one specific embodiment of the present invention, any foregoing cationic liposome preferably contains 20% to 80% cationic lipids represented by the general formula (1), 5% to 15% neutral lipids, 25% to 55% steroid lipids, and 0.5% to 10% PEGylated lipids, and said percentage is the molar percentage of each lipid relative to the total lipids in a solution containing the solvent.

In one specific embodiment of the present invention, in any foregoing cationic liposome, the molar percentage of cationic lipids relative to the total lipids in a solution containing the solvent is preferably 30% to 65%, more preferably about 35%, 40%, 45%, 46%, 47%, 48%, 49%, 50%, or 55%.

In one specific embodiment of the present invention, in any foregoing cationic liposomes, the molar percentage of neutral lipids relative to the total lipids in a solution containing the solvent is preferably 7.5% to 13%, more preferably about 8%, 9%, 10%, 11%, or 12%.

In one specific embodiment of the present invention, in any foregoing cationic liposomes, the molar percentage of steroid lipids relative to the total lipids in a solution containing the solvent is preferably 35% to 50%, more preferably about 40%, 41%, 42%, 43%, 44%, 45%, 46%, 47%, 48%, 49%, or 50%.

In one specific embodiment of the present invention, in any foregoing cationic liposomes, the molar percentage of PEGylated lipids relative to the total lipids in a solution containing the solvent is preferably 0.5% to 5%, more preferably 1% to 3%, more preferably about 1.5%, 1.6%, 1.7%, 1.8%, or 1.9%.

### 3.2. Preparation of cationic liposomes

In the present invention, cationic liposomes can be prepared by the following methods, including but not limited to thin-film dispersion method, ultrasonic dispersion method, reverse phase evaporation method, freeze-drying method, freeze-thaw method, double emulsion method and/or injection method, preferably thin-film dispersion method, ultrasonic dispersion method and/or reverse phase evaporation method.

In the preparation methods of the cationic liposomes of the present invention, said thin-film dispersion method could include the following steps:
Step (1): weigh cationic lipids, steroid lipids, neutral lipids, and PEGylated lipids, dissolve them fully in an organic solvent, shake well, remove the organic solvent by rotary evaporation under reduced pressure to form an oil film, and dry with a vacuum pump to remove the organic solvent;
Step (2): add a phosphate buffer solution containing dissolved cryoprotectants, and use water-bath ultrasonication to obtain a translucent emulsion;
Step (3): add the emulsion to a high-pressure homogenizer for overpressure, and then add the overpressurized emulsion to the liposome extruder to pass through films to obtain cationic liposomes;
Step (4): optionally, dry said cationic liposomes in a freeze dryer to obtain cationic liposome powders;
wherein, said organic solvent is preferably selected from dichloromethane, chloroform, and/or methanol, more preferably chloroform and methanol; the rotational speed of said rotary evaporation under reduced pressure is preferably 30 to 300 rpm, more preferably 50 to 200 rpm, and most preferably 100 to 170 rpm; the temperature of said rotary evaporation under reduced pressure is preferably 10 to 200°C, more preferably 20 to 200°C, and most preferably 40 to 80°C;
the time of said drying with a vacuum pump is preferably 1 to 72 h, more preferably 5 to 48 h, and most preferably 15 to 36 h;
the mass concentration of said cryoprotectants dissolved in a phosphate buffer solution is preferably 0.1 to 80%, preferably 1 to 50 %, and more preferably 5 to 20%;
the frequency of said water-bath ultrasonication is preferably 10 to 300 kHz, more preferably 30 to 200 kHz, and most preferably 60 to 150 kHz;
the time of said water-bath ultrasonication is preferably 0.1~5 h, more preferably 0.2 to 2 h, and most preferably 0.25 to 1 h;
the pressure of said high-pressure homogenizer is preferably 50 to 240 MPa, more preferably 80 to 200 MPa, and most preferably 100 to 150 MPa;
the times of said overpressure in the high-pressure homogenizer is preferably any integer from 1 to 50, more preferably any integer from 3 to 20, and most preferably any integer from 5 to 10;
the pressure of said liposome extruder is preferably 50 to 300 MPa, more preferably 80 to 250 MPa, and most preferably 120 to 200 MPa;
the times of said passing through films in the liposome extruder is preferably any integer from 1 to 50, more preferably any integer from 3 to 30, and most preferably any integer from 5 to 20;
the time of said drying in a freeze dryer is preferably 1 to 120 h, more preferably 5 to 72 h, and most preferably 10 to 36 h.

In the preparation methods of the cationic liposomes of the present invention, the ratio of the cationic liposomes to the phosphate buffer solution containing dissolved cryoprotectants could be 1 mg:(0.1~100) mL, preferably 1 mg:(0.3~50) mL, and more preferably 1 mg:(0.5~5) mL.

### 4. Cationic liposome-nucleic acid pharmaceutical composition

In one embodiment of the present invention, provided herein is a cationic liposome pharmaceutical composition containing any foregoing cationic liposome and a drug, wherein the cationic liposome contains any foregoing PEGylated lipid whose structure is represented by the general formula (2).

In one specific embodiment of the present invention, in a cationic liposome pharmaceutical composition, the drug is preferably a nucleic acid drug or an anti-tumor agent; said nucleic acid drug is selected from the group consisting of DNA, antisense nucleic acid, plasmid, mRNA (messenger RNA), interfering nucleic acid, aptamer, miRNA inhibitor (antagomir), microRNA (miRNA), ribozyme, and small interfering RNA (siRNA), and preferably the group consisting of RNA, miRNA and siRNA.

In one specific embodiment of the present invention, the cationic liposome pharmaceutical composition is preferably used as a drug, which is selected from the group consisting of drugs for treating cancer, drugs for treating malignant tumor, anti-infectious agents, antiviral agents, antifungal agents, vaccines, and drugs for treating esophageal carcinoma, gastric cancer, large intestine cancer, nasopharyngeal cancer, brain tumor, cervical carcinoma, leukemia, bone cancer, AIDS, and virus infection.

In the present invention, said drugs are further preferably selected from those including but not limited to doxorubicin, mitoxantrone, camptothecin, cisplatin, bleomycin, cyclophosphamide, streptozotocin, actinomycin D, vincristine, vinblastine, cytosine arabinoside, anthracycline, nitrogen mustard, tioteppa, chlorambucil, rachelmycin, melphalan, carmustine, romustine, busulfan, dibromannitol, mitomycin C, cis-diammineplatinum(II) dichloride, methotrexate, 6-mercaptopurine, 6-thioguanine, cytosine arabinoside, 5-fluorouracil dacarbazine, dibucaine, chlorpromazine, propranolol, demorol, labetalol, clonidine, hydralazine, imipramine, amitriptyline, doxepin, phenytoin, diphenhydramine, chlorpheniramine, promethazine, gentamicin, ciprofloxacin, cefoxitin, miconazole, terconazole, econazole, isoconazole, butoconazole, clotrimazole, itraconazole, nystatin, naftifine, amphotericin B, antiparasitic agents, hormones, hormone antagonists, immunomodulators, neurotransmitter antagonists, glaucoma drugs, vitamins, tranquilizers, imaging agents, taxol, cytochalasin B, gramicidin D, ethidium bromide, emetine, mitomycin, etoposide, teniposide, colchicine, daunorubicin, quinizarin, mithramycin, 1-dihydrotestosterone, glucocorticoid, procaine, tetracaine, lidocaine, puromycin, and maytansinoid.

In one specific embodiment of the present invention, the N/P ratio of said cationic liposomes to said nucleic acids is preferably (0.1~100):1, more preferably (0.2~30):1, and most preferably (0.5~20):1.

In one specific embodiment of the present invention, the working solution of said formulation of nucleic acid pharmaceutical composition is preferably deionized water, ultrapure water, phosphate buffer, or physiological saline, more preferably phosphate buffer or physiological saline, and most preferably physiological saline; the ratio of the cationic liposomes to the working solution is preferably (0.05~20) g:100 mL, more preferably (0.1~10) g:100 mL, and most preferably (0.2~5) g:100 mL.

### 5. Formulation of cationic liposome pharmaceutical composition

In the present invention, provided herein is a formulation of cationic liposome pharmaceutical composition containing any foregoing cationic liposome pharmaceutical composition and pharmaceutically acceptable diluents or excipients; said diluents or excipients are preferably selected from the group consisting of deionized water, ultrapure water, phosphate buffer, and physiological saline, more preferably selected from phosphate buffer and physiological saline, and most preferably physiological saline.

In the present invention, the preparation method of the formulation of cationic liposome-nucleic acid pharmaceutical composition includes the following steps:
Step (1): equilibrate said cationic liposome in said diluents or excipients;
Step (2): complex a nucleic acid drug with the equilibrated mixture of the cationic liposome and the diluents or excipients;
wherein, the time of said equilibration is 0.1~12 h, preferably 0.2~6 h, and more preferably 0.5~3 h; the time of said complexation is 0.1~12 h, preferably 0.2~5 h, and more preferably 0.5~2 h.

The following is a further description with specific examples of the preparation methods of PEGylated lipids, cationic liposomes, and cationic liposome pharmaceutical compositions, and the biological activity assays for cationic liposome pharmaceutical compositions. The specific examples are for further illustration of the present invention, not limiting the protected scope of present invention. Wherein, in the embodiments of preparing PEGylated lipids, the structures of end-products were characterized by NMR, and the molecular weight was determined by MALDI-TOF.

### Example-1: Synthesis of PEGylated lipids E1-1 and E1-3

### Example-1.1: Synthesis of methoxy-PEGylated lipid E1-1

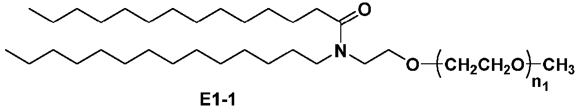

**E1-1** corresponds to the general formula (2), wherein, R₁ is a tridecyl group, R₂ is a tetradecyl group, B₃ and B₄ are both linking bonds, L₇ is a carbonyl group, L₈ is a linking bond, L₃ is -CH₂CH₂-, A is -OCH₂CH₂-, Rₐ and R_{b} are both hydrogen atoms, s is 2, n₁≈45, R is a methoxy group, and the total molecular weight is approximately 2460 Da.

The preparation process is as follows:
Step a: Compound **S1-1** (20.00 g, 10.0 mmol, Mw≈2000, n₁≈45, PDI=1.03) and toluene (200 mL) underwent the azeotropic water removal at 140°C. After 60 mL of solvent was steamed out, the temperature of reaction solution was cooled to room temperature. Triethylamine **(TEA,** 2.02 g, 20.0 mmol) and methyl sulfonyl chloride (**MsCl**, 2.05 g, 18.0 mmol) were added, and the reaction was stirred at room temperature overnight. After completion of the reaction, the reaction solution was poured into water (200 mL) and extracted twice with EtOAc (100 mL*2). The water phase was retained and extracted twice with dichloromethane (100 mL*2). The organic phases were combined, dried, filtered, concentrated, dissolved in isopropanol at 50°C, recrystallized in an ice bath, and filtered to obtain the compound **S1-2** (18.00 g).
Step b: The above compound **S1-2** (18.00 g,9.0 mmol) was added into 80 mL water and stirred at room temperature to dissolve. Potassium carbonate **(K₂CO₃,** 12.42 g, 90.0 mmol), compound **S1-3** (9.58 g, 45.0 mmol), and **tetra-*n*-butylammonium bromide (TBABr,** 0.29 g, 0.9 mmol) were added, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), retained, dried with anhydrous sodium sulfate, filtered, and concentrated to obtain the crude product of **S1-4.** Furthermore, the crude product was purified by column chromatography, concentrated, and dried with an oil pump to obtain the target compound **S1-4** (12.00 g).
Step c: The above compound **S1-4** (11.26 g, 5.0 mmol), **S1-5**(1.95 g, 6.0 mmol), and TEA (0.76 g, 7.5 mmol) were dissolved in dichloromethane (100 mL), followed by stirring at room temperature overnight. The reaction solution was concentrated, dissolved in 100 mL water, and extracted twice with EtOAc (100 mL*2). The water phase was retained, added with sodium chloride, and extracted twice with dichloromethane (100 mL*2). The organic phases were combined, backwashed once with saturated sodium chloride aqueous solution (100 mL), dried with anhydrous MgSO₄, filtered, and concentrated to obtain the crude product. The crude product was purified by column chromatography, concentrated, and dried with an oil pump to obtain the target compound **E1-1**(10.1 g). ¹H NMR (500 MHz, CDCl₃) δ: 3.84-3.45 (m, 182H), 3.37 (s, 3H), 3.35 (t, 2H), 3.18 (t, 2H), 2.27 (t, 2H), 1.56-1.40 (m, 4H), 1.36-1.18 (m, 42H), 0.87 (t, 6H). The molecular weight of **E1-1** was determined to be 2461 Da by MALDI-TOF, PDI=1.03.

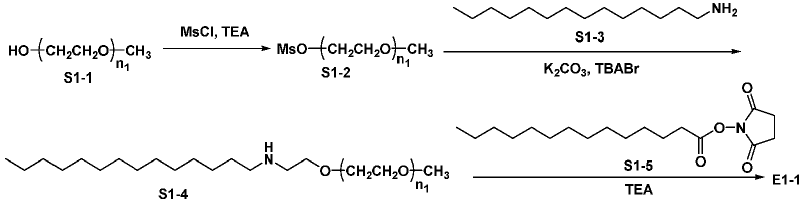

### Example-1.2: Synthesis of hydroxy-PEGylated lipid E1-3

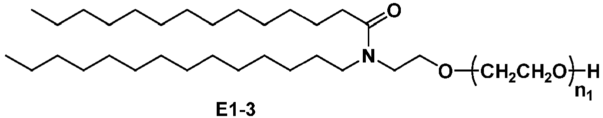

**E1-3** corresponds to the general formula (2), wherein, R₁ is a tridecyl group, R₂ is a tetradecyl group, B₃ and B₄ are both linking bonds, L₇ is a carbonyl group, L₈ is a linking bond, L₃ is -CH₂CH₂-, A is -OCH₂CH₂-, Rₐ and R_{b} are both hydrogen atoms, s is 2, n₁≈45, R is a hydroxy group, and the total molecular weight is approximately 2450 Da.

The preparation process is as follows:
Step a: Compound **S1-6** (19.95 g, 9.5 mmol, mPEG-OH, Mw≈2100, n₁≈45, PDI=1.03) and toluene (200 mL) underwent the azeotropic water removal at 140°C. After 60 mL of solvent was steamed out, the temperature of reaction solution was cooled to room temperature. TEA (2.02 g, 20.0 mmol) and MsCl (2.05 g, 18.0 mmol) were added, and the reaction was stirred at room temperature overnight. After completion of the reaction, the reaction solution was poured into water (200 mL), extracted twice with EtOAc (100 mL*2). The water phase was retained and extracted twice with dichloromethane (100 mL*2). The organic phases were combined, dried, filtered, concentrated, dissolved in isopropanol at 50°C, recrystallized in an ice bath, and filtered to obtain the compound **S1-7** (18.93 g).
Step b: The above compound **S1-7** (18.00 g, 8.5 mmol) was added into 80 mL water and stirred at room temperature to dissolve. Potassium carbonate (12.42 g, 90.0 mmol), compound **S1-3** (9.58 g, 45.0 mmol), and tetra-*n*-butylammonium bromide (0.29 g, 0.9 mmol) were added, and the reaction was stirred at room temperature for 72 h. After completion of the reaction, the reaction solution was extracted twice with dichloromethane (100 mL*2). The organic phases were combined and backwashed once with saturated sodium chloride aqueous solution (100 mL). The organic phase was retained, dried with anhydrous sodium sulfate, filtered, and concentrated to obtain the crude product of **S1-8**. The crude product was purified by column chromatography, concentrated, and dried with an oil pump to obtain the target compound **S1-8** (12.93 g).
Step c: The above compound **S1-8** (10.00 g, 4.3 mmol), **S1-5** (2.05 g, 6.3 mmol), and TEA (0.76 g, 7.5 mmol) were dissolved in dichloromethane (100 mL), followed by stirring at room temperature overnight. The reaction solution was concentrated, dissolved in 100 mL water, and extracted twice with EtOAc (100 mL*2). The water phase was retained, added with sodium chloride, and extracted twice with dichloromethane (100 mL*2). The organic phases were combined, backwashed once with saturated sodium chloride aqueous solution (100 mL), dried with anhydrous MgSO₄, filtered, and concentrated to obtain the crude product. The crude product was purified by column chromatography, concentrated, and dried with an oil pump to obtain the target compound **E1-2**(8.88 g).
Step d: Into a flask under nitrogen protection, the above substitution product **(E1-2,** 8.00 g, 3.1 mmol) was dissolved in THF solution (50 mL), tetrabutylammonium fluoride solution **(TBAF,** 50 mL, 1N in THF) was added, and the reaction was conducted overnight to remove the TBS protecting group. The reaction solution was dried with anhydrous sodium sulfate, filtered, and concentrated to obtain the crude product. The crude product was purified by column chromatography, and the target eluent was collected and concentrated to obtain the product **E1-3** (6.60 g). ¹H NMR (500 MHz, CDCl₃) δ: 3.85-3.44 (m, 182H), 3.35 (t, 2H), 3.17 (t, 2H), 2.28 (t, 2H), 1.56-1.43 (m, 4H), 1.36-1.20 (m, 42H), 0.88 (t, 6H). The molecular weight of **E1-3** was determined to be 2447 Da by MALDI-TOF, PDI=1.03.

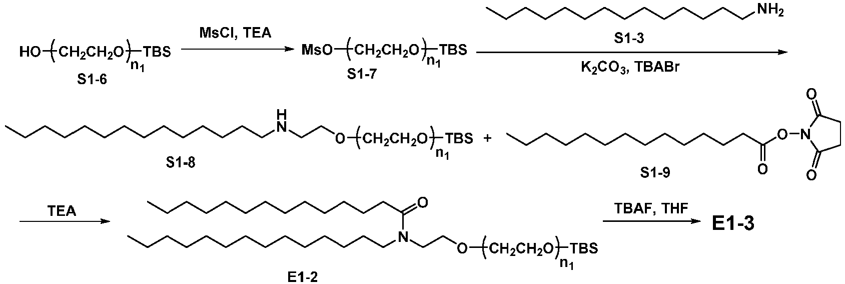

### Example-2: Synthesis of methoxy-PEGylated lipid E2-1

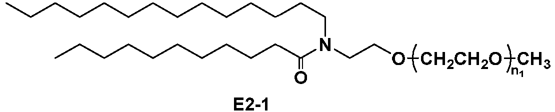

**E2-1** corresponds to the general formula (2), wherein, R₁ is a tetradecyl group, R₂ is a decyl group, B₃, B₄, and L₇ are all linking bonds, L₈ is a carbonyl group, L₃ is -CH₂CH₂-, A is -OCH₂CH₂-, Rₐ and R_{b} are both hydrogen atoms, s is 2, n₁≈60, R is a methoxy group, and the total molecular weight is approximately 3070 Da.

The preparation process is as follows:
Step a: Compound **S2-1** (27.00 g, 10.0 mmol, mPEG-OH, Mw≈2700, n₁≈60, PDI=1.03), and toluene (200 mL) underwent the azeotropic water removal at 140°C. After 60 mL of solvent was steamed out, the temperature of reaction solution was cooled to room temperature. TEA (2.02 g, 20.0 mmol) and MsCl (2.05 g, 18.0 mmol) were added, and the reaction was stirred at room temperature overnight. After completion of the reaction, the reaction solution was poured into water (200 mL), extracted twice with EtOAc (100 mL*2). The water phase was retained and extracted twice with dichloromethane (100 mL*2). The organic phases were combined, dried, filtered, concentrated, dissolved in isopropanol at 50°C, recrystallized in an ice bath, and filtered to obtain the compound **S2-2** (24.75 g).
Step b: The above compound **S2-2** (24.75 g, 9.0 mmol) was added into 80 mL water and stirred at room temperature to dissolve. Potassium carbonate (12.42 g, 90.0 mmol), compound **S1-3** (9.58 g, 45.0 mmol), and tetra-*n*-butylammonium bromide (0.29 g, 0.9 mmol) were added, and the reaction was stirred at room temperature for 72 h. After completion of the reaction, the reaction solution was extracted twice with dichloromethane (100 mL*2). The organic phases were combined, and then backwashed once with saturated sodium chloride aqueous solution (100 mL). The organic phase was retained, dried with anhydrous sodium sulfate, filtered, and concentrated to obtain the crude product of **S2-3.** Furthermore, the crude product was purified by column chromatography, concentrated and dried with an oil pump to obtain the target compound **S2-3** (17.55 g).
Step c: The above substitution product **S2-3** (14.55 g, 5.0 mmol), **S2-4**(1.22 g, 6.3 mmol), and TEA (0.76 g, 7.5 mmol) were dissolved in dichloromethane (100 mL), followed by stirring at room temperature overnight. The reaction solution was concentrated, dissolved in 100 mL water, and extracted twice with EtOAc (100 mL*2). The water phase was retained, added with sodium chloride, and extracted twice with dichloromethane (100 mL*2). The organic phases were combined, backwashed once with saturated sodium chloride aqueous solution (100 mL), dried with anhydrous MgSO₄, filtered, and concentrated to obtain the crude product. The crude product was purified by column chromatography, concentrated, and dried with an oil pump to obtain the target compound **E2-1**(13.13 g). ¹H NMR (500 MHz, CDCl₃) δ: 3.85-3.44 (m, 242H), 3.39 (s, 3H), 3.34 (t, 2H), 3.18 (t, 2H), 2.25 (t, 2H), 1.56-1.42 (m, 4H), 1.36-1.19 (m, 36H), 0.87 (t, 6H). The molecular weight of **E2-1** was determined to be 3079 Da by MALDI-TOF, PDI=1.03.

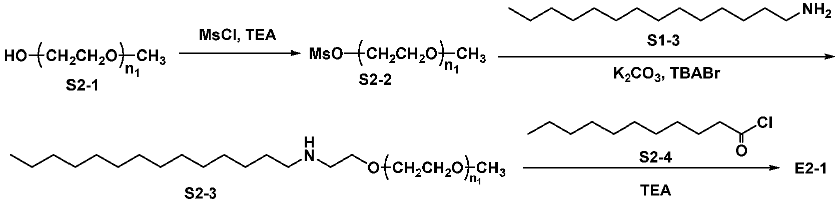

### Example-3: Synthesis of methoxy-PEGylated lipid E3-1

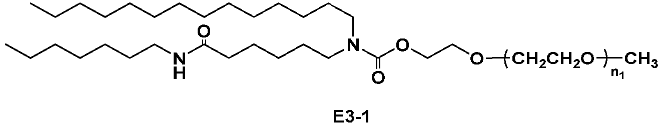

**E3-1** corresponds to the general formula (2), wherein, R₁ is a tetradecyl group, R₂ is a heptyl group, B₃ is a linking bond, B₄ is a pentylene group, L₇ is a linking bond, L₈ is an amide bond (-NHC(=O)-), L₃ is a carbonyl group, A is -OCH₂CH₂-, Rₐ and R_{b} are both hydrogen atoms, s is 2, n₁≈45, R is a methoxy group, and the total molecular weight is approximately 2500 Da.

The preparation process is as follows:
Step a: Compound **S3-2** (10.00 g, 34.4 mmol) was dissolved in 30 mL isopropanol, and then compound **S3-1** (12.40 g, 33.8 mmol), potassium carbonate (4.75 g, 34.4 mmol), and **potassium iodide** (0.16 g, 1.0 mmol) were added in sequence, and the reaction mixture was heated to reflux for 36 h. The reaction was monitored by the TLC until a small amount of starting materials remained, and then the isopropanol was concentrated. 200 mL purified water was added to quench the reaction. The reaction solution was extracted with dichloromethane (100 mL*2), dried with anhydrous MgSO₄, filtered, and concentrated to obtain the crude product. The crude product was purified by column chromatography and concentrated to obtain the product **S3-3** (10.83 g).
Step b: The above compound **S13-3** (2.13 g, 5.0 mmol), compound **S3-4** (8.80 g, 4.0 mmol), and TEA (0.76 g, 7.5 mmol) were dissolved in dichloromethane (100 mL), followed by stirring at room temperature overnight. The reaction solution was concentrated, dissolved in 100 mL water, and extracted twice with EtOAc (100 mL*2). The water phase was retained, added with sodium chloride, and extracted twice with dichloromethane (100 mL*2). The organic phases were combined, backwashed once with saturated sodium chloride aqueous solution (100 mL), dried with anhydrous MgSO₄, filtered, and concentrated to obtain the crude product. The crude product was purified by column chromatography, concentrated, and dried with an oil pump to obtain the target compound **E3-1** (9.25 g). ¹H NMR (500 MHz, CDCl₃) δ: 4.07 (t, 2H), 3.83-3.45 (m, 182H), 3.36 (s, 3H), 3.26 (t, 2H), 3.25-3.20 (q, 2H), 3.14 (t, 2H), 2.17 (t, 2H), 1.66-1.46 (m, 8H), 1.34-1.25 (m, 32H), 0.87 (t, 6H). The molecular weight of **E3-1** was determined to be 2508 Da by MALDI-TOF, PDI=1.02.

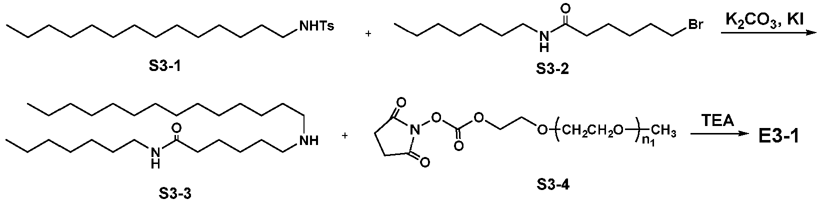

### Example-4: Synthesis of hydroxy-PEGylated lipid E4-2

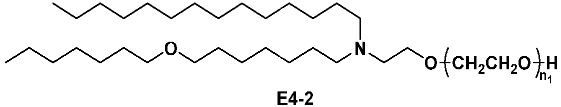

**E4-2** corresponds to the general formula (2), wherein, R₁ is a tetradecyl group, R₂ is a heptyl group, B₃ is a linking bond, B₄ is a heptyl group, L₇ is a linking bond, L₈ is an ether bond (-O-), L₃ is -CH₂CH₂-, A is -OCH₂CH₂-, Rₐ and R_{b} are both hydrogen atoms, s is 2, n₁≈45, R is a hydroxy group, and the total molecular weight is approximately 2450 Da.

The preparation process is as follows:
Step a: Compound **S4-1** (10.00 g, 34.2 mmol) was dissolved in 100 mL isopropanol, and then compound **S3-1** (12.40 g, 33.8 mmol), potassium carbonate (4.75 g, 34.4 mmol), and potassium iodide (0.16 g, 1.0 mmol) were added in sequence, and the reaction mixture was heated to reflux for 36 h. The reaction was monitored by the TLC until a small amount of starting materials remained, and then the isopropanol was concentrated. 100 mL purified water was added to quench the reaction. The reaction solution was extracted with dichloromethane (100 mL*2), dried with anhydrous MgSO₄, filtered, and concentrated to obtain the crude product. The crude product was purified by column chromatography and concentrated to obtain the product **S4-2** (10.53 g).
Step b: Into a 500 mL round-bottom flask, **naphthalene** (9.60 g, 75.0 mmol) was added and dissolved in 120 mL THF, **sodium bulk** (2.59 g, 112.5 mmol) was added, and the reaction solution was heated to 70°C, followed by stirring for 18 h. The reaction solution was cooled to 0°C, p-toluenesulfonamide compound **S4-2** (8.70 g, 15.0 mmol) dissolved in 20 mL THF was added dropwise slowly, after which the reaction was continued for 1.5 h, followed by stirring for 16 h after heating to 50°C. After completion of the reaction, the reaction solution was cooled to 0°C, methanol was added dropwise slowly to quench the reaction, and finally water was also added to quench the reaction. The reaction solution was extracted with PE, dried, concentrated, dissolved in methanol at 60°C, recrystallized in an ice bath, and filtered to obtain the amine compound **S4-3** (4.65 g).
Step c: The above compound **S1-7** (4.40 g, 2.0 mmol, Mw≈2200, n₁≈45, PDI=1.02) was added into 80 mL water, and stirred at room temperature to dissolve. Potassium carbonate (3.04 g, 22.0 mmol), compound **S4-3** (4.26 g, 10.0 mmol), and tetra-n-butylammonium bromide (0.06 g, 0.2 mmol) were added, and the reaction was stirred at room temperature for 72 h. After completion of the reaction, the reaction solution was extracted twice with dichloromethane solution (100 mL*2). The organic phases were combined, backwashed once with saturated sodium chloride aqueous solution (100 mL), and then the organic phase was retained, dried with anhydrous sodium sulfate, filtered, and concentrated to obtain the crude product of **E4-1.** The crude product was purified by column chromatography, concentrated, and dried with an oil pump to obtain the target compound **E4-1** (3.50 g).
Step d: Into a flask under nitrogen protection, the above substitution product **E4-1** (2.50 g, 1.0 mmol, Mw≈2500, n₁≈45, PDI=1.02) was dissolved in THF solution (50 mL), TBAF (50 mL, 1N in THF) was added, and the reaction was conducted overnight to remove the TBS protecting group. The reaction solution was dried with anhydrous sodium sulfate, filtered, and concentrated to obtain the crude product. The crude product was purified by column chromatography, and the target eluent was collected and concentrated to obtain the product **E4-2** (2.14 g). The characteristic peak of Fmoc group disappeared in the ¹H-NMR spectrum. ¹H NMR (500 MHz, CDCl₃) δ: 3.86-3.46 (m, 186H), 2.65 (t, 2H), 2.49 (m, 4H), 1.65-1.42 (m, 8H), 1.36-1.18 (m, 36H), 0.87 (t, 6H). The molecular weight of **E4-2** was determined to be 2449 Da by MALDI-TOF, PDI=1.02.

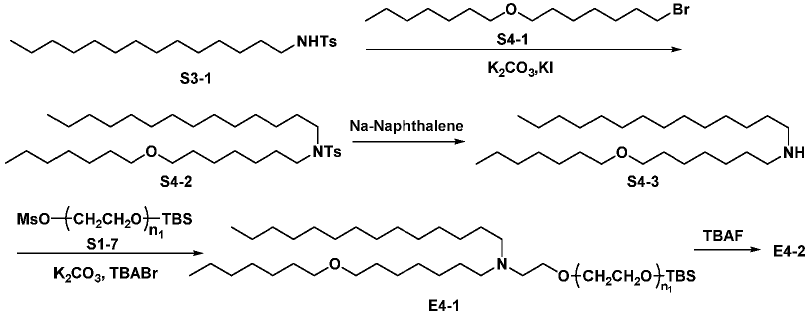

### Example-5: Synthesis of methoxy-PEGylated lipid E5-1

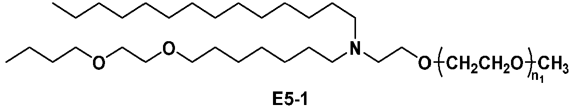

**E5-1** corresponds to the general formula (2), wherein, R₁ is a tetradecyl group, R₂ is a butyl group, B₃ is a linking bond, B₄ is a heptylene group, L₇ is a linking bond, L₈ is -OCH₂CH₂O-, L₃ is -CH₂CH₂-, A is -OCH₂CH₂-, Rₐ and R_{b} are both hydrogen atoms, s is 2, n₁≈45, R is a methoxy group, and the total molecular weight is approximately 2460 Da.

The preparation process is as follows:
Step a: Compound **S5-1** (10.00 g, 34.0 mmol) was dissolved in 30 mL isopropanol, and then compound **S3-1** (12.40 g, 33.8 mmol), potassium carbonate (4.75 g, 34.4 mmol), and potassium iodide (0.16 g, 1.0 mmol) were added in sequence, and the reaction mixture was heated to reflux for 36 h. The reaction was monitored by the TLC until a small amount of starting materials remained, and then the isopropanol was concentrated. 100 mL purified water was added to quench the reaction. The reaction solution was extracted with dichloromethane (100 mL*2), dried with anhydrous MgSO₄, filtered, and concentrated to obtain the crude product. The crude product was purified by column chromatography and concentrated to obtain the product **S5-2** (10.88 g).
Step b: Into a 500 mL round-bottom flask, naphthalene (9.60 g, 75.0 mmol) was added and dissolved in 120 mL THF, sodium bulk (2.59 g, 112.5 mmol) was added, and the reaction solution was heated to 70°C, followed by stirring for 18 h. The reaction solution was cooled to 0°C, p-toluenesulfonamide compound **S5-2** (8.73 g, 15.0 mmol) dissolved in 20 mL THF was added dropwise slowly, and the reaction was continued for 1.5 h, followed by stirring for 16 h after heating to 50°C. After completion of the reaction, the reaction solution was cooled to 0°C, methanol was added dropwise slowly to quench the reaction, and finally water was added to quench the reaction. The reaction solution was extracted with PE, dried, concentrated, dissolved in methanol at 60°C, recrystallized in an ice bath, and filtered to obtain the amine compound **S5-3** (4.75 g).
Step c: The above compound **S1-2** (4.20 g, 2.0 mmol, Mw≈2100, n₁≈45, PDI=1.02) was added into 80 mL water and stirred at room temperature to dissolve. Potassium carbonate (3.04 g, 22.0 mmol), compound **S5-3** (4.28 g, 10.0 mmol), and tetra-n-butylammonium bromide (0.06 g, 0.2 mmol) were added, and the reaction was stirred at room temperature for 72 h. After completion of the reaction, the reaction solution was extracted twice with dichloromethane solution (100 mL*2). The organic phases were combined, backwashed once with saturated sodium chloride aqueous solution (100 mL), and then the organic phase was retained, dried with anhydrous sodium sulfate, filtered, and concentrated to obtain the crude product of **E5-1.** The crude product was purified by column chromatography, concentrated, and dried with an oil pump to obtain the target compound **E5-1** (3.56 g). ¹H NMR (500 MHz, CDCl₃) δ: 3.85-3.45 (m, 190H), 3.38 (s, 3H), 2.65 (t, 2H), 2.49 (m, 4H), 1.66-1.43 (m, 8H), 1.36-1.19 (m, 30H), 0.87 (t, 6H). The molecular weight of **E5-1** was determined to be 2467 Da by MALDI-TOF, PDI=1.02.

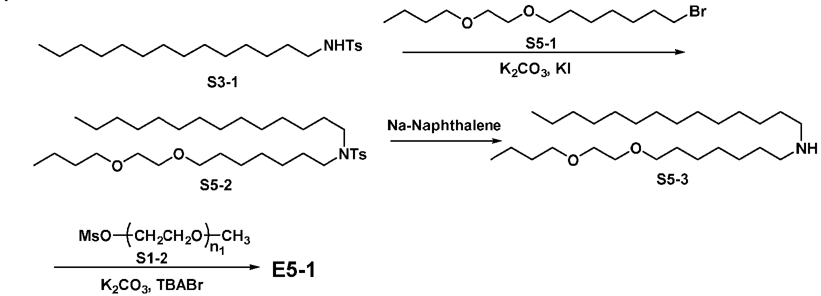

### Example-6: Synthesis of methoxy-PEGylated lipid E6-1

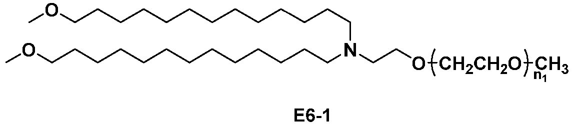

**E6-1** corresponds to the general formula (2), wherein, R₁ and R₂ are both methyl groups, B₃ and B₄ are both tridecylene groups, L₇ and L₈ are both ether bonds (-O-), L₃ is -CH₂CH₂O-, A is -CH₂CH₂O-, Rₐ and R_{b} are both hydrogen atoms, s is 2, n₁≈40, R is a methyl group, and the total molecular weight is approximately 2260 Da.

The preparation process is as follows:
Step a: Into a 500 mL round-bottom flask, compound 13-bromo-1-tridecanol **(S6-1,** 20.00 g, 71.9 mmol) was dissolved in THF (100 mL), **Na₂CO₃** (15.20 g, 143.8 mmol) was added, and **magnesium sulphate** (13.60 g, 107.8 mmol) was added dropwise slowly in an ice bath, followed by stirring for 24 h at room temperature. When the starting materials were consumed completely according to the TLC, saturated ammonium chloride solution was added to quench the reaction. The reaction solution was extracted with EtOAc (200 mL*2), dried with anhydrous MgSO₄, filtered, and concentrated to obtain the methyl ether compound **S6-2** (19.66 g).
Step b: Compound **S6-2** (19.66 g, 67.3 mmol) was dissolved in 60 mL isopropanol, ***p*-toluenesulfonamide (TsNH₂,** 3.82 g, 22.3 mmol), potassium carbonate (9.24 g, 67.3 mmol), and potassium iodide (0.36 g, 2.2 mmol) were added in sequence, and the reaction mixture was heated to reflux for 36 h. The reaction was monitored by the TLC until a small amount of starting materials remained, and then the isopropanol was concentrated. 200 mL purified water was added to quench the reaction. The reaction solution was extracted with dichloromethane (200 mL*2), dried with anhydrous MgSO₄, filtered, and concentrated to obtain the crude product. The crude product was purified by column chromatography and concentrated to obtain the product **S6-3** (9.30 g).
Step c: Into a 500 mL round-bottom flask, naphthalene (10.00 g, 78.2 mmol) was added and dissolved in 120 mL THF, sodium bulk (2.70 g, 117.2 mmol) was added, and the reaction solution was heated to 70°C, followed by stirring for 18 h. Then, the reaction solution was cooled to 0°C, p-toluenesulfonamide compound **S6-3** (9.30 g, 15.4 mmol) dissolved in 20 mL THF was added dropwise slowly, and the reaction was continued for 1.5 h, followed by stirring for 16 h after heating to 50°C. After completion of the reaction, the reaction solution was cooled to 0°C, methanol was added dropwise slowly to quench the reaction, and finally, water was added to quench the reaction. The reaction solution was extracted with PE, dried, and concentrated to obtain a pale-yellow solid. The solid was dissolved in methanol at 60°C, recrystallized in an ice bath, and filtered to obtain the amine compound **S6-4** (5.00 g).
Step d: Compound **S6-5** (4.37 g, 2.3 mmol, Mw≈1900, n₁≈40, PDI=1.02) was added into 80 mL water and stirred at room temperature to dissolve. Potassium carbonate (3.17 g, 23.01 mmol), amine compound **S6-4** (5.00 g, 11.3 mmol), and tetra-n-butylammonium bromide (0.74 g, 0.23 mmol) were added, and the reaction was stirred at room temperature for 72 h. After completion of the reaction, the reaction solution was extracted twice with dichloromethane solution (100 mL*2). The organic phases were combined, backwashed once with saturated sodium chloride aqueous solution (100 mL), and then the organic phase was retained, dried with anhydrous sodium sulfate, filtered, and concentrated to obtain the crude product of **E6-1.** The crude product was purified by column chromatography, concentrated, and dried with an oil pump to obtain the target compound **E6-1** (3.95 g). ¹H NMR (500 MHz, CDCl₃) δ: 3.83-3.45 (m, 166H), 3.38 (s, 3H), 3.32 (s, 6H), 2.65 (t, 2H), 2.49 (m, 4H), 1.66-1.42 (m, 8H), 1.35-1.19 (m, 36H). The molecular weight **of E6-1** was determined to be 2259 Da by MALDI-TOF, PDI=1.02.

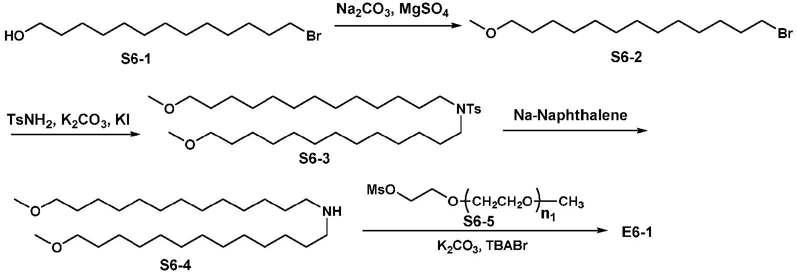

### Example-7: Synthesis of methoxy-PEGylated lipid E7-1

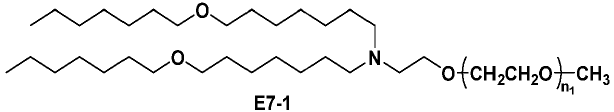

**E7-1** corresponds to the general formula (2), wherein, R₁ and R₂ are both heptyl groups, B₃ and B₄ are both heptylene groups, L₇ and L₈ are both ether bonds (-O-), L₃ is -CH₂CH₂-, A is -OCH₂CH₂-, Rₐ and R_{b} are both hydrogen atoms, s is 2, n₁=45, R is a methoxy group, and the total molecular weight is approximately 2482 Da.

The preparation process is as follows:
Step a: Into a 500 mL round-bottom flask, the above compound **S4-1** (10.00 g, 34.2 mmol) was dissolved in 100 mL isopropanol, *p*-toluenesulfonamide (TsNH₂, 1.95 g, 11.4 mmol), potassium carbonate (4.72 g, 34.2 mmol), and potassium iodide (0.18 g, 1.1 mmol) were added, and the reaction mixture was heated to reflux for 36 h. The reaction was monitored by the TLC until a small amount of starting materials remained, and then the isopropanol was concentrated. 100 mL purified water was added to quench the reaction. The reaction solution was extracted with dichloromethane (100 mL*2), dried with anhydrous MgSO₄, filtered, and concentrated to obtain the crude product. The crude product was purified by column chromatography and concentrated to obtain the product **S7-1** (5.01 g).
Step b: Into a 500 mL round-bottom flask, naphthalene (5.38 g, 42.0 mmol) was added and dissolved in 120 mL THF, sodium bulk (1.45 g, 63.0 mmol) was added, and the reaction solution was heated to 70°C, followed by reacting for 18 h. Then, the reaction solution was cooled to 0°C, p-toluenesulfonamide compound **S7-1** (5.00 g, 8.4 mmol) dissolved in 20 mL THF was added dropwise slowly, and the reaction was continued for 1.5 h, followed by reacting for 16 h after heating to 50°C. After completion of the reaction, the reaction solution was cooled to 0°C, methanol was added dropwise slowly to quench the reaction, and finally, water was added to quench the reaction. The reaction solution was extracted with PE, dried, and concentrated to obtain a pale-yellow solid. The solid was dissolved in methanol at 60°C, recrystallized in an ice bath, and filtered to obtain the compound **S7-2** (2.78 g).
Step c: Compound **S7-3** (2.78 g, 1.3 mmol, Mw=2137, n₁=45) was added into 50 mL water and stirred at room temperature to dissolve. Potassium carbonate (1.79 g, 13.0 mmol), compound **S7-2** (2.78 g, 6.3 mmol), and tetra-n-butylammonium bromide (0.03 g, 0.1 mmol) were added, and the reaction was stirred at room temperature for 72 h. After completion of the reaction, the reaction solution was extracted twice with dichloromethane (50 mL*2). The organic phases were combined, backwashed once with saturated sodium chloride aqueous solution (50 mL), and then the organic phase was retained, dried with anhydrous sodium sulfate, filtered, and concentrated to obtain the crude product of **E7-1.** The crude product was purified by column chromatography, concentrated, and dried with an oil pump to obtain the target compound **E7-1** (2.43 g). ¹H NMR (500 MHz, CDCl₃) δ: 3.83-3.43 (m, 190H), 3.37 (s, 3H), 2.65 (t, 2H), 2.49 (m, 4H), 1.61-1.47 (m, 12H), 1.34-1.19 (m, 28H), 0.88 (t, 6H). The molecular weight of **E7-1** was determined to be 2481 Da by MALDI-TOF, PDI=1.

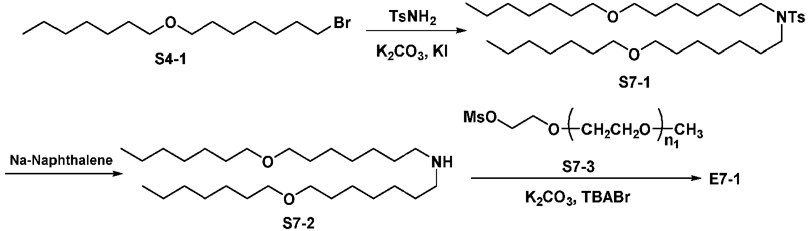

### Example-8: Synthesis of methoxy-PEGylated lipids E8-1 and E8-2

### Example-8.1: Synthesis of methoxy-PEGylated lipid E8-1

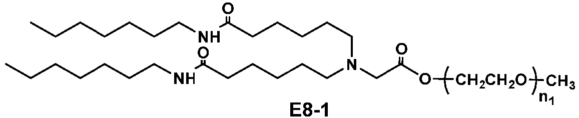

**E8-1** corresponds to the general formula (2), wherein, R₁ and R₂ are both heptyl groups, B₃ and B₄ are both pentylene groups, L₇ and L₈ are both amide bonds (-NHC(=O)-), L₃ is -CH₂C(=O)-, A is -OCH₂CH₂-, Rₐ and R_{b} are both hydrogen atoms, s is 2, n₁≈60, R is a methoxy group, and the total molecular weight is approximately 3150 Da.

The preparation process is as follows:
Step a: Compound **S8-1** (20.00 g, 103.1 mmol), 1-ethyl-3-(3-dimethylaminopropyl)carbodiimide hydrochloride **(EDCI,** 20.08 g, 104.6 mmol), and *N*-hydroxysuccinimide **(NHS,** 11.57 g, 101.5 mmol) were dissolved in dichloromethane (400 mL), and the reaction was stirred at room temperature overnight. After completion of the reaction, the reaction solution was backwashed twice with 0.1 mol/L HCl solution (200 mL*2) and backwashed once with saturated sodium chloride aqueous solution (200 mL). The dichloromethane phase was dried with anhydrous MgSO4, filtered, and concentrated to obtain the target compound **S8-2** (29.31 g).
Step b: The above compound **S8-2** (29.00 g, 99.7 mmol) was dissolved in dichloromethane (100 mL), and then added with compound **S8-3** (11.67 g, 101.5 mmol) and TEA (14.15 g, 140.1 mmol). The reaction was conducted at room temperature overnight, during which a large amount of white solid was precipitated. After filtration, the obtained solid was slurried with methanol (60 mL), filtered, rinsed twice with methanol, collected, and dried to obtain the target compound **S8-4** (24.55g).
Step c: The above compound **S8-4** (20.00 g, 68.7 mmol) was dissolved in 60 mL isopropanol, *p*-toluenesulfonamide (TsNH₂, 3.93 g, 22.9 mmol), potassium carbonate (9.48 g, 68.7 mmol), and potassium iodide (0.36 g, 2.2 mmol) were added, and the reaction mixture was heated to reflux for 36 h. The reaction was monitored by the TLC until a small amount of starting materials remained, and then the isopropanol was concentrated. 200 mL purified water was added to quench the reaction. The reaction solution was extracted with dichloromethane (200 mL*2), dried with anhydrous MgSO₄, filtered, and concentrated to obtain the crude product. The crude product was purified by column chromatography and concentrated to obtain the product **S8-5** (9.68 g).
Step d: Into a 500 mL round-bottom flask, naphthalene (9.60 g, 75.0 mmol) was added and dissolved in 120 mL THF, sodium bulk (2.59 g, 112.5 mmol) was added, and the reaction solution was heated to 70°C, followed by stirring for 18 h. Then, the reaction solution was cooled to 0°C, and *p*-toluenesulfonamide compound **S8-5** (8.91 g, 15.0 mmol) dissolved in 20 mL THF was added dropwise slowly. After completion of the dropwise addition, the reaction was continued for 1.5 h, followed by stirring for 16 h after heating to 50°C. After completion of the reaction, the reaction solution was cooled to 0°C, methanol was added dropwise slowly to quench the reaction, and finally, water was added to quench the reaction. The reaction solution was extracted with PE, dried, and concentrated to obtain a pale-yellow solid. The solid was dissolved in methanol at 60°C, recrystallized in an ice bath, and filtered to obtain the amine compound **S8-6** (4.79 g).
Step e: The compound **S8-7** (5.60 g, 2.0 mmol, Mw≈2800, n₁≈60, PDI=1.02) was added into 80 mL water and stirred at room temperature to dissolve. Potassium carbonate (2.76 g, 20.0 mmol), secondary amine compound **S8-6** (4.40 g, 10.0 mmol), and tetra-n-butylammonium bromide (0.06 g, 0.2 mmol) were added, and the reaction was stirred at room temperature for 72 h. After completion of the reaction, the reaction solution was extracted twice with dichloromethane solution (100 mL*2). The organic phases were combined, backwashed once with saturated sodium chloride aqueous solution (100 mL), and then the organic phase was retained, dried with anhydrous sodium sulfate, filtered, and concentrated to obtain the crude product of **E8-1.** The crude product was purified by column chromatography, concentrated, and dried with an oil pump to obtain the target compound **E8-1** (5.12 g). ¹H NMR (500 MHz, CDCl₃) δ: 4.17 (s, 2H), 3.85-3.45 (m, 238H), 3.37 (s, 3H), 3.26 (t, 2H), 3.25-3.20 (q, 4H), 3.14 (t, 2H), 3.06 (t, 2H), 2.17 (t, 4H), 1.68-1.41 (m, 12H), 1.33-1.25 (m, 20H), 0.88 (t, 6H). The molecular weight of **E8-1** was determined to be 3153 Da by MALDI-TOF, PDI=1.02.

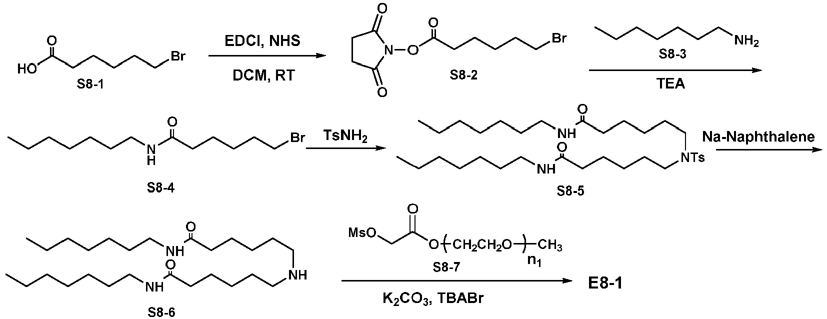

### Example-8.2: Synthesis of methoxy-PEGylated lipid E8-2

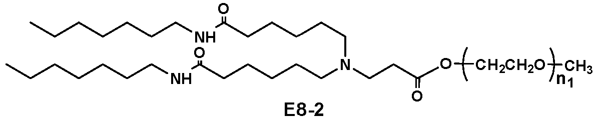

**E8-2** corresponds to the general formula (2), wherein, R₁ and R₂ are both heptyl groups, B₃ and B₄ are both pentylene groups, L₇ and L₈ are both amide bonds (-NHC(=O)-), L₃ is -CH₂CH₂C(=O)-, A is -OCH₂CH₂-, Rₐ and R_{b} are both hydrogen atoms, s is 2, n₁≈150, R is a methoxy group, and the total molecular weight is approximately 7130 Da.

The preparation process is as follows:
The compound **S8-8** (13.60 g, 2.0 mmol, Mw≈6800, n₁≈150, PDI=1.02) was added into 80 mL water and stirred at room temperature to dissolve. Potassium carbonate (2.76 g, 20.0 mmol), secondary amine compound **S8-6** (4.40 g, 10.0 mmol), and tetra-n-butylammonium bromide (0.06 g, 0.2 mmol) were added, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), and then the organic phase was retained, dried with anhydrous sodium sulfate, filtered, and concentrated to obtain the crude product of **E8-2.** The crude product was purified by column chromatography, concentrated, and dried with an oil pump to obtain the target compound **E8-2** (11.12 g). ¹H NMR (500 MHz, CDCl₃) δ: δ: 4.07 (t, 2H), 3.83-3.46 (m, 598H), 3.36 (s, 3H), 3.26 (t, 2H), 3.25-3.21 (q, 4H), 3.14 (t, 2H), 3.06 (t, 2H), 2.38 (t, 2H), 2.16 (t, 4H), 1.66-1.41 (m, 12H), 1.32-1.26 (m, 20H), 0.87 (t, 6H). The molecular weight of **E8-2** was determined to be 7129 Da by MALDI-TOF, PDI=1.02.

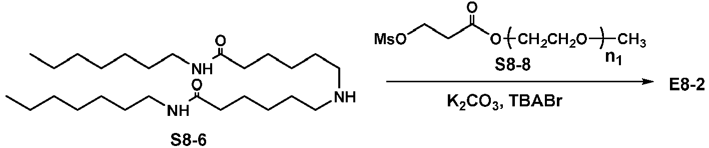

### Example-9: Synthesis of ethylamine-PEGylated lipid E9-2

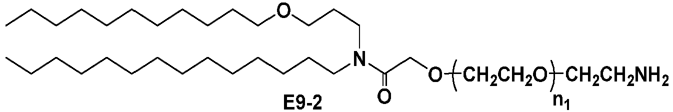

**E9-1** corresponds to the general formula (2), wherein, R₁ is an undecyl group, R₂ is a tetradecyl group, B₃ is a propylene group, B₄ is a linking bond, L₇ is an ether bond, L₈ is a linking bond, L₃ is -C(=O)CH₂-, A is -OCH₂CH₂-, Rₐ and R_{b} are both hydrogen atoms, s is 2, n₁≈45, R is -CH₂CH₂NH₂, and the total molecular weight is approximately 2500 Da.

The preparation process is as follows:
Step a: Into a dry and clean 500 mL round-bottom flask, 80 mL water was added to the compound **S9-1** (12.00 g, 41.1 mmol) and stirred at room temperature to dissolve. Potassium carbonate (56.72 g, 411.0 mmol), compound **S9-2** (47.06 g, 205.5 mmol), and tetra-n-butylammonium bromide (1.32 g, 4.1 mmol) were added, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), and then the organic phase was retained, dried with anhydrous sodium sulfate, filtered, and concentrated to obtain the crude product of **S9-3.** The crude product was purified by column chromatography, concentrated, and dried with an oil pump to obtain the target compound **S9-3** (10.37 g).
Step b: The compound **S9-4** (7.92 g, 3.6 mmol, Mw≈2200, n₁≈45, PDI=1.03), compound **S9-3** (2.29 g, 5.4 mmol), EDCI (1.38 g, 7.2 mmol), HOBt (0.73 g, 5.4 mmol), and TEA (0.73 g, 7.2 mmol) were dissolved in dichloromethane (100 mL) in sequence, followed by stirring at room temperature overnight. After completion of the reaction, the reaction solution was backwashed twice with 0.1 mol/L HCl solution (10%NaCl) (100 mL*2) and backwashed once with saturated sodium chloride aqueous solution (100 mL). The organic phase was dried with anhydrous MgSO₄, filtered, and concentrated to obtain the crude product. The crude product was purified by column chromatography, concentrated, and dried with an oil pump to obtain the target compound **E9-1** (7.75 g).
Step c: Removal of Boc protecting group. Into a dry and clean round-bottom flask, a solution of trifluoroacetic acid/dichloromethane (1:2, v/v) was prepared, dichloromethane solution of **E9-1** (7.00 g, 2.7 mmol) was added dropwise slowly in an ice bath, and the reaction was continued for 2 h at room temperature. The reaction solution was concentrated, added with purified water, and extracted with dichloromethane. The extract was dried with anhydrous MgSO₄, filtered, concentrated, and recrystallized to obtain the **E9-2** (6.16 g). ¹H NMR (500 MHz, CDCl₃) δ: 4.17 (s, 2H), 3.83-3.43 (m, 186H), 3.26 (t, 2H), 3.17 (t, 2H), 2.83 (t, 2H), 1.66-1.46 (m, 6H), 1.36-1.21 (m, 38H), 0.89 (t, 6H). The molecular weight of **E9-2** was determined to be 2506 Da by MALDI-TOF, PDI=1.03.

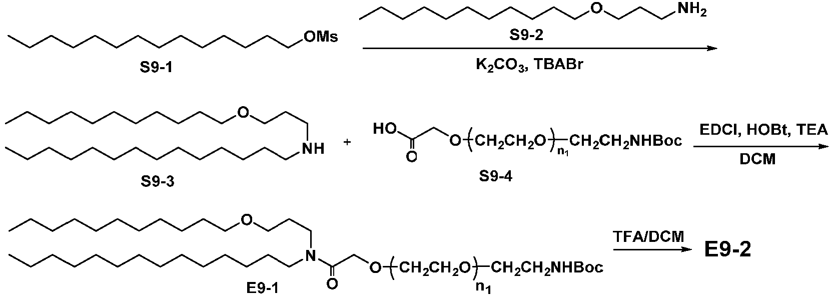

### Example-10: Synthesis of methoxy-PEGylated lipid E10-1

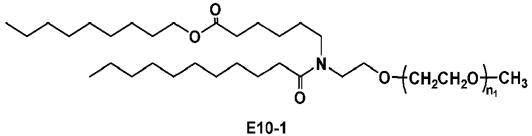

**E10-1** corresponds to the general formula (2), wherein, R₁ is a nonyl group, R₂ is a decyl group, L₇ is -OC(=O)-, L₈ is -C(=O)-, B₃ is a pentylene group, B₄ is a linking bond, L₃ is -CH₂CH₂O-, A is -CH₂CH₂O-, Rₐ and R_{b} are both hydrogen atoms, s is 2, n₁≈100, R is a methyl group, and the total molecular weight is approximately 4880 Da.

The preparation process is as follows:
Step a: Under argon atmosphere, into the round-bottom flask containing **S10-2** (1.45 g, 10.0 mmol), **S10-1** (3.88 g, 20.0 mmol), and 4-dimethylaminopyridine **(DMAP,** 0.30 g, 2.5 mmol) dissolved in dichloromethane (20 mL), *N,N*'-dicyclohexylcarbodiimide **(DCC,** 4.55 g, 22.0 mmol) was added, and the reaction was conducted for 16 h at room temperature. After completion of the reaction, the precipitate was removed by filtering. The filtrate was concentrated, and the residue was purified by silica gel column chromatography to obtain the colorless oily substance **S10-3** (2.65 g).
Step b: Under nitrogen protection, acetonitrile was added, the bromide **S10-3** (2.41 g, 7.5 mmol), methoxypolyethylene glycol amine **S10-4** (27.00 g, 6.0 mmol, Mw≈4500, n₁≈100, PDI=1.03), and ***N,N*-diisopropylethylamine (DIPEA,** 1.04 mL, 6.0 mmol) were added with stirring slowly, and the reaction was conducted for 20 h at room temperature. After completion of the reaction, the reaction solution was concentrated, dissolved in dichloromethane, and extracted with 0.6 M hydrochloric acid (10% NaCl) and saturated sodium bicarbonate solution in sequence. After ensuring the absence of material loss in the water phase, the organic phases were combined, dried with anhydrous MgSO₄, filtered, concentrated, and purified by silica gel column chromatography to obtain the **S10-5** (22.25 g).
Step c: Undecanoyl chloride **(S10-6,** 0.84 g, 4.1 mmol) dissolved in benzene (50 mL) was added dropwise slowly to the benzene solution (100 mL) of compound **S10-5** (16.10 g, 3.4 mmol), triethylamine (17.0 mmol, 2.3 mL, 5 equivalents), and DMAP (66.00 mg) with a constant pressure burette at room temperature. After completion of the reaction, the reaction solution was diluted with the mixed solution of hexane and ethyl acetate (about 5%), washed with water and saline, dried with anhydrous sodium sulfate, filtered, and concentrated to obtain the crude product. The crude product was purified by silica gel column chromatography to obtain the product **E10-1** (12.98 g). ¹H NMR (500 MHz, CDCl₃) δ: 4.07 (t, 2H), 3.84-3.45 (m, 402H), 3.39 (s, 3H), 3.34 (t, 2H), 3.18 (t, 2H), 2.36 (t, 2H), 2.25 (t, 2H), 1.71-1.19 (m, 36H), 0.86 (t, 6H). The molecular weight of **E10-1** was determined to be 4886 by MALDI-TOF, PDI=1.03.

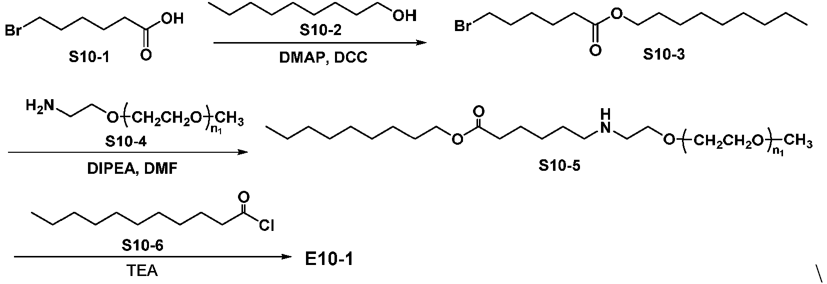

### Example-11: Synthesis of ethoxy-PEGylated lipid E11-1

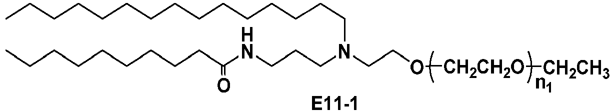

**E11-1** corresponds to the general formula (2), wherein, R₁ is a pentadecyl group, R₂ is a nonyl group, B₃ is a linking bond, B₄ is a propylene group, L₇ is a linking bond, L₈ is an amide bond (-C(=O)NH-), L₃ is -CH₂CH₂-, A is -OCH₂CH₂-, Rₐ and R_{b} are both hydrogen atoms, s is 2, n₁≈20, R is an ethoxy group, and the total molecular weight is approximately 1390 Da.

The preparation process is as follows:
Step a: The compound **S11-1** (5.53 g, 18.0 mmol) was added into 80 mL water and stirred at room temperature to dissolve. Potassium carbonate (24.84 g, 180.0 mmol), compound **S11-2** (20.52 g, 90.0 mmol), and tetra-n-butylammonium bromide (0.58 g, 1.8 mmol) were added, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), and then the organic phase was retained, dried with anhydrous sodium sulfate, filtered, and concentrated to obtain the crude product of **S11-3.** The crude product was purified by column chromatography, concentrated, and dried with an oil pump to obtain the target compound **S11-3** (5.27 g).
Step b: **S11-4** (2.00 g, 2.0 mmol, Mw≈1000, n₁=20, PDI=1.03) was added into 80 mL water and stirred at room temperature to dissolve. Potassium carbonate (2.76 g, 20.0 mmol), secondary amine compound **S11-3** (4.39 g, 10.0 mmol), and tetra-n-butylammonium bromide (0.06 g, 0.2 mmol) were added, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), and then the organic phase was retained, dried with anhydrous sodium sulfate, filtered, and concentrated to obtain the crude product of **E11-1.** The crude product was purified by column chromatography, concentrated, and dried with an oil pump to obtain the target compound ethoxy-PEGylated lipid **E11-1** (1.98 g). ¹H NMR (500 MHz, CDCl₃) δ: 3.83-3.47 (m, 84H), 3.25-3.20 (q, 2H), 2.62 (t, 2H), 2.49 (m, 4H), 2.19 (t, 2H), 1.74-1.46 (m, 6H), 1.33-1.25 (m, 36H), 1.12 (t, 3H), 0.86 (t, 6H). The molecular weight of **E11-1** was determined to be 1390 Da by MALDI-TOF, PDI=1.03.

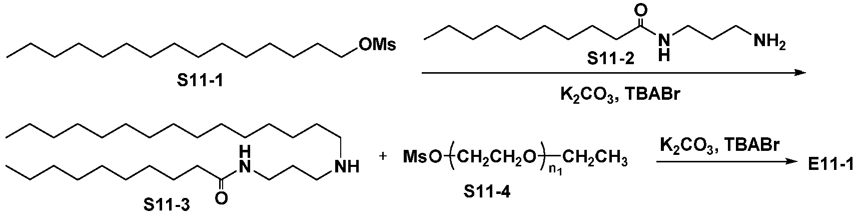

### Example-12: Synthesis of methoxy-PEGylated lipid E12-1

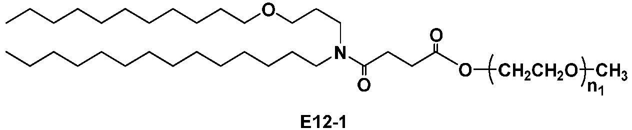

**E12-1** corresponds to the general formula (2), wherein, R₁ is an undecyl group, R₂ is a tetradecyl dodecyl group, B₃ is a propylene group, B₄ is a linking bond, L₇ is an ether bond, L₈ is a linking bond, L₃ is -C(=O)CH₂CH₂C(=O)-, A is -OCH₂CH₂-, Rₐ and R_{b} are both hydrogen atoms, s is 2, n₁≈45, R is a methoxy group, and the total molecular weight is approximately 2520 Da.

The preparation process is as follows:

The compound **S12-1** (10.50 g, 5.0 mmol, Mw≈2100, n₁≈45, PDI=1.02), compound **S9-3** (3.19 g, 7.5 mmol) prepared in Example-9, 1-ethyl-3-(3-dimethylaminopropyl)carbodiimide hydrochloride **(EDCI,** 1.92 g, 10.0 mmol), 1-hydroxybenzotriazole **(HOBt,** 1.01 g, 7.5 mmol), and TEA (1.01 g, 10.0 mmol) were dissolved in dichloromethane (100 mL) in sequence, followed by stirring at room temperature overnight. After completion of the reaction, the reaction solution was backwashed twice with 0.1 mol/L HCl aqueous solution (10% NaCl) (100 mL*2) and backwashed once with saturated sodium chloride aqueous solution (100 mL). The organic phase was dried with anhydrous MgSO₄, filtered, and concentrated to obtain the crude product. The crude product was purified by column chromatography, concentrated, and dried with an oil pump to obtain the target compound methoxy-PEGylated lipid **E12-1** (12.15 g). ¹H NMR (500 MHz, CDCl₃) δ: 4.17 (t, 2H), 3.84-3.45 (m, 182H), 3.39 (s, 3H), 3.20 (t, 2H),3.14 (t, 2H), 2.62 (t, 2H), 2.36 (t, 2H), 1.62-1.46 (m, 6H), 1.24-1.19 (m, 38H), 0.87 (t, 6H). The molecular weight of **E12-1** was determined to be 2519 Da by MALDI-TOF, PDI=1.02.

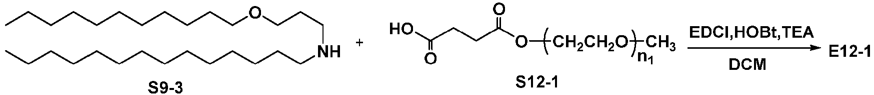

### Example-13: Synthesis of methoxy-PEGylated lipid E13-2

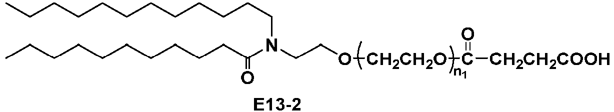

**E13-2** corresponds to the general formula (2), wherein, R₁ is a dodecyl group, R₂ is a decyl group, B₃ and B₄ are both linking bonds, L₇ is a linking bond, L₈ is a carbonyl group, L₃ is -CH₂CH₂-, A is -OCH₂CH₂-, Rₐ and R_{b} are both hydrogen atoms, s is 2, n₁≈45, R is -OC(=O)CH₂CH₂COOH, and the total molecular weight is approximately 2480 Da.

The preparation process is as follows:
Step a: Compound **S13-1** (10.50 g, 5.0 mmol, Mw≈2100, n₁≈45, PDI=1.03) and toluene (200 mL) underwent the azeotropic water removal at 140°C. After 60 mL of solvent was steamed out, the temperature of reaction solution was cooled to room temperature. TEA (1.01 g, 10.0 mmol) and MsCl (1.04 g, 9.0 mmol) were added, and the reaction was stirred at room temperature overnight. After completion of the reaction, the reaction solution was poured into water (200 mL) and extracted twice with EtOAc (100 mL*2). The water phase was retained and extracted twice with dichloromethane (100 mL*2). The organic phases were combined, dried, filtered, concentrated, dissolved in isopropanol at 50°C, recrystallized in an ice bath, and filtered to obtain the compound **S13-2** (10.01 g).
Step b: The above compound **S13-2** (8.80 g, 4.0 mmol, Mw≈2200, n₁≈45, PDI=1.03) was added into 80 mL water and stirred at room temperature to dissolve. Potassium carbonate (5.52 g, 40.0 mmol), compound **S13-3** (3.70 g, 20.0 mmol), and tetra-n-butylammonium bromide (0.13 g, 0.4 mmol) were added in sequence, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), retained, dried with anhydrous sodium sulfate, filtered, and concentrated to obtain the crude product of **S13-4.** The crude product was purified by column chromatography, concentrated, and dried with an oil pump to obtain the target compound **S13-4** (6.38 g).
Step c: The above compound **S13-4** (5.75 g, 2.5 mmol, Mw≈2300, n₁≈45, PDI=1.03), **S13-5** (0.79 g, 2.8 mmol), and TEA (0.38 g, 3.8 mmol) were dissolved in dichloromethane (100 mL), followed by stirring at room temperature overnight. The reaction solution was concentrated, dissolved in 100 mL water, and extracted twice with EtOAc (100 mL*2). The water phase was retained, added with sodium chloride, and extracted twice with dichloromethane (100 mL*2). The organic phases were combined, backwashed once with saturated sodium chloride aqueous solution (100 mL), dried with anhydrous sodium sulfate, filtered, and concentrated to obtain the crude product. The crude product was purified by column chromatography, concentrated, and dried with an oil pump to obtain the target compound **E13-1** (5.16 g).
Step d: Removal of tBu protecting group. Into a dry and clean 500 mL round-bottom flask, a solution of trifluoroacetic acid/dichloromethane (1:2, v/v) was prepared, dichloromethane solution of **E13-1** (5.00 g, 2.0 mmol, Mw≈2500, n₁≈45, PDI=1.03) was added dropwise slowly in an ice bath, and the reaction was continued for 2 h at room temperature. After completion of the reaction, the reaction solution was concentrated, added with purified water, and extracted with dichloromethane. The extract was dried with anhydrous MgSO₄, filtered, concentrated, and recrystallized to obtain the final product methoxy-PEGylated lipid **E13-2** (4.58 g). ¹H NMR (500 MHz, CDCl₃) δ: 4.21 (t, 2H), 3.84-3.45 (m, 180H), 3.35 (t, 2H), 3.18 (t, 2H), 2.38 (t, 2H), 2.30 (t, 2H), 2.25 (t, 2H), 1.56-1.45 (m, 4H), 1.36-1.20 (m, 32H), 0.86 (t, 6H). The molecular weight of **E13-2** was determined to be 2477 Da by MALDI-TOF, PDI=1.03.

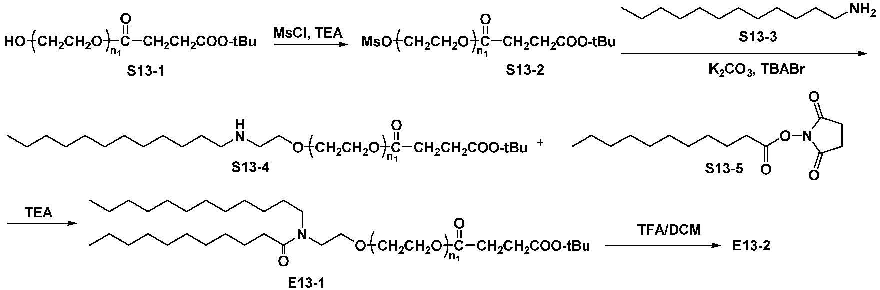

### Example-14: Synthesis of succinimide-PEGylated lipid E14-1

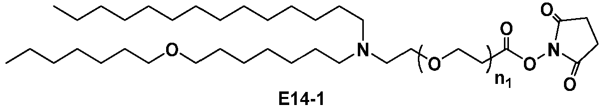

**E14-1** corresponds to the general formula (2), wherein, R₁ is a tetradecyl group, R₂ is a heptyl group, B₃ is a linking bond, B₄ is a heptylene group, L₇ is a linking bond, L₈ is an ether bond, L₃ is -CH₂CH₂-, A is -OCH₂CH₂-, Rₐ and R_{b} are both hydrogen atoms, s is 2, n₁≈45, R is 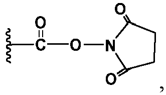 and the total molecular weight is approximately 2570 Da.

The preparation process is as follows:

The compound **S14-1** (8.80 g, 4.0 mmol, Mw≈2200, n₁≈45, PDI=1.03) was added into 80 mL water and stirred at room temperature to dissolve. Potassium carbonate (5.52 g, 50.0 mmol), secondary amine compound **S4-2** (8.52 g, 20.0 mmol), and tetra-*n*-butylammonium bromide (0.13 g, 0.4 mmol) were added, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), and then the organic phase was retained, dried with anhydrous sodium sulfate, filtered, and concentrated to obtain the crude product of **E14-1.** The crude product was purified by column chromatography, concentrated, and dried with an oil pump to obtain the target compound succinimide-PEGylated lipid **E14-1** (8.35 g). ¹H NMR (500 MHz, CDCl₃) δ: 3.87-3.45 (m, 184H), 2.86 (m, 4H), 2.62 (t, 2H), 2.45 (m, 4H), 2.28 (t, 2H), 1.61-1.46 (m, 8H), 1.35-1.19 (m, 36H), 0.88 (t, 6H). The molecular weight of **E14-1** was determined to be 2574 Da by MALDI-TOF, PDI=1.03.

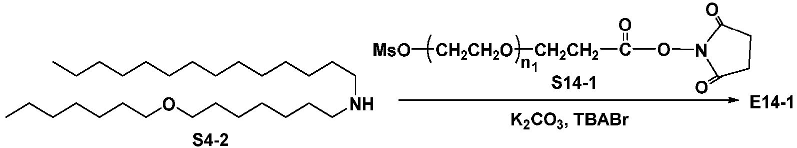

### Example-15: Synthesis of maleimide-PEGylated lipid E15-2

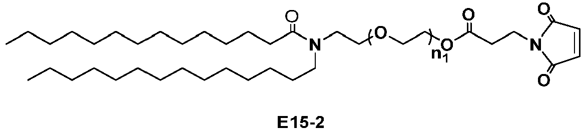

**E15-2** corresponds to the general formula (2), wherein, R₁ is a tridecyl group, R₂ is a tetradecyl group, B₃ and B₄ are both linking bonds, L₇ is a carbonyl group, L₈ is a linking bond, L₃ is -CH₂CH₂-, A is -OCH₂CH₂-, Rₐ and R_{b} are both hydrogen atoms, s is 2, n₁≈45, R is 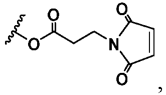 and the total molecular weight is approximately 2600 Da.

### Method-1:

The preparation process is as follows:
Step a: Compound **S15-1** (11.30 g, 5.0 mmol, Mw≈2260, n₁≈45, PDI=1.02) and toluene (200 mL) underwent the azeotropic water removal at 140°C. After 60 mL of solvent was steamed out, the temperature of reaction solution was cooled to room temperature. TEA (1.01 g, 10.0 mmol) and methyl sulfonyl chloride **(MsCl,** 1.03 g, 9.0 mmol) were added, and the reaction was stirred at room temperature overnight. After completion of the reaction, the reaction solution was poured into water (200 mL) and extracted twice with EtOAc (100 mL*2). The water phase was retained and extracted twice with dichloromethane (100 mL*2). The organic phases were combined, dried, filtered, concentrated, dissolved in isopropanol at 50°C, recrystallized in an ice bath, and filtered to obtain the compound **S15-2** (10.48 g).
Step b: The above compound **S15-2** (9.36 g, 4.0 mmol) was added into 80 mL water and stirred at room temperature to dissolve. Potassium carbonate (5.52 g, 40.0 mmol), compound **S1-3** (4.26 g, 20.0 mmol), and tetra-n-butylammonium bromide (0.13 g, 0.4 mmol) were added, and the reaction was stirred at room temperature for 72 h. After completion of the reaction, the reaction solution was extracted twice with dichloromethane solution (100 mL*2). The organic phases were combined, backwashed once with saturated sodium chloride aqueous solution (100 mL), retained, dried with anhydrous sodium sulfate, filtered, and concentrated to obtain the crude product of **S15-2.** The crude product was purified by column chromatography, concentrated, and dried with an oil pump to obtain the target compound **S15-3** (6.45 g).
Step c: The above compound **S15-3** (6.14 g, 2.5 mmol), compound **S1-5** (0.89 g, 2.8 mmol), and TEA (0.38 g, 3.8 mmol) were dissolved in dichloromethane (100 mL), followed by stirring at room temperature overnight. The reaction solution was concentrated, dissolved in 100 mL water, and extracted twice with EtOAc (100 mL*2). The water phase was retained, added with sodium chloride, and extracted twice with dichloromethane (100 mL*2). The organic phases were combined, backwashed once with saturated sodium chloride aqueous solution (100 mL), dried with anhydrous MgSO₄, filtered, and concentrated to obtain the crude product. The crude product was purified by column chromatography, concentrated and dried with an oil pump to obtain the target compound **E15-1** (5.23 g). The furan protecting group of **E15-1** was removed to obtain the end-product maleimide-PEGylated lipid **E15-2** (4.68 g). ¹H NMR (500 MHz, CDCl₃) δ: 6.49 (s, 2H), 4.25-4.10 (m, 4H), 3.85-3.46 (m, 180H), 3.35 (t, 2H), 3.17 (t, 2H), 2.73 (t, 2H), 2.24 (t, 2H), 1.58-1.48 (m, 4H), 1.36-1.17 (m, 42H), 0.88 (t, 6H). The molecular weight of **E15-2** was determined to be 2598 Da by MALDI-TOF, PDI=1.02.

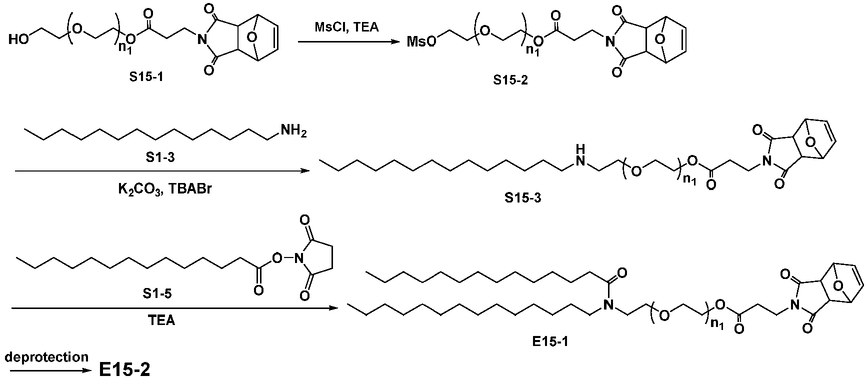

### Method-2:

The preparation process is as follows:
Under argon atmosphere, into the round-bottom flask containing **E1-3** (12.24 g, 5.0 mmol), **S15-4** (1.69 g, 10.0 mmol), and DMAP (0.15 g, 1.25 mmol) dissolved in dichloromethane (20 mL), DCC (2.28 g, 11.0 mmol) was added, and the reaction was conducted for 16 h at room temperature. After completion of the reaction, the precipitate was removed by filtering. The reaction solution was backwashed twice with saturated sodium chloride aqueous solution (100 mL). The organic phase was dried with anhydrous MgSO₄, filtered, and concentrated, and the residue was purified by silica gel column chromatography to obtain the end-product maleimide-PEGylated lipid **E15-2** (11.00 g, 84.7%). ¹H NMR (500 MHz, CDCl₃) δ: 6.49 (s, 2H), 4.25-4.10 (m, 4H), 3.85-3.46 (m, 180H), 3.35 (t, 2H), 3.17 (t, 2H), 2.73 (t, 2H), 2.24 (t, 2H), 1.58-1.48 (m, 4H), 1.36-1.17 (m, 42H), 0.88 (t, 6H). The molecular weight of **E15-2** was determined to be 2598 Da by MALDI-TOF, PDI=1.02.

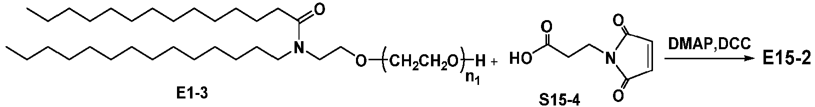

### Example-16: Synthesis of azide-PEGylated lipid E16-1

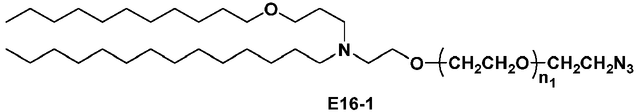

**E16-1** corresponds to the general formula (2), wherein, R₁ is an undecyl group, R₂ is a tetradecyl group, B₃ is a propylene group, B₄ is a linking bond, L₇ is an ether bond, L₈ is a linking bond, L₃ is -CH₂CH₂O-, A is -CH₂CH₂O-, Rₐ and R_{b} are both hydrogen atoms, s is 2, n₁≈45, R is -CH₂CH₂N₃, and the total molecular weight is approximately 2520 Da.

The preparation process is as follows:
The compound **S16-1** (8.80 g, 4.0 mmol, Mw≈2200, n₁≈45, PDI=1.03) was added into 80 mL water and stirred at room temperature to dissolve. Potassium carbonate (5.52 g, 50.0 mmol), secondary amine compound **S9-3** (8.52 g, 20.0 mmol), and tetra-*n*-butylammonium bromide (0.13 g, 0.4 mmol) were added, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), and then the organic phase was retained, dried with anhydrous sodium sulfate, filtered, and concentrated to obtain the crude product of **E16-1.** The crude product was purified by column chromatography, concentrated, and dried with an oil pump to obtain the target compound azide-PEGylated lipid **E16-1** (8.03 g). ¹H NMR (500 MHz, CDCl₃) δ: 3.85-3.42 (m, 186H), 3.99 (t, 2H), 2.66 (t, 2H), 2.48 (m, 4H), 1.58-1.48 (m, 8H), 1.38-1.19 (m, 38H), 0.85 (t, 6H). The molecular weight of **E16-1** was determined to be 2518 Da by MALDI-TOF, PDI=1.03.

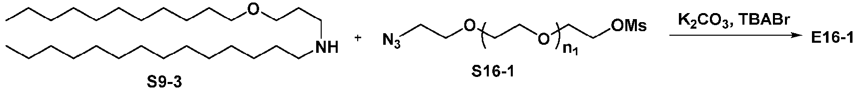

### Example-17: Synthesis of glycol-PEGylated lipid E17-2

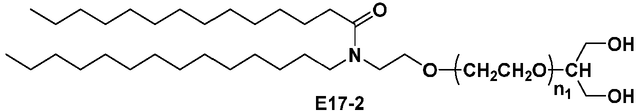

**E17-2** corresponds to the general formula (2), wherein, R₁ is a tridecyl group, R₂ is a tetradecyl group, B₃ and B₄ are both linking bonds, L₇ is a carbonyl group, L₈ is a linking bond, L₃ is -CH₂CH₂O-, A is -CH₂CH₂O-, Rₐ and R_{b} are both hydrogen atoms, s is 2, n₁≈250, R is 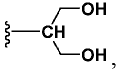 and the total molecular weight is approximately 11540 Da.

The preparation process is as follows:
Step a: Compound **S17-1** (56.50 g, 5.0 mmol, Mw≈11300, n₁≈250, PDI=1.02) and toluene (200 mL) underwent the azeotropic water removal at 140°C. After 60 mL of solvent was steamed out, the temperature of reaction solution was cooled to room temperature. TEA (1.01 g, 10.0 mmol) and methyl sulfonyl chloride **(MsCl,** 1.03 g, 9.0 mmol) were added, and the reaction was stirred at room temperature overnight. After completion of the reaction, the reaction solution was poured into water (200 mL) and extracted twice with EtOAc (100 mL*2). The water phase was retained and extracted twice with dichloromethane (100 mL*2). The organic phases were combined, dried, filtered, concentrated, dissolved in isopropanol at 50°C, recrystallized in an ice bath, and filtered to obtain the compound **S17-2** (51.63 g).
Step b: The above compound **S17-2** (45.60 g, 4.0 mmol) was added into 80 mL water and stirred at room temperature to dissolve. Potassium carbonate (5.52 g, 40.0 mmol), compound **S1-3** (4.26 g, 20.0 mmol), and tetra-*n-*butylammonium bromide (0.13 g, 0.4 mmol) were added, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), retained, dried with anhydrous sodium sulfate, filtered, and concentrated to obtain the crude product of **S17-3.** The crude product was purified by column chromatography, concentrated, and dried with an oil pump to obtain the target compound **S17-3** (31.16 g).
Step c: The above compound **S17-3** (28.90 g, 2.5 mmol), compound **S1-5** (0.89 g, 2.8 mmol), and TEA (0.38 g, 3.8 mmol) were dissolved in dichloromethane (100 mL), followed by stirring at room temperature overnight. The reaction solution was concentrated, dissolved in 100 mL water, and extracted twice with EtOAc (100 mL*2). The water phase was retained, added with sodium chloride, and extracted twice with dichloromethane (100 mL*2). The organic phases were combined, backwashed once with saturated sodium chloride aqueous solution (100 mL), dried with anhydrous MgSO₄, filtered, and concentrated to obtain the crude product. The crude product was purified by column chromatography, concentrated, and dried with an oil pump to obtain the compound **E17-1** (23.83 g).
Step d: Into a flask under nitrogen protection, the above substitution product **E17-1** (23.54 g, 2.0 mmol) was dissolved in THF solution (50 mL), TBAF (50 mL, 1N in THF) was added, and the reaction was conducted overnight to remove the TBS protecting group. The reaction solution was dried with anhydrous sodium sulfate, filtered, and concentrated to obtain the crude product. The crude product was purified by column chromatography, and the target eluent was collected and concentrated to obtain the glycol-PEGylated lipid **E17-2** (20.20 g). ¹H NMR (500 MHz, CDCl₃) δ: 3.83-3.45 (m, 1006H), 3.35 (t, 2H), 3.30 (m, 1H), 3.18 (t, 2H), 2.28 (t, 2H), 1.56-1.46 (m, 4H), 1.39-1.19 (m, 42H), 0.87 (t, 6H). The molecular weight of **E17-2** was determined to be 11452 Da by MALDI-TOF, PDI=1.02.

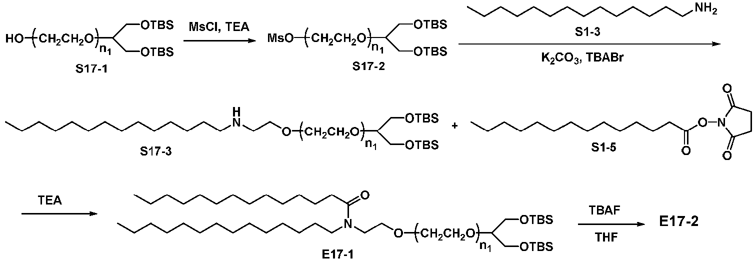

### Example-18: Synthesis of lysine-PEGylated lipid E18-2

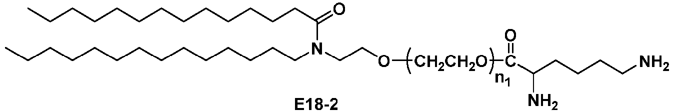

**E18-2** corresponds to the general formula (2), wherein, R₁ is a tridecyl group, R₂ is a tetradecyl group, B₃ and B₄ are both linking bonds, L₇ is a carbonyl group, L₈ is a linking bond, L₃ is -CH₂CH₂O-, A is -CH₂CH₂O-, Rₐ and R_{b} are both hydrogen atoms, s is 2, n₁≈45, R is 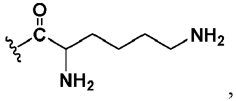 and the total molecular weight is approximately 2580 Da.

The preparation process is as follows:
Step a: Compound **S18-1** (13.00 g, 5.0 mmol, Mw≈2600, n₁≈45, PDI=1.02, a product of coupling reaction between lysine containing two Fmoc protecting groups and PEG) and toluene (200 mL) underwent the azeotropic water removal at 140°C. After 60 mL of solvent was steamed out, the temperature of reaction solution was cooled to room temperature. TEA (1.01 g, 10.0 mmol) and MsCl (1.03 g, 9.0 mmol) were added, and the reaction was stirred at room temperature overnight. After completion of the reaction, the reaction solution was poured into water (200 mL) and extracted twice with EtOAc (100 mL*2). The water phase was retained and extracted twice with dichloromethane (100 mL*2). The organic phases were combined, dried, filtered, concentrated, dissolved in isopropanol at 50°C, recrystallized in an ice bath, and filtered to obtain the compound **S18-2** (12.18 g).
Step b: The above compound **S18-2** (10.60 g, 4.0 mmol) was added into 80 mL water and stirred at room temperature to dissolve. Potassium carbonate (5.52 g, 40.0 mmol), compound **S1-3** (4.26 g, 20.0 mmol), and tetra-n-butylammonium bromide (0.13 g, 0.4 mmol) were added, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), retained, dried with anhydrous sodium sulfate, filtered, and concentrated to obtain the crude product of **S18-3.** The crude product was purified by column chromatography, concentrated, and dried with an oil pump to obtain the target compound **S18-3** (7.80 g).
Step c: The above compound **S18-3** (7.02 g, 2.5 mmol), compound **S1-5** (0.89 g, 2.8 mmol), and TEA (0.38 g, 3.8 mmol) were dissolved in dichloromethane (100 mL), followed by stirring at room temperature overnight. The reaction solution was concentrated, dissolved in 100 mL water, and extracted twice with EtOAc (100 mL*2). The water phase was retained, added with sodium chloride, and extracted twice with dichloromethane (100 mL*2). The organic phases were combined, backwashed once with saturated sodium chloride aqueous solution (100 mL), dried with anhydrous MgSO₄, filtered, and concentrated to obtain the crude product. The crude product was purified by column chromatography, concentrated, and dried with an oil pump to obtain the compound **E18-1** (6.24 g).
Step d: Removal of Fmoc protecting group. The above concentrated product **E18-1** (6.04 g, 2.0 mmol) was treated with 20% piperidine/DMF solution. The solvent was removed by rotary evaporation, and then the residue was dissolved in dichloromethane, precipitated by anhydrous ether, filtered, and recrystallized by isopropanol to obtain the lysinylated product containing two free amino groups **E18-2** (3.50 g). The main ¹H-NMR spectrum data of**E18-2** were as follows: 4.36 (t, 1H). The characteristic peak of Fmoc group disappeared in the ¹H-NMR spectrum. ¹H NMR (500 MHz, CDCl₃) δ: 4.36 (t, 1H), 4.20 (t, 2H), 3.83-3.45 (m, 180H), 3.35 (t, 2H), 3.16 (t, 2H), 2.38 (t, 2H), 2.27 (t, 2H), 2.00-1.76 (t, 2H), 1.56-1.46 (m, 6H), 1.36-1.19 (m, 44H), 0.86 (t, 6H). The molecular weight of **E18-2** was determined to be 2575 Da by MALDI-TOF, PDI=1.02.

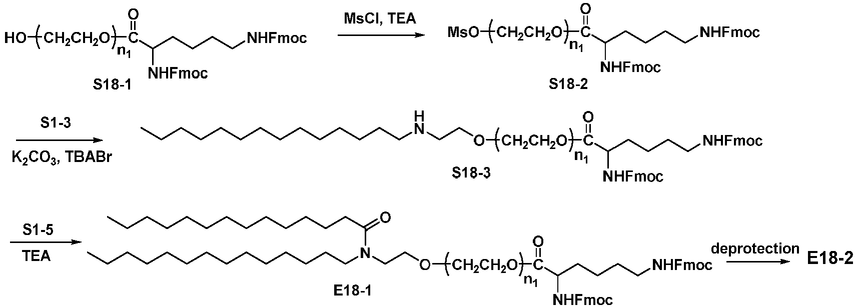

### Example-19: Synthesis of trimeric lysine-PEGylated lipid E19-2

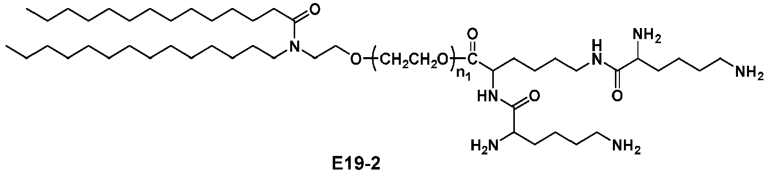

**E19-2** corresponds to the general formula (2), wherein, R₁ is a tridecyl group, R₂ is a tetradecyl group, B₃ and B₄ are both linking bonds, L₇ is a carbonyl group, L₈ is a linking bond, L₃ is -CH₂CH₂O-, A is -CH₂CH₂O-, Rₐ and R_{b} are both hydrogen atoms, s is 2, n₁≈45, R is 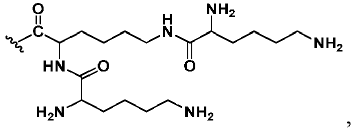 and the total molecular weight is approximately 2830 Da.

The preparation process is as follows:
Step a: Compound **S19-1** (14.00 g, 5.0 mmol, Mw≈2800, n₁≈45, PDI=1.03, a product of coupling reaction between polylysine containing two Boc protecting groups and PEG) and toluene (200 mL) underwent the azeotropic water removal at 140°C. After 60 mL of solvent was steamed out, the temperature of reaction solution was cooled to room temperature. TEA (1.01 g, 10.0 mmol) and MsCl (1.03 g, 9.0 mmol) were added, and the reaction was stirred at room temperature overnight. After completion of the reaction, the reaction solution was poured into water (200 mL) and extracted twice with EtOAc (100 mL*2). The water phase was retained and extracted twice with dichloromethane (100 mL*2). The organic phases were combined, dried, filtered, concentrated, dissolved in isopropanol at 50°C, recrystallized in an ice bath, and filtered to obtain the compound **S19-2** (12.92 g).
Step b: The above compound **S19-2** (11.38 g, 4.0 mmol) was added into 80 mL water and stirred at room temperature to dissolve. Potassium carbonate (5.52 g, 40.0 mmol), compound **S1-3** (4.26 g, 20.0 mmol), and tetra-n-butylammonium bromide (0.13 g, 0.4 mmol) were added in sequence, and the reaction was stirred at room temperature for 72 h. After completion of the reaction, the reaction solution was extracted twice with dichloromethane solution (100 mL*2). The organic phases were combined, backwashed once with saturated sodium chloride aqueous solution (100 mL), retained, dried with anhydrous sodium sulfate, filtered, and concentrated to obtain the crude product of **S19-3.** The crude product was purified by column chromatography, concentrated, and dried with an oil pump to obtain the target compound **S19-3** (8.66 g).
Step c: The above compound **S19-3** (7.52 g, 2.5 mmol), compound **S1-5** (0.89 g, 2.8 mmol), and TEA (0.38 g, 3.8 mmol) were dissolved in dichloromethane (100 mL), followed by stirring at room temperature overnight. The reaction solution was concentrated, dissolved in 100 mL water, and extracted twice with EtOAc (100 mL*2). The water phase was retained, added with sodium chloride, and extracted twice with dichloromethane (100 mL*2). The organic phases were combined, backwashed once with saturated sodium chloride aqueous solution (100 mL), dried with anhydrous MgSO₄, filtered, and concentrated to obtain the crude product. The crude product was purified by column chromatography, concentrated, and dried with an oil pump to obtain the compound **E19-1** (6.85 g).
Step d: Removal of Boc protecting group. Into a dry and clean 500 mL round-bottom flask, a solution of trifluoroacetic acid/dichloromethane (1:2, v/v) was prepared, dichloromethane solution of **E9-1** (6.43 g, 2.0 mmol) was added dropwise slowly in an ice bath, and the reaction was continued for 2 h at room temperature. After the reaction was over, the reaction solution was concentrated, added with purified water, and extracted with dichloromethane. The extract was dried with anhydrous MgSO₄, filtered, concentrated, and recrystallized to obtain the trimeric lysine-PEGylated lipid **E19-2** (5.25 g). ¹H NMR (500 MHz, CDCl₃) δ: 4.67 (t, 1H), 4.36 (t, 2H), 4.20 (t, 2H), 3.83-3.45 (m, 180H), 3.35 (t, 2H), 3.25 (t, 2H), 3.18 (t, 2H), 2.36 (t, 4H), 2.25 (t, 2H), 2.00-1.76 (t, 6H), 1.56-1.46 (m, 10H), 1.36-1.19 (m, 48H), 0.86 (t, 6H). The molecular weight of **E19-2** was determined to be 2831 Da by MALDI-TOF, PDI=1.03.

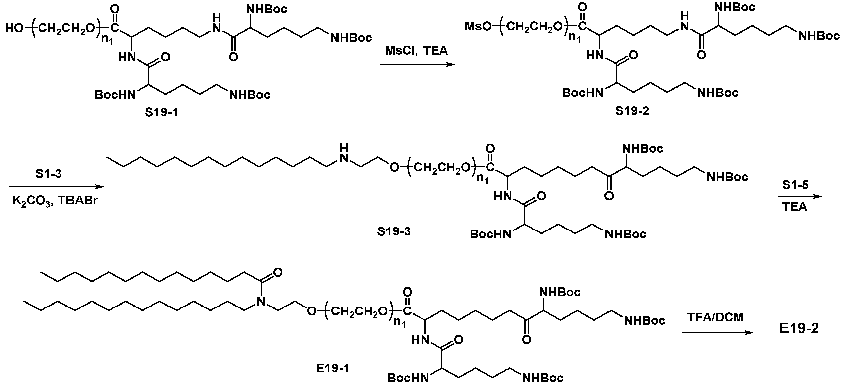

### Example-20: Synthesis of methoxy-PEGylated lipid E20-1

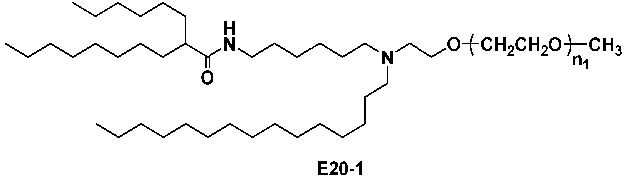

**E20-1** corresponds to the general formula (2), wherein, R₁ is 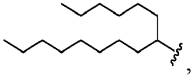 R₂ is a pentadecyl group, B₃ is a hexylene group, B₄ is a linking bond, L₇ is an amide bond (-C(=O)NH-), L₈ is a linking bond, L₃ is -CH₂CH₂-, A is -OCH₂CH₂-, Rₐ and R_{b} are both hydrogen atoms, s is 2, n₁≈40, R is an methoxy group, and the total molecular weight is approximately 2380 Da.

### Method-1:

The preparation process is as follows:
Step a: Compound **S20-1** (5.12 g, 20.0 mmol), EDCI (4.61 g, 24.0 mmol), and NHS (2.51 g, 22.0 mmol) were dissolved in dichloromethane (100 mL), and the reaction was stirred at room temperature overnight. After completion of the reaction, the reaction solution was backwashed twice with 0.1 mol/L HCl solution (50 mL*2), and backwashed once with saturated sodium chloride aqueous solution (50 mL). The dichloromethane phase was collected, dried with anhydrous MgSO₄, filtered, and concentrated to obtain the target compound **S20-2** (6.46 g).
Step b: The above compound **S20-2** (6.35 g, 18.0 mmol) was dissolved in dichloromethane solution (100 mL), compound **S20-3** (4.57 g, 19.8 mmol) and TEA (2.73 g, 27.0 mmol) were added, and the reaction was stirred at room temperature overnight, during which a large amount of solid was precipitated. After filtration, the obtained solid was slurried with methanol (30 mL), filtered, rinsed twice with methanol, collected, and dried to obtain the **substitution product** (4.51 g). Into a flask under nitrogen protection, the above **substitution product** (4.37 g, 15.0 mmol) was dissolved in THF solution (50 mL), TBAF (50 mL, 1N in THF) was added, and the reaction was conducted overnight to remove the TBS protecting group. The reaction solution was dried with anhydrous sodium sulfate, filtered, and concentrated to obtain the crude product. The crude product was purified by column chromatography, and the target eluent was collected and concentrated to obtain the product **S20-4** (4.50 g).
Step c: Compound **S20-4** (4.26 g, 12.0 mmol) and toluene (200 mL) underwent the azeotropic water removal at 140°C. After 60 mL of solvent was steamed out, the temperature of reaction solution was cooled to room temperature. TEA (2.42 g, 24.0 mmol) and MsCl (2.46 g, 21.6 mmol) were added, and the reaction was stirred at room temperature overnight. After completion of the reaction, the reaction solution was poured into water (200 mL) and extracted twice with EtOAc (100 mL*2). The water phase was retained and extracted twice with dichloromethane (100 mL*2). The organic phases were combined, dried, filtered, concentrated, dissolved in isopropanol at 50°C, recrystallized in an ice bath, and filtered to obtain the compound **S20-5** (4.70 g).
Step d: The above compound **S20-5** (4.33 g, 10.0 mmol) was added into 80 mL water and stirred at room temperature to dissolve. Potassium carbonate (13.80 g, 100.0 mmol), compound **S20-6** (11.35 g, 50.0 mmol), and tetra-*n*-butylammonium bromide (0.32 g, 1.0 mmol) were added, and the reaction was stirred at room temperature for 72 h. After completion of the reaction, the reaction solution was extracted twice with dichloromethane solution (100 mL*2). The organic phases were combined, backwashed once with saturated sodium chloride aqueous solution (100 mL), retained, dried with anhydrous sodium sulfate, filtered, and concentrated to obtain the crude product of **S20-7.** The crude product was purified by column chromatography, concentrated, and dried with an oil pump to obtain the target compound **S20-7** (3.98 g).
Step e: The compound **S20-8** (1.90 g, 1.0 mmol, Mw≈1900, n₁≈40, PDI=1.03) was added into 80 mL water and stirred at room temperature to dissolve. Potassium carbonate (1.38 g, 10.0 mmol), secondary amine compound **S20-7** (2.83 g, 5.0 mmol), and tetra-*n*-butylammonium bromide (0.03 g, 0.1 mmol) were added, and the reaction was stirred at room temperature for 72 h. After completion of the reaction, the reaction solution was extracted twice with dichloromethane solution (100 mL*2). The organic phases were combined, backwashed once with saturated sodium chloride aqueous solution (100 mL), and then the organic phase was retained, dried with anhydrous sodium sulfate, filtered, and concentrated to obtain the crude product of compound **E20-1.** The crude product was purified by column chromatography, concentrated, and dried with an oil pump to obtain the target compound methoxy-PEGylated lipid **E20-1** (1.75 g). ¹H NMR (500 MHz, CDCl₃) δ: 3.83-3.46 (m, 162H), 3.37 (s, 3H), 3.25-3.20 (q, 2H), 2.64 (t, 2H), 2.48 (m, 4H), 2.27-2.25 (m, 1H), 1.56-1.46 (m, 10H), 1.32-1.25 (m, 48H), 0.87 (t, 9H). The molecular weight of **E20-1** was determined to be 2382 Da by MALDI-TOF, PDI=1.03.

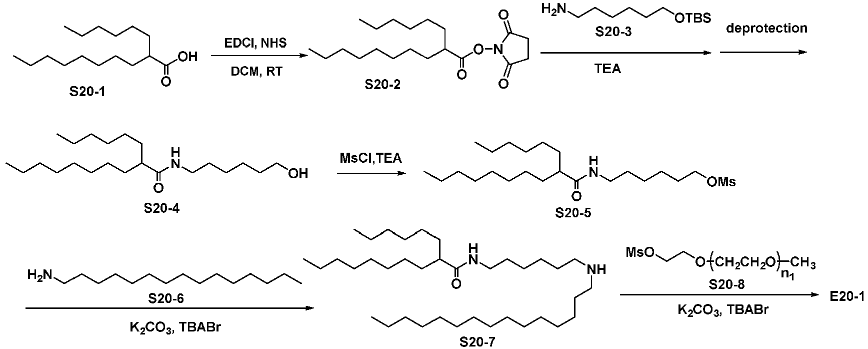

### Method-2:

The preparation process is as follows:
Step a: The above compound **S20-2** (6.35 g, 18.0 mmol) was dissolved in dichloromethane (100 mL), compound **S20-9** (4.57 g, 19.8 mmol) and TEA (2.73 g, 27.0 mmol) were added, and the reaction was stirred at room temperature overnight, during which a large amount of solid was precipitated. After filtration, the obtained solid was slurried with methanol (30 mL), filtered, rinsed twice with methanol, collected, and dried to obtain the compound **S20-10** (5.45 g).
Step b: The above compound **S20-10** (3.54 g, 10.0 mmol) was added into 80 mL water and stirred at room temperature to dissolve. Potassium carbonate (13.80 g, 100.0 mmol), compound **S20-11** (11.35 g, 50.0 mmol), and tetra-n-butylammonium bromide (0.32 g, 1.0 mmol) were added, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), retained, dried with anhydrous sodium sulfate, filtered, and concentrated to obtain the crude product of compound **S20-7.** The crude product was purified by column chromatography, concentrated, and dried with an oil pump to obtain the target compound **S20-7** (4.07 g).
Step c: The compound **S20-8** (1.90 g, 1.0 mmol, Mw≈1900, n₁≈40, PDI=1.03) was added into 80 mL water and stirred at room temperature to dissolve. Potassium carbonate (1.38 g, 10.0 mmol), secondary amine compound **S20-7** (2.83 g, 5.0 mmol), and tetra-n-butylammonium bromide (0.03 g, 0.1 mmol) were added, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), and then the organic phase was retained, dried with anhydrous sodium sulfate, filtered, and concentrated to obtain the crude product of compound **E20-1.** The crude product was purified by column chromatography, concentrated, and dried with an oil pump to obtain the target compound methoxy-PEGylated lipid **E20-1** (1.82 g). ¹H NMR (500 MHz, CDCl₃) δ: 3.83-3.46 (m, 162H), 3.37 (s, 3H), 3.26 (t, 2H), 3.25-3.20 (q, 2H), 3.14 (t,2H), 2.27-2.25 (m, 3H), 1.56-1.46 (m, 8H), 1.32-1.25 (m, 50H), 0.87 (t, 9H). The molecular weight of **E20-1** was determined to be 2382 Da by MALDI-TOF, PDI=1.03.

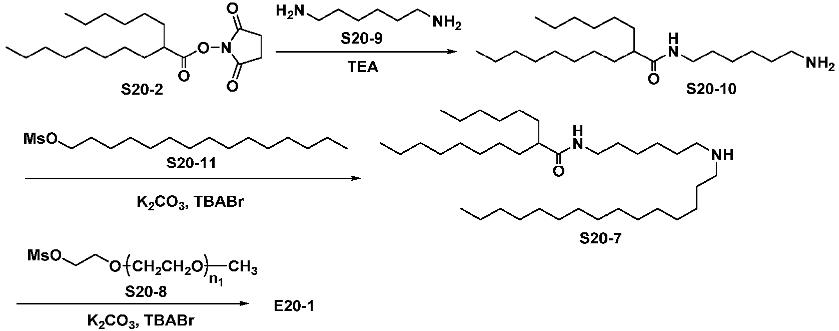

### Example-21: Synthesis of methoxy-PEGylated lipid E21-1

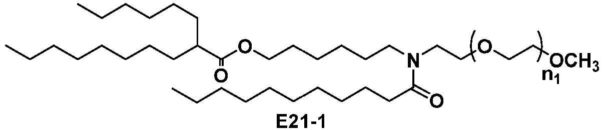

**E21-1** corresponds to the general formula (2), wherein, R₁ is 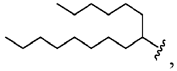 R₂ is a decyl group, B₃ is a hexylene group, B₄ is a linking bond, L₇ is a carboxyl group (-C(=O)O-), L₈ is a carbonyl group, L₃ is -CH₂CH₂-, A is -OCH₂CH₂-, Rₐ and R_{b} are both hydrogen atoms, s is 2, n₁≈45, R is an methoxy group, and the total molecular weight is approximately 2560 Da.

The preparation process is as follows:
Step a: Into a flask under nitrogen protection, compound 2-hexyldecanoic acid **(S21-1,** 10.00 g, 39.1 mmol) was dissolved in anhydrous dichloromethane (DCM, 100 mL). After the temperature of the above mixture was cooled to 0~10°C, 1,6-hexanediol **(S21-2,** 9.23 g, 78.1 mmol) and DMAP (5.72 g, 46.9 mmol) were added carefully, and then EDCI (81.60 g, 430.0 mmol) was added in batches. The reaction solution was returned to room temperature, and the reaction was continued. After reaction for 16 h, **S21-1** was completely consumed according to the TLC, then the reaction solution was washed twice with 500 mL mixed solution of 0.4N HCl/10% NaCl, and then washed once with saturated saline. The organic phases were dried with anhydrous MgSO₄, filtered, and concentrated to obtain the crude product. The crude product was separated and purified by silica gel column chromatography. The target eluent was collected and concentrated to obtain the product **S21-3** (8.12 g).
Step b: The above condensation product **(S21-3,** 5.00 g, 14.1 mmol) was dissolved in 500 mL DCM solution. After the temperature of the above solution was cooled to 0°C, 2,2,6,6-tetramethylpiperidinooxy **(Tempo,** 1.10 mg) and **KBr** solution (2.01 g, 16.9 mmol) dissolved in 50 mL purified water were added, and the **NaClO** solution (18.3 mmol) was added slowly dropwise. After completion of the dropwise addition, the reaction was continued until the starting materials were consumed completely according to the TLC. Then, sodium sulfite solution was added to quench the reaction. The reaction was returned to room temperature, and extracted twice with 50 mL DCM. The organic phases were dried with anhydrous MgSO₄, filtered, and concentrated to obtain the crude product. The crude product was separated and purified by silica gel column chromatography. The target eluent was collected and concentrated to obtain the oxidation product **S21-4** (2.40 g).
Step c: The above oxidation product **S21-4** (2.00 g, 5.7 mmol) was dissolved in a mixed solution of 50 mL THF and 50 mL methanol. After the temperature of the above solution was cooled to 0°C, methoxy polyethylene glycol ethylamine **(S21-5,** 12.92 g, 6.3 mmol, Mw≈2050, n₁≈45, PDI=1.02) and **glacial acetic acid** (0.38 g, 6.3 mmol) were added, and then **NaBH(OAc)₃** (5.38 g, 19.4 mmol) was added slowly in batches. After the feeding, the reaction was continued for 2 h, and the starting materials were consumed completely according to the TLC. After completion of the reaction, saturated sodium bicarbonate solution was added to quench the reaction. The reaction solution was returned to room temperature and concentrated to remove THF and methanol. The concentrated liquid was extracted twice with 200 mL DCM. The organic phases were combined, dried with anhydrous MgSO₄, filtered, and concentrated to obtain the crude product. The crude product was separated and purified by silica gel column chromatography. The target eluent was collected and concentrated to obtain the end-product **S21-6** (8.41 g).
Step d: The above compound **S21-6** (8.00 g, 3.3 mmol), compound **S21-7** (0.82 g, 4.0 mmol), and TEA (0.51 g, 5.0 mmol) were dissolved in dichloromethane (100 mL), followed by stirring at room temperature overnight. The reaction solution was concentrated, dissolved in 100 mL water, and extracted twice with EtOAc (100 mL*2). The water phase was retained, added with sodium chloride, and extracted twice with dichloromethane (100 mL*2). The organic phases were combined, backwashed once with saturated sodium chloride aqueous solution (100 mL), dried with anhydrous MgSO₄, filtered, and concentrated to obtain the crude product. The crude product was purified by column chromatography, concentrated, and dried with an oil pump to obtain the target compound **E21-1**(7.02 g). ¹H NMR (500 MHz, CDCl₃) δ: 4.09 (m, 2H), 3.83-3.43 (m, 182H), 3.37 (s, 3H), 3.35 (t, 2H), 3.15 (t, 2H), 2.26-2.25 (m, 3H), 1.56-1.46 (m, 10H), 1.32-1.25 (m, 38H), 0.88 (t, 9H). The molecular weight of **E21-1** was determined to be 2561 Da by MALDI-TOF, PDI=1.02.

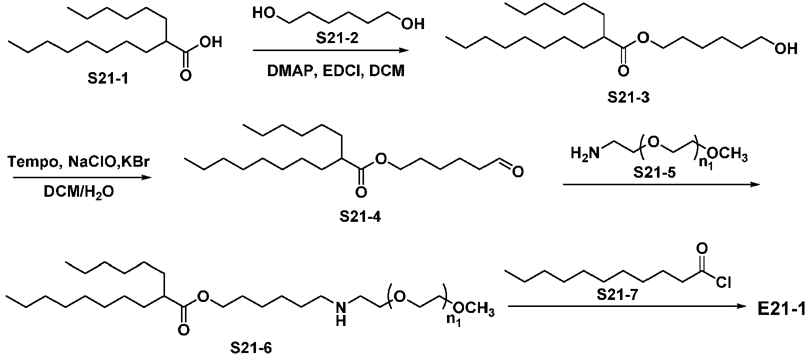

### Example-22: Synthesis of folic acid-PEGylated lipid E22-1

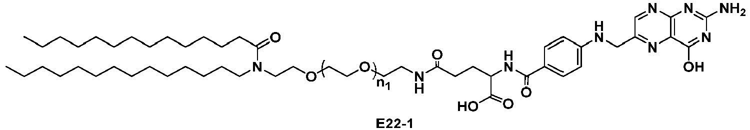

**E22-1** corresponds to the general formula (2), wherein, R₁ is a tridecyl group, R₂ is a tetradecyl group, B₃ and B₄ are both linking bonds, L₇ is a carbonyl group, L₈ is a linking bond, L₃ is -CH₂CH₂O-, A is -CH₂CH₂O-, Rₐ and R_{b} are both hydrogen atoms, s is 2, n₁≈45, R is 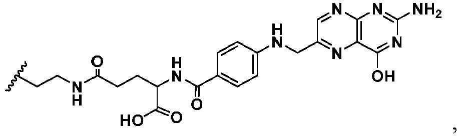 and the total molecular weight is approximately 2910 Da.

Step a: Compound **S22-1** (13.60 g, 6.8 mmol, Mw≈2000, n₁≈45, PDI=1.03) was dissolved in dichloromethane (300 mL), TEA (1.01 g, 10.1 mmol) and di-t-butyl decarbonate **(Boc₂O,** 2.02 g, 10.1mmol) were added at room temperature, and the reaction was conducted overnight. After completion of the reaction, the reaction solution was concentrated and dissolved in isopropanol at 50°C, recrystallized in an ice bath, and filtered to obtain the compound **S22-2** (12.50 g).

Step b: Compound **S22-2** (10.70 g, 5.0 mmol) and toluene (200 mL) underwent the azeotropic water removal at 140°C. After 60 mL of solvent was steamed out, the temperature of reaction solution was cooled to room temperature. TEA (1.01 g, 10.0 mmol) and MsCl (1.02 g, 9.0 mmol) were added, and the reaction was stirred at room temperature overnight. After completion of the reaction, the reaction solution was poured into water (200 mL) and extracted twice with EtOAc (100 mL*2). The water phase was retained and extracted twice with dichloromethane (100 mL*2). The organic phases were combined, dried, filtered, concentrated, dissolved in isopropanol at 50°C, recrystallized in an ice bath, and filtered to obtain the compound **S22-3** (9.00 g).

Step c: The above compound **S22-3** (8.88 g, 4.0 mmol) was added into 80 mL water and stirred at room temperature to dissolve. Potassium carbonate (5.52 g, 40.0 mmol), compound **S1-3** (4.26 g, 20.0 mmol), and tetra-n-butylammonium bromide (0.12 g, 0.4 mmol) were added, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), retained, dried with anhydrous sodium sulfate, filtered, and concentrated to obtain the crude product of compound **S22-4.** The crude product was purified by column chromatography, concentrated, and dried with an oil pump to obtain the target compound **S22-4** (7.27 g).

Step d: The above compound **S22-4** (7.14 g, 3.0 mmol), compound **S1-5** (1.46 g, 4.5 mmol), and TEA (0.76 g, 7.5 mmol) were dissolved in dichloromethane (100 mL), followed by stirring at room temperature overnight. The reaction solution was concentrated, dissolved in 100 mL water, and extracted twice with EtOAc (100 mL*2). The water phase was retained, added with sodium chloride, and extracted twice with dichloromethane (100 mL*2). The organic phases were combined, backwashed once with saturated sodium chloride aqueous solution (100 mL), dried with anhydrous MgSO₄, filtered, and concentrated to obtain the crude product. The crude product was purified by column chromatography, concentrated, and dried with an oil pump to obtain the target compound **S22-5** (6.41 g).

Step e: Removal of Boc protecting group. Into a dry and clean 500 mL round-bottom flask, a solution of trifluoroacetic acid/dichloromethane (1:2, v/v) was prepared, dichloromethane solution of **S22-5** (6.00 g, 2.3 mmol) was added dropwise slowly in an ice bath, and the reaction was continued for 2 h at room temperature. After the reaction was over, the reaction solution was concentrated, added with purified water, and then extracted with dichloromethane. The extract was dried with anhydrous MgSO₄, filtered, concentrated, and recrystallized to obtain the **S22-6** (5.35 g).

Step f: Into a dry and clean 1000 mL round-bottom flask, folic acid **(S22-7,** 1.49 g, 3.4 mmol) dissolved in DMSO, ethylamine-PEGylated lipid **(S22-6,** 5.00 g, 2.0 mmol), pyridine (20 mL), and DCC (1.85 g, 9.0 mmol) were added, followed by stirring for 4 h at room temperature. The reaction was monitored by TLC, the reaction was continued until the end-product was occurred and the starting material ethylamine-PEGylated lipid was consumed completely in the reaction mixture according to the TLC. After completion of the reaction, pyridine was removed by rotary evaporation under reduced pressure, then 300 mL water was added to the solution, and centrifugation was carried out to remove trace insoluble substances. The supernatant obtained from the centrifugation was dialyzed in saline and water until the TLC monitoring indicated that the dialysate contained only the end-product monitored by TLC (i.e., only one spot in the TLC plate). The aqueous solution of dialysate was freeze-dried to obtain the end-product PEGylated lipid folic acid derivative **(E22-1,** 5.35 g). ¹H NMR (500 MHz, CDCl₃) δ: 8.71 (s, 1H), 7.66-7.64 (d, 2H), 6.67 (d, 2H), 4.49-4.48 (d, 2H), 4.32-4.20 (m, 1H), 3.83-3.45 (m, 184H), 3.35 (t, 2H), 3.25-3.20 (q, 2H), 3.19 (t, 2H), 2.34 (t, 2H), 2.28 (t, 2H), 2.08-1.94 (m, 2H), 1.56-1.46 (m, 4H), 1.36-1.19 (m, 42H), 0.86 (t, 6H). The molecular weight of **E22-1** was determined to be 2913 Da by MALDI-TOF, PDI=1.03.

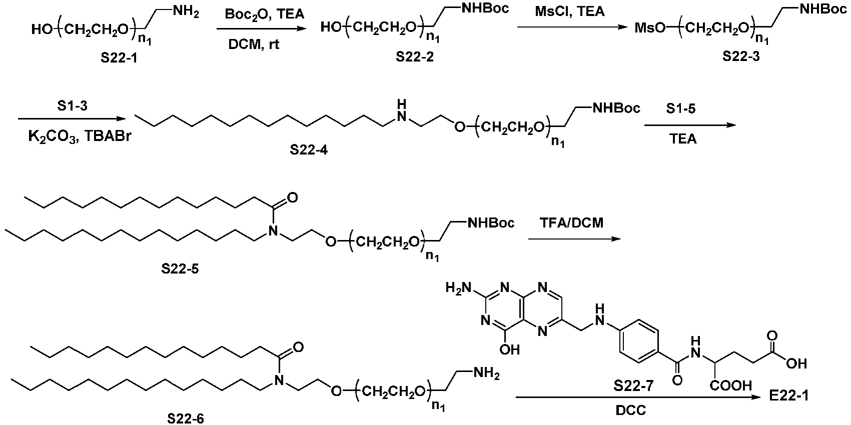

### Example-23: Synthesis of biotin-PEGylated lipid E23-1

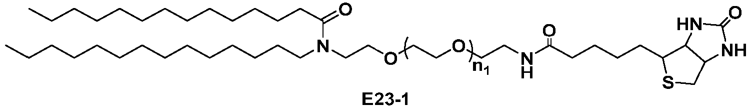

**E23-1** corresponds to the general formula (2), wherein, R₁ is a tridecyl group, R₂ is a tetradecyl group, B₃ and B₄ are both linking bonds, L₇ is a carbonyl group, L₈ is a linking bond, L₃ is -CH₂CH₂O-, A is -CH₂CH₂O-, Rₐ and R_{b} are both hydrogen atoms, s is 2, n₁≈45, R is 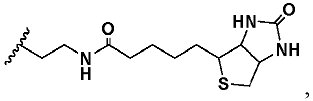 and the total molecular weight is approximately 2720 Da.

The preparation process is as follows:
Into a dry and clean 1000 mL round-bottom flask, biotin succinimidyl ester **(S23-1,** 1.87 g, 5.5 mmol) dissolved in DMF and ethylamine-PEGylated lipid **(S22-6,** 12.50 g, 5.0 mmol, Mw≈2500, n₁≈45, PDI=1.03) dissolved in DCM were added, followed by stirring well. Then, TEA (1.52 g, 15.0 mmol) was added, and the reaction was continued at room temperature until completion as indicated by TLC. After completion of the reaction, the reaction solution was filtered, concentrated, and purified by column chromatography. The eluents were collected, combined, concentrated, and freeze-dried to obtain the PEGylated lipid biotin derivative **(E23-1,** 9.82 g). ¹H NMR (CDCl₃) δ: 4.36-4.25 (m, 2H), 3.83-3.45 (m, 184H), 3.35 (t, 2H), 3.20 (m,1H), 3.18 (t, 2H), 2.81 (t, 2H), 2.26 (t, 2H), 2.22-2.18 (m, 2H), 1.70-1.40 (m, 8H), 1.36-1.21 (m, 42H), 0.87 (t, 6H). The molecular weight of **E23-1** was determined to be 2716 Da by MALDI-TOF, PDI=1.03.

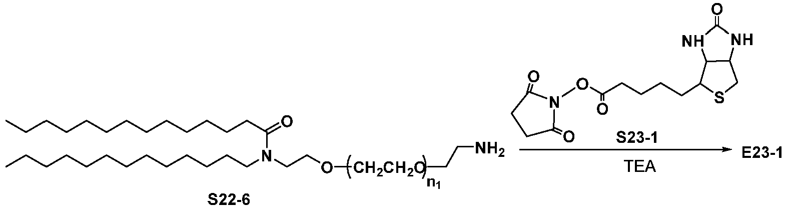

### Example-24: Synthesis of methoxy-PEGylated lipid E24-1

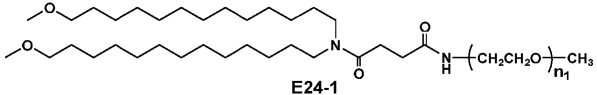

**E24-1** corresponds to the general formula (2), wherein, R₁ and R₂ are both methyl groups, B₃ and B₄ are both tridecylene groups, L₇ and L₈ are both ether bonds (-O-), L₃ is -C(=O)CH₂CH₂C(=O)NH-, A is -CH₂CH₂O-, Rₐ and R_{b} are both hydrogen atoms, s is 2, n₁≈45, R is a methyl group, and the total molecular weight is approximately 2530 Da.

The preparation process is as follows:
Methoxy polyethylene glycol succinate derivative **S24-1** containing an amide bond (10.50 g, 5.0 mmol, Mw≈2100, n₁≈45, PDI=1.02), compound **S6-4** (3.32 g, 7.5 mmol), EDCI (1.92 g, 10.0 mmol), HOBt (1.01 g, 7.5 mmol), and TEA (1.01 g, 10.0 mmol) were dissolved in dichloromethane (100 mL) in sequence, followed by stirring at room temperature overnight. After completion of the reaction, the reaction solution was backwashed twice with 0.1 mol/L HCl aqueous solution (10% NaCl) (100 mL*2) and backwashed once with saturated sodium chloride aqueous solution (100 mL). The organic phase was dried with anhydrous MgSO₄, filtered, and concentrated to obtain the crude product. The crude product was purified by column chromatography, concentrated, and dried with an oil pump to obtain the target compound methoxy-PEGylated lipid **E24-1** (10.55 g). ¹H NMR (500 MHz, CDCl₃) δ: 3.83-3.45 (m, 184H), 3.38 (s, 3H), 3.32 (s, 6H), 3.26-3.17 (m, 4H), 2.43-2.36 (m, 4H), 1.66-1.42 (m, 8H), 1.35-1.19 (m, 36H). The molecular weight of **E24-1** was determined to be 2536 Da by MALDI-TOF, PDI=1.02.

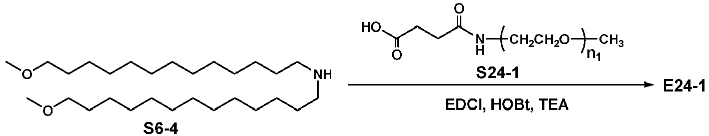

### Example-25: Synthesis of methoxy-PEGylated lipid E25-1

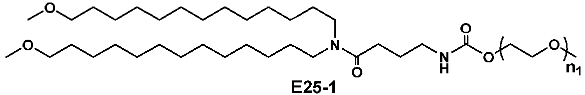

**E25-1** corresponds to the general formula (2), wherein, R₁ and R₂ are both methyl groups, B₃ and B₄ are both tridecylene groups, L₇ and L₈ are both ether bonds (-O-), L₃ is -C(=O)CH₂CH₂CH₂NHC(=O)O-, A is -CH₂CH₂O-, Rₐ and R_{b} are both hydrogen atoms, s is 2, n₁≈45, R is a methyl group, and the total molecular weight is approximately 2560 Da.

The preparation process is as follows:
Step a: The compound **S25-1** (1.91 g, 12.0 mmol) and **S25-2** (16.80 g, 8.0 mmol, Mw≈2100, n₁≈45, PDI=1.02) were dissolved in DCM and cooled in an ice bath. Pyridine (20 mL) was added into the mixture, followed by stirring overnight. After completion of the reaction, the solvent was removed, and the residue was dried under high vacuum. The residue was dissolved with dichloromethane, and washed with bicarbonate solution and water. The crude product was purified by column chromatography to obtain compound **S25-3** (15.84 g).
Step b: The above compound **S25-3** (15.40 g, 7.0 mmol, Mw≈2200, n₁≈45, PDI=1.02) was dissolved in dichloromethane (80 mL), trifluoroacetic acid (80 mL) was added, and the reaction was conducted for 12 h at room temperature. After completion of the reaction, the solvent was evaporated off under reduced pressure to obtain **S25-4** (10.85 g).
Step c: **S25-1** (10.50 g, 5.0 mmol, Mw≈2100, n₁≈45, PDI=1.02), compound **S6-4** (3.32 g, 7.5 mmol), EDCI (1.92 g, 10.0 mmol), HOBt (1.01 g, 7.5 mmol), and TEA (1.01 g, 10.0 mmol) were dissolved in dichloromethane (100 mL) in sequence, followed by stirring at room temperature overnight. After completion of the reaction, the reaction solution was backwashed twice with 0.1 mol/L HCl aqueous solution (10% NaCl) (100 mL*2) and backwashed once with saturated sodium chloride aqueous solution (100 mL). The organic phase was dried with anhydrous MgSO₄, filtered, and concentrated to obtain the crude product. The crude product was purified by column chromatography, concentrated, and dried with an oil pump to obtain the target compound methoxy-PEGylated lipid **E25-1** (10.30 g). ¹H NMR (500 MHz, CDCl₃) δ: 4.04-3.45 (m, 184H), 3.38 (s, 3H), 3.32 (s, 6H), 3.26-3.17 (m, 4H), 3.23 (m, 2H), 2.34 (m, 2H), 1.84 (m, 2H), 1.66-1.42 (m, 8H), 1.35-1.19 (m, 36H). The molecular weight of **E25-1** was determined to be 2566 Da by MALDI-TOF, PDI=1.02.

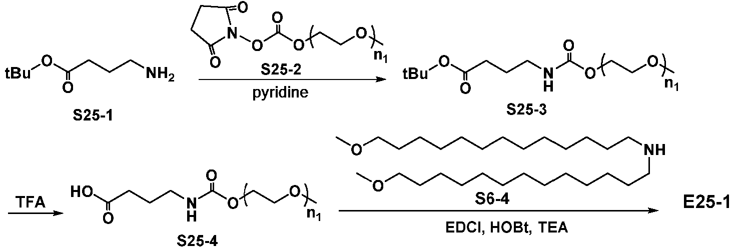

### Example-26: Synthesis of methoxy-PEGylated lipid E26-1

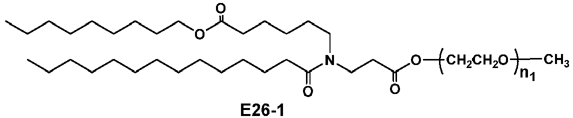

**E26-1** corresponds to the general formula (2), wherein, R₁ is a nonyl group, R₂ is a tridecyl group, B₃ is a pentylene group, B₄ is a linking bond, L₇ is -OC(=O)-, L₈ is -C(=O)-, L₃ is -CH₂CH₂C(=O)O-, A is -CH₂CH₂O-, Rₐ and R_{b} are both hydrogen atoms, s is 2, n₁≈45, R is a methyl group, and the total molecular weight is approximately 2530.

### Method-1:

The preparation process is as follows:
Step a: Under nitrogen protection, acetonitrile was added, bromide **S10-3** (2.41 g, 7.5 mmol), methoxy polyethylene glycol alanine ester derivative **S26-1** (12.60 g, 6.0 mmol, Mw≈2100, n₁≈45, PDI=1.03, a product of reaction between methoxy-polyethylene glycol and β-alanine containing a Boc protected amino group), and DIPEA (1.04 mL, 6.0 mmol) were added in sequence with stirring slowly, and the reaction was stirred at room temperature for about 20 h. After completion of the reaction, the reaction solution was concentrated, dissolved in dichloromethane, and extracted with 0.6 M HCl solution (10% NaCl) and saturated sodium bicarbonate solution in sequence. After ensuring the absence of material loss in the water phase, the organic phases were combined, dried with anhydrous MgSO₄, filtered, concentrated, and purified by silica gel column chromatography to obtain the **S26-2** (10.63 g).
Step b: Compound **S26-2** (9.20 g, 4.0 mmol, Mw≈2300, n₁≈45, PDI=1.03), **S1-5** (1.56 g, 4.8 mmol), and TEA (0.61 g, 6.0 mmol) were dissolved in dichloromethane (100 mL), followed by stirring at room temperature overnight. The reaction solution was concentrated, dissolved in 100 mL water, and extracted twice with EtOAc (100 mL*2). The water phase was retained, added with sodium chloride, and extracted twice with dichloromethane (100 mL*2). The organic phases were combined, backwashed once with saturated sodium chloride aqueous solution (100 mL), dried with anhydrous MgSO₄, filtered, and concentrated to obtain the crude product. The crude product was purified by column chromatography, concentrated, and dried with an oil pump to obtain the target compound **E26-1** (8.16 g). ¹H NMR (500 MHz, CDCl₃) δ: 4.15 (m, 2H), 4.07 (t, 2H), 3.83-3.45 (m, 178H), 3.39 (s, 3H), 3.45-3.18 (m, 4H), 2.36 (t, 2H), 2.25 (t, 2H), 2.61 (m, 2H), 1.71-1.19 (m, 42H), 0.86 (t, 6H). The molecular weight of **E26-1** was determined to be 2535 Da by MALDI-TOF, PDI=1.03.

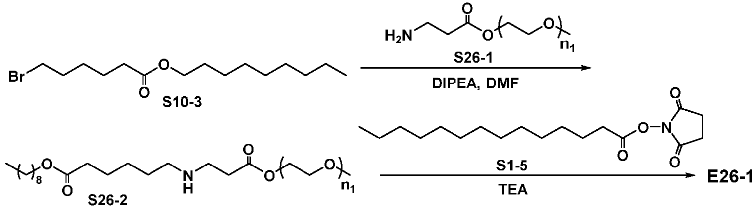

### Method-2:

The preparation process is as follows:
Step a: 3-hydroxypropionate derivative of methoxy-polyethylene glycol **S26-3** (21.00 g, 10.0 mmol, Mw≈2100, n₁≈45, PDI=1.03, a deprotected product of reaction between methoxy-polyethylene glycol and 3-hydroxypropionic acid containing a TBS protected hydroxyl group) and toluene (200 mL) underwent the azeotropic water removal at 140°C. After 60 mL of solvent was steamed out, the reaction solution was cooled to room temperature. TEA (2.02 g, 20.0 mmol) and MsCl (2.05 g, 18.0 mmol) were added and the reaction was stirred at room temperature overnight. After completion of the reaction, the reaction solution was poured into water (200 mL) and extracted twice with EtOAc (100 mL*2). The water phase was retained and extracted twice with dichloromethane (100 mL*2). The organic phases were combined, dried, filtered, concentrated, dissolved in isopropanol at 50°C, recrystallized in an ice bath, and filtered to obtain the compound **S26-4** (19.62 g).
Step b: Compound **S26-4** (17.60 g, 8.0 mmol, Mw≈2200, n₁≈45, PDI=1.03) was added into 80 mL water and stirred at room temperature to dissolve. Potassium carbonate (11.04 g, 80.0 mmol), compound **S26-5** (10.28 g, 40.0 mmol, a deprotected product of reaction between nonanol and 6-aminohexanoic acid containing a Boc protected amino group, referring to the Step a in Example-10), and tetra-n-butylammonium bromide (0.26 g, 0.8 mmol) were added, and the reaction was stirred at room temperature for 72 h. After completion of the reaction, the reaction solution was extracted twice with dichloromethane (100 mL*2). The organic phases were combined, backwashed once with saturated sodium chloride aqueous solution (100 mL), retained, dried with anhydrous sodium sulfate, filtered, concentrated, and purified by column chromatography to obtain the compound **S26-2** (5.72 g).
Step c: Compound **S26-2** (4.60 g, 2.0 mmol, Mw≈2300, n₁≈45, PDI=1.03), **S1-5** (0.78 g, 2.4 mmol), and TEA (0.30 g, 3.0 mmol) were dissolved in dichloromethane (100 mL), followed by stirring at room temperature overnight. The reaction solution was concentrated, dissolved in 100 mL water, and extracted twice with EtOAc (100 mL*2). The water phase was retained, added with sodium chloride, and extracted twice with dichloromethane (100 mL*2). The organic phases were combined, backwashed once with saturated sodium chloride aqueous solution (100 mL), dried with anhydrous MgSO₄, filtered, and concentrated to obtain the crude product. The crude product was purified by column chromatography, concentrated, and dried with an oil pump to obtain the compound **E26-1** (4.03 g). ¹H NMR (500 MHz, CDCl₃) δ: 4.15 (m, 2H), 4.07 (t, 2H), 3.83-3.45 (m, 178H), 3.39 (s, 3H), 3.45-3.18 (m, 4H), 2.36 (t, 2H), 2.25 (t, 2H), 2.61 (m, 2H), 1.71-1.19 (m, 42H), 0.86 (t, 6H). The molecular weight of **E26-1** was determined to be 2535 Da by MALDI-TOF, PDI=1.03.

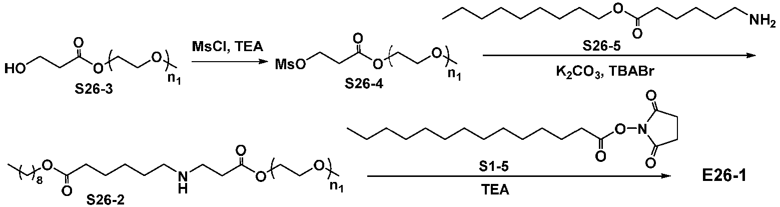

### Example-27: Synthesis of cationic lipid P-9

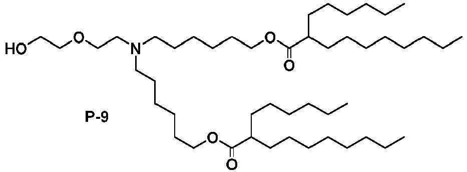

**P-9** corresponds to the general formula (1), wherein, L₁ and L₂ are both -C(=O)O-, L₃ is a linking bond, B₁ and B₂ are both 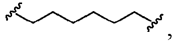 R₁ and R₂ are both 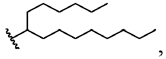 R₃ is a hydrogen atom, A is -(CRₐR_{b})ₛO-, Rₐ and R_{b} are both hydrogen atoms, s is 2, and n is 2.

Step a: Into a flask under nitrogen protection, compound **2-hexyldecanoic acid (S27-1,** 100.00 g, 390.6 mmol) was dissolved in anhydrous dichloromethane (DCM, 1 L). After the temperature of the above mixture was cooled to 0~10°C, **1,6-hexanediol (S27-2,** 92.30 g, 781.3 mmol) and DMAP (57.20 g, 468.7 mmol) were added carefully, and then EDCI (81.60 g, 430.0 mmol) was added in batches. The reaction solution was returned to room temperature, and the reaction was continued. After 16 h of reaction, **S27-1** was completely consumed according to the TLC. The reaction solution was washed twice with 500 mL mixed solution of 0.4N HCl/10% NaCl, and then washed once with saturated saline. The organic phases were dried with anhydrous MgSO₄, filtered, and concentrated to obtain the crude product. The crude product was separated and purified by silica gel column chromatography. The target eluent was collected and concentrated to obtain the product **S27-3** (79.00 g).

Step b: The above condensation product **(S27-3,** 50.0 g, 140.5 mmol) was dissolved in 500 mL DCM solution. After the temperature of the above solution was cooled to 0°C, 2,2,6,6-tetramethylpiperidinooxy (Tempo, 11.00 mg) and **KBr** solution (20.10 g, 168.6 mmol) dissolved in 50 mL purified water were added, and the reaction solution was dissolved in 50 mL purified water, NaClO solution (182.6 mmol) was added dropwise slowly. After completion of the dropwise addition, the reaction was continued until the starting materials were consumed completely according to the TLC. Then, sodium sulfite solution was added to quench the reaction. The reaction was returned to room temperature and extracted twice with 500 mL DCM. The organic phases were combined, dried with anhydrous MgSO₄, filtered, and concentrated to obtain the crude product. The crude product was separated and purified by silica gel column chromatography. The target eluent was collected and concentrated to obtain the oxidation product **S27-4** (23.00 g).

Step c: The above oxidation product **(S27-4,** 20.00 g, 56.5 mmol) was dissolved in a mixed solution of 200 mL THF and 20 mL methanol. After the temperature of the above solution was cooled to 0°C, diglycolamine **(S27-5,** 2.80 g, 26.9 mmol) and glacial acetic acid (1.61 g, 26.9 mmol) were added, and then NaBH(OAc)₃ (17.50 g, 82.5 mmol) was added slowly in batches. After the feeding, the reaction was continued for 2 h, and the starting materials were consumed completely according to the TLC. After completion of the reaction, saturated sodium bicarbonate solution was added to quench the reaction. The reaction solution was returned to room temperature and concentrated to remove THF and methanol. The concentrated liquid was extracted twice with 200 mL DCM. The organic phases were combined, dried with anhydrous MgSO₄, filtered, and concentrated to obtain the crude product. The crude product was separated and purified by silica gel column chromatography. The target eluent was collected and concentrated to obtain the product **P-9** (12.00 g). The main ¹H-NMR spectrum data of **P-9** were as follows: ¹H NMR (400 MHz, CDCl₃) δ 4.07 (t, *J* = 5.9 Hz, 4H), 3.70 (s, 2H), 3.63 (m, 4H), 2.65 (m, 2H), 2.49 (m, 4H), 2.32 (m, 2H), 1.70-1.22 (m, 64H), 0.88 (d, *J =* 6.3 Hz, 12H).

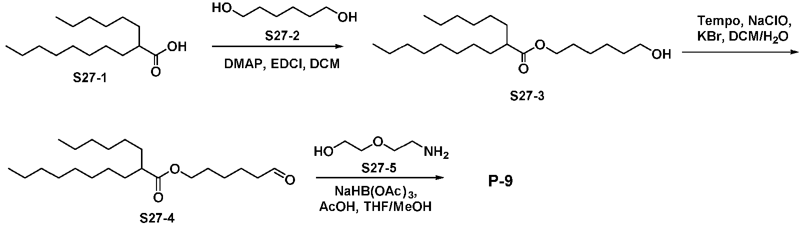

### Example-28: Preparation of cationic liposomes

In this example, multiple groups of cationic liposomes were prepared for comparison. In the compositions of every group of cationic liposomes, the neutral lipids contained were all DSPC, the sterol lipids contained were all cholesterol, and the differences lied in the cationic lipid and the PEGylated lipid components. Wherein, for the control group **(L-0),** the cationic lipid was DLin-MC3-DMA (MC3 for short) and the PEGylated lipid was PEG2k-DMG (DMG for short); for the experimental groups **(L-1** to **L-28,** and **L-31),** the cationic lipid was MC3, and the PEGylated lipids were the PEGylated lipids prepared in the present application; for the experimental groups **(L-29** to **L-30),** the cationic lipid was the cationic lipid **P-9** prepared in the present application, and the PEGylated lipids were the PEGylated lipids **E1-1** or **E22-1** prepared in the present application; specifically as shown in Table 1.

The preparation method of cationic liposomes is as follows:
Step a: The cationic lipid **CL** (15.0 µmol), distearoylphosphatidylcholine (DSPC, 3.0 µmol), cholesterol (12.0 µmol), and the PEGylated lipid **PL** (0.45 µmol) of each group from **L-0** to **L-30** listed in Table 1 were weighed at a molar ratio of 50:10:40:1.5 as cationic lipid : DSPC : cholesterol : PEGylated lipid, and added into a 100 mL round-bottom flask. 30 mL chloroform was added to dissolve the solid, and the solution was thoroughly shaken;
Step b: The solvent chloroform was removed by a rotary evaporator at a speed of 140 rpm and a temperature of 55°C to form a thin oil film. Subsequently, the film was dried with a vacuum pump for 12 h to ensure complete removal of chloroform;
Step c: Into a flask, 30 mL phosphate buffer (PBS, pH=7.4) containing 10% lactose was added. The reaction solution was ultrasonicated by an ultrasonic cleaner at 90% frequency for 30 min to form a translucent emulsion;
Step d: The emulsion was added into a high-pressure homogenizer and overpressurized for 5 times under the pressure of 100 MPa; then, the emulsion was added into the liposome extruder and overpressurized for 10 times under the pressure of 150 MPa to prepare the cationic liposome powders of groups **L-0** to **L-30.**

Following the above Steps a-d, the molar ratio of cationic lipid : DSPC : cholesterol : PEGylated lipid was adjusted to 48:9:42:1.5. The cationic lipid **CL** (16.0 µmol), distearoylphosphatidylcholine (DSPC, 3.0 µmol), cholesterol (14.0 µmol), and the PEGylated lipid **PL** (0.5 µmol) of group **L-31** listed in Table 1 were weighed. The other conditions remained unchanged to prepare the cationic liposome powder of**L-31**.

### Example-29: Preparation of the formulations of cationic liposome-nucleic acid pharmaceutical compositions

Step a: 0.03 mL physiological saline was used as the working solution for the formulations of nucleic acid pharmaceutical compositions;

Step b: 0.1 mg cationic liposome (from **L-0** to **L-31)** prepared in Example-**28** was weighed, dissolved in physiological saline, and equilibrated for 30 min. According to the N/P ratio of 10/1 for the complexation, 1.00 µg siRNA was weighed, dissolved in 10 µL physiological saline, and complexed with the physiological saline containing cationic liposomes for 30 min to prepare 1 control group (**L-0**/siRNA) and said formulations of cationic liposome-nucleic acid pharmaceutical compositions of the present invention ("LNP/siRNA formulation" for short, **L-1**/siRNA ~ **L-31**/siRNA).

### Example-30: Biological activity assays for the formulations of cationic liposome-nucleic acid pharmaceutical compositions

### (1) Determination of gene complexation ability

Gel electrophoresis experiment was used to evaluate the gene complexation ability of LNP/siRNA in each group. 0.8 g agarose was weighed and dissolved in 40 mL TAE solution. The solution was heated in a microwave until the granules of the agarose were dissolved completely, and then cooled. 5 µL nucleic acid dye GelGreen was added into the cooled agarose gel. The agarose gel was added into the gel slot and dried naturally in air. A mixed solution of LNP/siRNA and 2 µL loading buffer was added into the agarose gel well, and the electrophoresis voltage was set to 90 V for the electrophoresis experiment. The electrophoresis was carried out at room temperature for 10 min. The results showed that free siRNA was essentially absent in both the experimental groups (**L-1**/siRNA ~ **L-31**/siRNA) and the control group (**L-0**/siRNA), indicating that the formulations of cationic liposome-nucleic acid pharmaceutical compositions modified by the PEGylated lipids of the present invention had a stronger gene complexation ability of mRNA.

**Table 1 Summary of the liposome formulations and the particle size and encapsulation efficiency of the prepared LNP/siRNA**

| **Liposome** | **L-0** | **L-1** | **L-2** | **L-3** | **L-4** | **L-5** |
|---|---|---|---|---|---|---|
| **PEGylated lipid PL** | DMG | E1-1 | E1-3 | E2-1 | E3-1 | E4-2 |
| **Cationic lipid CL** | MC3 | | | | | |
| **Particle size /nm** | 103.83 | 119.38 | 92.64 | 121.17 | 110.26 | 121.80 |
| **Encapsulation efficiency /%** | 93.2 | 94.8 | 88.9 | 95.2 | 82.6 | 84.6 |

| **Liposome** | **L-6** | **L-7** | **L-8** | **L-9** | **L-10** | **L-11** |
|---|---|---|---|---|---|---|
| **PEGylated lipid PL** | E5-1 | E6-1 | E7-1 | E8-1 | E8-2 | E9-2 |
| **Cationic lipid CL** | MC3 | | | | | |
| **Particle size /nm** | 102.90 | 107.78 | 111.61 | 118.89 | 136.47 | 93.90 |
| **Encapsulation efficiency /%** | 82.2 | 97.9 | 90.1 | 88.9 | 86.8 | 81.8 |

| **Liposome** | **L-12** | **L-13** | **L-14** | **L-15** | **L-16** | **L-17** |
|---|---|---|---|---|---|---|
| **PEGylated lipid PL** | E10-1 | E11-1 | E12-1 | E13-2 | E14-1 | E15-2 |
| **Cationic lipid CL** | MC3 | | | | | |
| **Particle size /nm** | 126.13 | 83.11 | 102.31 | 94.00 | 116.60 | 99.69 |
| **Encapsulation efficiency /%** | 95.0 | 90.1 | 91.4 | 90.5 | 93.4 | 95.6 |

| **Liposome** | **L-18** | **L-19** | **L-20** | **L-21** | **L-22** | **L-23** |
|---|---|---|---|---|---|---|
| **PEGylated lipid PL** | E16-1 | E17-2 | E18-2 | E19-2 | E20-1 | E21-1 |
| **Cationic lipid CL** | MC3 | | | | | |
| **Particle size /nm** | 121.42 | 144.41 | 102.19 | 111.42 | 119.47 | 115.86 |
| **Encapsulation efficiency /%** | 84.4 | 82.5 | 87.5 | 82.0 | 90.9 | 81.3 |

| **Liposome** | **L-24** | **L-25** | **L-26** | **L-27** | **L-28** | **L-29** |
|---|---|---|---|---|---|---|
| **PEGylated lipid PL** | E22-1 | E23-1 | E24-1 | E25-1 | E26-1 | E1-1 |
| **Cationic lipid CL** | MC3 | | | | | P-9 |
| **Particle size /nm** | 94.39 | 116.12 | 115.84 | 122.56 | 111.30 | 103.91 |
| **Encapsulation efficiency /%** | 88.1 | 92.0 | 97.8 | 82.3 | 92.6 | 90.0 |

| **Liposome** | **L-30** | **L-31** | | | | |
|---|---|---|---|---|---|---|
| **PEGylated lipid PL** | E22-1 | E1-1 | | | | |
| **Cationic lipid CL** | P-9 | MC3 | | | | |
| **Particle size /nm** | 111.20 | 92.90 | | | | |
| **Encapsulation efficiency /%** | 87.2 | 93.0 | | | | |

Determination of encapsulation efficiency: The LNP/siRNA was ultracentrifuged (4 °C, 60000 rpm, 1 h) with an ultracentrifuge, the concentration of unencapsulated siRNA in the supernatant was determined by a nucleic acid quantifier, and the encapsulation efficiency of the liposome for siRNA was calculated. The results were summarized in Table 1, showing that the cationic liposomes of the present invention **(L-1~L-31)** had higher encapsulation efficiency for nucleic acid drugs. The DMG is a PEGylated lipid which has been successfully applied to mRNA vaccines. In contrast, the encapsulation efficiency for siRNA of **L-1** (containing the PEGylated lipid **E1-1), L-3** (containing the PEGylated lipid **E2-1), L-7** (containing the PEGylated lipid **E6-1), L-12** (containing the PEGylated lipid **E10-1), L-16** (containing the PEGylated lipid **E14-1), L-17** (containing the PEGylated lipid **E15-2),** and **L-26** (containing the PEGylated lipid **E24-1)** using the same cationic lipid MC3 were all higher than the group containing DMG; **L-30~L-31** using the cationic lipid prepared in the present invention also showed higher encapsulation efficiency; **L-29** adopted a different ratio of lipids, and the prepared cationic liposome showed higher encapsulation efficiency for nucleic acid as well.

Determination of particle size: In this example, the particle size of LNP/siRNA was determined by dynamic light scattering (DLS). The measured sizes of LNP/siRNA had a relatively high uniformity with PDI values all smaller than 0.3. With the PEGylated lipids of the present invention being used, when n₁ was in the range of approximately 40 to 100, the particle sizes of the prepared LNP/siRNA were in the range of 90 to 130 nm.

### (2) Study on the stability in serum

In this experiment, the stability of the PEGylated lipid-modified formulation of cationic liposome-nucleic acid pharmaceutical composition in serum was determined by measuring the variation of the size of liposome particles. In this example, 0.5 mL LNP/siRNA formulation **L-0~L-31** whose N/P ratio was 10/1 was added into the 0.5 mL culture medium containing 10% fetal bovine serum (FBS), followed by stirring at 37°C. Samples were taken at a regular interval to measure the variation of the particle size of the formulation of cationic liposome-nucleic acid pharmaceutical composition, based on which the stability of the formulation of cationic liposome-nucleic acid pharmaceutical composition in serum was analyzed. In other groups, 0.5 mL PBS was added to replace the culture medium to determine the stability of the formulation of cationic liposome-nucleic acid pharmaceutical composition in serum, using the same method as aforementioned. Results of the experiments showed that, in 48 h, compared with **L-0,** the variations in the particle size of the formulation of cationic liposome-nucleic acid pharmaceutical composition of **L-1~L-31** in PBS solution and serum were all relatively small, indicating that the PEGylated lipid-modified cationic liposomes of the present invention were relatively stable, which had good stability in serum and met the requirements of long circulating cationic liposomes.

### (3) Study on the cytotoxicity (biocompatibility)

The cytotoxicity of the formulation of cationic liposome-nucleic acid pharmaceutical composition of the present invention was examined by the MTT assay. The formulations of cationic liposome-nucleic acid pharmaceutical compositions were dissolved in culture medium to the required concentration, and an appropriate amount of solubilizer could be added when necessary. With HeLa cells as a cell model, the cell suspension was inoculated onto a 96 well plate at 100 µL/well and a density of 1×10⁴ cells/well. After the inoculation, the cells were incubated in a cell incubator at 37°C, 4% CO₂ for 24 h. Then, the culture medium was aspirated and 100 µL culture medium containing 3.3 µg/mL LNP/siRNA (formulation of cationic liposome-nucleic acid pharmaceutical composition prepared in Example-41) was added to each well, 100 µL fresh culture medium was added to the blank control group, and each concentration of each group corresponded to 6 replicate wells. After the formulations of cationic liposome-nucleic acid pharmaceutical compositions were incubated with the HeLa cells for 24 h, 20 µL PBS buffer solution containing 5 mg/mL MTT was added to each well. After the MTT was incubated with the cancer cells for 4 h, the mixed solution of the culture medium and the buffer solution containing MTT was aspirated, and DMSO was added at 150 µL/well to dissolve the purple formazan crystals in living cells. After shaking thoroughly, the absorbance was measured by a microplate reader. The result was calculated according to the measured absorbance value, and showed that compared with the blank control group, the cell viability in groups containing the formulations of cationic liposome-nucleic acid pharmaceutical compositions prepared in the present invention were all over 93%, indicating that the PEGylated lipid-modified formulations of cationic liposome-nucleic acid pharmaceutical composition of the present invention had good biocompatibility

### (4) Study on the nucleic acid transport efficiency

In order to examine the nucleic acid transport efficiency of each group of formulation of cationic liposome-nucleic acid pharmaceutical composition prepared with the PEGylated lipid of the present invention, the Luc-HeLa cells which could stably express the luciferase were used as the cell model to evaluate the nucleic acid transport efficiency of each group of the formulation of cationic liposome-nucleic acid pharmaceutical compositions (as described in **Example-29)** whose N/P ratio was 10/1. In a 96-well plate, when the cells were cultured to a cell density of 80%, each group of formulation of cationic liposome-nucleic acid pharmaceutical composition was added to the culture medium, and the cells were incubated in a cell incubator at 37 °C, 4% CO₂ for 24 h. The culture medium was then aspirated, and the culture medium containing 3.3 µg/mL LNP/siRNA was added for further incubation for 24 h. The cells were treated with lyse buffer, and the fluorescence intensity was measured using a luciferase assay kit and a chemiluminescence detector. On the other hand, the naked siRNA at the same dosage was added into the cultured cells to set a **blank**/siRNA group. Considering the siRNA which was transported into cells could inhibit the expression of the luciferase gene Fluc mRNA, the untreated cells were used as the negative control group, and the expression (i.e., fluorescence intensity) of the target gene in the blank cell group was designated as 100%. The result showed that the fluorescence intensity of the **blank**/siRNA group was approximately 78%, the fluorescence intensity of the **L-0**/siRNA group was approximately 43%, the fluorescence intensity of the **L-1**/siRNA group was approximately 29%, the fluorescence intensity of the **L-7/**siRNA group was approximately 31%, the fluorescence intensity of the **L-26**/siRNA group was approximately 39%, and the fluorescence intensity of the **L-27**/siRNA group was approximately 36%. Compared with the blank cell group and the siRNA group, the control group and the experimental groups showed better inhibitory effects on the luciferase gene, indicating that cationic liposomes could improve the transport efficiency for nucleic acid drugs; compared with the control group **L-0**/siRNA, the experimental groups had better inhibitory effects on the luciferase gene (the fluorescence intensity was less than 43%) except the group **L-10**/siRNA, **L-12**/siRNA, and **L-19**/siRNA, indicating that the cationic liposomes prepared with the PEGylated lipids of the present invention could improve the nucleic acid transport efficiency of liposomes.

### (5) Study on the uptake efficiency of tumor cells

In order to investigate whether the PEGylated lipid-modified cationic liposomes containing the targeting group folic acid can increase the uptake of cationic liposomes by tumor cells, in the present invention, the nasopharymneal carcinoma cells (KB) with high expression of folate receptors on the cell surface and the hepatoma cells (HepG2) with low expression of folate receptors on the cell surface were respectively used as models to evaluate the cellular uptake of liposomes. The cells were inoculated onto 6-well plates. The supernatant was discarded when the cell growth density reached approximately 80%. Then, 0.2 mL cationic liposome formulation of **L-29**/siRNA without the targeting group folic acid or **L-30**/siRNA with the targeting group folic acid, together with 1.8 mL culture medium, was added to each well, and each sampling point corresponded to 3 replicate wells. After 1, 3 and 6 hours of incubation, respectively, the supernatant was retrieved, and the cells were trypsinized, washed with PBS, and transferred into a flow cytometer tube. 1×10⁴ cells were counted and collected with a flow cytometer to calculate the percentage of positive cells (i.e., cells whose fluorescence intensity exceeded 10 units). The uptake efficiency was represented by the percentage of positive cells. Results of the experiments showed that, for the hepatoma cells with low expression of folate receptors, the difference of cellular uptake efficiency between **L-29**/siRNA without the targeting group folic acid and **L-30**/siRNA with the targeting group folic acid was relatively small. Compared with the **L-29**/siRNA without the targeting group folic acid, the **L-30**/siRNA with the targeting group folic acid permitted a higher cellular uptake efficiency of HepG2 cells by approximately 4%; however, for the nasopharymneal carcinoma cells with high expression of folate receptors, compared with the **L-29**/siRNA without the targeting group folic acid, the **L-30**/siRNA with the targeting group folic acid permitted a higher cellular uptake efficiency of KB cells by approximately 16%. It meant that the targeting effect of folic acid and the enhanced transport efficiency of cationic liposomes for nucleic acid drugs can act synergistically and further improve the effects of gene therapy on tumor.

Those described above are only embodiments of the present invention, and are not for setting a limitation on the patent scope of the present invention. Any modification of equivalent structures or equivalent routes according to the present invention, which may be applied in other related art in a direct or an indirect way, should be included into the scope of the present invention.

For those skilled in the art, without departing from the spirit and scope of the present invention, and without unnecessary experimentation, the present invention can be implemented in a wide range under equivalent parameters, concentrations and conditions. While the present invention has given particular examples, it should be understood that the present invention can be further modified. In conclusion, in accordance with the principles of the present invention, the present application is intended to cover any alterations, uses, or improvements of the present invention, including changes departing from the scope disclosed in this application but made using conventional techniques known in the art.

## Claims

1. A PEGylated lipid, wherein, the structure is represented by the general formula (2): 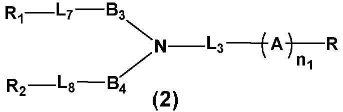 or a pharmaceutically acceptable salt, tautomer, or stereoisomer thereof,
wherein, one of L₇ and L₈ is -OC(=O)O-, -C(=O)-, -O-, -O(CR_{c}R_{c})ₛO-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, or -NR_{c}C(=O)S-, and the other is a linking bond, -OC(=O), -C(=O)O-, -OC(=O)O-, -C(=O)-, -O-, -O(CR_{c}R_{c})ₛO-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, or -NR_{c}C(=O)S-, wherein, R_{c} is independently, at each occurrence, a hydrogen atom or an alkyl group, s is 2, 3, or 4;
wherein, L₃ is a linking bond, -L₄-, -Z-L₄-, -L₄-Z-, -Z-L₄-Z-, -L₄-Z-L₅-, -Z-L₄-Z-L₅-, -L₄-Z-L₅-Z-, -Z-L₄-Z-L₅-Z-, or -L₄-Z-L₄-Z-L₅-Z-; said L₄ and L₅ are carbon-chain linking groups, which are each independently represented by -(CRₐR_{b})ₜ-(CRₐR_{b})ₒ-(CRₐR_{b})ₚ-, wherein, t, o, and p are each independently an integer from 0 to 12, and t, o, and p are not simultaneously 0; Rₐ and R_{b} are, at each occurrence, independently a hydrogen atom or a C₁₋₁₂ alkyl group; said Z is, at each occurrence, independently selected from the group consisting of -C(=O)-, -OC(=O)-, -C(=O)O-, -OC(=O)O-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NR_{c}C(=O)S-, and 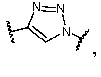 wherein, R_{c} is, at each occurrence, independently a hydrogen atom or a C₁₋₁₂ alkyl group;
wherein, B₃ and B₄ are each independently a linking bond or a C₁₋₃₀ alkylene group;
wherein, R₁ and R₂ are each independently a C₁₋₃₀ alkyl group;
wherein, R contains any atom or group selected from the group consisting of a hydrogen atom, an alkyl group, an alkoxy group, an alcoholic hydroxyl group, a protected alcoholic hydroxyl group, a thiol group, a protected thiol group, a carboxyl group, a protected carboxyl group, an amino group, a protected amino group, an aldehyde group, a protected aldehyde group, an ester group, a carbonate group, a urethane group, a succinimidyl group, a maleimide group, a protected maleimide group, a dimethylamino group, an alkenyl group, an alkenoate group, an azido group, an alkynyl group, a folate group, a rhodamine group, a biotinyl group, a monosaccharide group, and a polysaccharide group;
wherein, A is -(CRₐR_{b})ₛO- or -O(CRₐR_{b})ₛ-, wherein, s is 2, 3, or 4;
wherein, n₁ is an integer from 20 to 250;
said alkyl group, alkylene group, alkoxy group, and aliphatic hydrocarbon group are each independently substituted or unsubstituted.

2. The PEGylated lipid according to claim **1,** wherein, said B₃ and B₄ are each independently a linking bond or a C₁₋₂₀ alkylene group; more preferably selected from any one of the following Groups:
Group (1): B₃ and B₄ are both linking bonds;
Group (2): one of B₃ and B₄ is a linking bond, and the other is a C₁₋₂₀ alkylene group;
Group (3): B₃ and B₄ are each independently selected from the group consisting of a methylene group, an ethylene group, a propylene group, a butylene group, a pentylene group, a hexylene group, a heptylene group, an octylene group, a nonylene group, a decylene group, an undecylene group, a dodecylene group, a tridecylene group, a tetradecylene group, a pentadecylene group, a hexadecylene group, a heptadecylene group, an octadecylene group, a nonadecylene group, and an eicosylene group.

3. The PEGylated lipid according to claim **1,** wherein, one of said L₇ and L₈ is -OC(=O)O-, -C(=O)-, -O-, -O(CH₂)ₛO-, -S-, -C(=O)S-, -SC(=O)-, -NHC(=O)-, -C(=O)NH-, -NHC(=O)NH-, -OC(=O)NH-, -NHC(=O)O-, -SC(=O)NH-, or -NHC(=O)S-, and the other is a linking bond, -OC(=O)-, -C(=O)O-, -OC(=O)O-, -C(=O)-, -O-, -O(CH₂)ₛO-, -S-, -C(=O)S-, -SC(=O)-, -NHC(=O)-, -C(=O)NH-, -NHC(=O)NH-, -OC(=O)NH-, -NHC(=O)O-, -SC(=O)NH-, or -NHC(=O)S-;
preferably one of said L₇ and L₈ is -OC(=O)O-, -C(=O)-, -O-, -O(CH₂)ₛO-, -S-, -C(=O)S-, -SC(=O)-, -NHC(=O)-, -C(=O)NH-, -NHC(=O)NH-, -OC(=O)NH-, -NHC(=O)O-, -SC(=O)NH-, or -NHC(=O)S-, and the other is a linking bond, -O-, -OC(=O)-, or -C(=O)O-;
more preferably said L₇ and L₈ are selected from any one of the following Groups:
Group (1): one of L₇ and L₈ is -C(=O)-, and the other is a linking bond;
Group (2): one of L₇ and L₈ is -C(=O)-, and the other is -OC(=O)- or -C(=O)O-;
Group (3): L₇ and L₈ are both -O-.

4. The PEGylated lipid according to claim 1, wherein, said L₃ contains degradable groups, and said degradable group refers to the group which can degrade under any condition selected from the group consisting of light, heat, low temperature, enzymatic condition, oxidation-reduction condition, acidic condition, basic condition, physiological condition, and simulated physiological environment in vitro; L₄ and L₅ in L₃ are preferably each independently -(CH₂)ₜ-; L₃ is selected from the group consisting of -(CH₂)ₜ-, -(CH₂)ₜZ-, -Z(CH₂)ₜ-, -(CH₂)ₜZ(CH₂)ₜ-, -Z(CH₂)ₜZ-, -(CH₂)ₜZ(CH₂)ₜZ-, -Z(CH₂)ₜZ(CH₂)ₜ-, and -Z(CH₂)ₜZ(CH₂)ₜZ-, wherein, t is an integer from 1 to 12, and Z is, at each occurrence, independently selected from the group consisting of -C(=O)-, -OC(=O)-, -C(=O)O-, -OC(=O)O-, -O-, -S-, -C(=O)S-, -SC(=O)-, -NHC(=O)-, -C(=O)NH-, -NHC(=O)NH-, -OC(=O)NH-, -NHC(=O)O-, -SC(=O)NH-, and -NHC(=O)S-; L₃ is preferably selected from the group consisting of -(CH₂)ₜ-, -(CH₂)ₜO-, -(CH₂)ₜC(=O)-, -(CH₂)ₜC(=O)O-, -(CH₂)ₜOC(=O)-, -(CH₂)ₜC(=O)NH-, -(CH₂)ₜNHC(=O)-, -(CH₂)ₜOC(=O)O-, -(CH₂)ₜNHC(=O)O-, -(CH₂)ₜOC(=O)NH-, -(CH₂)ₜNHC(=O)NH-, -O(CH₂)ₜ-, -C(=O)(CH₂)ₜ-, -C(=O)O(CH₂)ₜ-, -OC(=O)(CH₂)ₜ-, -C(=O)NH(CH₂)ₜ-, -NHC(=O)(CH₂)ₜ-, -OC(=O)O(CH₂)ₜ-, -NHC(=O)O(CH₂)ₜ-, -OC(=O)NH(CH₂)ₜ-, -NHC(=O)NH(CH₂)ₜ-, -(CH₂)ₜO(CH₂)ₜ-, -(CH₂)ₜC(=O)(CH₂)ₜ-, -(CH₂)ₜC(=O)O(CH₂)ₜ-, -(CH₂)ₜOC(=O)(CH₂)ₜ-, -(CH₂)ₜC(=O)NH(CH₂)ₜ-, -(CH₂)ₜNHC(=O)(CH₂)ₜ-, -(CH₂)ₜOC(=O)O(CH₂)ₜ-, -(CH₂)ₜNHC(=O)O(CH₂)ₜ-, -(CH₂)ₜOC(=O)NH(CH₂)ₜ-, -(CH₂)ₜNHC(=O)NH(CH₂)ₜ-, -O(CH₂)ₜO-, -C(=O)(CH₂)ₜC(=O)-, -C(=O)O(CH₂)ₜC(=O)O-, -OC(=O)(CH₂)ₜOC(=O)-, -C(=O)O(CH₂)ₜOC(=O)-, -OC(=O)(CH₂)ₜC(=O)O-, -OC(=O)O(CH₂)ₜOC(=O)O-, -C(=O)NH(CH₂)ₜC(=O)NH-, -NHC(=O)(CH₂)ₜNHC(=O)-, -NHC(=O)(CH₂)ₜC(=O)NH-, -C(=O)NH(CH₂)ₜNHC(=O)-, -NHC(=O)O(CH₂)ₜNHC(=O)O-, -OC(=O)NH(CH₂)ₜOC(=O)NH-, -NHC(=O)O(CH₂)ₜOC(=O)NH-, -OC(=O)NH(CH₂)ₜNHC(=O)O-, -NHC(=O)NH(CH₂)ₜNHC(=O)NH-, -C(=O)(CH₂)ₜO-, -C(=O)(CH₂)ₜC(=O)O-, -C(=O)(CH₂)ₜOC(=O)-, -C(=O)(CH₂)ₜOC(=O)O-, -C(=O)(CH₂)ₜNHC(=O)O-, -C(=O)(CH₂)ₜOC(=O)NH-, -C(=O)(CH₂)ₜNHC(=O)NH-, -C(=O)(CH₂)ₜC(=O)O(CH₂)ₜ-, -C(=O)(CH₂)ₜOC(=O)(CH₂)ₜ-, -C(=O)(CH₂)ₜOC(=O)O(CH₂)ₜ-, -C(=O)(CH₂)ₜNHC(=O)O(CH₂)ₜ-, -C(=O)(CH₂)ₜOC(=O)NH(CH₂)ₜ-, -C(=O)(CH₂)ₜNHC(=O)NH(CH₂)ₜ-, and -C(=O)(CH₂)ₜC(=O)(CH₂)ₜNHC(=O)O-, wherein, t is an integer from 2 to 12, and more preferably selected from the group consisting of -C(=O)O-, -(CH₂)ₜO-, -(CH₂)ₜC(=O)O-, -(CH₂)ₜOC(=O)-, -(CH₂)ₜO-, -C(=O)(CH₂)ₜO-, -C(=O)(CH₂)ₜOC(=O)-, -C(=O)(CH₂)ₜC(=O)O-, -C(=O)(CH₂)ₜC(=O)NH-, -C(=O)(CH₂)ₜOC(=O)NH-, -C(=O)(CH₂)ₜNHC(=O)O-, -C(=O)(CH₂)ₜOC(=O)NH(CH₂)ₜ-, and -C(=O)(CH₂)ₜC(=O)(CH₂)ₜNHC(=O)O-.

5. The PEGylated lipid according to claim **1,** wherein, the number-average molecular weight determined by MALDI_TOF of said polyethylene glycol chain is selected from the group consisting of 900, 1000, 1500, 2000, 2500, 3000, 3350, 3500, 4000, 5000, 5500, 6000, 6500, 7000, 7500, 8000, 8500, 9000, 9500, 10000, and 11,000.

6. The PEGylated lipid according to claim **1,** wherein, said polyethylene glycol chain is polydisperse, and the number-average degree of polymerization n₁ determined by MALDI_TOF is preferably an integer from 20 to 100, more preferably an integer from 20 to 60, more preferably an integer from 40 to 60, and most preferably selected from the group consisting of 44, 45, 46, 48, 50, 52, 54, 56, 58, and 60; or
wherein, said polyethylene glycol chain is monodisperse, and the number of EO units determined by MALDI_TOF is preferably from 20 to 70, more preferably from 20 to 60, most preferably selected from the group consisting of 20, 22, 24, 26, 27, 28, 29, 30, 32, 34, 36, 38, 40, 42, 44, 45, 46, 48, 50, 52, 54, 56, 58, and 60.

7. The PEGylated lipid according to claim **1,** wherein, said R₁ and R₂ are each independently a C₅₋₃₀ alkyl group, more preferably a C₁₀₋₃₀ alkyl group, and most preferably a C₁₀₋₂₀ alkyl group;
wherein, said R₁ and R₂ are each independently a C₁₋₃₀ linear alkyl group or a C₁₋₃₀ branched alkyl group; preferably a C₁₋₃₀ linear alkyl group; more preferably a C₁₋₂₅ linear alkyl group; more preferably a C₁₋₂₀ linear alkyl group; more preferably selected from the group consisting of a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, and an eicosyl group; most preferably each independently a C₁₋₃₀ branched alkyl group, and each independently represented as 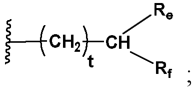
wherein, Rₑ and R_{f} are each independently a C₁₋₁₅ alkyl group, more preferably selected from the group consisting of a methyl group, an ethyl group, a propyl group, a butyl group, a pentyl group, a hexyl group, a heptyl group, an octyl group, a nonyl group, a decyl group, an undecyl group, a dodecyl group, a tridecyl group, and a tetradecyl group;
and further more preferably said R₁ and R₂ are each independently selected from the group consisting of the following structures: 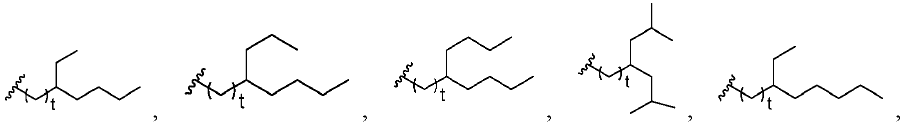 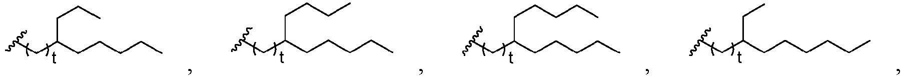 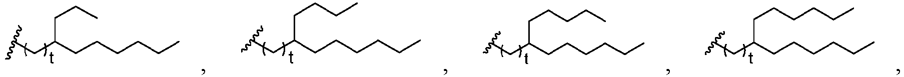 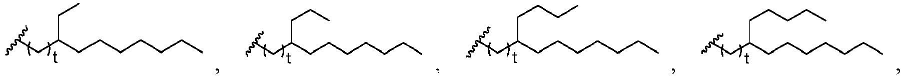 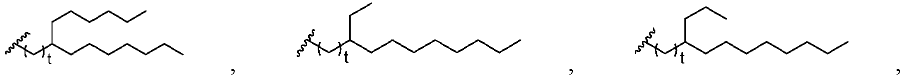 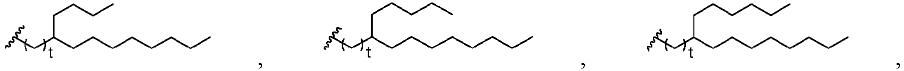 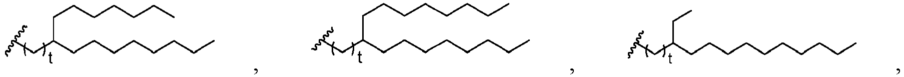 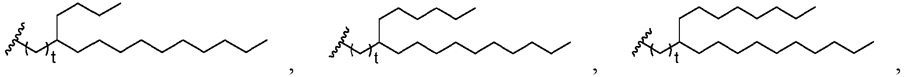 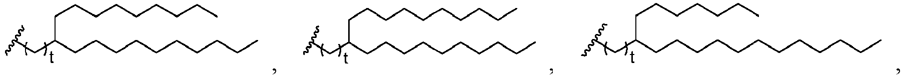 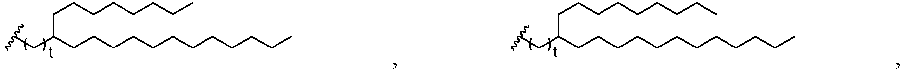 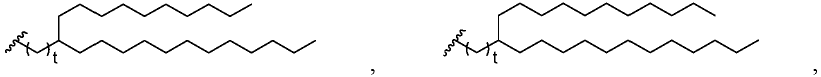
and 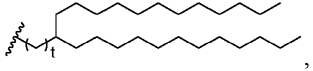
wherein, t is an integer from 0 to 12.

8. The PEGylated lipid according to claim **1,** wherein, said R is selected from the group consisting of H, -CH₃, -CH₂CH₃, -(CH₂)ₜOH, -(CH₂)ₜSH, -OCH₃, -OCH₂CH₃, -(CH₂)ₜNH₂, -(CH₂)ₜC(=O)OH, -C(=O)(CH₂)ₜC(=O)OH, -C(=O)CH₃, -(CH₂)ₜN₃, -C(=O)CH₂CH₃, -C(=O)OCH₃, -OC(=O)OCH₃, -C(=O)OCH₂CH₃, -OC(=O)OCH₂CH₃, -(CH₂)ₜN(CH₃)₂, -(CH₂)ₜN(CH₂CH₃)₂, -(CH₂)ₜCHO,

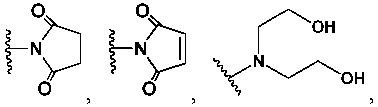

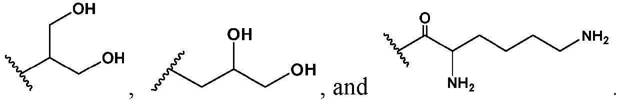

9. The PEGylated lipid according to claim **1,** wherein, the structure of said PEGylated lipid is selected from the group consisting of the following formulas: 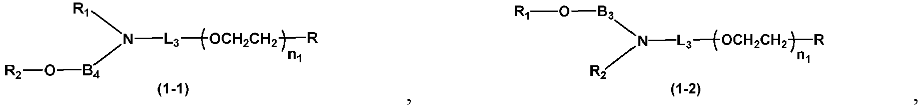 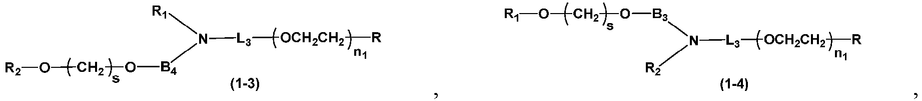 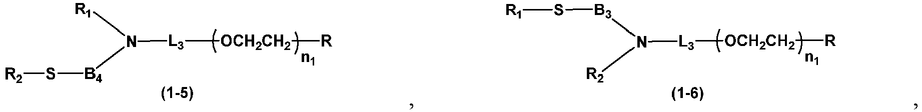 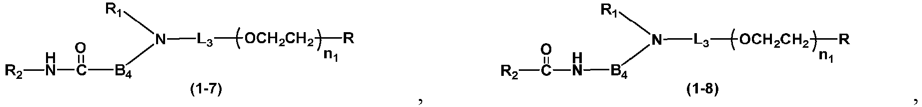 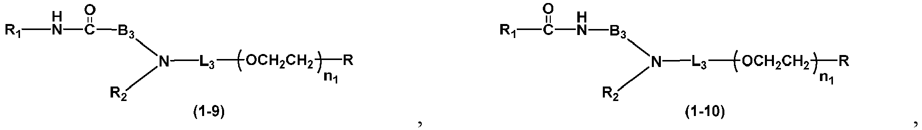 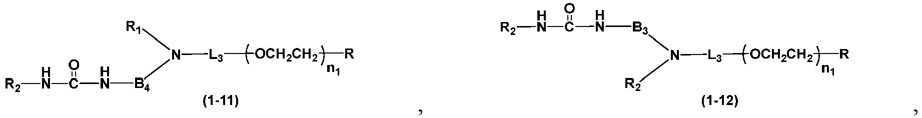 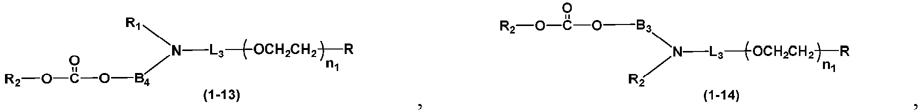 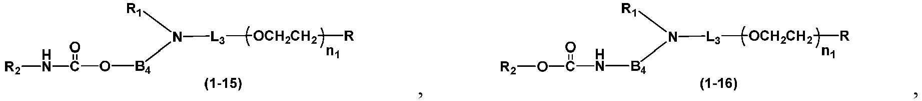 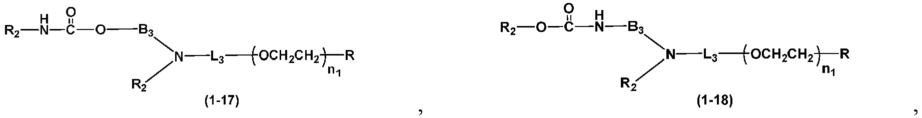 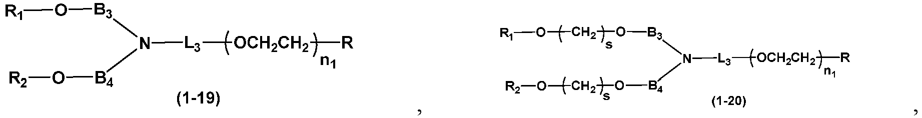 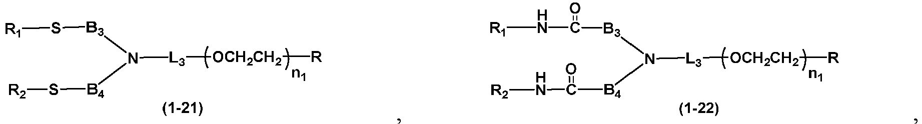 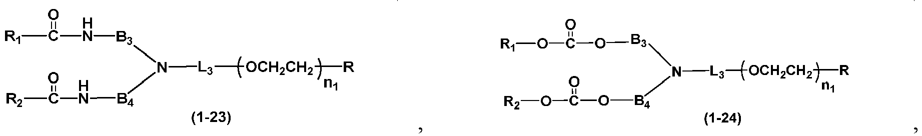 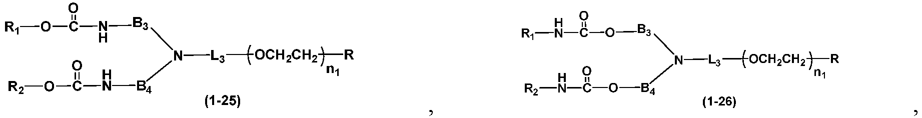 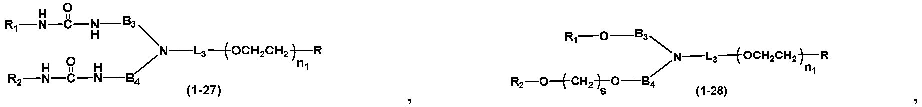 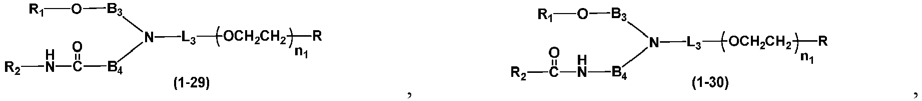 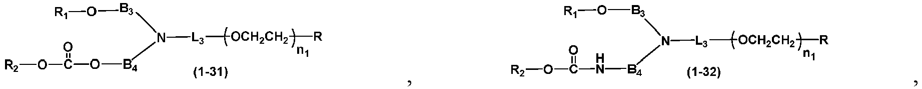 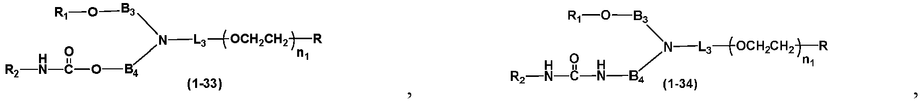 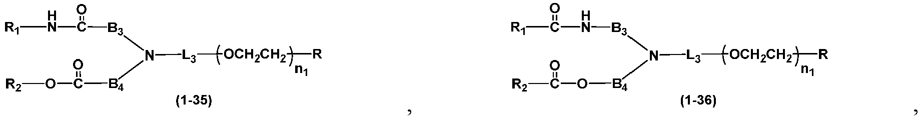 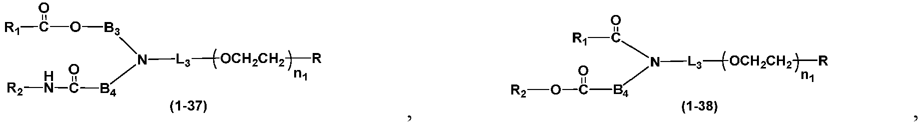 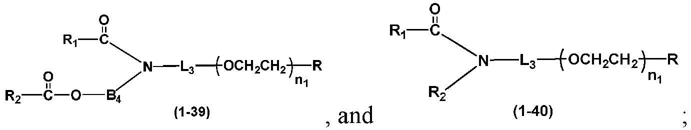 or
wherein, the structure of said PEGylated lipid is selected from the group consisting of the following structures: 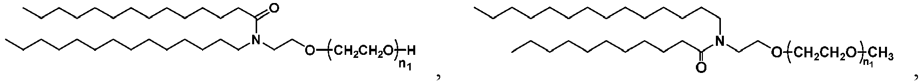 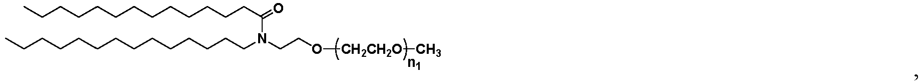 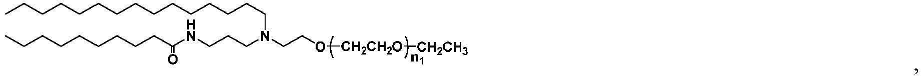 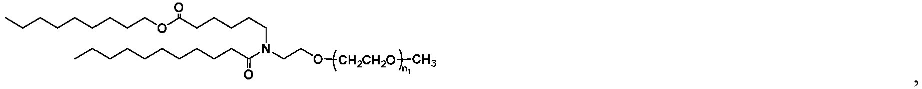 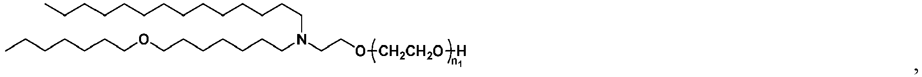 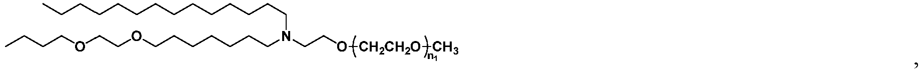 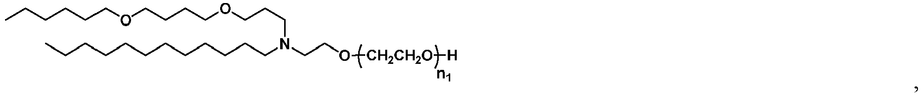 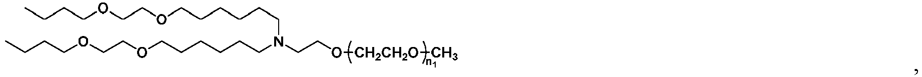 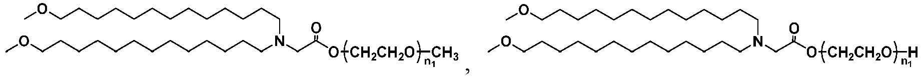 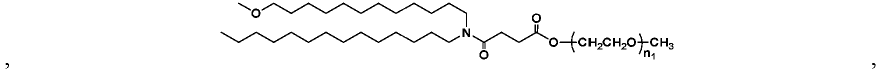 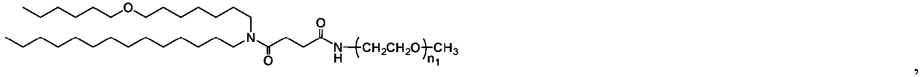 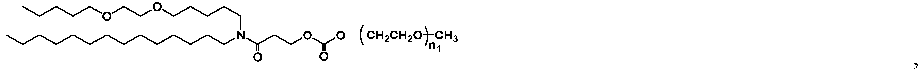 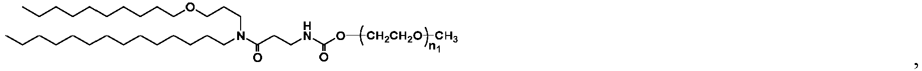 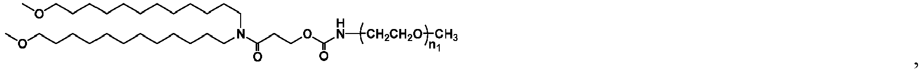 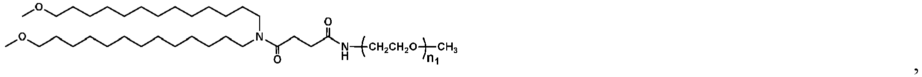 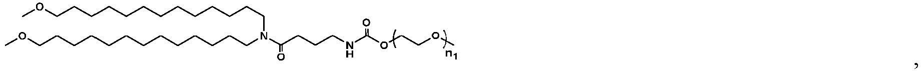 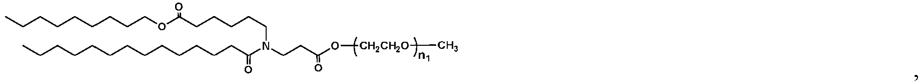 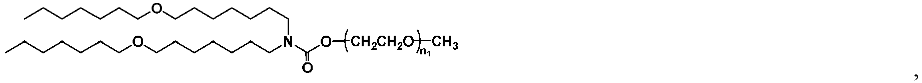 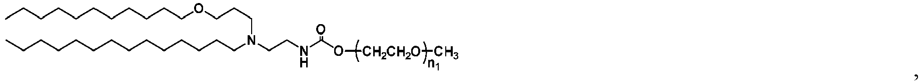 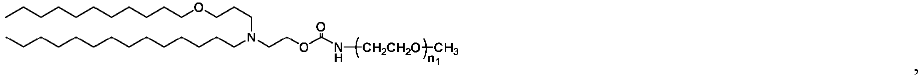 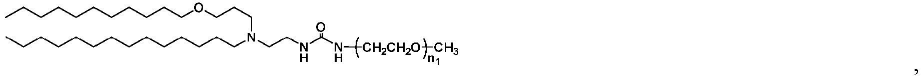 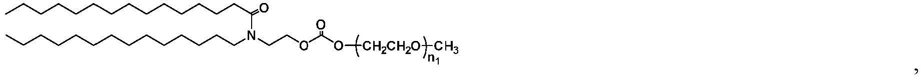 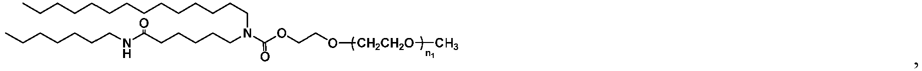 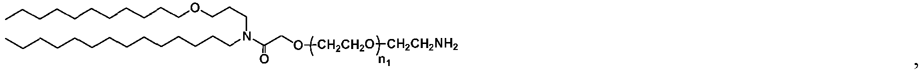 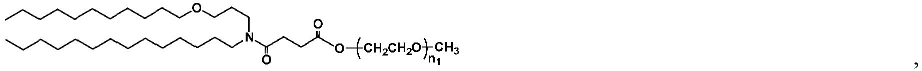 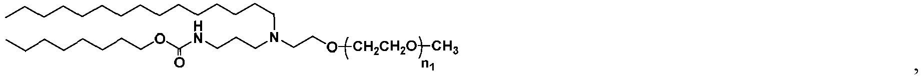 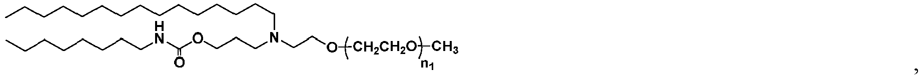 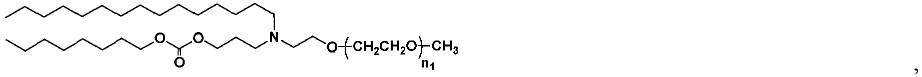 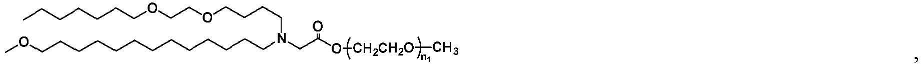 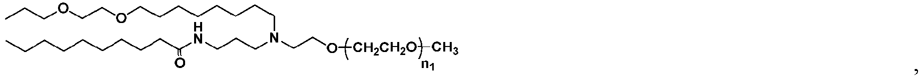 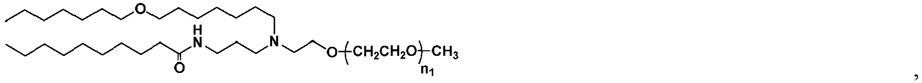 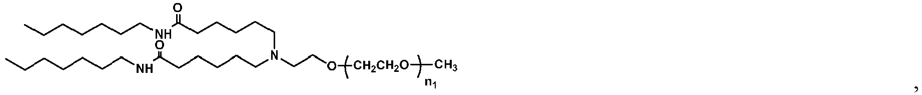 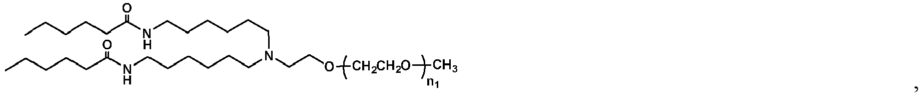 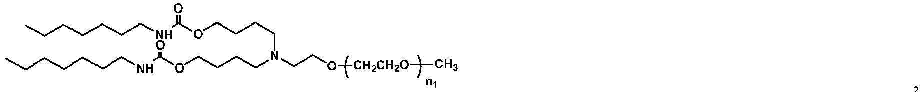 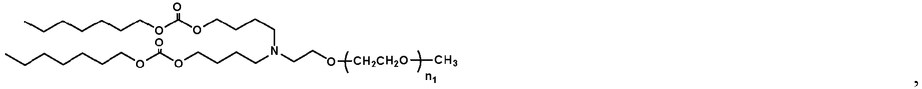 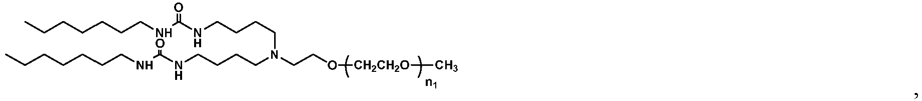 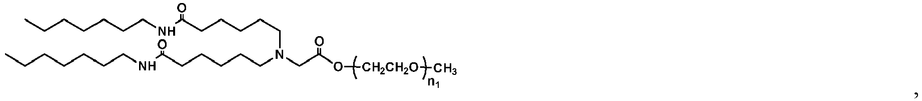 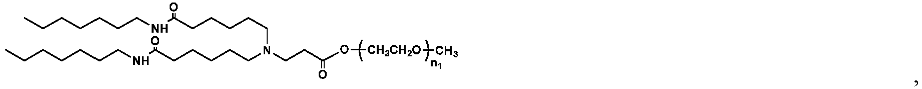 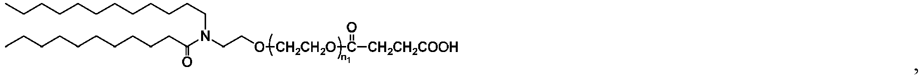 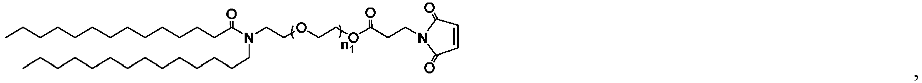 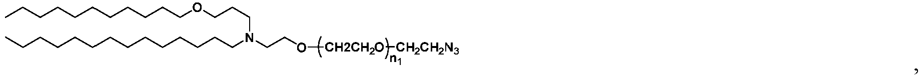 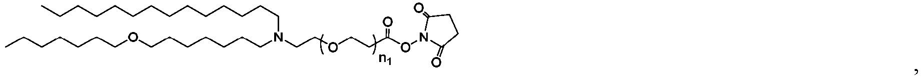 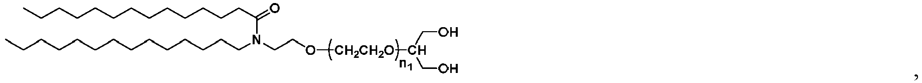 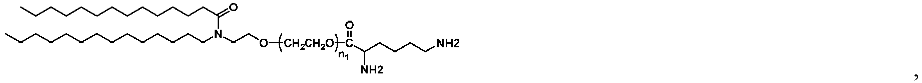 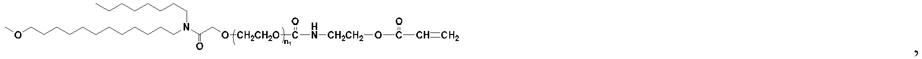 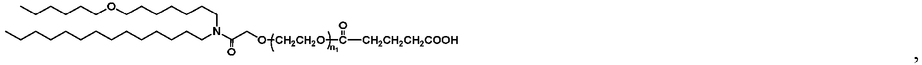 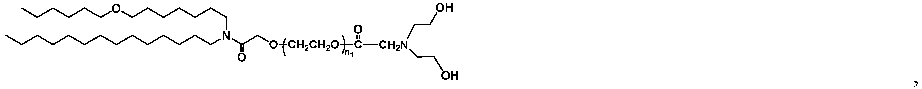 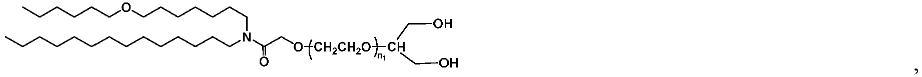 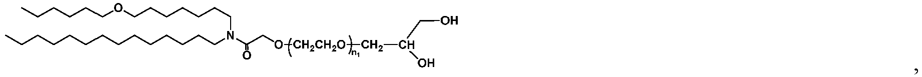 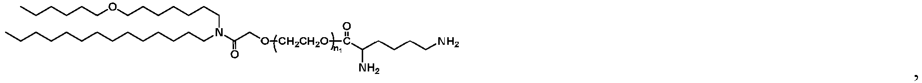 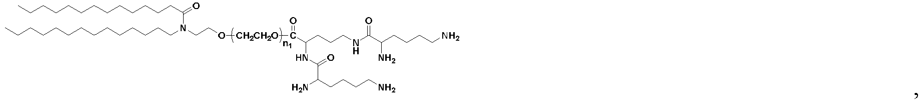 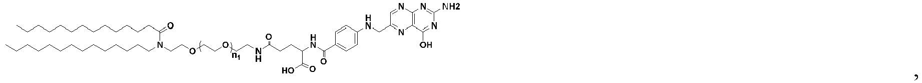 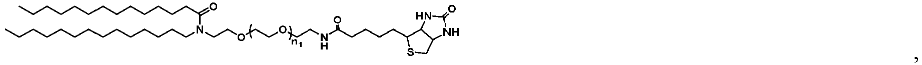 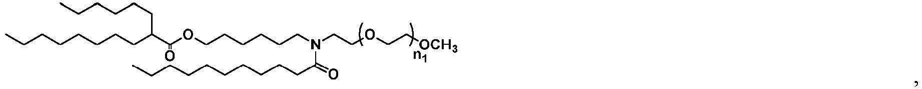 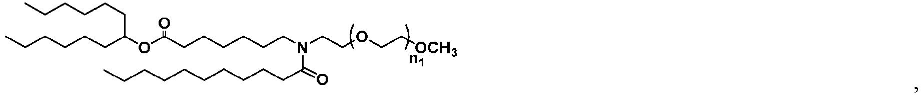 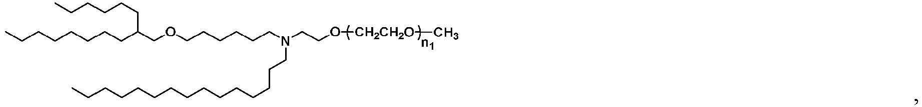 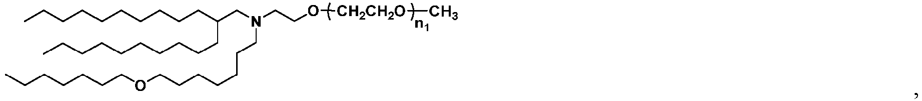 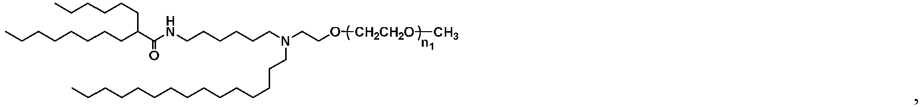 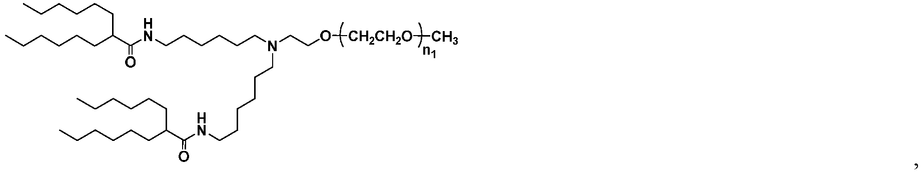 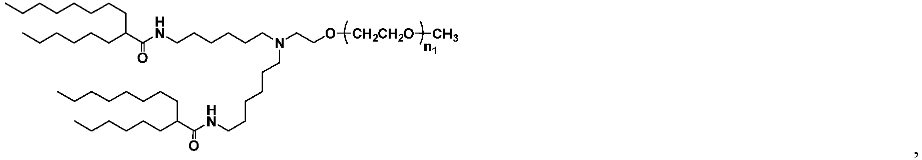 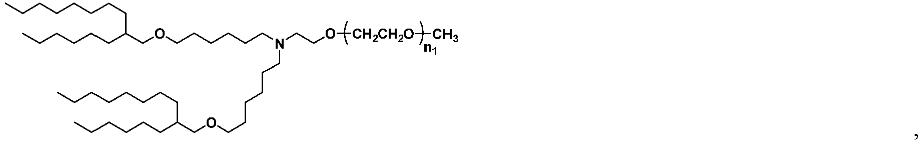 and

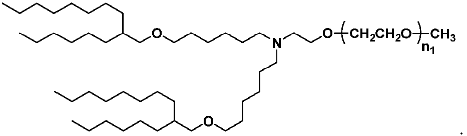

10. A cationic liposome, wherein, the cationic liposome contains any foregoing PEGylated lipid according to one of claims **1 to 9.**

11. The cationic liposome according to claim **10,** wherein, said cationic liposome also contains one or more types of lipids selected from the group consisting of neutral lipid, steroid lipid, and cationic lipid; said neutral lipid is preferably phospholipid.

12. The cationic liposome according to claim **11,** wherein, said neutral lipid is selected from the group consisting of 1,2-dilinoleoyl-sn-glycero-3-phosphocholines, 1,2-dimyristoleoyl-sn-glycero-3-phosphocholines, 1,2-dioleoyl-sn-glycero-3-phosphocholines, 1,2-dipalmitoyl-sn-glycero-3-phosphocholines, 1,2-distearoyl-sn-glycero-3-phosphatidylcholines, 1,2-diundecanoyl-sn-glycero-3-phosphatidylcholines, 1-plamitoyl-2-oleoyl-sn-glycero-3-phosphocholines, 1,2-di-*O*-octadecenyl-sn-glycero-3-phosphatidylcholines, 1-oleoyl-2-cholesterylhemisuccinyl-sn-glycero-3-phosphocholines, 1-*O*-hexadecyl-sn-glycero-3-phosphatidylcholines, 1,2-dilinolenoyl-sn-glycero-3-phosphatidylcholines, 1,2-diarachidonoyl-sn-glycero-3-phosphatidylcholines, 1,2-didecosahexaenoyl-sn-glycero-3-phosphocholines, 1,2-dioleoyl-sn-glycero-3-phosphoethanolamines, 1,2-diphytanyl-sn-glycero-3-phosphoethanolamines, 1,2-distearoyl-sn-glycero-3-phosphoethanolamines, 1,2-dilinoleoyl-sn-glycero-3-phosphoethanolamines, 1,2-dilinolenoyl-sn-glycero-3-phosphoethanolamines, 1,2-diarachidonoyl-sn-glycero-3-phosphoethanolamines, 1,2-didecosahexaenoyl-sn-glycero-3-phosphoethanolamines, 1,2-dioleoyl-sn-glycero-3-phospho-rac-(1-glycerol) sodium salts, dioleoyl phosphatidylserines, dipalmitoylphosphatidylglycerols, palmitoyloleoyl phosphatidylethanolamines, distearoyl phosphatidylethanolamines, dipalmitoyl phosphatidylethanolamines, dimyristoleoyl phosphoethanolamines, 1-stearoyl-2-oleoyl-stearoylethanolamines, 1-stearoyl-2-oleoyl-phosphatidylcholines, sphingomyelins, phosphatidylcholines, phosphatidylethnolamines, phosphatidylserines, phosphatidylinositols, phosphatidic acids, palmitoyloleoyl phosphatidylcholines, lysophosphatidylcholines, lysophosphatidylethanolamines, and combinations thereof; or
said steroid lipid is selected from the group consisting of a cholesterol, a coprostanol, a sitosterol, an ergosterol, a campesterol, a stigmasterol, a brassicasterol tomatidine, an ursolic acid, an α-tocopherol, and any mixture thereof; or
wherein, the structure of said cationic lipid is represented by the general formula (1): 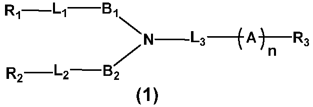
wherein, N is the nitrogen branching center;
L₁ and L₂ are divalent linking groups, each independently selected from the group consisting of -OC(=O)-, -C(=O)O-, -OC(=O)O-, -C(=O)-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; wherein, R_{c} is a hydrogen atom or a C₁₋₁₂ alkyl group;
L₃ is a linking bond or -L₄-Z-L₅-; said L₄ and L₅ are carbon chain linking groups, each independently -(CRₐR_{b})ₜ- or -(CRₐR_{b})ₜ-(CRₐR_{b})ₚ-(CRₐR_{b})_{q}-; wherein, t, p and q are each independently an integer from 0 to 12, Rₐ and R_{b} are each independently a hydrogen atom or a C₁₋₁₂ alkyl group; said Z is a linking bond or a divalent linking group selected from the group consisting of -OC(=O)-, -C(=O)O-, -OC(=O)O-, -C(=O)-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-;
B₁ and B₂ are each independently a C₁₋₁₂ alkylene group;
R₁ and R₂ are each independently a C₂₋₃₀ aliphatic hydrocarbon group;
R₃ is a hydrogen atom, an alkyl group, an alkoxy group, -C(=O)R_{d}, -C(=O)OR_{d}, -OC(=O)R_{d}, or -OC(=O)OR_{d}; wherein, R_{d} is a C₁₋₁₂ alkyl group;
A is selected from the group consisting of -(CRₐR_{b})ₛO-, -O(CRₐR_{b})ₛ-, -(CRₐR_{b})ₛS-, -S(CRₐR_{b})ₛ-, -(CRₐR_{b})ₛO(CRₐR_{b})ₛS-, -(CRₐR_{b})ₛS(CRₐR_{b})ₛO-, -(CRₐR_{b})ₛNR_{c}(CRₐR_{b})ₛS-, -(CRaR_{b})ₛS(CRₐR_{b})ₛNR_{c}-, -(CRₐR_{b})ₛNR_{c}(CRₐR_{b})ₛO-, and -(CRₐR_{b})ₛO(CRₐR_{b})ₛNR_{c}-; wherein, s is 2, 3 or 4, Rₐ and R_{b} are each independently a hydrogen atom or a C₁₋₁₂ alkyl group;
when A is -(CRₐR_{b})ₛO- or -O(CRₐR_{b})ₛ-, n is an integer from 2 to 6; when A is not -(CRₐR_{b})ₛO- or -O(CRₐR_{b})ₛ-, n is an integer from 1 to 6;
said alkyl group, alkylene group, alkoxy group, and aliphatic hydrocarbon group are each independently substituted or unsubstituted; or
wherein, the structure of said cationic lipid is selected from the following structures: 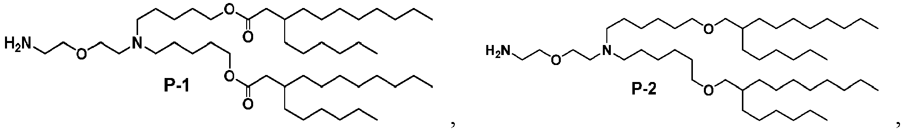 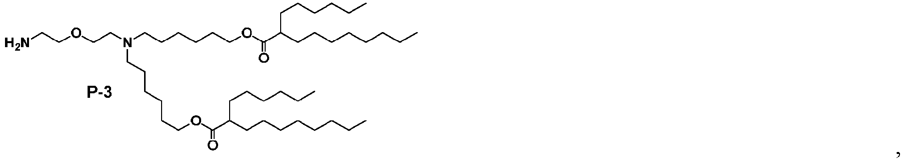 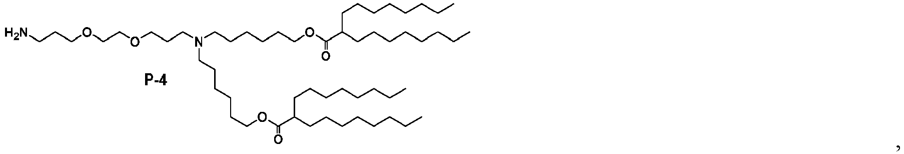 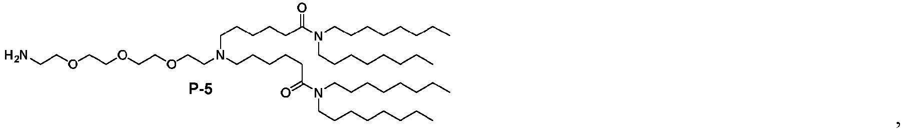 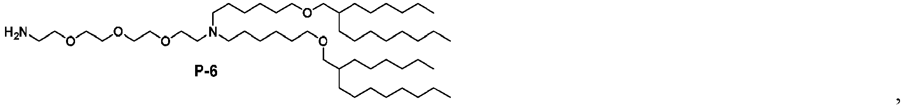 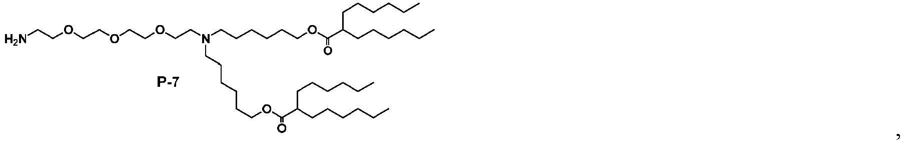 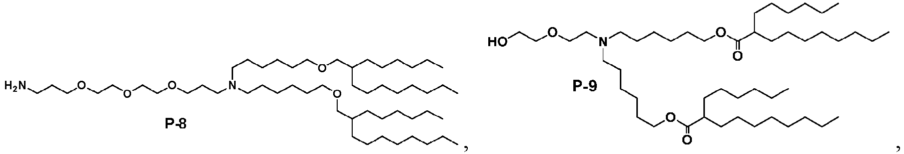 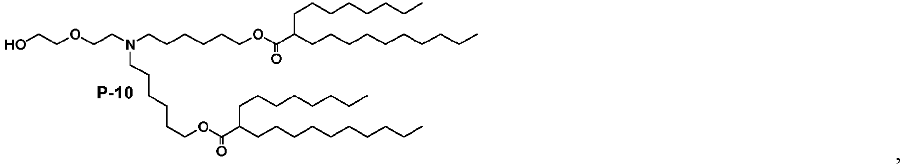 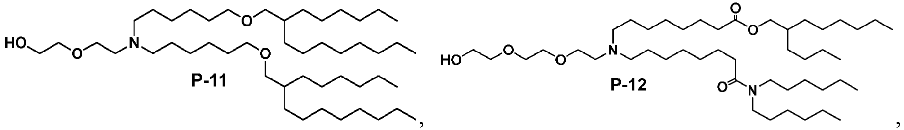 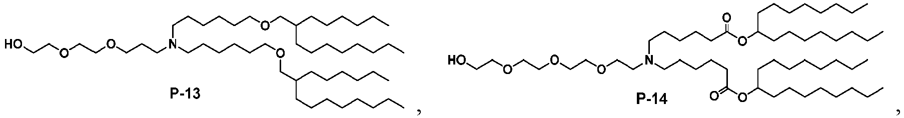 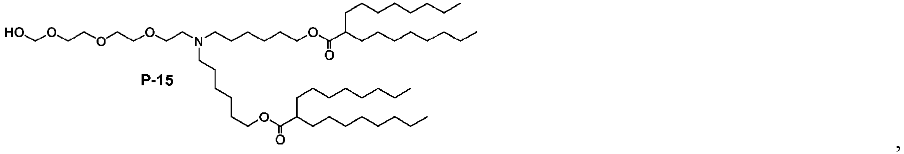 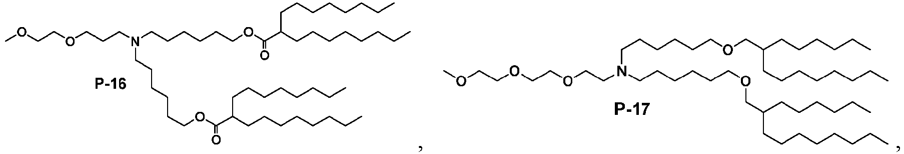 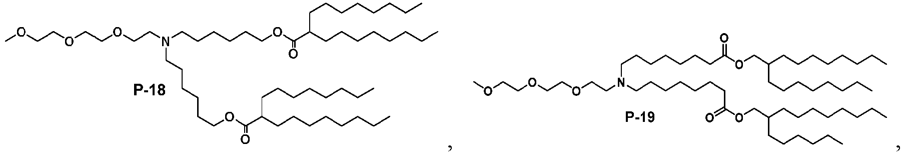 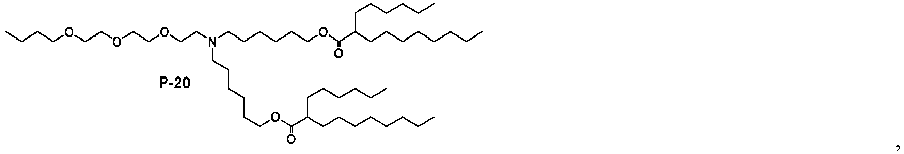 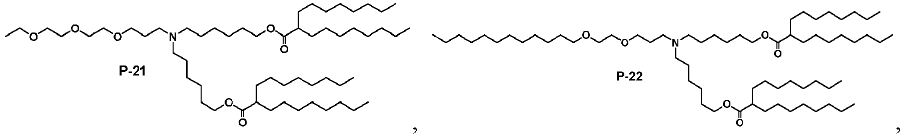 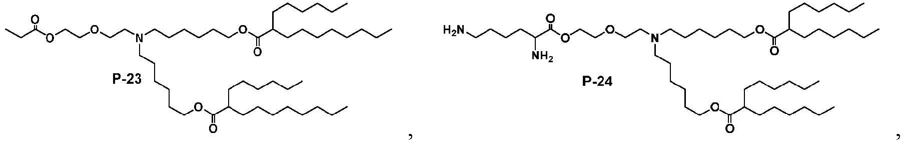 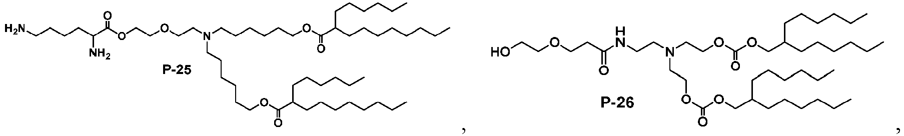 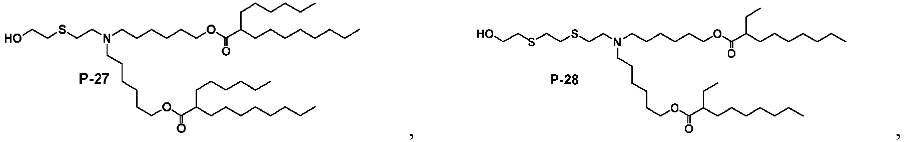 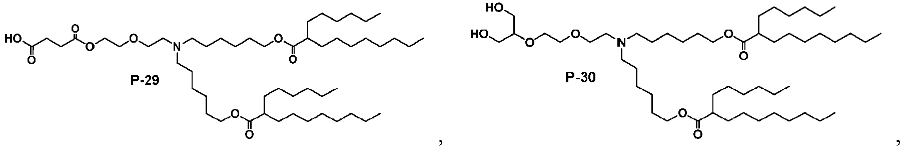 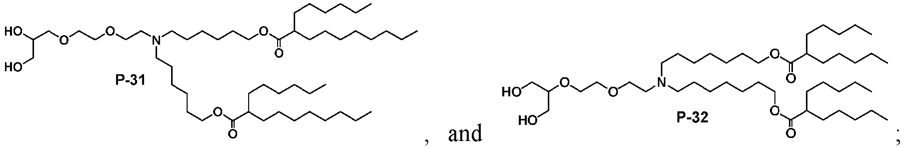 or
wherein, said cationic lipids in the cationic liposomes can be selected from the group consisting of *N,N-*dioleyl-*N,N*-dimethylammonium chloride, *N,N*-distearyl-*N,N*-dimethylammonium bromide, 1,2-dioleoyl-3-trimethylammonium-propane, *N*-[1-(2,3-dioleoyloxy)propyl]-*N,N,N*-trimethylammonium chloride, *1,2-dioleyloxy-N,N-dimethylaminopropane,* 3-(didodecylamino)-*N1*,*N1*,4-tridodecyl-1-piperazineethanamine, *N1*-[2-(didodecylamino)ethyl]-*N1*,*N4*,*N4*-tridodecyl-1,4-piperazinediethanamine, 14,25-ditridecyl-15,18,21,24-tetraaza-octatriacontane, 1,2-dilinoleyloxy-*N,N*-dimethylaminopropane, 2,2-dilinoleyl-4-dimethylaminomethyl-[1,3]-dioxolane, heptatriaconta-6,9,28,31-tetraen-19-yl 4-(dimethylamino)butanoate, 2,2-dilinoleyl-4-(2-dimethylaminoethyl)-[1,3]-dioxolane, and mixtures thereof.

13. The cationic liposome according to one of claims **11 to 12,** wherein, said cationic liposome contains 20% to 80% cationic lipids, 5% to 15% neutral lipids, 25% to 55% steroid lipids, and 0.5% to 10% PEGylated lipids represented by the general formula (2), said percentage is the molar percentage of each lipid relative to the total lipids in a solution containing the solvent.

14. The cationic liposome according to one of claims **11 to 12,** wherein, the molar percentage of said cationic lipids relative to the total lipids in a solution containing the solvent is 30% to 65%; more preferably 35%, 40%, 45%, 46%, 47%, 48%, 49%, 50%, or 55%; or
wherein, the molar percentage of said neutral lipids relative to the total lipids in a solution containing the solvent is 7.5% to 13%, more preferably 8%, 9%, 10%, 11%, or 12%; or
wherein, the molar percentage of said steroid lipids relative to the total lipids in a solution containing the solvent is 35% to 50%, more preferably 40%, 41%, 42%, 43%, 44%, 45%, 46%, 47%, 48%, 49%, or 50%; or
wherein, the molar percentage of said PEGylated lipids relative to the total lipids in a solution containing the solvent is 0.5% to 5%, preferably 1% to 3%, more preferably 1.5%, 1.6%, 1.7%, 1.8%, or 1.9%.

15. A cationic liposome pharmaceutical composition, wherein, the cationic liposome pharmaceutical composition contains the cationic liposome according to one of claims **11 to 12** and drugs; said drug is preferably a nucleic acid drug or an anti-tumor agent, said nucleic acid drug is selected from the group consisting of DNA, antisense nucleic acid, plasmid, mRNA, interfering nucleic acid, aptamer, antagomir, miRNA, ribozyme, and siRNA, and more preferably selected from the group consisting of DNA, mRNA, miRNA, and siRNA.

## Patentansprüche

1. PEGyliertes Lipid, wobei die Struktur durch die allgemeine Formel (2) wiedergegeben wird: 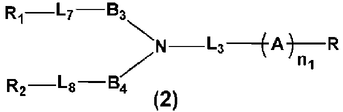
oder pharmazeutisch unbedenkliches Salz, Tautomer oder Stereoisomer davon,
wobei eine von L₇ und L₈ für -OC(=O)O-, -C(=O)-, -O-, -O(CR_{c}R_{c})ₛO-, - S-, -C(=O)S-, -SC(=O)-, -NR_{c}C(=O)-, -C(=O)NR_{c}-, -NR_{c}C(=O)NR_{c}-, -OC(=O)NR_{c}-, - NR_{c}C(=O)O-, -SC(=O)NR_{c}- oder -NR_{c}C(=O)S- steht und die jeweils andere für eine Verknüpfungsbindung, -OC(=O), -C(=O)O-, -OC(=O)O-, -C(=O)-, -O-, -O(CR_{c}R_{c})ₛO-, - S-, -C(=O)S-, -SC(=O)-, -NR_{c}C(=O)-, -C(=O)NR_{c}-, -NR_{c}C(=O)NR_{c}-, -OC(=O)NR_{c}-, - NR_{c}C(=O)O-, -SC(=O)NR_{c}- oder -NR_{c}C(=O)S- steht, wobei die R_{c} unabhängig jeweils für ein Wasserstoffatom oder eine Alkylgruppe stehen, s für 2, 3 oder 4 steht;
wobei L₃ für eine Verknüpfungsbindung, -L₄-, -Z-L₄-, -L₄-Z-, -Z-L₄-Z-, -L₄-Z-L₅-, -Z-L₄-Z-L₅-, -L₄-Z-L₅-Z-, -Z-L₄-Z-L₅-Z- oder -L₄-Z-L₄-Z-L₅-Z- steht, die L₄ und L₅ kohlenstoffkettige Verknüpfungsgruppen sind, die jeweils unabhängig durch -(CRₐR_{b})ₜ-(CRₐR_{b})ₒ-(CRₐR_{b})ₚ- dargestellt sind, wobei t, o und p jeweils unabhängig für eine ganze Zahl von 0 bis 12 stehen und t, o und p nicht gleichzeitig gleich 0 sind, die Rₐ und R_{b} unabhängig jeweils für ein Wasserstoffatom oder eine C₁₋₁₂-Alkylgruppe stehen, die Z jeweils unabhängig aus der Gruppe bestehend aus -C(=O)-, -OC(=O)-, -C(=O)O-, - OC(=O)O-, -O-, -S-, -C(=O)S-, -SC(=O)-, -NR_{c}C(=O)-, -C(=O)NR_{c}-, -NR_{c}C(=O)NR_{c}-, - OC(=O)NR_{c}-, -NR_{c}C(=O)O-, -SC(=O)NR_{c}-, -NR_{c}C(=O)S- und 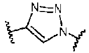
ausgewählt sind, wobei die R_{c} jeweils unabhängig für ein Wasserstoffatom oder eine C₁₋₁₂-Alkylgruppe stehen;
wobei B₃ und B₄ jeweils unabhängig für eine Verknüpfungsbindung oder eine C₁₋₃₀-Alkylengruppe stehen;
wobei R₁ und R₂ jeweils unabhängig für eine C₁₋₃₀-Alkylgruppe stehen; wobei R ein Atom oder eine Gruppe enthält, das bzw. die aus der Gruppe bestehend aus einem Wasserstoffatom, einer Alkylgruppe, einer Alkoxygruppe, einer alkoholischen Hydroxylgruppe, einer geschützten alkoholischen Hydroxylgruppe, einer Thiolgruppe, einer geschützten Thiolgruppe, einer Carboxylgruppe, einer geschützten Carboxylgruppe, einer Aminogruppe, einer geschützten Aminogruppe, einer Aldehydgruppe, einer geschützten Aldehydgruppe, einer Estergruppe, einer Carbonatgruppe, einer Urethangruppe, einer Succinimidylgruppe, einer Maleimidgruppe, einer geschützten Maleimidgruppe, einer Dimethylaminogruppe, einer Alkenylgruppe, einer Alkenoatgruppe, einer Azidogruppe, einer Alkinylgruppe, einer Folatgruppe, einer Rhodamingruppe, einer Biotinylgruppe, einer Monosaccharidgruppe und einer Polysaccharidgruppe ausgewählt ist;
wobei A für -(CRₐR_{b})ₛO- oder -O(CRₐR_{b})ₛ- steht, wobei s für 2, 3 oder 4 steht;
wobei n₁ für eine ganze Zahl von 20 bis 250 steht,
die Alkylgruppe, Alkylengruppe, Alkoxygruppe und aliphatische Kohlenwasserstoffgruppe jeweils unabhängig substituiert oder unsubstituiert sind.

2. PEGyliertes Lipid nach Anspruch 1, wobei die B₃ und B₄ jeweils unabhängig für eine Verknüpfungsbindung oder eine C₁₋₂₀-Alkylengruppe stehen, bevorzugter aus einer der folgenden Gruppen ausgewählt sind:
Gruppe (1): B₃ und B₄ sind jeweils Verknüpfungsbindungen;
Gruppe (2): eine von B₃ und B₄ ist eine Verknüpfungsbindung, und die jeweils andere steht für eine C₁₋₂₀-Alkylengruppe;
Gruppe (3): B₃ und B₄ sind jeweils unabhängig aus der Gruppe bestehend aus einer Methylengruppe, einer Ethylengruppe, einer Propylengruppe, einer Butylengruppe, einer Pentylengruppe, einer Hexylengruppe, einer Heptylengruppe, einer Octylengruppe, einer Nonylengruppe, einer Decylengruppe, einer Undecylengruppe, einer Dodecylengruppe, einer Tridecylengruppe, einer Tetradecylengruppe, einer Pentadecylengruppe, einer Hexadecylengruppe, einer Heptadecylengruppe, einer Octadecylengruppe, einer Nonadecylengruppe und einer Eicosylengruppe ausgewählt.

3. PEGyliertes Lipid nach Anspruch 1, wobei eine der L₇ und L₈ -OC(=O)O-, -C(=O)-, -O-, -O(CH₂)ₛO-, -S-, -C(=O)S-, -SC(=O)-, -NHC(=O)-, -C(=O)NH-, - NHC(=O)NH-, -OC(=O)NH-, -NHC(=O)O-, -SC(=O)NH- oder -NHC(=O)S- ist und die jeweils andere eine Verknüpfungsbindung, -OC(=O)-, -C(=O)O-, -OC(=O)O-, -C(=O)-, -O-, -O(CH₂)ₛO-, -S-, -C(=O)S-, -SC(=O)-, -NHC(=O)-, -C(=O)NH-, -NHC(=O)NH-, - OC(=O)NH-, -NHC(=O)O-, -SC(=O)NH- oder -NHC(=O)S- ist;
bevorzugt eine der L₇ und L₈ -OC(=O)O-, -C(=O)-, -O-, -O(CH₂)ₛO-, -S-, -C(=O)S-, -SC(=O)-, -NHC(=O)-, -C(=O)NH-, -NHC(=O)NH-, -OC(=O)NH-, - NHC(=O)O-, -SC(=O)NH- oder -NHC(=O)S- ist und die jeweils andere eine Verknüpfungsbindung, -O-, -OC(=O)- oder -C(=O)O- ist;
bevorzugter die L₇ und L₈ aus einer der folgenden Gruppen ausgewählt sind:
Gruppe (1): eine von L₇ und L₈ ist -C(=O)-, und die jeweils andere ist eine Verknüpfungsbindung;
Gruppe (2): eine von L₇ und L₈ ist -C(=O)-, und die jeweils andere ist - OC(=O)- oder -C(=O)O-;
Gruppe (3): L₇ und L₈ sind jeweils -O-.

4. PEGyliertes Lipid nach Anspruch 1, wobei die L₃ abbaubare Gruppen enthält und sich die abbaubare Gruppe auf die Gruppe bezieht, die unter einer Bedingung abgebaut werden kann, die aus der Gruppe bestehend aus Licht, Wärme, niedriger Temperatur, enzymatischer Bedingung, Oxidation-Reduktion-Bedingung, saurer Bedingung, basischer Bedingung, physiologischer Bedingung und simuliertem physiologischem Milieu in vitro ausgewählt ist, L₄ und L₅ in L₃ vorzugsweise jeweils unabhängig für -(CH₂)ₜ- stehen, L₃ aus der Gruppe bestehend aus -(CH₂)ₜ-, -(CH₂)ₜZ-, -Z(CH₂)ₜ-, -(CH₂)ₜZ(CH₂)ₜ-, -Z(CH₂)ₜZ-, -(CH₂)ₜZ(CH₂)ₜZ-, -Z(CH₂)ₜZ(CH₂)ₜ- und - Z(CH₂)ₜZ(CH₂)ₜZ- ausgewählt ist, wobei t für eine ganze Zahl von 1 bis 12 steht und die Z unabhängig jeweils aus der Gruppe bestehend aus -C(=O)-, -OC(=O)-, -C(=O)O-, -OC(=O)O-, -O-, -S-, -C(=O)S-, -SC(=O)-, -NHC(=O)-, -C(=O)NH-, -NHC(=O)NH-, - OC(=O)NH-, -NHC(=O)O-, -SC(=O)NH- und -NHC(=O)S- ausgewählt sind, L₃ bevorzugt aus der Gruppe bestehend aus -(CH₂)ₜ-, -(CH₂)ₜO-, -(CH₂)ₜC(=O)-, - (CH₂)ₜC(=O)O-, -(CH₂)ₜOC(=O)-, -(CH₂)ₜC(=O)NH-, -(CH₂)ₜNHC(=O)-, - (CH₂)ₜOC(=O)O-, -(CH₂)ₜNHC(=O)O-, -(CH₂)ₜOC(=O)NH-, -(CH₂)ₜNHC(=O)NH-, - O(CH₂)ₜ-, -C(=O)(CH₂)ₜ-, -C(=O)O(CH₂)ₜ-, -OC(=O)(CH₂)ₜ-, -C(=O)NH(CH₂)ₜ-, - NHC(=O)(CH₂)ₜ-, -OC(=O)O(CH₂)ₜ-, -NHC(=O)O(CH₂)ₜ-, -OC(=O)NH(CH₂)ₜ-, - NHC(=O)NH(CH₂)ₜ-, -(CH₂)ₜO(CH₂)ₜ-, -(CH₂)ₜC(=O)(CH₂)ₜ-, -(CH₂)ₜC(=O)O(CH₂)ₜ-, - (CH₂)ₜOC(=O)(CH₂)ₜ-, -(CH₂)ₜC(=O)NH(CH₂)ₜ-, -(CH₂)ₜNHC(=O)(CH₂)ₜ-, - (CH₂)ₜOC(=O)O(CH₂)ₜ-, -(CH₂)ₜNHC(=O)O(CH₂)ₜ-, -(CH₂)ₜOC(=O)NH(CH₂)ₜ-, - (CH₂)ₜNHC(=O)NH(CH₂)ₜ-, -O(CH₂)ₜO-, -C(=O)(CH₂)ₜC(=O)-, -C(=O)O(CH₂)ₜC(=O)O-, - OC(=O)(CH₂)tOC(=O)-, -C(=O)O(CH₂)ₜOC(=O)-, -OC(=O)(CH₂)ₜC(=O)O-, - OC(=O)O(CH₂)ₜOC(=O)O-, -C(=O)NH(CH₂)ₜC(=O)NH-, -NHC(=O)(CH₂)ₜNHC(=O)-, - NHC(=O)(CH₂)ₜC(=O)NH-, -C(=O)NH(CH₂)ₜNHC(=O)-, -NHC(=O)O(CH₂)ₜNHC(=O)O-, -OC(=O)NH(CH₂)ₜOC(=O)NH-, -NHC(=O)O(CH₂)ₜOC(=O)NH-, - OC(=O)NH(CH₂)ₜNHC(=O)O-, -NHC(=O)NH(CH₂)ₜNHC(=O)NH-, -C(=O)(CH₂)ₜO-, - C(=O)(CH₂)ₜC(=O)O-, -C(=O)(CH₂)ₜOC(=O)-, -C(=O)(CH₂)ₜOC(=O)O-, - C(=O)(CH₂)ₜNHC(=O)O-, -C(=O)(CH₂)ₜOC(=O)NH-, -C(=O)(CH₂)ₜNHC(=O)NH-, - C(=O)(CH₂)ₜC(=O)O(CH₂)ₜ-, -C(=O)(CH₂)ₜOC(=O)(CH₂)ₜ-, - C(=O)(CH₂)ₜOC(=O)O(CH₂)ₜ-, -C(=O)(CH₂)ₜNHC(=O)O(CH₂)ₜ-, - C(=O)(CH₂)ₜOC(=O)NH(CH₂)ₜ-, -C(=O)(CH₂)ₜNHC(=O)NH(CH₂)ₜ- und - C(=O)(CH₂)ₜC(=O)(CH₂)ₜNHC(=O)O- ausgewählt ist, wobei t für eine ganze Zahl von 2 bis 12 steht, und bevorzugter aus der Gruppe bestehend aus -C(=O)O-, -(CH₂)ₜO-, - (CH₂)ₜC(=O)O-, -(CH₂)ₜOC(=O)-, -(CH₂)ₜO-, -C(=O)(CH₂)ₜO-, -C(=O)(CH₂)ₜOC(=O)-, - C(=O)(CH₂)ₜC(=O)O-, -C(=O)(CH₂)ₜC(=O)NH-, -C(=O)(CH₂)ₜOC(=O)NH-, - C(=O)(CH₂)ₜNHC(=O)O-, -C(=O)(CH₂)ₜOC(=O)NH(CH₂)ₜ- und - C(=O)(CH₂)ₜC(=O)(CH₂)ₜNHC(=O)O- ausgewählt ist.

5. PEGyliertes Lipid nach Anspruch 1, wobei das durch MALDI_TOF bestimmte zahlenmittlere Molekulargewicht der Polyethylenglykolkette aus der Gruppe bestehend aus 900, 1000, 1500, 2000, 2500, 3000, 3350, 3500, 4000, 5000, 5500, 6000, 6500, 7000, 7500, 8000, 8500, 9000, 9500, 10000, und 11.000 ausgewählt ist.

6. PEGyliertes Lipid nach Anspruch 1, wobei die Polyethylenglykolkette polydispers ist und der durch MALDI_TOF bestimmte zahlenmittlere Polymerisationsgrad n₁ bevorzugt eine ganze Zahl von 20 bis 100, bevorzugter eine ganze Zahl von 20 bis 60, bevorzugter eine ganze Zahl von 40 bis 60 ist und am meisten bevorzugt aus der Gruppe bestehend aus 44, 45, 46, 48, 50, 52, 54, 56, 58 und 60 ausgewählt ist; oder
wobei die Polyethylenglykolkette monodispers ist und die durch MALDI_TOF bestimmte Anzahl der EO-Einheiten bevorzugt 20 bis 70, bevorzugter 20 bis 60 beträgt, am meisten bevorzugt aus der Gruppe bestehend aus 20, 22, 24, 26, 27, 28, 29, 30, 32, 34, 36, 38, 40, 42, 44, 45, 46, 48, 50, 52, 54, 56, 58 und 60 ausgewählt ist.

7. PEGyliertes Lipid nach Anspruch 1, wobei die R₁ und R₂ jeweils unabhängig für eine C₅₋₃₀ -Alkylgruppe, bevorzugter eine C₁₀₋₃₀ -Alkylgruppe und am meisten bevorzugt eine C₁₀₋₂₀-Alkylgruppe stehen;
wobei die R₁ und R₂ jeweils unabhängig für eine lineare C₁₋₃₀-Alkylgruppe oder eine verzweigte C₁₋₃₀-Alkylgruppe sind, bevorzugt eine lineare C₁₋₃₀-Alkylgruppe, bevorzugter eine lineare C₁₋₂₅-Alkylgruppe, bevorzugter eine lineare C₁₋₂₀-Alkylgruppe stehen, bevorzugter aus der Gruppe bestehend aus einer Methylgruppe, einer Ethylgruppe, einer Propylgruppe, einer Butylgruppe, einer Pentylgruppe, einer Hexylgruppe, einer Heptylgruppe, einer Octylgruppe, einer Nonylgruppe, einer Decylgruppe, einer Undecylgruppe, einer Dodecylgruppe, einer Tridecylgruppe, einer Tetradecylgruppe, einer Pentadecylgruppe, einer Hexadecylgruppe, einer Heptadecylgruppe, einer Octadecylgruppe, einer Nonadecylgruppe und einer Eicosylgruppe ausgewählt sind, am meisten bevorzugt jeweils unabhängig für eine verzweigte C₁₋₃₀-Alkylgruppe stehen und jeweils unabhängig als 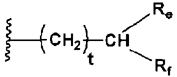
dargestellt sind; wobei Rₑ und R_{f} jeweils unabhängig für eine C₁₋₁₅ -Alkylgruppe stehen, bevorzugter aus der Gruppe bestehend aus einer Methylgruppe, einer Ethylgruppe, einer Propylgruppe, einer Butylgruppe, einer Pentylgruppe, einer Pentylgruppe, einer Hexylgruppe, einer Heptylgruppe, einer Octylgruppe, einer Nonylgruppe, einer Decylgruppe, einer Undecylgruppe, einer Dodecylgruppe, einer Tridecylgruppe und einer Tetradecylgruppe ausgewählt sind;
und noch bevorzugter R₁ und R₂ jeweils unabhängig aus der Gruppe bestehend aus den folgenden Strukturen ausgewählt sind: 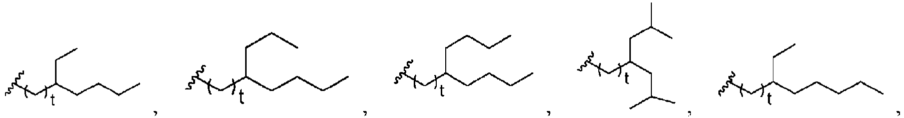 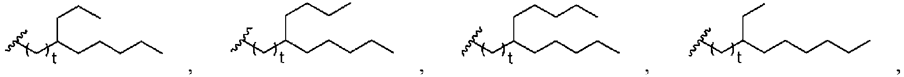 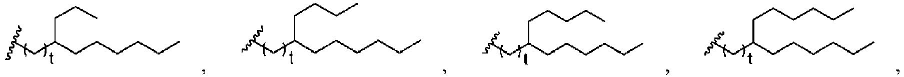 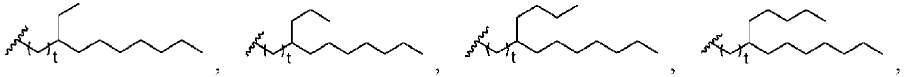 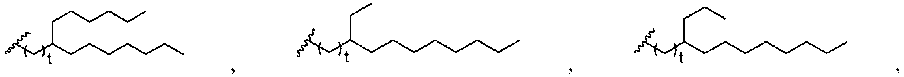 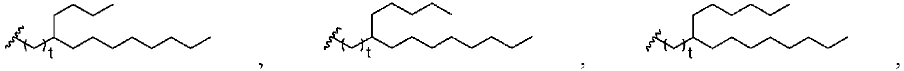 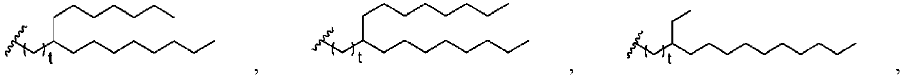 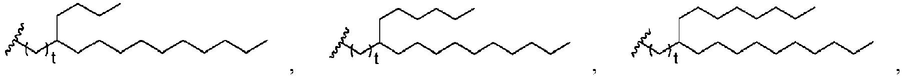 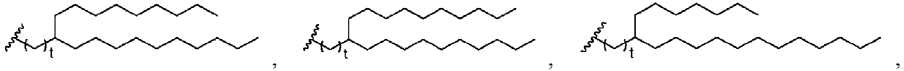 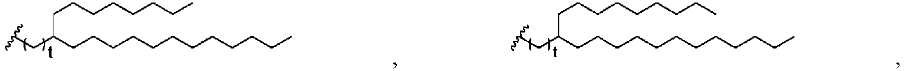 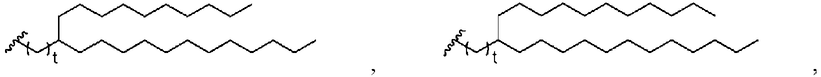 und 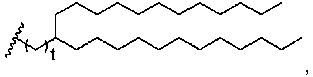 wobei t für eine ganze Zahl von 0 bis 12 steht.

8. PEGyliertes Lipid nach Anspruch 1, wobei das R aus der Gruppe bestehend aus H, -CH₃, -CH₂CH₃, -(CH₂)ₜOH, -(CH₂)ₜSH, -OCH₃, -OCH₂CH₃, - (CH₂)ₜNH₂, -(CH₂)ₜC(=O)OH, -C(=O)(CH₂)ₜC(=O)OH, -C(=O)CH₃, -(CH₂)ₜN₃, - C(=O)CH₂CH₃, -C(=O)OCH₃, -OC(=O)OCH₃, -C(=O)OCH₂CH₃, -OC(=O)OCH₂CH₃, - (CH₂)ₜN(CH₃)₂, -(CH₂)ₜN(CH₂CH₃)₂, -(CH₂)ₜCHO, 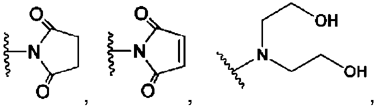 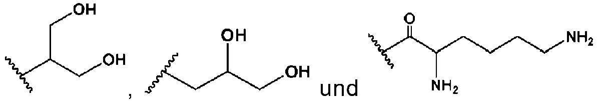 ausgewählt ist.

9. PEGyliertes Lipid nach Anspruch 1, wobei die Struktur des PEGylierten Lipids aus der Gruppe bestehend aus den folgenden Formeln ausgewählt ist: 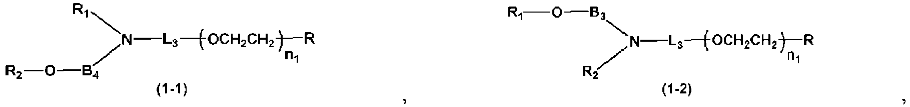 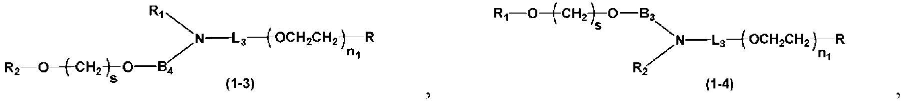 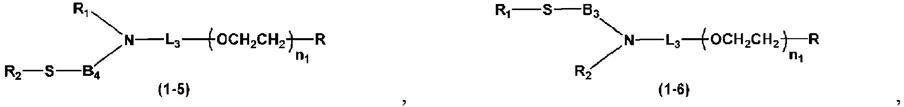 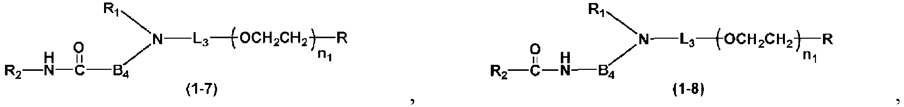 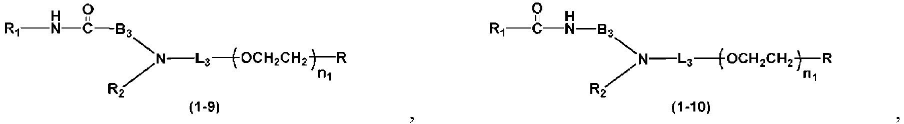 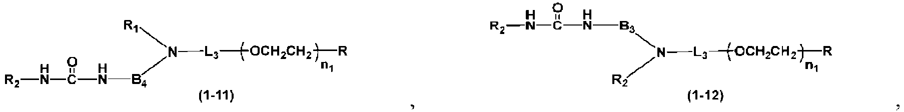 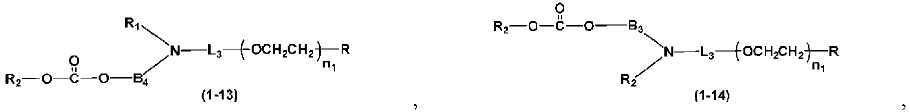 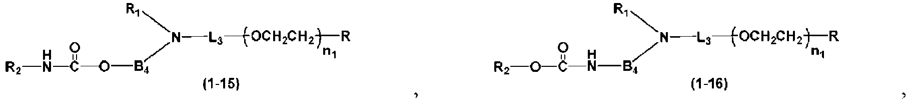 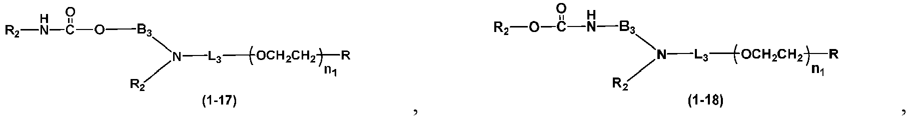 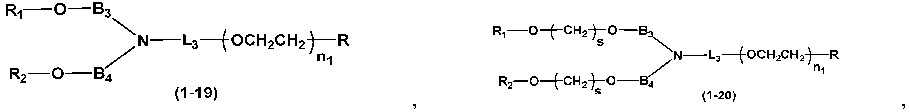 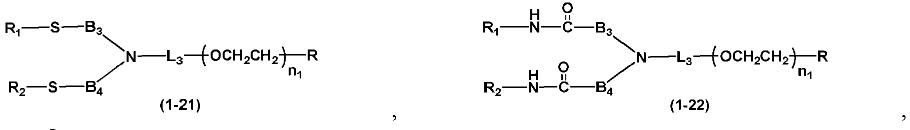 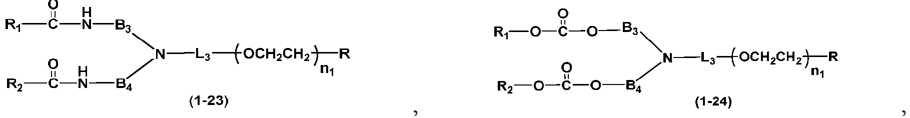 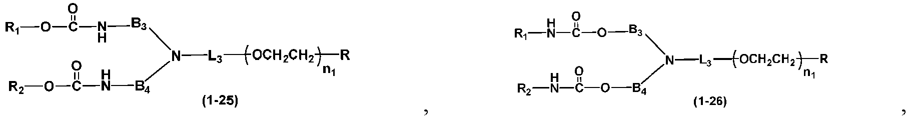 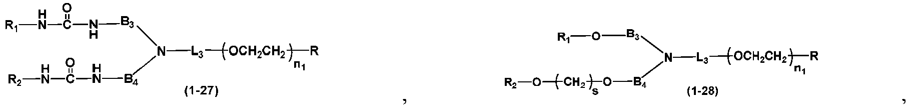 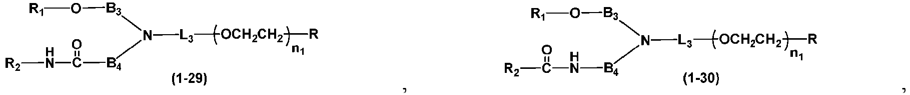 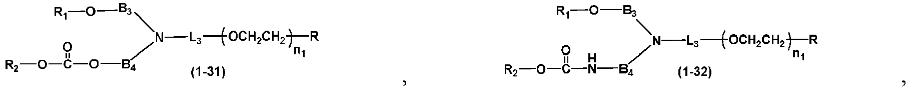 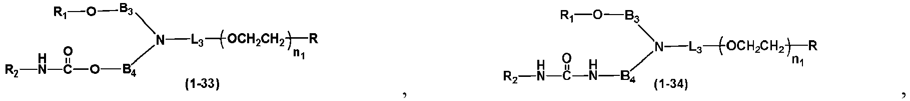 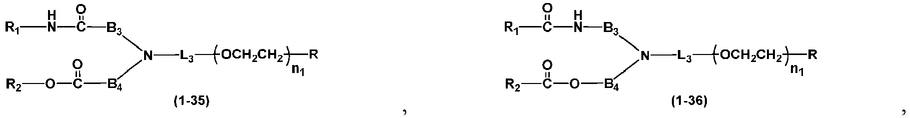 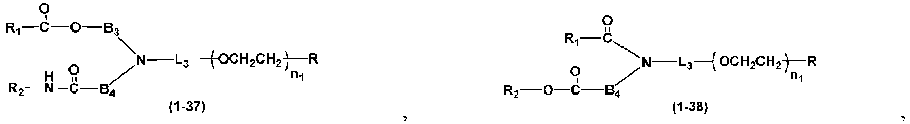 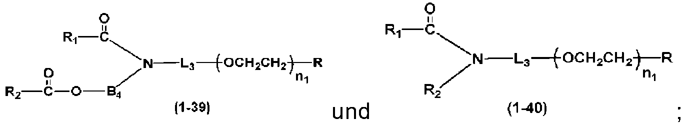
oder
wobei die Struktur des PEGylierten Lipids aus der Gruppe bestehend aus den folgenden Strukturen ausgewählt ist: 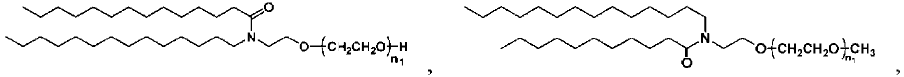 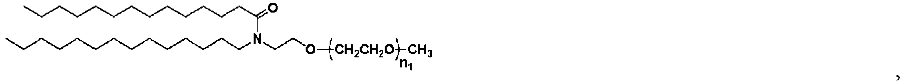 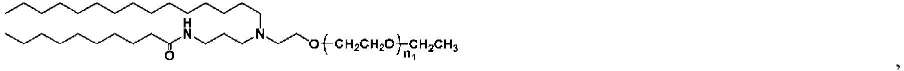 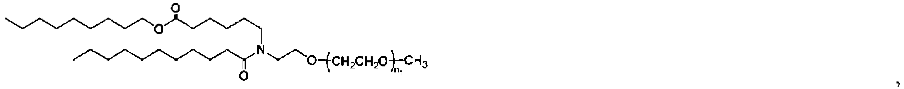 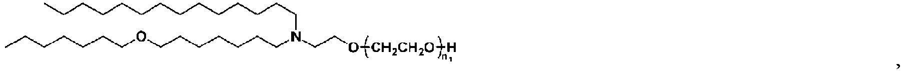 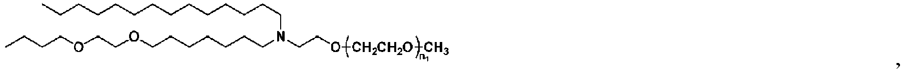 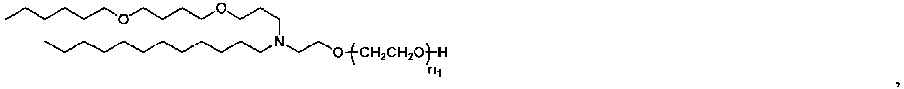 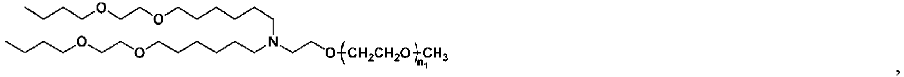 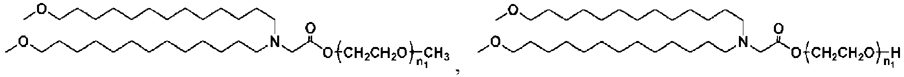 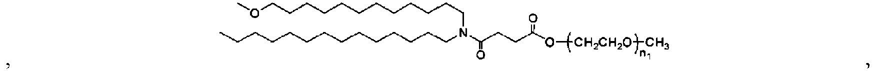 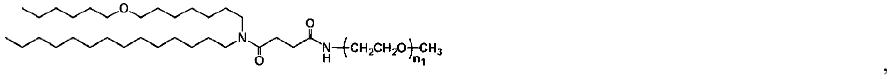 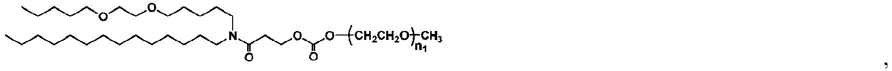 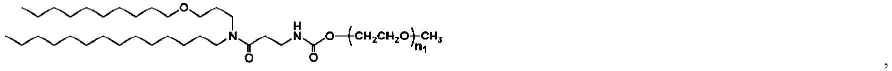 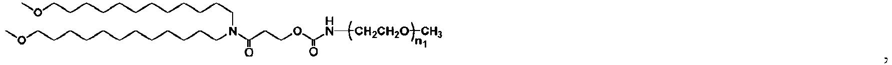 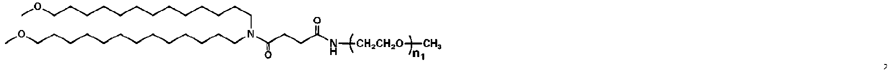 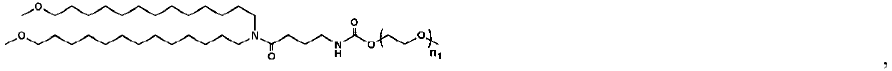 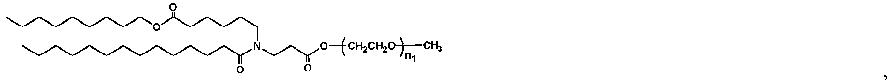 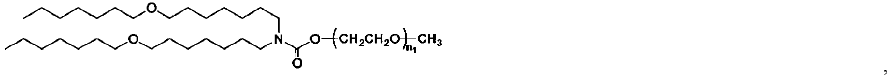 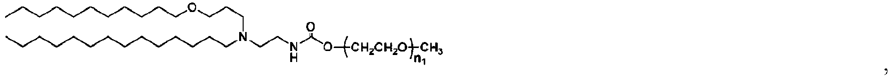 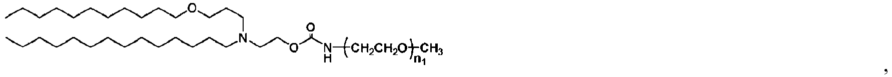 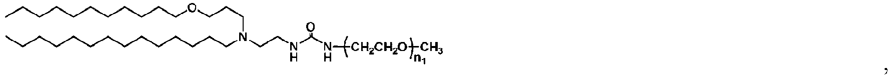 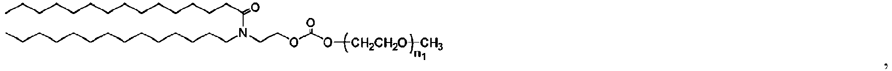 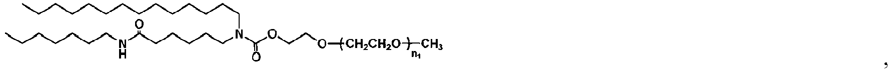 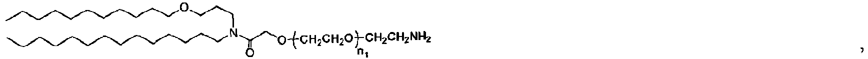 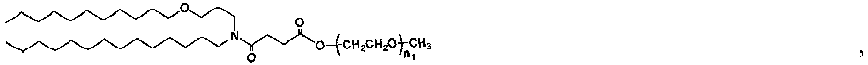 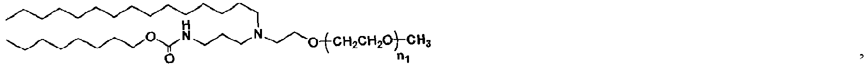 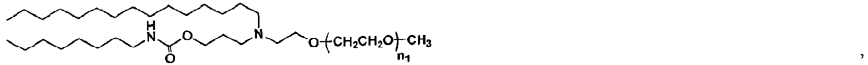 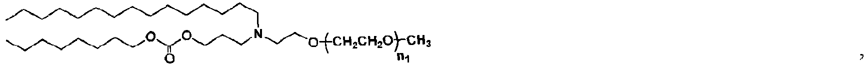 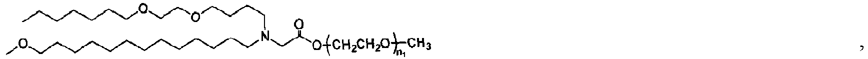 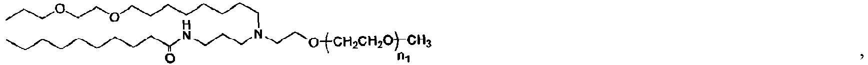 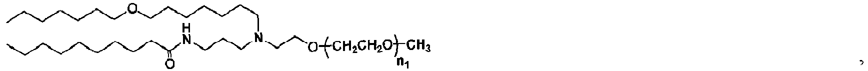 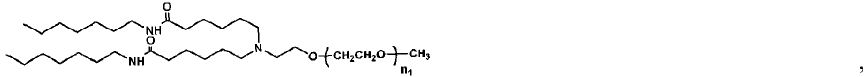 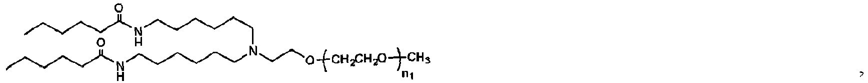 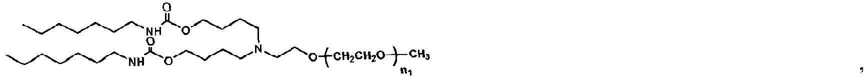 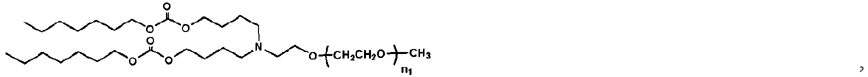 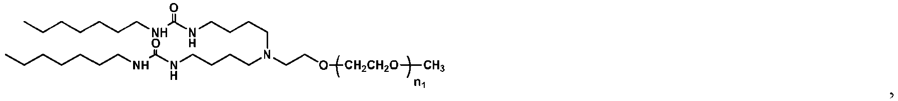 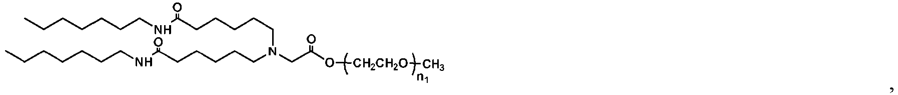 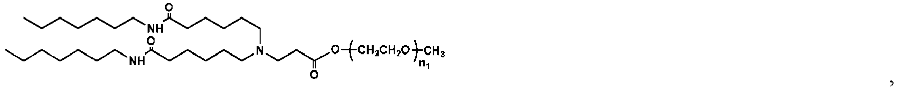 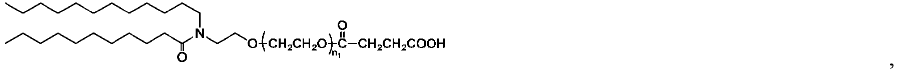 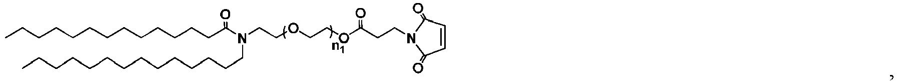 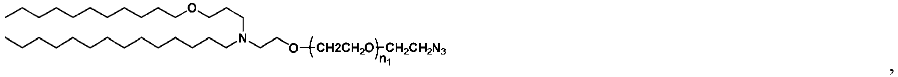 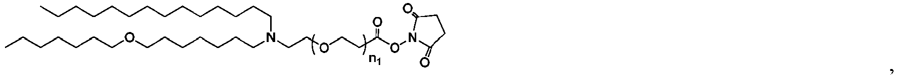 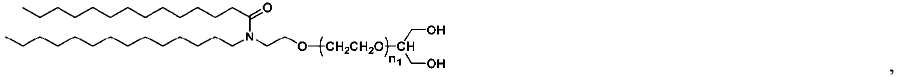 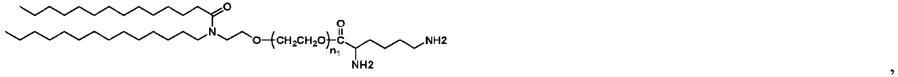 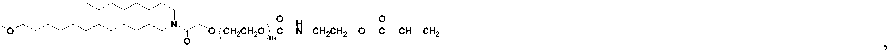 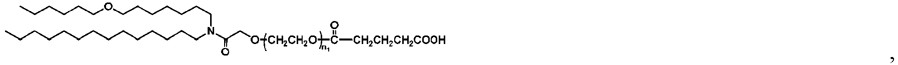 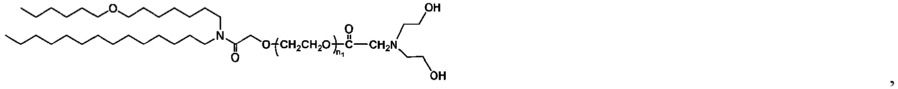 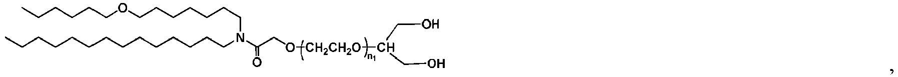 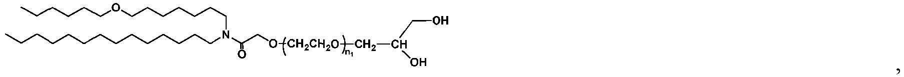 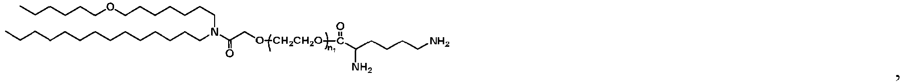 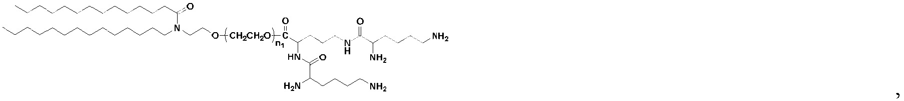 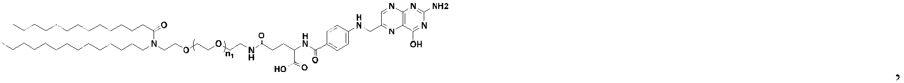 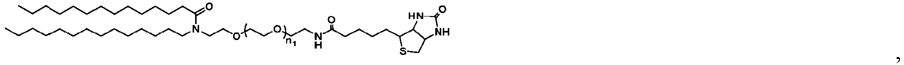 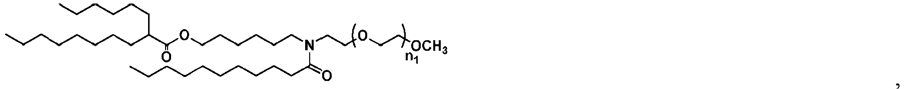 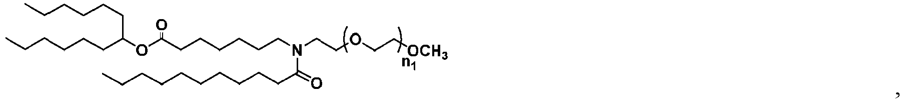 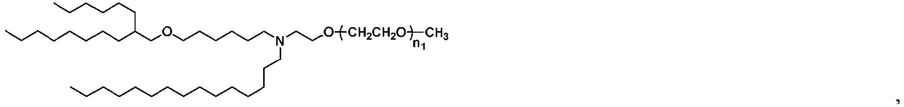 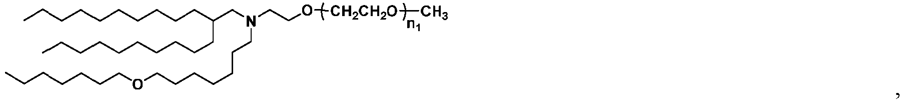 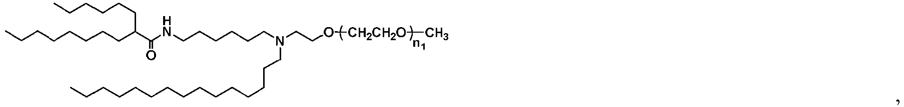 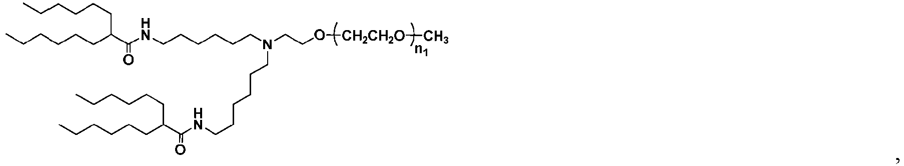 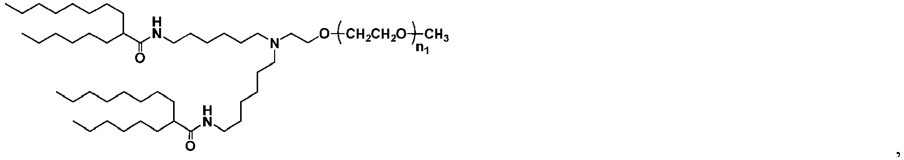 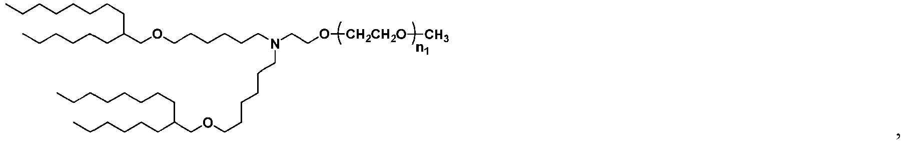 und

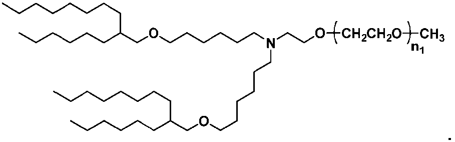

10. Kationisches Liposom, wobei das kationische Liposom ein vorstehendes PEGyliertes Lipid nach einem der Ansprüche 1 bis 9 enthält.

11. Kationisches Liposom nach Anspruch 10, wobei das kationische Liposom auch eine oder mehrere Arten von Lipiden enthält, die aus der Gruppe bestehend aus neutralem Lipid, Steroidlipid und kationischem Lipid ausgewählt sind, wobei es sich bei dem neutralen Lipid vorzugsweise um Phospholipid handelt.

12. Kationisches Liposom nach Anspruch 11, wobei das neutrale Lipid aus der Gruppe bestehend aus 1,2-Dilinoleoyl-sn-glycero-3-phosphocholinen, 1,2-Dimyristoleoyl-sn-glycero-3-phosphocholinen, 1,2-Dioleoyl-sn-glycero-3-phosphocholinen, 1,2-Dipalmitoyl-sn-glycero-3-phosphocholinen, 1,2-Distearoyl-sn-glycero-3-phosphatidylcholinen, 1,2-Diundecanoyl-sn-glycero-3-phosphatidylcholinen, 1-Palmitoyl-2-oleoyl-sn-glycero-3-phosphocholinen, 1,2-Di-O-octadecenyl-sn-glycero-3-phosphatidylcholinen, 1-Oleoyl-2-cholesterylhemisuccinyl-sn-glycero-3-phosphocholinen, 1-O-Hexadecyl-sn-glycero-3-phosphatidylcholinen, 1,2-Dilinolenoyl-sn-glycero-3-phosphatidylcholinen, 1,2-Diarachidonoyl-sn-glycero-3-phosphatidylcholinen, 1,2-Didecosahexaenoyl-sn-glycero-3-phosphocholinen, 1,2-Dioleoyl-sn-glycero-3-phosphoethanolaminen, 1,2-Diphytanyl-sn-glycero-3-phosphoethanolaminen, 1,2-Distearoyl-sn-glycero-3-phosphoethanolaminen, 1,2-Dilinoleoyl-sn-glycero-3-phosphoethanolaminen, 1,2-Dilinolenoyl-sn-glycero-3-phosphoethanolaminen, 1,2-Diarachidonoyl-sn-glycero-3-phosphoethanolaminen, 1,2-Didecosahexaenoyl-sn-glycero-3-phosphoethanolaminen, 1,2-Dioleoyl-sn-glycero-3-phospho-rac-(1-glycerin)-Natriumsalzen, Dioleoylphosphatidylserinen, Dipalmitoylphosphatidylglycerinen, Palmitoyloleoylphosphatidylethanolaminen, Distearoylphosphatidylethanolaminen, Dipalmitoylphosphatidylethanolaminen, Dimyristoleoylphosphoethanolaminen, 1-Stearoyl-2-oleoyl-stearoylethanolaminen, 1-Stearoyl-2-oleoyl-phosphatidylcholinen, Sphingomyelinen, Phosphatidylcholinen, Phosphatidylethnolaminen, Phosphatidylserinen, Phosphatidylinositolen, Phosphatidsäuren, Palmitoyloleoylphosphatidylcholinen, Lysophosphatidylcholinen, Lysophosphatidylethanolaminen und Kombinationen davon ausgewählt ist; oder
das Steroidlipid aus der Gruppe bestehend aus einem Cholesterin, einem Coprostanol, einem Sitosterol, einem Ergosterol, einem Campesterol, einem Stigmasterol, einem Brassicasteroltomatidin, einer Ursolsäure, einem α-Tocopherol und irgendeinem beliebigen Gemisch davon ausgewählt ist; oder wobei die Struktur des kationischen Lipids durch die allgemeine Formel (1) wiedergegeben wird: 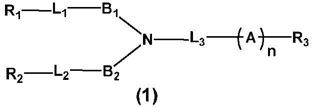
wobei N das Stickstoffverzweigungszentrum ist,
L₁ und L₂ zweiwertige Verknüpfungsgruppen sind, die jeweils unabhängig aus der Gruppe bestehend aus -OC(=O)-, -C(=O)O-, -OC(=O)O-, -C(=O)-, -O-, -S-, -C(=O)S-, -SC(=O)-, -NR_{c}C(=O)-, -C(=O)NR_{c}-, -NR_{c}C(=O)NR_{c}-, - OC(=O)NR_{c}-, -NR_{c}C(=O)O-, -SC(=O)NR_{c}- und -NR_{c}C(=O)S- ausgewählt sind, wobei R_{c} für ein Wasserstoffatom oder eine C₁₋₁₂-Alkylgruppe steht,
L₃ für eine Verknüpfungsbindung oder -L₄-Z-L₅- steht, die L₄ und L₅ Kohlenstoffketten-Verknüpfungsgruppen sind, die jeweils unabhängig aus -(CRₐR_{b})ₜ-oder -(CRₐR_{b})ₜ-(CRₐR_{b})ₚ-(CRₐR_{b})_{q}- ausgewählt sind, wobei t, p und q jeweils unabhängig für eine ganze Zahl von 0 bis 12 stehen, Rₐ und R_{b} jeweils unabhängig für ein Wasserstoffatom oder eine C₁₋₁₂-Alkylgruppe stehen, das Z für eine Verknüpfungsbindung oder eine zweiwertige Verknüpfungsgruppe steht, die aus der Gruppe bestehend aus -OC(=O)-, -C(=O)O-, -OC(=O)O-, -C(=O)-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, - SC(=O)NR_{c}- und -NR_{c}C(=O)S- ausgewählt ist,
B₁ und B₂ jeweils unabhängig für eine C₁₋₁₂-Alkylengruppe stehen,
R₁ und R₂ jeweils unabhängig für eine aliphatische C₂₋₃₀-Kohlenwasserstoffgruppe stehen,
R₃ für ein Wasserstoffatom, eine Alkylgruppe, eine Alkoxygruppe, - C(=O)R_{d}, -C(=O)OR_{d}, -OC(=O)R_{d} oder -OC(=O)OR_{d} stehen, wobei Rₐ für eine C₁₋₁₂ - Alkylgruppe steht,
A aus der Gruppe bestehend aus -(CRₐR_{b})ₛO-, -O(CRₐR_{b})ₛ-, -(CRₐR_{b})ₛS-
, -S(CRₐR_{b})ₛ-, -(CRₐR_{b})ₛO(CRₐR_{b})ₛS-, -(CRₐR_{b})ₛS(CRₐR_{b})ₛO-, -(CRₐR_{b})ₛNR_{c}(CRₐR_{b})ₛS-, -(CRₐR_{b})ₛS(CRₐR_{b})ₛNR_{c}-, -(CRₐR_{b})ₛNR_{c}(CRₐR_{b})ₛO- und -(CRₐR_{b})ₛO(CRₐR_{b})ₛNR_{c}-ausgewählt ist, wobei s für 2, 3 oder 4 steht, Rₐ und R_{b} jeweils unabhängig für ein Wasserstoffatom oder eine C₁₋₁₂-Alkylgruppe stehen,
n, wenn A -(CRₐR_{b})ₛO- oder -O(CRₐR_{b})ₛ- ist, für eine ganze Zahl von 2 bis 6 steht, n, wenn A nicht -(CRₐR_{b})ₛO- oder -O(CRₐR_{b})ₛ- ist, für eine ganze Zahl von 1 bis 6 steht,
die Alkylgruppe, Alkylengruppe, Alkoxygruppe und aliphatische Kohlenwasserstoffgruppe jeweils unabhängig substituiert oder unsubstituiert sind; oder
wobei die Struktur des kationischen Lipids aus den folgenden Strukturen ausgewählt ist: 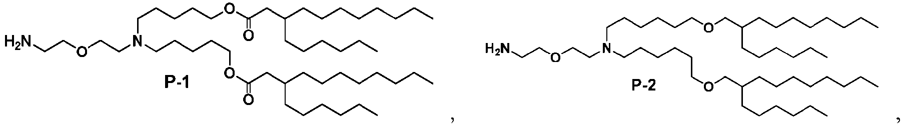 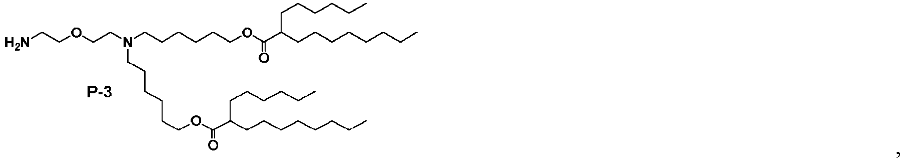 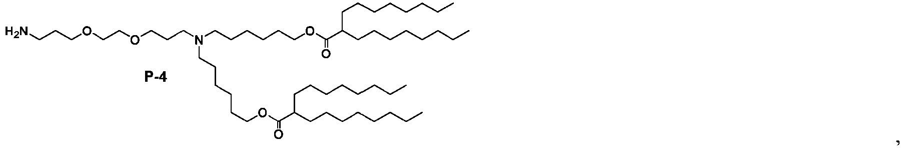 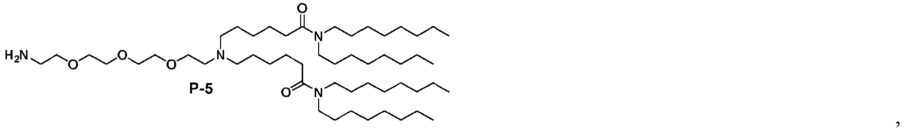 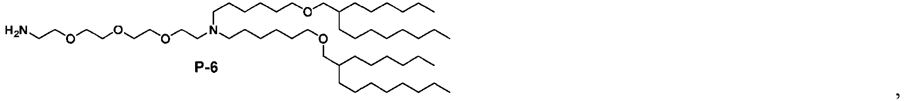 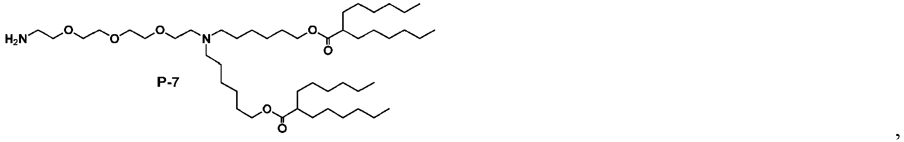 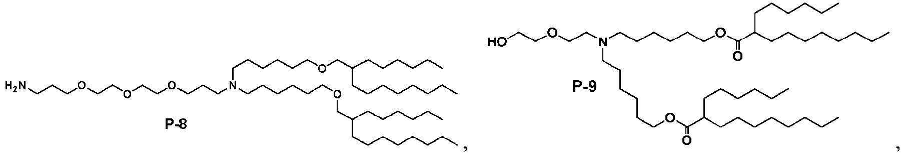 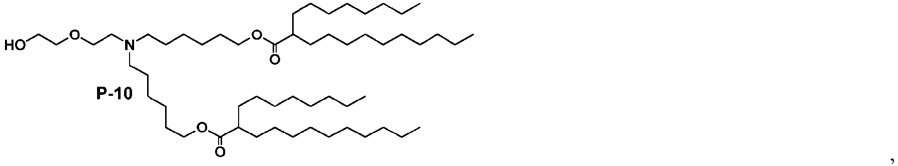 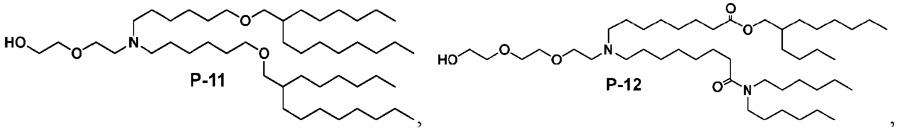 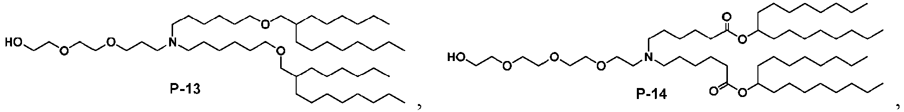 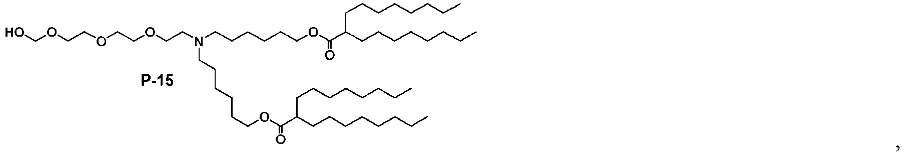 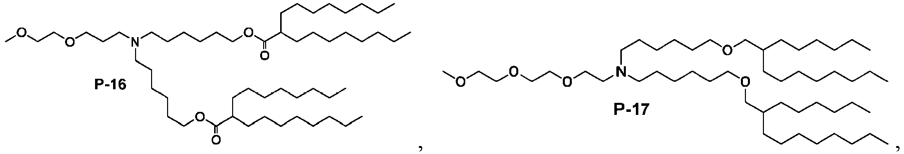 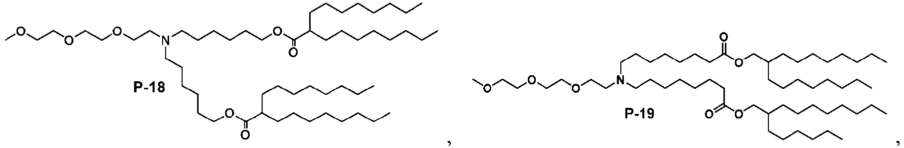 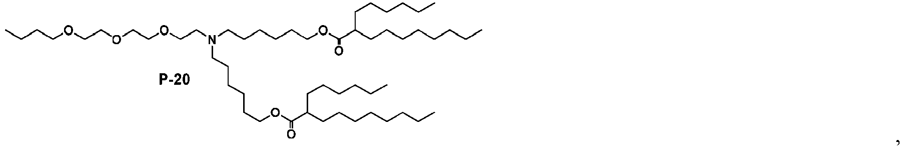 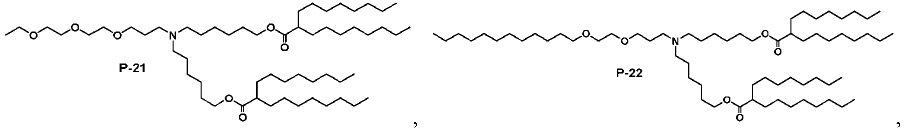 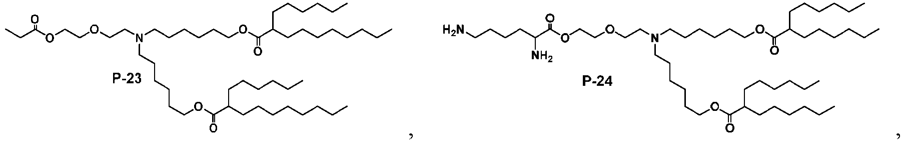 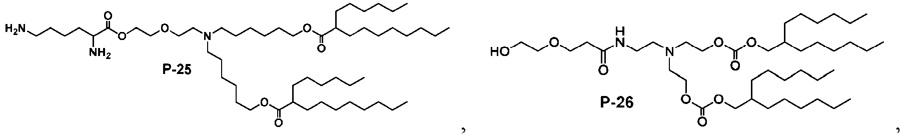 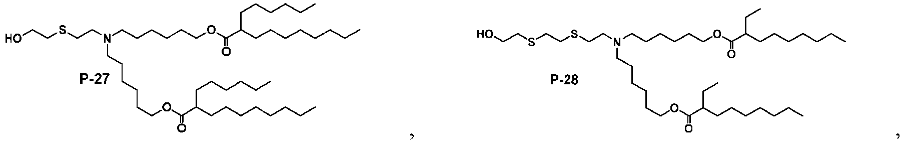 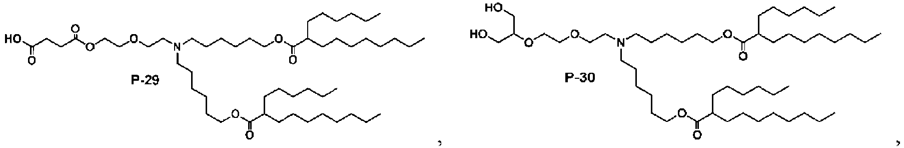 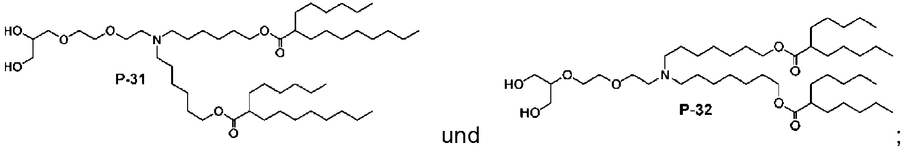 oder
wobei die kationischen Lipide in den kationischen Liposomen aus der Gruppe bestehend aus *N,N*-Dioleyl-*N,N*-dimethylammoniumchlorid, N,N-Distearyl-N,N-dimethylammoniumbromid, 1,2-Dioleoyl-3-trimethylammoniumpropan, N-[1-(2,3-Dioleoyloxy)propyl]-N,N,N-trimethylammoniumchlorid, 1,2-Dioleyloxy-*N,N-*dimethylaminopropan, 3-(Didodecylamino)-*N1*,*N1*,4-tridodecyl-1-piperazinethanamin, *N1*-[2-(Didodecylamino)ethyl]-*N1*,*N4*,*N4*-tridodecyl-1,4-piperazindiethanamin, 14,25-Ditridecyl-15,18,21,24-tetraaza-octatriacontan, 1,2-Dilinoleyloxy-*N,N-*dimethylaminopropan, 2,2-Dilinoleyl-4-dimethylaminomethyl-[1,3]-dioxolan, Heptatriaconta-6,9,28,31-tetraen-19-yl 4-(dimethylamino)butanoat, 2,2-Dilinoleyl-4-(2-dimethylaminoethyl)-[1,3]-dioxolan und Gemischen davon ausgewählt sein können.

13. Kationisches Liposom nach einem der Ansprüche **11** bis 12, wobei das kationische Liposom 20 % bis 80 % kationische Lipide, 5 % bis 15 % neutrale Lipide, 25 % bis 55 % Steroidlipide und 0,5 % bis 10 % PEGylierte Lipide der allgemeinen Formel (2) enthält, wobei es sich bei den Prozentsätzen jeweils um die molaren Prozentsätze der einzelnen Lipide im Verhältnis zu den gesamten Lipiden in einer das Lösungsmittel enthaltenden Lösung handelt.

14. Kationisches Liposom nach einem der Ansprüche **11** bis 12, wobei der molare Prozentsatz der kationischen Lipide im Verhältnis zu den gesamten Lipiden in einer das Lösungsmittel enthaltenden Lösung bei 30 % bis 65 %, bevorzugter bei 35 %, 40 %, 45 %, 46 %, 47 %, 48 %, 49 %, 50 % oder 55 % liegt; oder
wobei der molare Prozentsatz der neutralen Lipide im Verhältnis zu den gesamten Lipiden in einer das Lösungsmittel enthaltenden Lösung bei 7,5 % bis 13 %, bevorzugter bei 8 %, 9 %, 10 %, 11 % oder 12 % liegt; oder
wobei der molare Prozentsatz der Steroidlipide im Verhältnis zu den gesamten Lipiden in einer das Lösungsmittel enthaltenden Lösung bei 35 % bis 50 %, bevorzugter bei 40 %, 41 %, 42 %, 43 %, 44 %, 45 %, 46 %, 47 %, 48 %, 49 % oder 50 % liegt; oder
wobei der molare Prozentsatz der PEGylierten Lipide im Verhältnis zu den gesamten Lipiden in einer das Lösungsmittel enthaltenden Lösung bei 0,5 % bis 5 %, bevorzugt bei 1 % bis 3 %, bevorzugter bei 1,5 %, 1,6 %, 1,7 %, 1,8% oder 1,9 % liegt.

15. Kationische pharmazeutische Liposomenzusammensetzung, wobei die kationische pharmazeutische Liposomenzusammensetzung das kationische Liposom nach einem der Ansprüche 11 bis 12 und Arzneistoffe enthält, es sich bei dem Arzneistoff vorzugsweise um einen Nukleinsäurearzneistoff oder ein Antitumormittel handelt, der Nukleinsäurearzneistoff aus der Gruppe bestehend aus DNA, Antisense-Nukleinsäure, Plasmid, mRNA, Interferenz-Nukleinsäure, Aptamer, Antagomir, miRNA, Ribozym und siRNA ausgewählt ist und bevorzugter aus der Gruppe bestehend aus DNA, mRNA, miRNA und siRNA ausgewählt ist.

## Revendications

1. Lipide PEGylé, dans lequel la structure est représentée par la formule générale (2) : 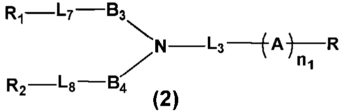 ou un sel, un tautomère ou un stéréoisomère pharmaceutiquement acceptable de celui-ci,
dans laquelle l'un de L₇ et L₈ est -OC(=O)O-, -C(=O)-, -O-, -O(CR_{c}R_{c})ₛO-, -S-, -C(=O)S-, -SC(=O)-, -NR_{c}C(=O)-, -C(=O)NR_{c}-, -NR_{c}C(=O)NR_{c}-, -OC(=O)NR_{c}-, - NR_{c}C(=O)O-, -SC(=O)NR_{c}-, ou -NR_{c}C(=O)S-, et l'autre est une liaison, -OC(=O), - C(=O)O-, -OC(=O)O-, -C(=O)-, -O-, -O(CR_{c}R_{c})ₛO-, -S-, -C(=O)S-, -SC(=O)-, - NR_{c}C(=O)-, -C(=O)NR_{c}-, -NR_{c}C(=O)NR_{c}-, -OC(=O)NR_{c}-, -NR_{c}C(=O)O-, - SC(=O)NR_{c}-, ou -NR_{c}C(=O)S-, où R_{c} est indépendamment, à chaque occurrence, un atome d'hydrogène ou un groupe alkyle, s est 2, 3 ou 4 ;
dans laquelle L₃ est une liaison, -L₄-, -Z-L₄-, -L₄-Z-, -Z-L₄-Z-, -L₄-Z-L₅-, -Z-L₄-Z-L₅-, -L₄-Z-L₅-Z-, -Z-L₄-Z-L₅-Z-, ou -L₄-Z-L₄-Z-L₅-Z- ; lesdits L₄ et L₅ sont des groupes de liaison à chaîne carbonée, qui sont chacun représentés indépendamment par -(CRₐR_{b})ₜ-(CRₐR_{b})ₒ-(CRₐR_{b})ₚ-, où t, o et p sont chacun indépendamment un nombre entier de 0 à 12, et t, o et p ne sont pas simultanément 0 ; Rₐ et R_{b} sont, à chaque occurrence, indépendamment un atome d'hydrogène ou un groupe alkyle en C₁ à C₁₂ ; ledit Z est, à chaque occurrence, indépendamment choisi dans le groupe constitué par -C(=O)-, -OC(=O)-, -C(=O)O-, -OC(=O)O-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NR_{c}C(=O)S- et - 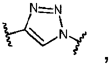 où R_{c} est, à chaque occurrence, indépendamment un atome d'hydrogène ou un groupe alkyle en C₁ à C12 ;
dans laquelle B₃ et B₄ sont chacun indépendamment une liaison ou un groupe alkylène en C₁ à C₃₀ ;
dans laquelle R₁ et R₂ sont chacun indépendamment un groupe alkyle en C₁ à C₃₀ ;
dans laquelle R contient tout atome ou groupe choisi dans le groupe constitué par un atome d'hydrogène, un groupe alkyle, un groupe alcoxy, un groupe hydroxyle alcoolique, un groupe hydroxyle alcoolique protégé, un groupe thiol, un groupe thiol protégé, un groupe carboxyle, un groupe carboxyle protégé, un groupe amino, un groupe amino protégé, un groupe aldéhyde, un groupe aldéhyde protégé, un groupe ester, un groupe carbonate, un groupe uréthane, un groupe succinimidyle, un groupe maléimide, un groupe maléimide protégé, un groupe diméthylamino, un groupe alcényle, un groupe alcénate, un groupe azido, un groupe alcynyle, un groupe folate, un groupe rhodamine, un groupe biotinyle, un groupe monosaccharide et un groupe polysaccharide ;
dans laquelle A est -(CRₐR_{b})ₛO- ou -O(CRₐR_{b})ₛ-, où s est 2, 3 ou 4 ;
dans laquelle n₁ est un nombre entier compris entre 20 et 250 ;
ledit groupe alkyle, ledit groupe alkylène, ledit groupe alcoxy et ledit groupe hydrocarbure aliphatique sont chacun indépendamment substitués ou non substitués.

2. Lipide PEGylé selon la revendication **1,** dans lequel lesdits B₃ et B₄ sont chacun indépendamment une liaison ou un groupe alkylène en C₁ à C₂₀ ; de préférence choisis parmi l'un quelconque des groupes suivants :
Groupe (1) : B₃ et B₄ sont tous deux des liaisons ;
Groupe (2) : l'un de B₃ et B₄ est une liaison, et l'autre est un groupe alkylène en C₁ à C₂₀ ;
Groupe (3) : B₃ et B₄ sont chacun indépendamment choisis dans le groupe constitué par un groupe méthylène, un groupe éthylène, un groupe propylène, un groupe butylène, un groupe pentylène, un groupe hexylène, un groupe heptylène, un groupe octylène, un groupe nonylène, un groupe décylène, un groupe undécylène, un groupe dodécylène, un groupe tridécylène, un groupe tétradécylène, un groupe pentadécylène, un groupe hexadécylène, un groupe heptadécylène, un groupe octadécylène, un groupe nonadécylène et un groupe eicosylène.

3. Lipide PEGylé selon la revendication **1,** dans lequel l'un desdits L₇ et L₈ est -OC(=O)O-, -C(=O)-, -O-, -O(CH₂)ₛO-, -S-, -C(=O)S-, -SC(=O)-, -NHC(=O)-, - C(=O)NH-, -NHC(=O)NH-, -OC(=O)NH-, -NHC(=O)O-, -SC(=O)NH-, ou - NHC(=O)S-, et l'autre est une liaison, -OC(=O)-, -C(=O)O-, -OC(=O)O-, -C(=O)-, - O-, -O(CH₂)ₛO-, -S-, -C(=O)S-, -SC(=O)-, -NHC(=O)-, -C(=O)NH-, -NHC(=O)NH-, - OC(=O)NH-, -NHC(=O)O-, -SC(=O)NH-, ou -NHC(=O)S- ;
de préférence, l'un desdits L₇ et L₈ est -OC(=O)O-, -C(=O)-, -O-, -O(CH₂)ₛ0-, -S-, -C(=O)S-, -SC(=O)-, -NHC(=O)-, -C(=O)NH-, -NHC(=O)NH-, -OC(=O)NH-, - NHC(=O)O-, -SC(=O)NH-, ou -NHC(=O)S-, et l'autre est une liaison, -O-, -OC(=O)-, ou -C(=O)O- ;
plus préférablement, lesdits L₇ et L₈ sont choisis parmi l'un quelconque des groupes suivants :
Groupe (1) : l'un de L₇ et L₈ est -C(=O)-, et l'autre est une liaison ;
Groupe (2) : l'un de L₇ et L₈ est -C(=O)-, et l'autre est -OC(=O)- ou -C(=O)O- ;
Groupe (3) : L₇ et L₈ sont tous deux -O-.

4. Lipide PEGylé selon la revendication **1,** dans lequel ledit L₃ contient des groupes dégradables, et ledit groupe dégradable désigne le groupe qui peut se dégrader dans n'importe quelle condition choisie dans le groupe constitué par la lumière, la chaleur, une température basse, une condition enzymatique, une condition d'oxydoréduction, une condition acide, une condition basique, une condition physiologique et un environnement physiologique simulé in vitro ; L₄ et L₅ dans L₃ sont de préférence chacun indépendamment -(CH₂)ₜ- ; L₃ est choisi dans le groupe constitué par -(CH₂)ₜ-, -(CH₂)ₜZ-, -Z(CH₂)ₜ-, -(CH₂)ₜZ(CH₂)ₜ-, -Z(CH₂)ₜZ-, - (CH₂)ₜZ(CH₂)ₜZ-, -Z(CH₂)ₜZ(CH₂)ₜ-, et -Z(CH₂)ₜZ(CH₂)ₜZ-, où t est un nombre entier compris entre 1 et 12, et Z est, à chaque occurrence, indépendamment choisi dans le groupe constitué par -C(=O)-, -OC(=O)-, -C(=O)O-, -OC(=O)O-, -O-, -S-, - C(=O)S-, -SC(=O)-, -NHC(=O)-, -C(=O)NH-, -NHC(=O)NH-, -OC(=O)NH-, - NHC(=O)O-, -SC(=O)NH- et -NHC(=O)S- ; L₃ est de préférence choisi dans le groupe constitué par -(CH₂)ₜ-, -(CH₂)ₜO-, -(CH₂)ₜC(=O)-, -(CH₂)ₜC(=O)O-, - (CH₂)ₜOC(=O)-, -(CH₂)ₜC(=O)NH-, -(CH₂)ₜNHC(=O)-, -(CH₂)ₜOC(=O)O-, - (CH₂)ₜNHC(=O)O-, -(CH₂)ₜOC(=O)NH-, -(CH₂)ₜNHC(=O)NH-, -O(CH₂)ₜ-, - C(=O)(CH₂)ₜ-, -C(=O)O(CH₂)ₜ-, -OC(=O)(CH₂)ₜ-, -C(=O)NH(CH₂)ₜ-, -NHC(=O)(CH₂)ₜ-, -OC(=O)O(CH₂)ₜ-, -NHC(=O)O(CH₂)ₜ-, -OC(=O)NH(CH₂)ₜ-, -NHC(=O)NH(CH₂)ₜ-, - (CH₂)ₜO(CH₂)ₜ-, -(CH₂)ₜC(=O)(CH₂)ₜ-, -(CH₂)ₜC(=O)O(CH₂)ₜ-, -(CH₂)ₜOC(=O)(CH₂)ₜ-, - (CH₂)ₜC(=O)NH(CH₂)ₜ-, -(CH₂)ₜNHC(=O)(CH₂)ₜ-, -(CH₂)ₜOC(=O)O(CH₂)ₜ-, - (CH₂)ₜNHC(=O)O(CH₂)ₜ-, -(CH₂)ₜOC(=O)NH(CH₂)ₜ-, -(CH₂)ₜNHC(=O)NH(CH₂)ₜ-, - O(CH₂)ₜO-, -C(=O)(CH₂)ₜC(=O)-, -C(=O)O(CH₂)ₜC(=O)O-, -OC(=O)(CH₂)ₜOC(=O)-, - C(=O)O(CH₂)ₜOC(=O)-, -OC(=O)(CH₂)ₜC(=O)O-, -OC(=O)O(CH₂)ₜOC(=O)O-, - C(=O)NH(CH₂)ₜC(=O)NH-, -NHC(=O)(CH₂)ₜNHC(=O)-, NHC(=O)(CH₂)ₜC(=O)NH-, - C(=O)NH(CH₂)ₜNHC(=O)-, -NHC(=O)O(CH₂)ₜNHC(=O)O-, - OC(=O)NH(CH₂)ₜOC(=O)NH-, -NHC(=O)O(CH₂)ₜOC(=O)NH-, - OC(=O)NH(CH₂)ₜNHC(=O)O-, -NHC(=O)NH(CH₂)ₜNHC(=O)NH-, -C(=O)(CH₂)ₜO-, - C(=O)(CH₂)ₜC(=O)O-, -C(=O)(CH₂)ₜOC(=O)-, -C(=O)(CH₂)ₜOC(=O)O-, - C(=O)(CH₂)ₜNHC(=O)O-, -C(=O)(CH₂)ₜOC(=O)NH-, -C(=O)(CH₂)ₜNHC(=O)NH-, - C(=O)(CH₂)ₜC(=O)O(CH₂)ₜ-, -C(=O)(CH₂)ₜOC(=O)(CH₂)ₜ-, - C(=O)(CH₂)ₜOC(=O)O(CH₂)ₜ-, -C(=O)(CH₂)ₜNHC(=O)O(CH₂)ₜ-, - C(=O)(CH₂)ₜOC(=O)NH(CH₂)ₜ-, -C(=O)(CH₂)ₜNHC(=O)NH(CH₂)ₜ-, et - C(=O)(CH₂)ₜC(=O)(CH₂)ₜNHC(=O)O-, où t est un nombre entier compris entre 2 et 12, et plus préférablement choisi dans le groupe constitué par -C(=O)O-, -(CH₂)ₜO-, -(CH₂)ₜC(=O)O-, -(CH₂)ₜOC(=O)-, -(CH₂)ₜO-, -C(=O)(CH₂)ₜO-, - C(=O)(CH₂)ₜOC(=O)-, -C(=O)(CH₂)ₜC(=O)O-, -C(=O)(CH₂)ₜC(=O)NH-, - C(=O)(CH₂)ₜOC(=O)NH-, -C(=O)(CH₂)ₜNHC(=O)O-, -C(=O)(CH₂)ₜOC(=O)NH(CH₂)ₜ-, et -C(=O)(CH₂)ₜC(=O)(CH₂)ₜNHC(=O)O-.

5. Lipide PEGylé selon la revendication **1,** dans lequel le poids moléculaire moyen en nombre déterminé par MALDI_TOF de ladite chaîne de polyéthylène glycol est choisi dans le groupe constitué par 900, 1 000, 1 500, 2 000, 2 500, 3 000, 3 350, 3 500, 4 000, 5 000, 5 500, 6 000, 6 500, 7 000, 7 500, 8 000, 8 500, 9 000, 9 500, 10 000 et 11 000.

6. Lipide PEGylé selon la revendication **1,** dans lequel ladite chaîne de polyéthylène glycol est polydispersée, et le degré de polymérisation moyen en nombre n₁ déterminé par MALDI_TOF est de préférence un nombre entier compris entre 20 et 100, plus préférablement un nombre entier compris entre 20 et 60, plus préférablement un nombre entier compris entre 40 et 60, et le plus préférablement choisi dans le groupe constitué par 44, 45, 46, 48, 50, 52, 54, 56, 58 et 60 ; ou
dans lequel ladite chaîne de polyéthylène glycol est monodispersée, et le nombre d'unités EO déterminé par MALDI_TOF est de préférence compris entre 20 et 70, plus préférablement entre 20 et 60, et le plus préférablement choisi dans le groupe constitué par 20, 22, 24, 26, 27, 28, 29, 30, 32, 34, 36, 38, 40, 42, 44, 45, 46, 48, 50, 52, 54, 56, 58 et 60.

7. Lipide PEGylé selon la revendication **1,** dans lequel lesdits R₁ et R₂ sont chacun indépendamment un groupe alkyle en C₅ à C₃₀, plus préférablement un groupe alkyle en C₁₀ à C₃₀, et le plus préférablement un groupe alkyle en C₁₀ à C₂₀ ;
dans lequel lesdits R₁ et R₂ sont chacun indépendamment un groupe alkyle linéaire en C₁ à C₃₀ ou un groupe alkyle ramifié en C₁ à C₃₀ ; de préférence un groupe alkyle linéaire en C₁ à C₃₀ ; plus préférablement un groupe alkyle linéaire en C₁ à C₂₅ ; plus préférablement un groupe alkyle linéaire en C₁ à C₂₀ ; plus préférablement choisi dans le groupe constitué par un groupe méthyle, un groupe éthyle, un groupe propyle, un groupe butyle, un groupe pentyle, un groupe hexyle, un groupe heptyle, un groupe octyle, un groupe nonyle, un groupe décyle, un groupe undécyle, un groupe dodécyle, un groupe tridécyle, un groupe tétradécyle, un groupe pentadécyle, un groupe hexadécyle, un groupe heptadécyle, un groupe octadécyle, un groupe nonadécyle et un groupe eicosyle ; plus préférablement, chacun indépendamment un groupe alkyle ramifié en C₁ à C₃₀, et chacun indépendamment représentés par 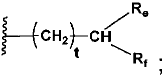
dans laquelle Rₑ et R_{f} sont chacun indépendamment un groupe alkyle en C₁ à C₁₅, plus préférablement choisi dans le groupe constitué par un groupe méthyle, un groupe éthyle, un groupe propyle, un groupe butyle, un groupe pentyle, un groupe hexyle, un groupe heptyle, un groupe octyle, un groupe nonyle, un groupe décyle, un groupe undécyle, un groupe dodécyle, un groupe tridécyle et un groupe tétradécyle ;
et plus préférablement encore, lesdits R₁ et R₂ sont chacun indépendamment choisis dans le groupe constitué par les structures suivantes : 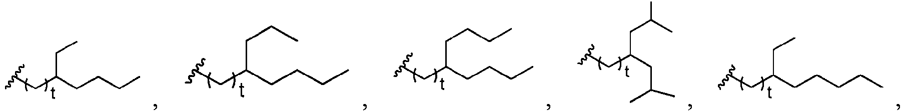 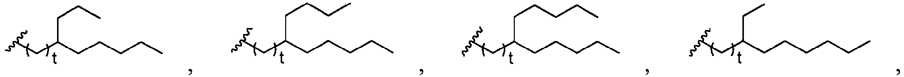 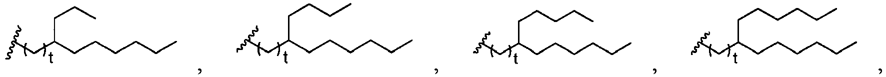 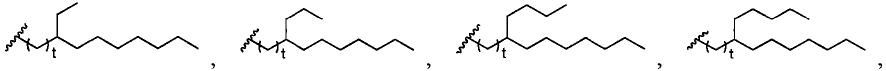 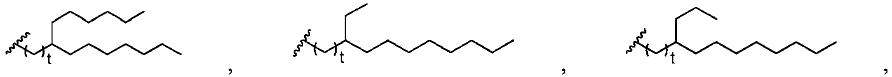 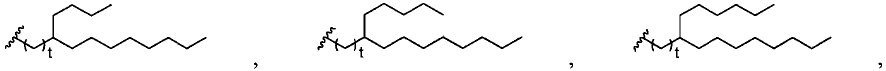 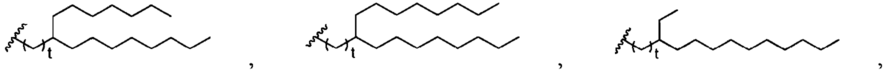 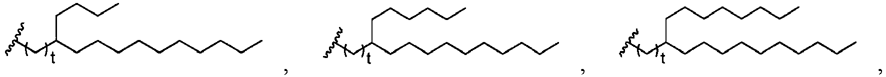 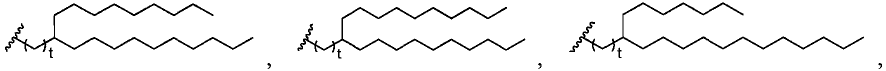 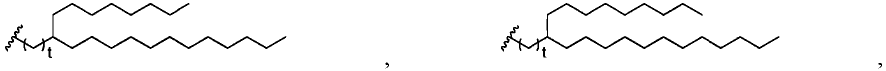 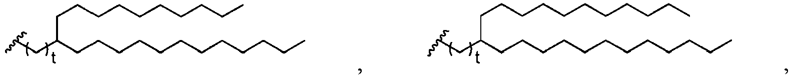 et 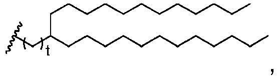 où t est un nombre entier compris entre 0 et 12.

8. Lipide PEGylé selon la revendication **1,** dans lequel ledit R est choisi dans le groupe constitué par H, -CH₃, -CH₂CH₃, -(CH₂)ₜOH, -(CH₂)ₜSH, -OCH₃, - OCH₂CH₃, -(CH₂)ₜNH₃, -(CH₂)ₜC(=O)OH, -C(=O)(CH₂)ₜC(=O)OH, -C(=O)CH₃, - (CH₂)ₜN₃, -C(=O)CH₂CH₃, -C(=O)OCH₃, - OC(=O)OCH₃, -C(=O)OCH₂CH₃, - OC(=O)OCH₂CH₃, -(CH₂)ₜN(CH₃)₂, -(CH₂)ₜN(CH₂CH₃)₂, -(CH₂)ₜCHO,

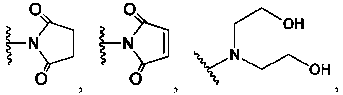

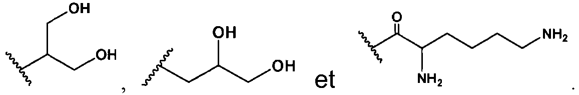

9. Lipide PEGylé selon la revendication **1,** dans lequel la structure dudit lipide PEGylé est choisie dans le groupe constitué par les formules suivantes : 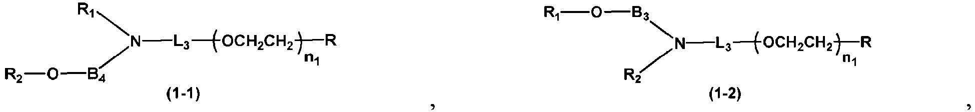 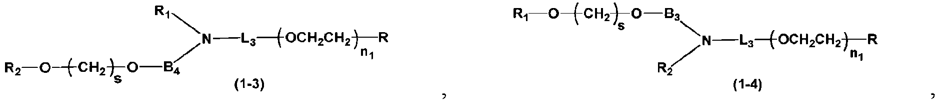 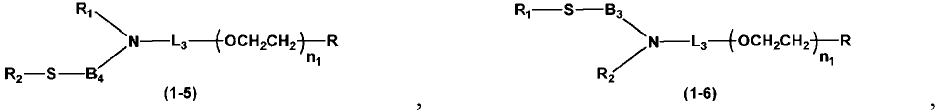 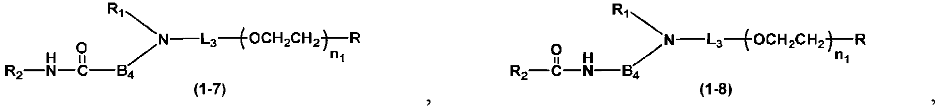 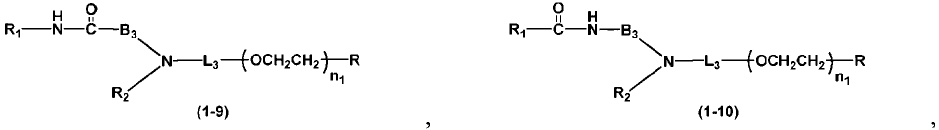 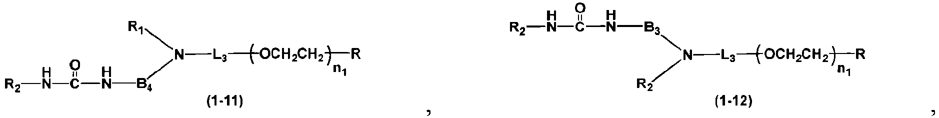 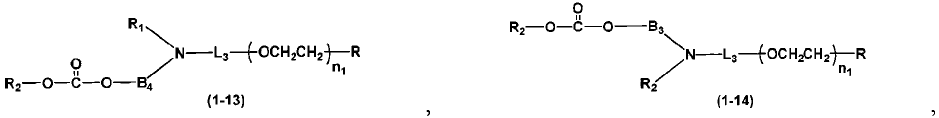 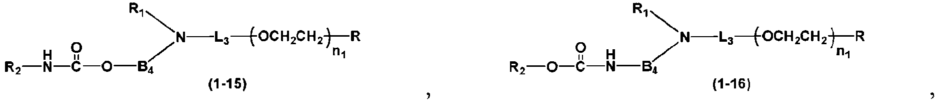 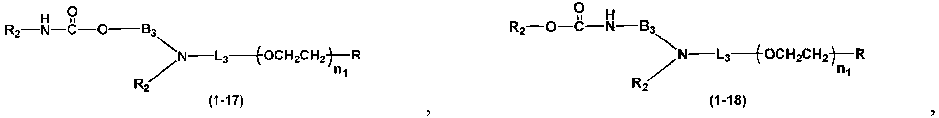 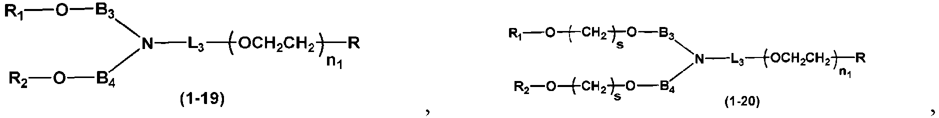 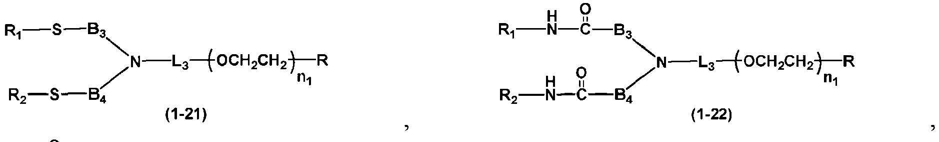 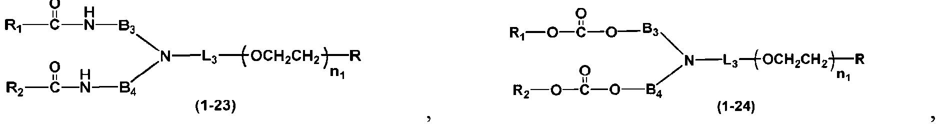 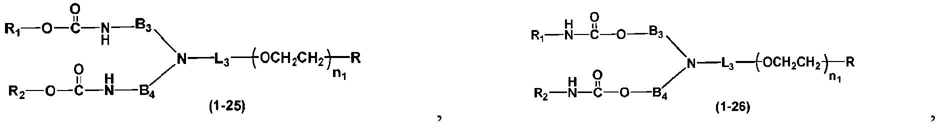 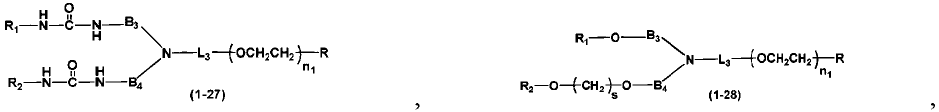 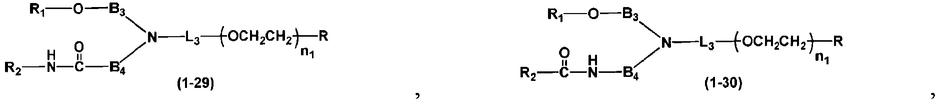 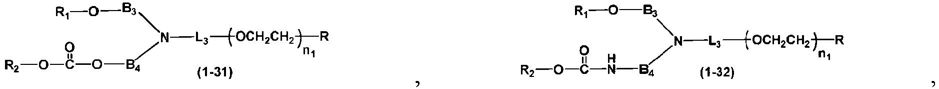 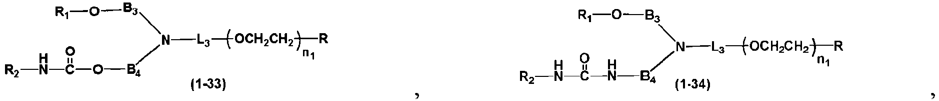 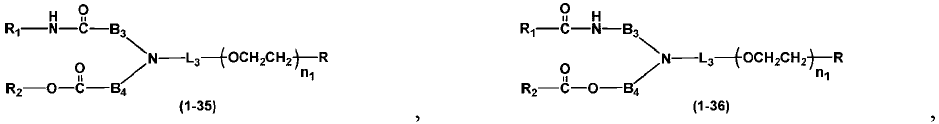 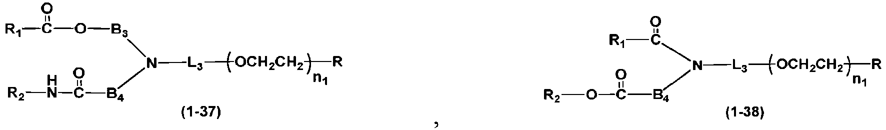 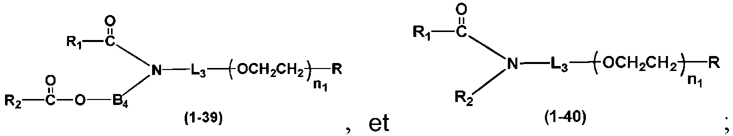 ou
dans lequel la structure dudit lipide PEGylé est choisie dans le groupe constitué par les structures suivantes : 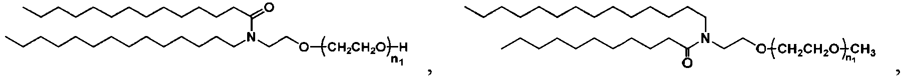 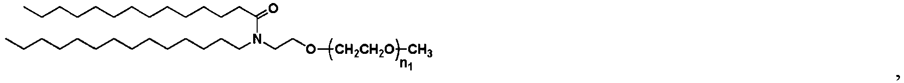 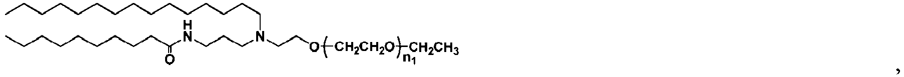 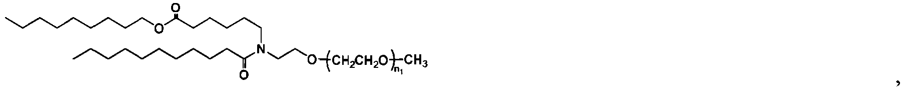 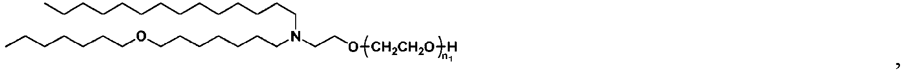 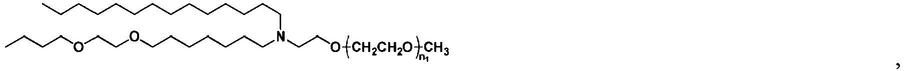 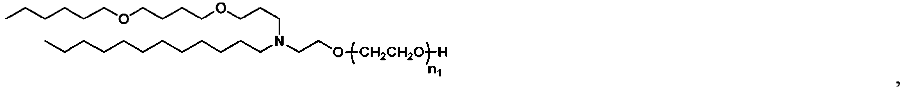 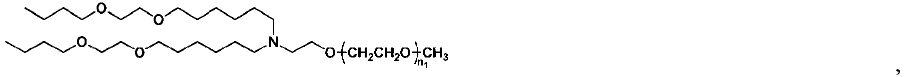 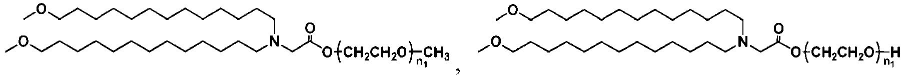 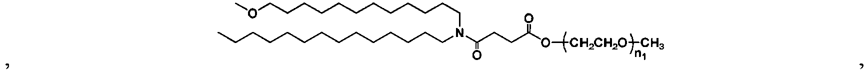 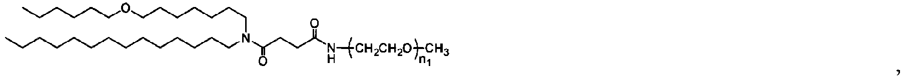 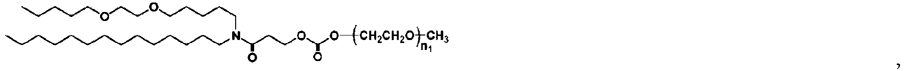 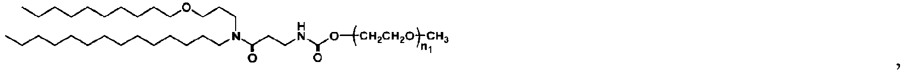 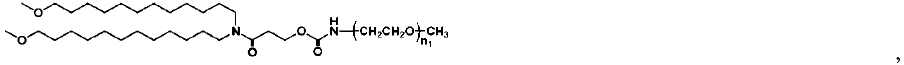 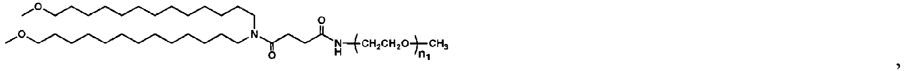 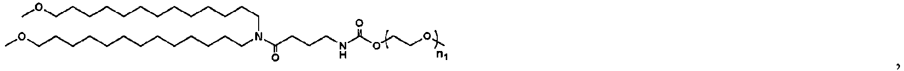 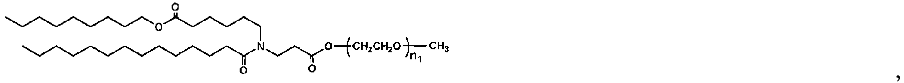 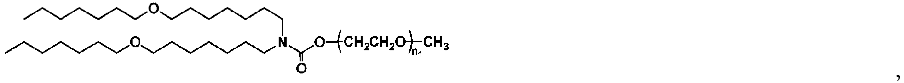 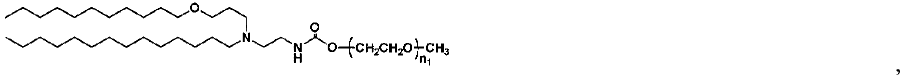 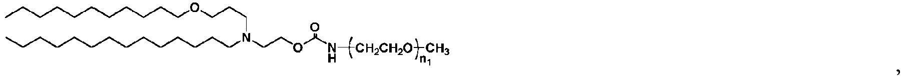 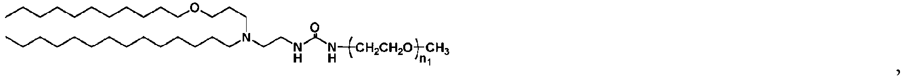 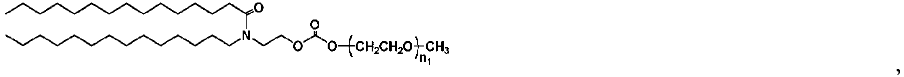 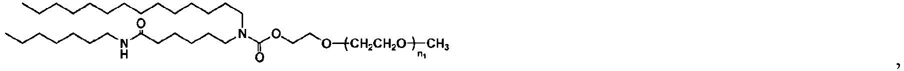 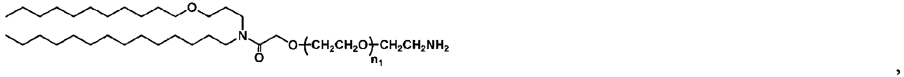 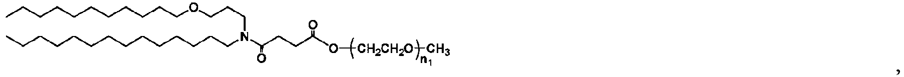 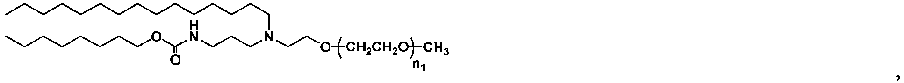 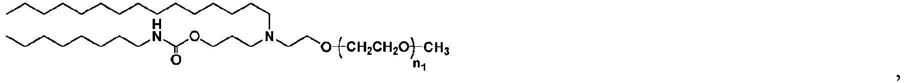 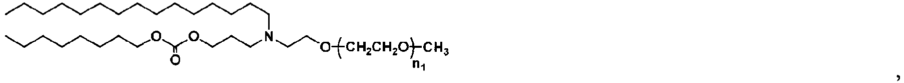 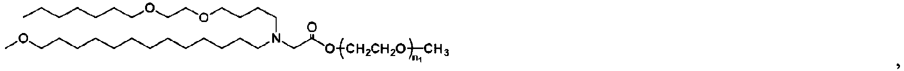 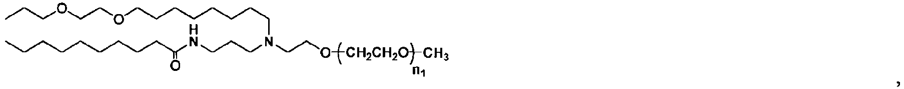 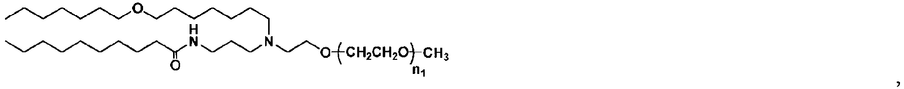 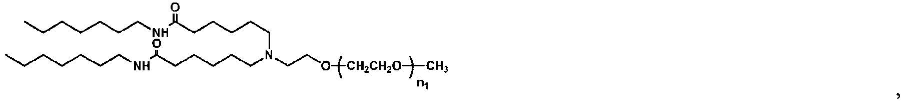 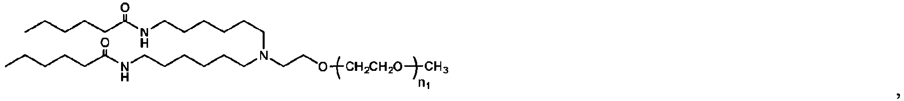 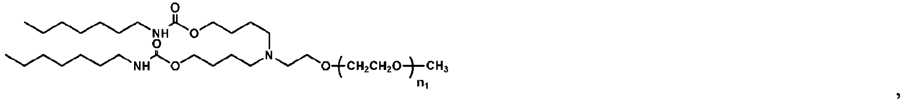 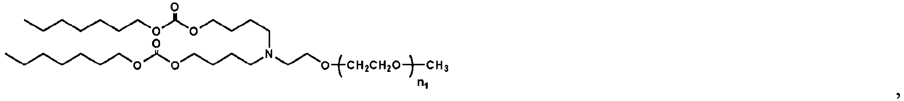 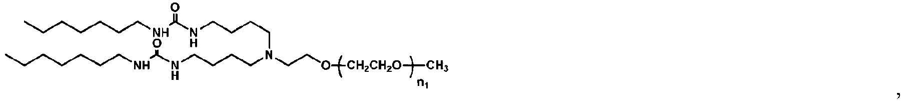 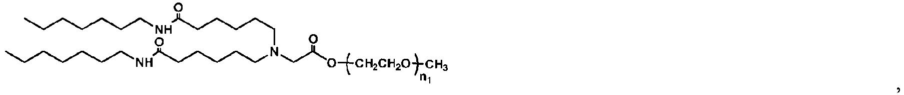 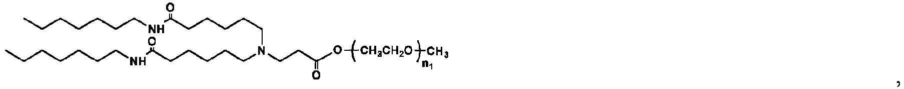 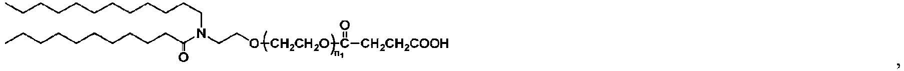 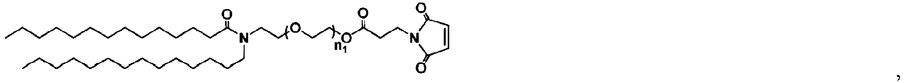 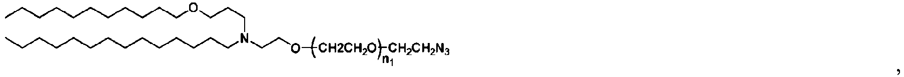 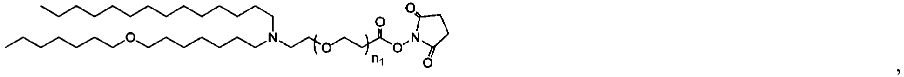 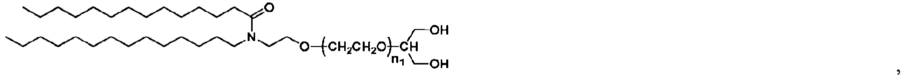 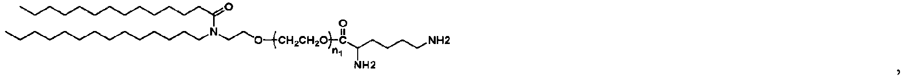 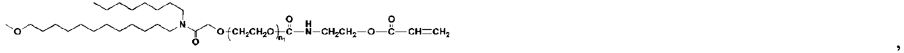 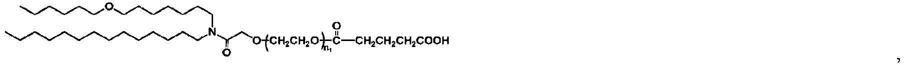 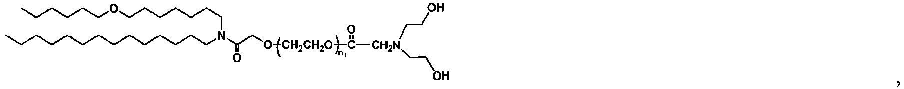 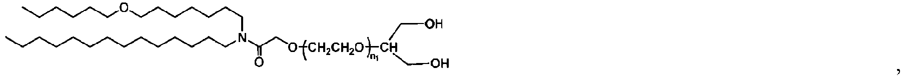 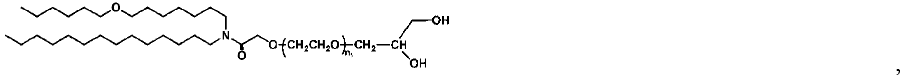 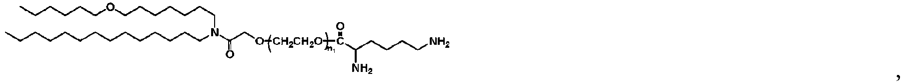 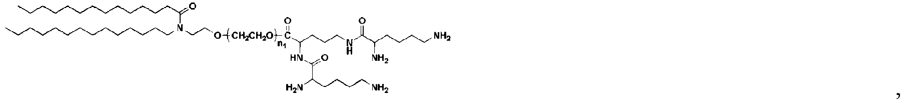 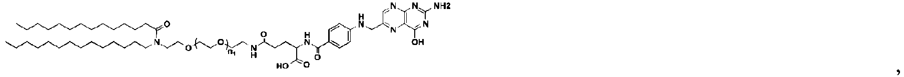 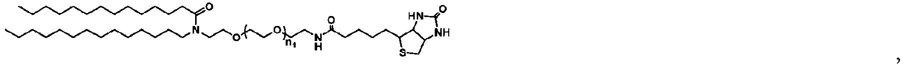 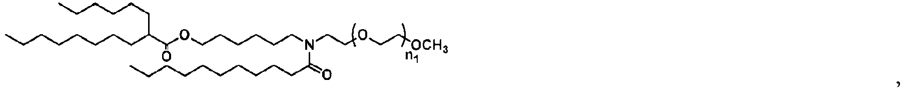 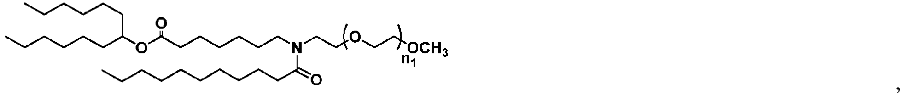 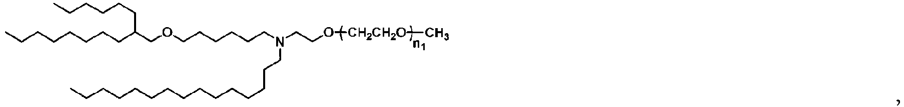 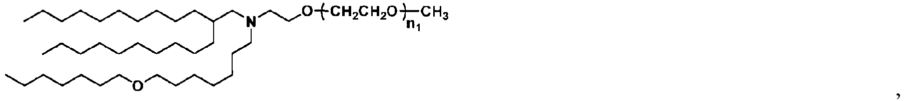 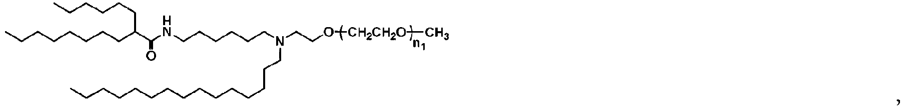 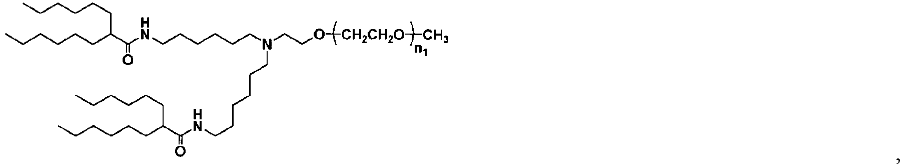 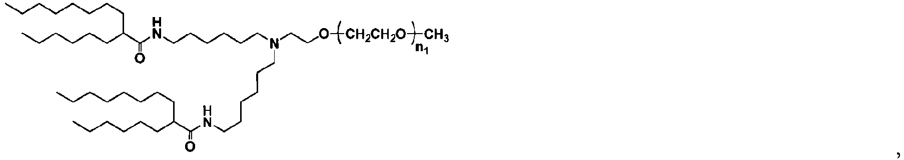 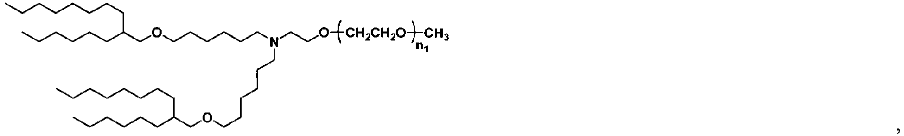 et

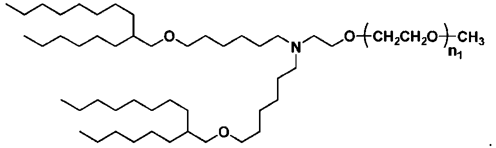

10. Liposome cationique, dans lequel le liposome cationique contient tout lipide PEGylé précédent selon l'une des revendications **1 à 9.**

11. Liposome cationique selon la revendication **10,** dans lequel ledit liposome cationique contient également un ou plusieurs types de lipides choisis dans le groupe constitué par un lipide neutre, un lipide stéroïdien et un lipide cationique ; ledit lipide neutre étant de préférence un phospholipide.

12. Liposome cationique selon la revendication **11,** dans lequel ledit lipide neutre est choisi dans le groupe constitué par les 1,2-dilinoléoyl-sn-glycéro-3-phosphocholines, les 1,2-dimyristoléoyl-sn-glycéro-3-phosphocholines, les 1,2-dioléoyl-sn-glycéro-3-phosphocholines, les 1,2-dipalmitoyl-sn-glycéro-3-phosphocholines, les 1,2-distéaroyl-sn-glycéro-3-phosphatidylcholines, les 1,2-diundécanoyl-sn-glycéro-3-phosphatidylcholines, les 1-palmitoyl-2-oléoyl-sn-glycéro-3-phosphocholines, les 1,2-di-O-octadécényl-sn-glycéro-3-phosphatidylcholines, les 1-oléoyl-2-cholestérylhemisuccinyl-sn-glycéro-3-phosphocholines, les 1-O-hexadécyl-sn-glycéro-3-phosphatidylcholines, les 1,2-dilinolénoyl-sn-glycéro-3-phosphatidylcholines, les 1,2-diarachidonoyl-sn-glycéro-3-phosphatidylcholines, les 1,2-didécosahexaénoyl-sn-glycéro-3-phosphocholines, les 1,2-dioléoyl-sn-glycéro-3-phosphoéthanolamines, les 1,2-diphytanyl-sn-glycéro-3-phosphoéthanolamines, les 1,2-distéaroyl-sn-glycéro-3-phosphoéthanolamines, les 1,2-dilinoléoyl-sn-glycéro-3-phosphoéthanolamines, les 1,2-dilinolénoyl-sn-glycéro-3-phosphoéthanolamines, les 1,2-diarachidonoyl-sn-glycéro-3-phosphoéthanolamines, les 1,2-didécosahexaénoyl-sn-glycéro-3-phosphoéthanolamines, les sels sodiques de 1,2-dioléoyl-sn-glycéro-3-phosphorac-(1-glycérol), les dioléoyl phosphatidylsérines, les dipalmitoylphosphatidylglycérols, les palmitoyloléoyl phosphatidyléthanolamines, les distéaroyl phosphatidyléthanolamines, les dipalmitoyl phosphatidyléthanolamines, les dimyristoleoyl phosphoéthanolamines, les 1-stéaroyl-2-oléoyl-stéaroyléthanolamines, les 1-stéaroyl-2-oléoyl-phosphatidylcholines, les sphingomyélines, les phosphatidylcholines, les phosphatidyléthanolamines, les phosphatidylsérines, les phosphatidylinositols, les acides phosphatidiques, les palmitoyloléoyl phosphatidylcholines, les lysophosphatidylcholines, les lysophosphatidyléthanolamines, et leurs combinaisons ; ou
ledit lipide stéroïdien est choisi dans le groupe constitué par un cholestérol, un coprostanol, un sitostérol, un ergostérol, un campestérol, un stigmastérol, un brassicasterol tomatidine, un acide ursolique, un α-tocophérol, et tout mélange de ceux-ci ; ou
dans lequel la structure dudit lipide cationique est représentée par la formule générale (1) : 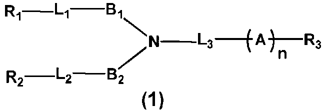
dans laquelle N est le centre de ramification azoté ;
L₁ et L₂ sont des groupes de liaison divalents, chacun indépendamment choisi dans le groupe constitué par -OC(=O)-, -C(=O)O-, -OC(=O)O-, -C(=O)-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, et -NR_{c}C(=O)S- ; dans laquelle R_{c} est un atome d'hydrogène ou un groupe alkyle en C₁ à C₁₂ ;
L₃ est une liaison ou -L₄-Z-L₅- ; lesdits L₄ et L₅ sont des groupes de liaison à chaîne carbonée, chacun indépendamment -(CRₐR_{b})ₜ- ou -(CRₐR_{b})ₜ-(CRₐR_{b})ₚ-(CRₐR_{b})_{q}- ; où t, p et q sont chacun indépendamment un nombre entier compris entre 0 et 12, Rₐ et R_{b} sont chacun indépendamment un atome d'hydrogène ou un groupe alkyle en C₁ à C₁₂ ; ledit Z est une liaison ou un groupe de liaison divalent choisi dans le groupe constitué par -OC(=O)-, -C(=O)O-, -OC(=O)O-, -C(=O)-, -O-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}- et -NR_{c}C(=O)S- ;
B₁ et B₂ représentent chacun indépendamment un groupe alkylène en C₁ à C12 ;
R₁ et R₂ sont chacun indépendamment un groupe hydrocarboné aliphatique en C₂ à C₃₀ ;
R₃ est un atome d'hydrogène, un groupe alkyle, un groupe alcoxy, -C(=O)R_{d}, -C(=O)OR_{d}, -OC(=O)R_{d} ou -OC(=O)OR_{d} ; où R_{d} est un groupe alkyle en C₁ à C₁₂ ;
A est choisi dans le groupe constitué par -(CRₐR_{b})ₛO-, -O(CRₐR_{b})ₛ-, - (CRₐR_{b})ₛS-, -S(CRₐR_{b})ₛ-, -(CRₐR_{b})ₛO(CRₐR_{b})ₛS-, -(CRₐR_{b})ₛS(CRₐR_{b})ₛO-, - (CRₐR_{b})ₛNR_{c}(CRₐR_{b})ₛS-, -(CRₐR_{b})ₛS(CRₐR_{b})ₛNR_{c}-, -(CRₐR_{b})ₛNR_{c}(CRₐR_{b})ₛO-, et - (CRₐR_{b})ₛO(CRₐR_{b})ₛNR_{c}- ; où s est 2, 3 ou 4, Rₐ et R_{b} sont chacun indépendamment un atome d'hydrogène ou un groupe alkyle en C₁ à C₁₂ ;
lorsque A est -(CRₐR_{b})ₛO- ou -O(CRₐR_{b})ₛ-, n est un nombre entier compris entre 2 et 6 ; lorsque A n'est pas -(CRₐR_{b})ₛO- ou -O(CRₐR_{b})ₛ-, n est un nombre entier compris entre 1 et 6 ;
ledit groupe alkyle, ledit groupe alkylène, ledit groupe alcoxy et ledit groupe hydrocarboné aliphatique sont chacun indépendamment substitués ou non substitués ; ou
dans lequel la structure dudit lipide cationique est choisie parmi les structures suivantes : 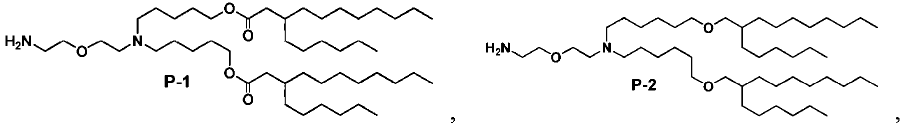 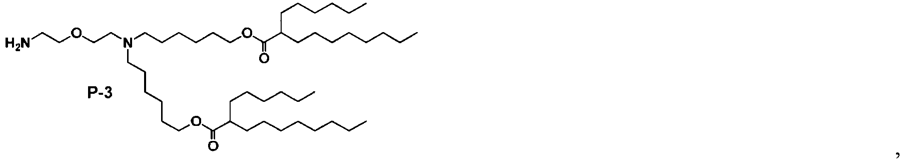 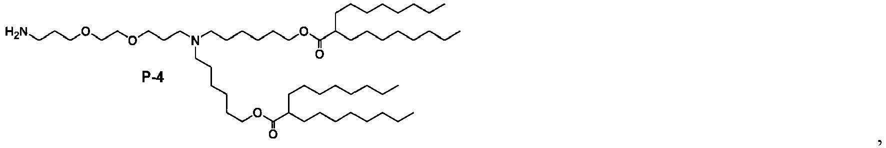 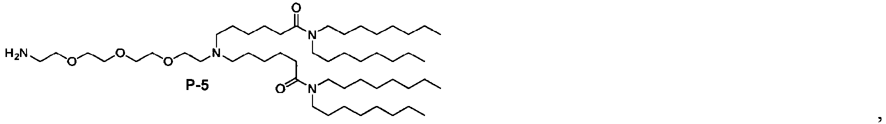 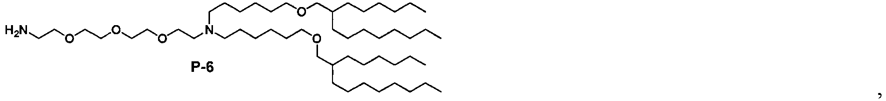 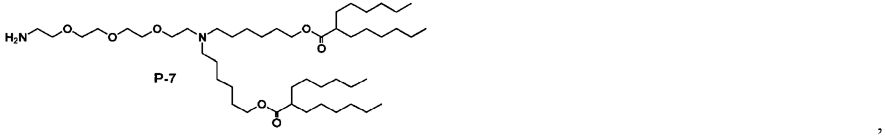 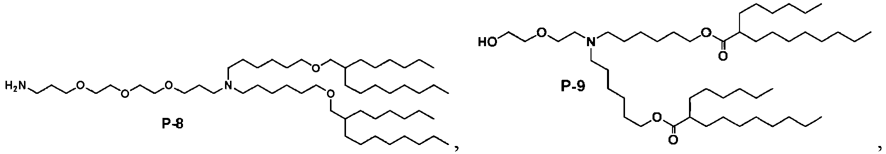 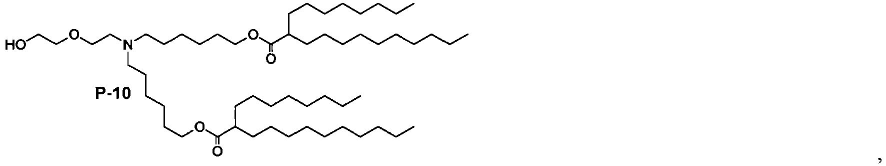 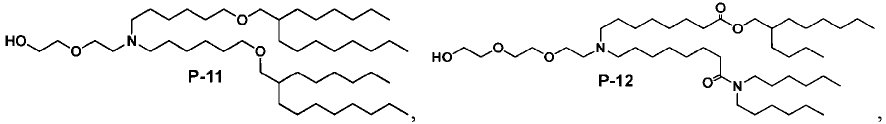 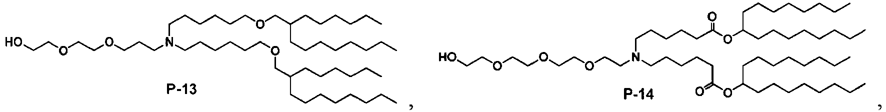 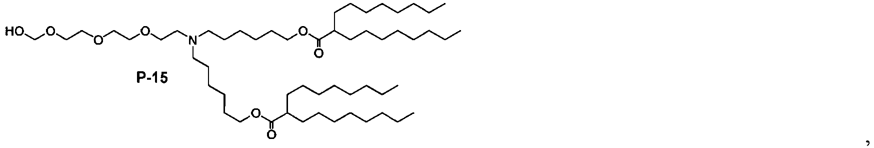 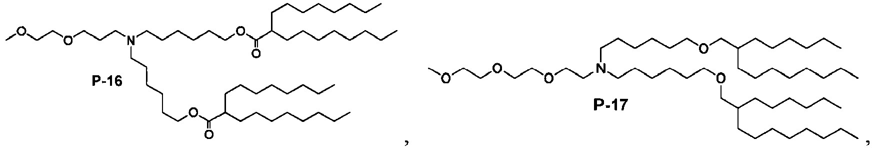 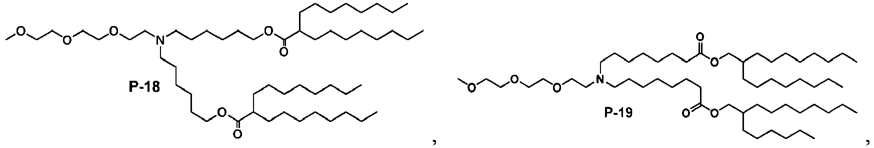 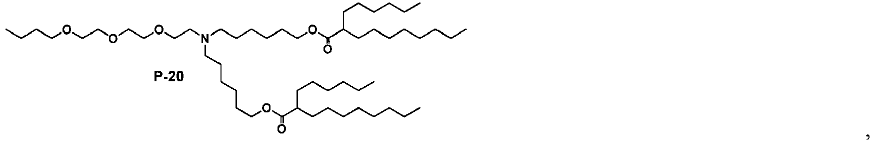 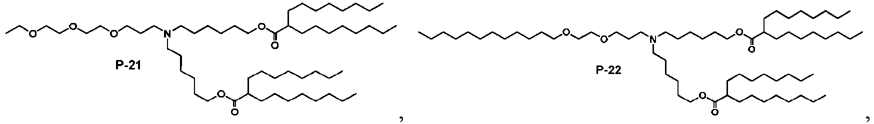 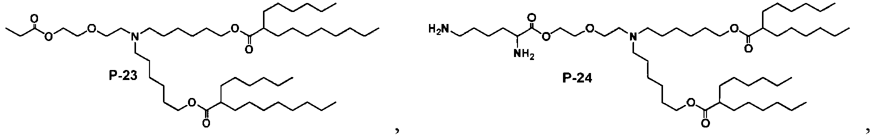 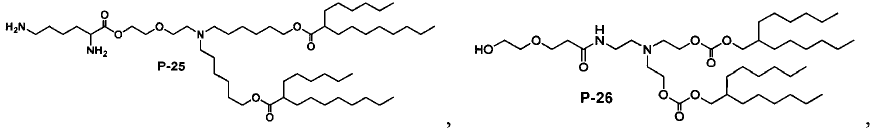 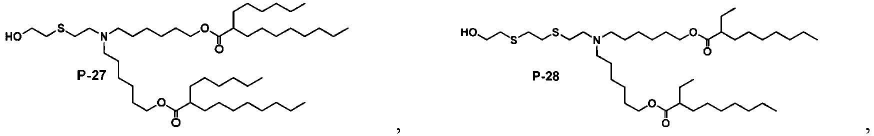 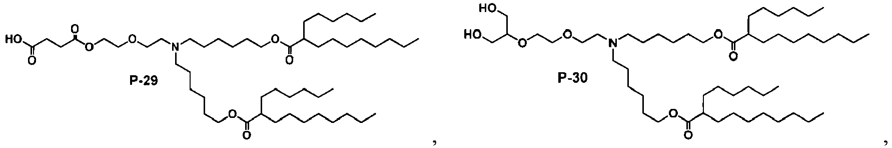 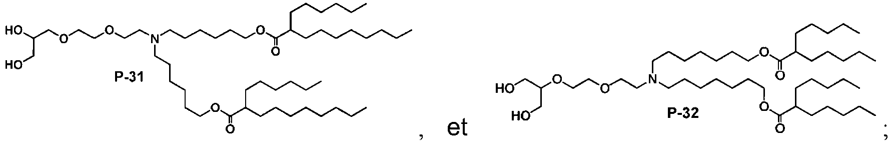 ou
dans lequel lesdits lipides cationiques dans les liposomes cationiques peuvent être choisis dans le groupe constitué par le chlorure de *N,N-dioleyl-N,N-*diméthylammonium, le bromure de *N,N*-distéaryl-*N,N*-diméthylammonium, le 1,2-dioléoyl-3-triméthylammonium-propane, le chlorure de *N*-[1-(2,3-dioléoyloxy)propyl]-*N,N,N*-triméthylammonium, le 1,2-dioleyloxy-*N,N-*diméthylaminopropane, le 3-(didodécylamino)-*N1,N1*,4-tridodécyl-1-pipérazineéthanamine, le *N1*-[2-(didodécylamino)éthyl]-*N1,N4,N4*-tridodécyl-1,4-pipérazinediéthanamine, le 14,25-ditridécyl-15,18,21,24-tétraaza-octatriacontane, le 1,2-dilinoleyloxy-*N,N*-diméthylaminopropane, le 2,2-dilinoleyl-4-diméthylaminométhyl-[1,3]-dioxolane, le heptatriaconta-6,9,28,31-tétraène-19-yl 4-(diméthylamino)butanoate, le 2,2-dilinoleyl-4-(2-diméthylaminoéthyl)-[1,3]-dioxolane, et leurs mélanges.

13. Liposome cationique selon l'une des revendications **11 à 12,** dans lequel ledit liposome cationique contient 20% à 80% de lipides cationiques, 5% à 15% de lipides neutres, 25% à 55% de lipides stéroïdiens et 0,5% à 10% de lipides PEGylés représentés par la formule générale (2), ledit pourcentage étant le pourcentage molaire de chaque lipide par rapport au total des lipides dans une solution contenant le solvant.

14. Liposome cationique selon l'une des revendications **11 à 12,** dans lequel le pourcentage molaire desdits lipides cationiques par rapport au total des lipides dans une solution contenant le solvant est de 30% à 65% ; plus préférablement de 35%, 40%, 45%, 46%, 47%, 48%, 49%, 50% ou 55% ; ou
dans lequel le pourcentage molaire desdits lipides neutres par rapport au total des lipides dans une solution contenant le solvant est de 7,5% à 13%, plus préférablement de 8%, 9%, 10%, 11% ou 12% ; ou
dans lequel le pourcentage molaire desdits lipides stéroïdiens par rapport au total des lipides dans une solution contenant le solvant est de 35% à 50%, plus préférablement de 40%, 41%, 42%, 43%, 44%, 45%, 46%, 47%, 48%, 49% ou 50% ; ou
dans lequel le pourcentage molaire desdits lipides PEGylés par rapport au total des lipides dans une solution contenant le solvant est de 0,5% à 5%, de préférence de 1% à 3%, plus préférablement de 1,5%, 1,6%, 1,7%, 1,8% ou 1,9%.

15. Composition pharmaceutique à base de liposomes cationiques, dans laquelle la composition pharmaceutique à base de liposomes cationiques contient les liposomes cationiques selon l'une des revendications 11 à 12 et des médicaments ; ledit médicament est de préférence un médicament à base d'acide nucléique ou un agent antitumoral, ledit médicament à base d'acide nucléique est choisi dans le groupe constitué par l'ADN, l'acide nucléique antisens, le plasmide, l'ARNm, l'acide nucléique interférant, l'aptamère, l'antagomir, l'ARNmi, le ribozyme, et l'ARNsi, et plus préférablement choisi dans le groupe constitué par l'ADN, l'ARNm, l'ARNmi et l'ARNsi.
